# EUROPEAN PATENT SPECIFICATION

(11) **EP 3 649 258 B1**
(45) Date of publication and mention of the grant of the patent: **04.05.2022**
(21) Application number: 18745509.2
(22) Date of filing: 06.07.2018
(51) Int. Cl.: C12Q 1/6806, C12Q 1/6883, C12Q 1/6886

(54) **TARGET-ENRICHED MULTIPLEXED PARALLEL ANALYSIS FOR ASSESSMENT OF FETAL DNA SAMPLES**
ZIELANGEREICHERTE, MULTIPLEXIERTE PARALLELANALYSE ZUR BEWERTUNG VON FETALEN DNA-PROBEN
ANALYSE PARALLÈLE MULTIPLEXÉE ENRICHIE EN CIBLE POUR L'ÉVALUATION D'ÉCHANTILLONS D'ADN F TAL

(30) Priority: 07.07.2017 US 201762529790 P
(43) Date of publication of application: 13.05.2020
(73) Proprietor: NIPD GENETICS PUBLIC COMPANY LIMITED, Nicosia 2409 (CY)
(72) Inventor: KOUMBARIS, George, Lithrodontas 2565 (CY); IOANNIDES, Marios, Nicosia 2416 (CY); KYPRI,Elena, Nicosia 2007 (CY); ACHILLEOS, Acilleas, Limassol 4045 (CY); MINA, Petros, Nicosia 2015 (CY); TSANGARAS, Kyriakos, Limassol 3085 (CY); PATSALIS, Philippos, Nicosia 2402 (CY)
(74) Representative: CH Kilger Anwaltspartnerschaft mbB
(86) International application number: PCT/EP2018/068414
(87) International publication number: WO 2019/008153

(56) References cited:
- EP-A1- 2 902 500
- WO-A1-2016/189388
- WO-A2-2008/027548
- WO-A2-2016/024134
- US-A1- 2008 194 414
- US-A1- 2011 039 304
- US-A1- 2011 160 076
- US-A1- 2015 203 907
- US-A1- 2016 068 889
- US-A1- 2017 051 355
- ERIC J DUNCAVAGE ET AL: "Targeted next generation sequencing of clinically significant gene mutations and translocations in leukemia", MODERN PATHOLOGY, vol. 25, no. 6, 16 March 2012 (2012-03-16) , pages 795-804, XP055235397, GB ISSN: 0893-3952, DOI: 10.1038/modpathol.2012.29
- XI LIN ET AL: "Applications of targeted gene capture and next-generation sequencing technologies in studies of human deafness and other genetic disabilities", HEARING RESEARCH, ELSEVIER SCIENCE PUBLISHERS, AMSTERDAM, NL, vol. 288, no. 1, 6 January 2012 (2012-01-06), pages 67-76, XP028519246, ISSN: 0378-5955, DOI: 10.1016/J.HEARES.2012.01.004 [retrieved on 2012-01-14]
- STEPHAN BAU ET AL: "Targeted next-generation sequencing by specific capture of multiple genomic loci using low-volume microfluidic DNA arrays", ANALYTICAL AND BIOANALYTICAL CHEMISTRY, SPRINGER, BERLIN, DE, vol. 393, no. 1, 29 October 2008 (2008-10-29), pages 171-175, XP019652995, ISSN: 1618-2650

### SEQUENCE LISTING

<110> NIPD Genetics
<120> TARGET-ENRICHED MULTIPLEXED PARALLEL ANALYSIS FOR ASSESSMENT OF FETAL DNA SAMPLES
<130> B281-0006WO1
<160> 2550
<170> PatentIn version 3.5
<210> 1
   <211> 20
   <212> DNA
   <213> Homo sapiens
<400> 1
   tcttctcgtt caagatgccg 20
<210> 2
   <211> 22
   <212> DNA
   <213> Homo sapiens
<400> 2
   aaattacatg caaggctacc ac 22
<210> 3
   <211> 21
   <212> DNA
   <213> Homo sapiens
<400> 3
   acaggaagaa aggggagtta c 21
<210> 4
   <211> 20
   <212> DNA
   <213> Homo sapiens
<400> 4
   ttcaggttgt gtgatgtgtc 20
<210> 5
   <211> 20
   <212> DNA
   <213> Homo sapiens
<400> 5
   taagcagagg ttttgttgcc 20
<210> 6
   <211> 20
   <212> DNA
   <213> Homo sapiens
<400> 6
   gtgaagtatt tgctgccacc 20
<210> 7
   <211> 20
   <212> DNA
   <213> Homo sapiens
<400> 7
   atggtcatct caacagcaca 20
<210> 8
   <211> 20
   <212> DNA
   <213> Homo sapiens
<400> 8
   tccactgacg ttgagattcg 20
<210> 9
   <211> 20
   <212> DNA
   <213> Homo sapiens
<400> 9
   cgaacctcct tgacctctta 20
<210> 10
   <211> 22
   <212> DNA
   <213> Homo sapiens
<400> 10
   gttatgttga taggggaagc tt 22
<210> 11
   <211> 20
   <212> DNA
   <213> Homo sapiens
<400> 11
   aagccctcta ctccatctgt 20
<210> 12
   <211> 20
   <212> DNA
   <213> Homo sapiens
<400> 12
   tcagaactcg tcagtggaag 20
<210> 13
   <211> 19
   <212> DNA
   <213> Homo sapiens
<400> 13
   gaagggccag acagcttat 19
<210> 14
   <211> 21
   <212> DNA
   <213> Homo sapiens
<400> 14
   atttgctttg tttttgtccc t 21
<210> 15
   <211> 21
   <212> DNA
   <213> Homo sapiens
<400> 15
   gatcattgct ttgtttggac c 21
<210> 16
   <211> 21
   <212> DNA
   <213> Homo sapiens
<400> 16
   aggagaggaa aaatcttgac c 21
<210> 17
   <211> 20
   <212> DNA
   <213> Homo sapiens
<400> 17
   ctgcctgcag ccattattgt 20
<210> 18
   <211> 22
   <212> DNA
   <213> Homo sapiens
<400> 18
   ccttggggta tgtttgttat gt 22
<210> 19
   <211> 22
   <212> DNA
   <213> Homo sapiens
<400> 19
   actttttctg tatcagtcac gg 22
<210> 20
   <211> 20
   <212> DNA
   <213> Homo sapiens
<400> 20
   ctgcttttgt gtgttccctt 20
<210> 21
   <211> 20
   <212> DNA
   <213> Homo sapiens
<400> 21
   ccagttcctg tttttctgcc 20
<210> 22
   <211> 21
   <212> DNA
   <213> Homo sapiens
<400> 22
   gccttcactg atcctacttt c 21
<210> 23
   <211> 21
   <212> DNA
   <213> Homo sapiens
<400> 23
   ggatatgggg taggtttttg t 21
<210> 24
   <211> 20
   <212> DNA
   <213> Homo sapiens
<400> 24
   actcatagaa ctggggcttt 20
<210> 25
   <211> 20
   <212> DNA
   <213> Homo sapiens
<400> 25
   ctttgtaggt cctccagaga 20
<210> 26
   <211> 20
   <212> DNA
   <213> Homo sapiens
<400> 26
   cctgttttca gtgggttgaa 20
<210> 27
   <211> 20
   <212> DNA
   <213> Homo sapiens
<400> 27
   ccacgttgta cctttccatg 20
<210> 28
   <211> 21
   <212> DNA
   <213> Homo sapiens
<400> 28
   gagattcaaa acagtggtgg c 21
<210> 29
   <211> 19
   <212> DNA
   <213> Homo sapiens
<400> 29
   acaacactgt ccttgggtt 19
<210> 30
   <211> 20
   <212> DNA
   <213> Homo sapiens
<400> 30
   taaatgtcct gtgtgctcgg 20
<210> 31
   <211> 20
   <212> DNA
   <213> Homo sapiens
<400> 31
   gacaggacac atggagagag 20
<210> 32
   <211> 21
   <212> DNA
   <213> Homo sapiens
<400> 32
   aactgatggt gatttgcatg t 21
<210> 33
   <211> 20
   <212> DNA
   <213> Homo sapiens
<400> 33
   ggcttctgga aacatcttgc 20
<210> 34
   <211> 21
   <212> DNA
   <213> Homo sapiens
<400> 34
   caatagaagt agggggtgag g 21
<210> 35
   <211> 20
   <212> DNA
   <213> Homo sapiens
<400> 35
   agctgggatg ggttgtttat 20
<210> 36
   <211> 20
   <212> DNA
   <213> Homo sapiens
<400> 36
   gccagttgtc agaagaatcc 20
<210> 37
   <211> 20
   <212> DNA
   <213> Homo sapiens
<400> 37
   caactgcatt ccaaaacagc 20
<210> 38
   <211> 21
   <212> DNA
   <213> Homo sapiens
<400> 38
   gaagggaaac agtgagaaag a 21
<210> 39
   <211> 20
   <212> DNA
   <213> Homo sapiens
<400> 39
   tcaagcgccg taagtatgta 20
<210> 40
   <211> 20
   <212> DNA
   <213> Homo sapiens
<400> 40
   taaccataac atccagggca 20
<210> 41
   <211> 20
   <212> DNA
   <213> Homo sapiens
<400> 41
   ttgtaagctg gcacactgaa 20
<210> 42
   <211> 20
   <212> DNA
   <213> Homo sapiens
<400> 42
   ttaagaaagt gccgtgttgc 20
<210> 43
   <211> 20
   <212> DNA
   <213> Homo sapiens
<400> 43
   atgggtccat gaagagaagc 20
<210> 44
   <211> 20
   <212> DNA
   <213> Homo sapiens
<400> 44
   agttgtttcc agtactgcca 20
<210> 45
   <211> 20
   <212> DNA
   <213> Homo sapiens
<400> 45
   gtatgctttc aagtgacgcc 20
<210> 46
   <211> 21
   <212> DNA
   <213> Homo sapiens
<400> 46
   gaagacaatg caatgaggtg t 21
<210> 47
   <211> 20
   <212> DNA
   <213> Homo sapiens
<400> 47
   agacccaatg agaacaggaa 20
<210> 48
   <211> 20
   <212> DNA
   <213> Homo sapiens
<400> 48
   tgatggagga agtgtgaagc 20
<210> 49
   <211> 20
   <212> DNA
   <213> Homo sapiens
<400> 49
   tggcttttct gtagtttggg 20
<210> 50
   <211> 20
   <212> DNA
   <213> Homo sapiens
<400> 50
   agcagcaaga gttgagaaga 20
<210> 51
   <211> 21
   <212> DNA
   <213> Homo sapiens
<400> 51
   ggctttgaaa aatcaccatg g 21
<210> 52
   <211> 20
   <212> DNA
   <213> Homo sapiens
<400> 52
   agaggtttcc atcgttgcta 20
<210> 53
   <211> 20
   <212> DNA
   <213> Homo sapiens
<400> 53
   aacagcccca aacttcctac 20
<210> 54
   <211> 20
   <212> DNA
   <213> Homo sapiens
<400> 54
   atcactgcca acaagccatt 20
<210> 55
   <211> 20
   <212> DNA
   <213> Homo sapiens
<400> 55
   tgagttgtgg gggataaagg 20
<210> 56
   <211> 20
   <212> DNA
   <213> Homo sapiens
<400> 56
   tctgggcact ttccttatga 20
<210> 57
   <211> 20
   <212> DNA
   <213> Homo sapiens
<400> 57
   gcagttttga ggggagaaga 20
<210> 58
   <211> 19
   <212> DNA
   <213> Homo sapiens
<400> 58
   ttcatgatgc cacctcctc 19
<210> 59
   <211> 20
   <212> DNA
   <213> Homo sapiens
<400> 59
   gcccaaatac cccttcagta 20
<210> 60
   <211> 20
   <212> DNA
   <213> Homo sapiens
<400> 60
   gcacctcaat cccgtacaat 20
<210> 61
   <211> 20
   <212> DNA
   <213> Homo sapiens
<400> 61
   gcgttttaag cagctgtgta 20
<210> 62
   <211> 20
   <212> DNA
   <213> Homo sapiens
<400> 62
   tctccagatc gaaacagcat 20
<210> 63
   <211> 20
   <212> DNA
   <213> Homo sapiens
<400> 63
   tcattcaaag ccaagatgcc 20
<210> 64
   <211> 20
   <212> DNA
   <213> Homo sapiens
<400> 64
   acacgctaca tagacactgg 20
<210> 65
   <211> 20
   <212> DNA
   <213> Homo sapiens
<400> 65
   caatcgaggt cacattcacc 20
<210> 66
   <211> 21
   <212> DNA
   <213> Homo sapiens
<400> 66
   tgacacgctg aagaaaatag c 21
<210> 67
   <211> 20
   <212> DNA
   <213> Homo sapiens
<400> 67
   ggagggaatg gcagaagtaa 20
<210> 68
   <211> 21
   <212> DNA
   <213> Homo sapiens
<400> 68
   ggattttcag agcagaggtt g 21
<210> 69
   <211> 20
   <212> DNA
   <213> Homo sapiens
<400> 69
   aagagttgcc tgtacccttc 20
<210> 70
   <211> 19
   <212> DNA
   <213> Homo sapiens
<400> 70
   gtctctttac tgggagcgt 19
<210> 71
   <211> 20
   <212> DNA
   <213> Homo sapiens
<400> 71
   acattgcccc tgacaacata 20
<210> 72
   <211> 19
   <212> DNA
   <213> Homo sapiens
<400> 72
   tcattatctg aggagccgg 19
<210> 73
   <211> 21
   <212> DNA
   <213> Homo sapiens
<400> 73
   ggctccataa tcttctgcaa t 21
<210> 74
   <211> 20
   <212> DNA
   <213> Homo sapiens
<400> 74
   cgtacatctg atttgtgggt 20
<210> 75
   <211> 21
   <212> DNA
   <213> Homo sapiens
<400> 75
   tgccactgat gatacaaaag c 21
<210> 76
   <211> 20
   <212> DNA
   <213> Homo sapiens
<400> 76
   gaaacgtgtg gtgtcctcta 20
<210> 77
   <211> 20
   <212> DNA
   <213> Homo sapiens
<400> 77
   agtgtttggg gctctatcag 20
<210> 78
   <211> 20
   <212> DNA
   <213> Homo sapiens
<400> 78
   ctttcccttt tgagtcctgc 20
<210> 79
   <211> 20
   <212> DNA
   <213> Homo sapiens
<400> 79
   tctcgcttac cttgctacat 20
<210> 80
   <211> 20
   <212> DNA
   <213> Homo sapiens
<400> 80
   aaagcgggaa ttggaacttt 20
<210> 81
   <211> 20
   <212> DNA
   <213> Homo sapiens
<400> 81
   ggggaacatt gggaagagat 20
<210> 82
   <211> 20
   <212> DNA
   <213> Homo sapiens
<400> 82
   tctaccagct acaaacccat 20
<210> 83
   <211> 20
   <212> DNA
   <213> Homo sapiens
<400> 83
   tcagccaata cccatagcag 20
<210> 84
   <211> 20
   <212> DNA
   <213> Homo sapiens
<400> 84
   aatcagagga agatgggtcg 20
<210> 85
   <211> 20
   <212> DNA
   <213> Homo sapiens
<400> 85
   gagaactcca ccctgtcttt 20
<210> 86
   <211> 20
   <212> DNA
   <213> Homo sapiens
<400> 86
   gggagtgtgt gaatgtgtct 20
<210> 87
   <211> 21
   <212> DNA
   <213> Homo sapiens
<400> 87
   aagctttaca tcatggcact g 21
<210> 88
   <211> 20
   <212> DNA
   <213> Homo sapiens
<400> 88
   aaaggtccat aggctcacat 20
<210> 89
   <211> 20
   <212> DNA
   <213> Homo sapiens
<400> 89
   ttgaccaatg ccattaagcc 20
<210> 90
   <211> 21
   <212> DNA
   <213> Homo sapiens
<400> 90
   accagggaaa tgttagcttc t 21
<210> 91
   <211> 21
   <212> DNA
   <213> Homo sapiens
<400> 91
   tgctctgtta tggttggagt t 21
<210> 92
   <211> 20
   <212> DNA
   <213> Homo sapiens
<400> 92
   gaagcggcag taattcagga 20
<210> 93
   <211> 20
   <212> DNA
   <213> Homo sapiens
<400> 93
   aactgtgtcc taagcagtga 20
<210> 94
   <211> 20
   <212> DNA
   <213> Homo sapiens
<400> 94
   gtcacctcca gagctttcat 20
<210> 95
   <211> 21
   <212> DNA
   <213> Homo sapiens
<400> 95
   gaacaatgca acctgagaac t 21
<210> 96
   <211> 21
   <212> DNA
   <213> Homo sapiens
<400> 96
   tgattgtcct ctaccatgca t 21
<210> 97
   <211> 21
   <212> DNA
   <213> Homo sapiens
<400> 97
   aggcttgaaa caccaccttt a 21
<210> 98
   <211> 20
   <212> DNA
   <213> Homo sapiens
<400> 98
   ttgatttgtt gggtggttgg 20
<210> 99
   <211> 20
   <212> DNA
   <213> Homo sapiens
<400> 99
   tgccaatctg aggtttttcc 20
<210> 100
   <211> 20
   <212> DNA
   <213> Homo sapiens
<400> 100
   actctgcttt agggcttctg 20
<210> 101
   <211> 20
   <212> DNA
   <213> Homo sapiens
<400> 101
   gcagaaaagc tcccaaacaa 20
<210> 102
   <211> 20
   <212> DNA
   <213> Homo sapiens
<400> 102
   gaaaagaggt ggagagggag 20
<210> 103
   <211> 20
   <212> DNA
   <213> Homo sapiens
<400> 103
   aggacccttt tgctgatttc 20
<210> 104
   <211> 20
   <212> DNA
   <213> Homo sapiens
<400> 104
   ctggcccaag tgcatacata 20
<210> 105
   <211> 20
   <212> DNA
   <213> Homo sapiens
<400> 105
   aaccagagag acaccttgac 20
<210> 106
   <211> 20
   <212> DNA
   <213> Homo sapiens
<400> 106
   tcctccctat ctcctgtgac 20
<210> 107
   <211> 20
   <212> DNA
   <213> Homo sapiens
<400> 107
   actctgccag aaaagcctac 20
<210> 108
   <211> 20
   <212> DNA
   <213> Homo sapiens
<400> 108
   cctttgtctt gaagcctcct 20
<210> 109
   <211> 20
   <212> DNA
   <213> Homo sapiens
<400> 109
   ttgactgagc agagtagagc 20
<210> 110
   <211> 22
   <212> DNA
   <213> Homo sapiens
<400> 110
   ttctacctac aagcaaagag ag 22
<210> 111
   <211> 21
   <212> DNA
   <213> Homo sapiens
<400> 111
   tcaccaacag aggatcaaac t 21
<210> 112
   <211> 20
   <212> DNA
   <213> Homo sapiens
<400> 112
   ccgagggata acatacagct 20
<210> 113
   <211> 20
   <212> DNA
   <213> Homo sapiens
<400> 113
   cactagtcac agaagcaggt 20
<210> 114
   <211> 20
   <212> DNA
   <213> Homo sapiens
<400> 114
   gtagaaaccc cgagacaact 20
<210> 115
   <211> 20
   <212> DNA
   <213> Homo sapiens
<400> 115
   caatttgctg ttcagaggct 20
<210> 116
   <211> 20
   <212> DNA
   <213> Homo sapiens
<400> 116
   tcatgatgtg gcttagtggg 20
<210> 117
   <211> 20
   <212> DNA
   <213> Homo sapiens
<400> 117
   tacacccaca tgcatacaca 20
<210> 118
   <211> 19
   <212> DNA
   <213> Homo sapiens
<400> 118
   gaagtgtgca tgggagagt 19
<210> 119
   <211> 21
   <212> DNA
   <213> Homo sapiens
<400> 119
   tttgggtggc tctatgttag g 21
<210> 120
   <211> 20
   <212> DNA
   <213> Homo sapiens
<400> 120
   ctccttgact catttcccgt 20
<210> 121
   <211> 20
   <212> DNA
   <213> Homo sapiens
<400> 121
   agagtaccac tgccaagaaa 20
<210> 122
   <211> 20
   <212> DNA
   <213> Homo sapiens
<400> 122
   ctgctagtct gtcaggagag 20
<210> 123
   <211> 20
   <212> DNA
   <213> Homo sapiens
<400> 123
   gggcagcagt ataaacatcc 20
<210> 124
   <211> 20
   <212> DNA
   <213> Homo sapiens
<400> 124
   gacattccct tccattgagc 20
<210> 125
   <211> 20
   <212> DNA
   <213> Homo sapiens
<400> 125
   ttcctggtaa atgtgctggt 20
<210> 126
   <211> 20
   <212> DNA
   <213> Homo sapiens
<400> 126
   ggccagattt gcagtgattt 20
<210> 127
   <211> 21
   <212> DNA
   <213> Homo sapiens
<400> 127
   agaatcaaca acaatggcag g 21
<210> 128
   <211> 21
   <212> DNA
   <213> Homo sapiens
<400> 128
   ctctgaggaa agcttgtagg a 21
<210> 129
   <211> 20
   <212> DNA
   <213> Homo sapiens
<400> 129
   aggttttcgt tctgcttcag 20
<210> 130
   <211> 19
   <212> DNA
   <213> Homo sapiens
<400> 130
   cgggtcagtg attctagct 19
<210> 131
   <211> 20
   <212> DNA
   <213> Homo sapiens
<400> 131
   gtcactatgg aatgggggtt 20
<210> 132
   <211> 20
   <212> DNA
   <213> Homo sapiens
<400> 132
   gcttcttccc cgcaatatga 20
<210> 133
   <211> 20
   <212> DNA
   <213> Homo sapiens
<400> 133
   cggttcagag tcaatgccta 20
<210> 134
   <211> 21
   <212> DNA
   <213> Homo sapiens
<400> 134
   tacccaacaa gccagagaaa t 21
<210> 135
   <211> 20
   <212> DNA
   <213> Homo sapiens
<400> 135
   agagggaaag tgcaaggaat 20
<210> 136
   <211> 20
   <212> DNA
   <213> Homo sapiens
<400> 136
   gtttttcagc acactgtccc 20
<210> 137
   <211> 20
   <212> DNA
   <213> Homo sapiens
<400> 137
   accacattac tcacaaccct 20
<210> 138
   <211> 20
   <212> DNA
   <213> Homo sapiens
<400> 138
   aagccctttt catctccaca 20
<210> 139
   <211> 20
   <212> DNA
   <213> Homo sapiens
<400> 139
   ccagagctga gacaactact 20
<210> 140
   <211> 20
   <212> DNA
   <213> Homo sapiens
<400> 140
   gaagcaattc ctcacaccac 20
<210> 141
   <211> 20
   <212> DNA
   <213> Homo sapiens
<400> 141
   ttcagtcaga atgaggagcc 20
<210> 142
   <211> 20
   <212> DNA
   <213> Homo sapiens
<400> 142
   ctcctctccc ctctgatttt 20
<210> 143
   <211> 21
   <212> DNA
   <213> Homo sapiens
<400> 143
   ttttaaagcg acagtcacac g 21
<210> 144
   <211> 20
   <212> DNA
   <213> Homo sapiens
<400> 144
   tcctctgcct tctacccttt 20
<210> 145
   <211> 20
   <212> DNA
   <213> Homo sapiens
<400> 145
   tgttgtgcct tttgttctgg 20
<210> 146
   <211> 22
   <212> DNA
   <213> Homo sapiens
<400> 146
   ttaggaggta aggctggaaa aa 22
<210> 147
   <211> 21
   <212> DNA
   <213> Homo sapiens
<400> 147
   ctgtcagcaa tttcaggtca g 21
<210> 148
   <211> 20
   <212> DNA
   <213> Homo sapiens
<400> 148
   agacaaaggc ttcacggaac 20
<210> 149
   <211> 20
   <212> DNA
   <213> Homo sapiens
<400> 149
   ttcctctgtg tcttgaaggt 20
<210> 150
   <211> 20
   <212> DNA
   <213> Homo sapiens
<400> 150
   atcaatgcag gtgagtgtga 20
<210> 151
   <211> 20
   <212> DNA
   <213> Homo sapiens
<400> 151
   cactccacat aagcctcaga 20
<210> 152
   <211> 20
   <212> DNA
   <213> Homo sapiens
<400> 152
   aggatgtagt tgggtgagga 20
<210> 153
   <211> 20
   <212> DNA
   <213> Homo sapiens
<400> 153
   cagtcatcac ggggagatac 20
<210> 154
   <211> 20
   <212> DNA
   <213> Homo sapiens
<400> 154
   gacagatatt tgtgcagggt 20
<210> 155
   <211> 20
   <212> DNA
   <213> Homo sapiens
<400> 155
   tcctagccct tacctttcct 20
<210> 156
   <211> 20
   <212> DNA
   <213> Homo sapiens
<400> 156
   agcctgaatg tcactgatca 20
<210> 157
   <211> 19
   <212> DNA
   <213> Homo sapiens
<400> 157
   actcatcact tctggctgc 19
<210> 158
   <211> 20
   <212> DNA
   <213> Homo sapiens
<400> 158
   tcttgtgttt cctgccctat 20
<210> 159
   <211> 20
   <212> DNA
   <213> Homo sapiens
<400> 159
   ttgctgtgga tgagaatgga 20
<210> 160
   <211> 20
   <212> DNA
   <213> Homo sapiens
<400> 160
   tgagggcaga aagaaacaga 20
<210> 161
   <211> 20
   <212> DNA
   <213> Homo sapiens
<400> 161
   ggggtcacac atcacttttc 20
<210> 162
   <211> 20
   <212> DNA
   <213> Homo sapiens
<400> 162
   atagggtcac aatccactgc 20
<210> 163
   <211> 20
   <212> DNA
   <213> Homo sapiens
<400> 163
   atattgagcc ccgcatgtta 20
<210> 164
   <211> 20
   <212> DNA
   <213> Homo sapiens
<400> 164
   ggttgcagga gaaagaacat 20
<210> 165
   <211> 20
   <212> DNA
   <213> Homo sapiens
<400> 165
   tgccatgtaa ttgccaagat 20
<210> 166
   <211> 19
   <212> DNA
   <213> Homo sapiens
<400> 166
   cctgttctcc atccctctg 19
<210> 167
   <211> 21
   <212> DNA
   <213> Homo sapiens
<400> 167
   tcacaaacta cccaacacct a 21
<210> 168
   <211> 21
   <212> DNA
   <213> Homo sapiens
<400> 168
   cagaattagt tggggagctg t 21
<210> 169
   <211> 20
   <212> DNA
   <213> Homo sapiens
<400> 169
   gccatctcct gaaatagtgc 20
<210> 170
   <211> 20
   <212> DNA
   <213> Homo sapiens
<400> 170
   gcaagtgttc ccatctagaa 20
<210> 171
   <211> 20
   <212> DNA
   <213> Homo sapiens
<400> 171
   attgataccc ctctccccag 20
<210> 172
   <211> 19
   <212> DNA
   <213> Homo sapiens
<400> 172
   aagtaagctg tctcctggc 19
<210> 173
   <211> 20
   <212> DNA
   <213> Homo sapiens
<400> 173
   aggttggttg gcatgaagaa 20
<210> 174
   <211> 20
   <212> DNA
   <213> Homo sapiens
<400> 174
   agcagagttt caagacaagc 20
<210> 175
   <211> 20
   <212> DNA
   <213> Homo sapiens
<400> 175
   ttttacacag caggcctctt 20
<210> 176
   <211> 21
   <212> DNA
   <213> Homo sapiens
<400> 176
   gcaactccaa attatcaggg c 21
<210> 177
   <211> 20
   <212> DNA
   <213> Homo sapiens
<400> 177
   cagcaccttc ccttagcaaa 20
<210> 178
   <211> 20
   <212> DNA
   <213> Homo sapiens
<400> 178
   cttgttgtct tgtagccctg 20
<210> 179
   <211> 20
   <212> DNA
   <213> Homo sapiens
<400> 179
   cacagataca gacgtccaca 20
<210> 180
   <211> 21
   <212> DNA
   <213> Homo sapiens
<400> 180
   agctcagcaa ttaaacagtc c 21
<210> 181
   <211> 20
   <212> DNA
   <213> Homo sapiens
<400> 181
   tgttgagagt gccagagatg 20
<210> 182
   <211> 20
   <212> DNA
   <213> Homo sapiens
<400> 182
   cttccacctt ctgccaatga 20
<210> 183
   <211> 20
   <212> DNA
   <213> Homo sapiens
<400> 183
   ttctgacatt tgcaagcacc 20
<210> 184
   <211> 20
   <212> DNA
   <213> Homo sapiens
<400> 184
   ctgttgctag tttcttgggc 20
<210> 185
   <211> 19
   <212> DNA
   <213> Homo sapiens
<400> 185
   ttttccagtc ccagcacat 19
<210> 186
   <211> 21
   <212> DNA
   <213> Homo sapiens
<400> 186
   tctctctctt cctgaaacag c 21
<210> 187
   <211> 21
   <212> DNA
   <213> Homo sapiens
<400> 187
   aacctctgct ttgtgtagtg a 21
<210> 188
   <211> 20
   <212> DNA
   <213> Homo sapiens
<400> 188
   gagagaatgc aaggttcagc 20
<210> 189
   <211> 20
   <212> DNA
   <213> Homo sapiens
<400> 189
   tacagcatca aagaggaagc 20
<210> 190
   <211> 20
   <212> DNA
   <213> Homo sapiens
<400> 190
   gaggggatga ggggaaaaag 20
<210> 191
   <211> 20
   <212> DNA
   <213> Homo sapiens
<400> 191
   catgacctct gacggatctg 20
<210> 192
   <211> 19
   <212> DNA
   <213> Homo sapiens
<400> 192
   aggcaatgag gtcaaggac 19
<210> 193
   <211> 20
   <212> DNA
   <213> Homo sapiens
<400> 193
   atgatggccc caacttcttc 20
<210> 194
   <211> 20
   <212> DNA
   <213> Homo sapiens
<400> 194
   ttctagacac tgagggagca 20
<210> 195
   <211> 20
   <212> DNA
   <213> Homo sapiens
<400> 195
   aacgctacac tttacgagct 20
<210> 196
   <211> 20
   <212> DNA
   <213> Homo sapiens
<400> 196
   tcaacactac ctgccaatca 20
<210> 197
   <211> 19
   <212> DNA
   <213> Homo sapiens
<400> 197
   tgccgacaca aaagaatgc 19
<210> 198
   <211> 19
   <212> DNA
   <213> Homo sapiens
<400> 198
   aaagtgttcc tccctgctg 19
<210> 199
   <211> 20
   <212> DNA
   <213> Homo sapiens
<400> 199
   aggagcaaaa tagtctggct 20
<210> 200
   <211> 21
   <212> DNA
   <213> Homo sapiens
<400> 200
   accactcttg aatcattgca g 21
<210> 201
   <211> 21
   <212> DNA
   <213> Homo sapiens
<400> 201
   ccagccaatt ttctctttcc c 21
<210> 202
   <211> 20
   <212> DNA
   <213> Homo sapiens
<400> 202
   actgtcccta ctgccaattt 20
<210> 203
   <211> 21
   <212> DNA
   <213> Homo sapiens
<400> 203
   atctggtttg aacttgccaa c 21
<210> 204
   <211> 20
   <212> DNA
   <213> Homo sapiens
<400> 204
   cactctgaat agctctcccc 20
<210> 205
   <211> 20
   <212> DNA
   <213> Homo sapiens
<400> 205
   ttatccggga cagtttcagg 20
<210> 206
   <211> 20
   <212> DNA
   <213> Homo sapiens
<400> 206
   gaatcttttg gcccacactg 20
<210> 207
   <211> 20
   <212> DNA
   <213> Homo sapiens
<400> 207
   gtcagacaca cttagctggt 20
<210> 208
   <211> 20
   <212> DNA
   <213> Homo sapiens
<400> 208
   acacatttca ccttcaccct 20
<210> 209
   <211> 20
   <212> DNA
   <213> Homo sapiens
<400> 209
   gcactaatcc aggggcttaa 20
<210> 210
   <211> 20
   <212> DNA
   <213> Homo sapiens
<400> 210
   ccccttacca ccacttctac 20
<210> 211
   <211> 20
   <212> DNA
   <213> Homo sapiens
<400> 211
   ttcagatccc ttaagcacgc 20
<210> 212
   <211> 20
   <212> DNA
   <213> Homo sapiens
<400> 212
   acctaaggcc tcaaattcca 20
<210> 213
   <211> 20
   <212> DNA
   <213> Homo sapiens
<400> 213
   cagaccacgg gcataagaaa 20
<210> 214
   <211> 20
   <212> DNA
   <213> Homo sapiens
<400> 214
   tggatgtgtg gatttggaga 20
<210> 215
   <211> 19
   <212> DNA
   <213> Homo sapiens
<400> 215
   ctggctgtct tctgggaaa 19
<210> 216
   <211> 21
   <212> DNA
   <213> Homo sapiens
<400> 216
   caagcagaac tgagaagagt c 21
<210> 217
   <211> 20
   <212> DNA
   <213> Homo sapiens
<400> 217
   agtggaacga ggattgtgtt 20
<210> 218
   <211> 20
   <212> DNA
   <213> Homo sapiens
<400> 218
   ctcccatctg aaactgctga 20
<210> 219
   <211> 19
   <212> DNA
   <213> Homo sapiens
<400> 219
   acatcacaac caccctgac 19
<210> 220
   <211> 20
   <212> DNA
   <213> Homo sapiens
<400> 220
   gtgttgacct gatttgccaa 20
<210> 221
   <211> 20
   <212> DNA
   <213> Homo sapiens
<400> 221
   gaacaaagag gaacagagcc 20
<210> 222
   <211> 20
   <212> DNA
   <213> Homo sapiens
<400> 222
   ttcatgattc cagggtcctc 20
<210> 223
   <211> 22
   <212> DNA
   <213> Homo sapiens
<400> 223
   ggacggattt agtgtacatt gg 22
<210> 224
   <211> 20
   <212> DNA
   <213> Homo sapiens
<400> 224
   tttccttcca acaccacaga 20
<210> 225
   <211> 20
   <212> DNA
   <213> Homo sapiens
<400> 225
   tccttagggt tctgcgaaat 20
<210> 226
   <211> 20
   <212> DNA
   <213> Homo sapiens
<400> 226
   ggaaactccc tgccttctac 20
<210> 227
   <211> 20
   <212> DNA
   <213> Homo sapiens
<400> 227
   tcttccaaac accaggtcta 20
<210> 228
   <211> 19
   <212> DNA
   <213> Homo sapiens
<400> 228
   actcaatgga aggaagggc 19
<210> 229
   <211> 20
   <212> DNA
   <213> Homo sapiens
<400> 229
   aaggacttgt gctgtattgc 20
<210> 230
   <211> 20
   <212> DNA
   <213> Homo sapiens
<400> 230
   gacgggagcc agtattctac 20
<210> 231
   <211> 20
   <212> DNA
   <213> Homo sapiens
<400> 231
   gggattgaga gcttggttct 20
<210> 232
   <211> 20
   <212> DNA
   <213> Homo sapiens
<400> 232
   ctccccacca agatgttcaa 20
<210> 233
   <211> 20
   <212> DNA
   <213> Homo sapiens
<400> 233
   gttttgggtc atgcagtgtt 20
<210> 234
   <211> 20
   <212> DNA
   <213> Homo sapiens
<400> 234
   gacaaaaaca cttgccagac 20
<210> 235
   <211> 20
   <212> DNA
   <213> Homo sapiens
<400> 235
   aaccatggct ttgcaagtac 20
<210> 236
   <211> 20
   <212> DNA
   <213> Homo sapiens
<400> 236
   ggaaccctct gctattttgc 20
<210> 237
   <211> 20
   <212> DNA
   <213> Homo sapiens
<400> 237
   tactccttgt gtgaacccct 20
<210> 238
   <211> 20
   <212> DNA
   <213> Homo sapiens
<400> 238
   acctttaccc cataccatcc 20
<210> 239
   <211> 20
   <212> DNA
   <213> Homo sapiens
<400> 239
   cattcctttg gttggtgtcc 20
<210> 240
   <211> 20
   <212> DNA
   <213> Homo sapiens
<400> 240
   ctaatgggcc tgttgttcct 20
<210> 241
   <211> 20
   <212> DNA
   <213> Homo sapiens
<400> 241
   caacctacct gcccatagtt 20
<210> 242
   <211> 20
   <212> DNA
   <213> Homo sapiens
<400> 242
   cgtagcaaat tatggcgagg 20
<210> 243
   <211> 20
   <212> DNA
   <213> Homo sapiens
<400> 243
   ggtcagaagg gaaagggttc 20
<210> 244
   <211> 20
   <212> DNA
   <213> Homo sapiens
<400> 244
   ccagattaaa acgtggtgcc 20
<210> 245
   <211> 20
   <212> DNA
   <213> Homo sapiens
<400> 245
   cccacaacta taggtcgcat 20
<210> 246
   <211> 21
   <212> DNA
   <213> Homo sapiens
<400> 246
   agttgttcca tttgtaccag c 21
<210> 247
   <211> 19
   <212> DNA
   <213> Homo sapiens
<400> 247
   ttggctgcac tttgagtca 19
<210> 248
   <211> 20
   <212> DNA
   <213> Homo sapiens
<400> 248
   cagatggccc attgtaacaa 20
<210> 249
   <211> 19
   <212> DNA
   <213> Homo sapiens
<400> 249
   tattgaggtt cccgtgctg 19
<210> 250
   <211> 20
   <212> DNA
   <213> Homo sapiens
<400> 250
   agaatgtgaa gtggctccat 20
<210> 251
   <211> 20
   <212> DNA
   <213> Homo sapiens
<400> 251
   tttgggtttg tgtgtgtgtg 20
<210> 252
   <211> 20
   <212> DNA
   <213> Homo sapiens
<400> 252
   aggaatctct ctctgccaag 20
<210> 253
   <211> 20
   <212> DNA
   <213> Homo sapiens
<400> 253
   tcttcaaggc aggtcatagg 20
<210> 254
   <211> 21
   <212> DNA
   <213> Homo sapiens
<400> 254
   gaaacctaag acgttccact g 21
<210> 255
   <211> 20
   <212> DNA
   <213> Homo sapiens
<400> 255
   gagtgaaggg attggagcaa 20
<210> 256
   <211> 20
   <212> DNA
   <213> Homo sapiens
<400> 256
   atgtctcagg ctaggtgttc 20
<210> 257
   <211> 20
   <212> DNA
   <213> Homo sapiens
<400> 257
   acactcacaa agcccagtta 20
<210> 258
   <211> 20
   <212> DNA
   <213> Homo sapiens
<400> 258
   gtgcagactc atgttatggc 20
<210> 259
   <211> 20
   <212> DNA
   <213> Homo sapiens
<400> 259
   aggatctcaa agcaccacag 20
<210> 260
   <211> 20
   <212> DNA
   <213> Homo sapiens
<400> 260
   aacgggaaga gggaaacttt 20
<210> 261
   <211> 20
   <212> DNA
   <213> Homo sapiens
<400> 261
   atgttcaaca gagtcaggct 20
<210> 262
   <211> 20
   <212> DNA
   <213> Homo sapiens
<400> 262
   cagtaacagt ccagggtctt 20
<210> 263
   <211> 20
   <212> DNA
   <213> Homo sapiens
<400> 263
   agtctgggag cctagaatca 20
<210> 264
   <211> 22
   <212> DNA
   <213> Homo sapiens
<400> 264
   cattgtagtt tcaggacacc aa 22
<210> 265
   <211> 20
   <212> DNA
   <213> Homo sapiens
<400> 265
   accacagaat gacttgcagc 20
<210> 266
   <211> 20
   <212> DNA
   <213> Homo sapiens
<400> 266
   tttcacgtgt aacaggagca 20
<210> 267
   <211> 20
   <212> DNA
   <213> Homo sapiens
<400> 267
   tgctacaggg aaaatggtct 20
<210> 268
   <211> 20
   <212> DNA
   <213> Homo sapiens
<400> 268
   tccactgctt agtttgcctt 20
<210> 269
   <211> 20
   <212> DNA
   <213> Homo sapiens
<400> 269
   acaggtgggg agaaaaggta 20
<210> 270
   <211> 20
   <212> DNA
   <213> Homo sapiens
<400> 270
   ctgccactac tacacagcta 20
<210> 271
   <211> 20
   <212> DNA
   <213> Homo sapiens
<400> 271
   atgggtctct ggaatgcatg 20
<210> 272
   <211> 20
   <212> DNA
   <213> Homo sapiens
<400> 272
   agttctccac agcacatcat 20
<210> 273
   <211> 20
   <212> DNA
   <213> Homo sapiens
<400> 273
   tctttcatct cagctctgca 20
<210> 274
   <211> 20
   <212> DNA
   <213> Homo sapiens
<400> 274
   ccgaacagta ttttgagggg 20
<210> 275
   <211> 20
   <212> DNA
   <213> Homo sapiens
<400> 275
   tttggctgtt tcctgtttcc 20
<210> 276
   <211> 20
   <212> DNA
   <213> Homo sapiens
<400> 276
   acctaacttg ccttgtcctt 20
<210> 277
   <211> 20
   <212> DNA
   <213> Homo sapiens
<400> 277
   acaggagaac aagcagcata 20
<210> 278
   <211> 21
   <212> DNA
   <213> Homo sapiens
<400> 278
   cagcctagta tatgggaacg t 21
<210> 279
   <211> 23
   <212> DNA
   <213> Homo sapiens
<400> 279
   acccatatgt agtatcgctc ttg 23
<210> 280
   <211> 21
   <212> DNA
   <213> Homo sapiens
<400> 280
   tatgggtttt tctgctccac t 21
<210> 281
   <211> 20
   <212> DNA
   <213> Homo sapiens
<400> 281
   aaatgtgagg gagagtcgtc 20
<210> 282
   <211> 19
   <212> DNA
   <213> Homo sapiens
<400> 282
   gcaggaccct tcagcatta 19
<210> 283
   <211> 20
   <212> DNA
   <213> Homo sapiens
<400> 283
   gactggatga tgcaaaggtg 20
<210> 284
   <211> 20
   <212> DNA
   <213> Homo sapiens
<400> 284
   aacatttgca gggggatcaa 20
<210> 285
   <211> 20
   <212> DNA
   <213> Homo sapiens
<400> 285
   aaagatgcct ccttgtgtct 20
<210> 286
   <211> 20
   <212> DNA
   <213> Homo sapiens
<400> 286
   aagttatctg cccagggaaa 20
<210> 287
   <211> 20
   <212> DNA
   <213> Homo sapiens
<400> 287
   tcctggctag ttttgctgaa 20
<210> 288
   <211> 20
   <212> DNA
   <213> Homo sapiens
<400> 288
   ctccttgctt gcctttacac 20
<210> 289
   <211> 20
   <212> DNA
   <213> Homo sapiens
<400> 289
   agccttaatt ccccatgcat 20
<210> 290
   <211> 20
   <212> DNA
   <213> Homo sapiens
<400> 290
   tttttctgtg gagtgtggct 20
<210> 291
   <211> 20
   <212> DNA
   <213> Homo sapiens
<400> 291
   aaagagtcaa ccatgcactg 20
<210> 292
   <211> 20
   <212> DNA
   <213> Homo sapiens
<400> 292
   ggtgaagcag cctgaataaa 20
<210> 293
   <211> 19
   <212> DNA
   <213> Homo sapiens
<400> 293
   tctttgtacc aagctgcca 19
<210> 294
   <211> 20
   <212> DNA
   <213> Homo sapiens
<400> 294
   gcttctactt tcccctccag 20
<210> 295
   <211> 20
   <212> DNA
   <213> Homo sapiens
<400> 295
   agaaaagcca acctcctctt 20
<210> 296
   <211> 20
   <212> DNA
   <213> Homo sapiens
<400> 296
   ccagggtact aaaaggggac 20
<210> 297
   <211> 20
   <212> DNA
   <213> Homo sapiens
<400> 297
   ttgtgggtca atgtcaacac 20
<210> 298
   <211> 20
   <212> DNA
   <213> Homo sapiens
<400> 298
   agaaaaggtg gaggaaggga 20
<210> 299
   <211> 21
   <212> DNA
   <213> Homo sapiens
<400> 299
   ggagttgttt acaggtggac t 21
<210> 300
   <211> 21
   <212> DNA
   <213> Homo sapiens
<400> 300
   tagcttccaa ttcacaggtc a 21
<210> 301
   <211> 20
   <212> DNA
   <213> Homo sapiens
<400> 301
   ttaaatgcgc caagtcccta 20
<210> 302
   <211> 20
   <212> DNA
   <213> Homo sapiens
<400> 302
   atttcctggg tcaagctctt 20
<210> 303
   <211> 20
   <212> DNA
   <213> Homo sapiens
<400> 303
   cttggaccag gaatgctcta 20
<210> 304
   <211> 20
   <212> DNA
   <213> Homo sapiens
<400> 304
   aggcagtcag atccacctat 20
<210> 305
   <211> 20
   <212> DNA
   <213> Homo sapiens
<400> 305
   aaaattgcct gctgtttgga 20
<210> 306
   <211> 20
   <212> DNA
   <213> Homo sapiens
<400> 306
   gcaccatcat gaaacctcct 20
<210> 307
   <211> 20
   <212> DNA
   <213> Homo sapiens
<400> 307
   ataggccagt ctcaggtaga 20
<210> 308
   <211> 20
   <212> DNA
   <213> Homo sapiens
<400> 308
   agattgcagc ctacccaaag 20
<210> 309
   <211> 20
   <212> DNA
   <213> Homo sapiens
<400> 309
   ttgactgaag tgttccaggt 20
<210> 310
   <211> 20
   <212> DNA
   <213> Homo sapiens
<400> 310
   gcttctttca accatccacc 20
<210> 311
   <211> 20
   <212> DNA
   <213> Homo sapiens
<400> 311
   gcagcactca actattccac 20
<210> 312
   <211> 20
   <212> DNA
   <213> Homo sapiens
<400> 312
   caggagttat ggcaccagtg 20
<210> 313
   <211> 20
   <212> DNA
   <213> Homo sapiens
<400> 313
   gagagtgtgg aggcagaaaa 20
<210> 314
   <211> 20
   <212> DNA
   <213> Homo sapiens
<400> 314
   gcccaactta ttttccagct 20
<210> 315
   <211> 21
   <212> DNA
   <213> Homo sapiens
<400> 315
   tcaagcccct tagattgaac a 21
<210> 316
   <211> 20
   <212> DNA
   <213> Homo sapiens
<400> 316
   gctgggcatg tagaactcaa 20
<210> 317
   <211> 20
   <212> DNA
   <213> Homo sapiens
<400> 317
   gggaaattgt caagggcttt 20
<210> 318
   <211> 21
   <212> DNA
   <213> Homo sapiens
<400> 318
   cttagttgct gttgtgcttc t 21
<210> 319
   <211> 20
   <212> DNA
   <213> Homo sapiens
<400> 319
   agttgtagct gtatctgggt 20
<210> 320
   <211> 20
   <212> DNA
   <213> Homo sapiens
<400> 320
   agctgagtca tgtttaaggc 20
<210> 321
   <211> 21
   <212> DNA
   <213> Homo sapiens
<400> 321
   caattcagac tttgcccaaa c 21
<210> 322
   <211> 21
   <212> DNA
   <213> Homo sapiens
<400> 322
   caatcattcc cacagttcca a 21
<210> 323
   <211> 20
   <212> DNA
   <213> Homo sapiens
<400> 323
   ctgtgatggt ccattcaagg 20
<210> 324
   <211> 20
   <212> DNA
   <213> Homo sapiens
<400> 324
   tagctggaaa ttgcaaggag 20
<210> 325
   <211> 19
   <212> DNA
   <213> Homo sapiens
<400> 325
   tctgttcacc tgagccttt 19
<210> 326
   <211> 20
   <212> DNA
   <213> Homo sapiens
<400> 326
   taacttggac tgtgaaccca 20
<210> 327
   <211> 20
   <212> DNA
   <213> Homo sapiens
<400> 327
   cgcaacagga tgaaggaaat 20
<210> 328
   <211> 20
   <212> DNA
   <213> Homo sapiens
<400> 328
   gactcacact ctgaaagcct 20
<210> 329
   <211> 21
   <212> DNA
   <213> Homo sapiens
<400> 329
   aatttaggta gcactgaccc c 21
<210> 330
   <211> 20
   <212> DNA
   <213> Homo sapiens
<400> 330
   ctggggaatt aggaagcaga 20
<210> 331
   <211> 19
   <212> DNA
   <213> Homo sapiens
<400> 331
   atgacaaggc tggctcatc 19
<210> 332
   <211> 20
   <212> DNA
   <213> Homo sapiens
<400> 332
   ttagttttgg catgtggtgg 20
<210> 333
   <211> 20
   <212> DNA
   <213> Homo sapiens
<400> 333
   tggtgaggga gtgttctttt 20
<210> 334
   <211> 20
   <212> DNA
   <213> Homo sapiens
<400> 334
   tcattggggg agtcattcac 20
<210> 335
   <211> 20
   <212> DNA
   <213> Homo sapiens
<400> 335
   gaagtggtgt gatgagggtg 20
<210> 336
   <211> 20
   <212> DNA
   <213> Homo sapiens
<400> 336
   aagttaggcc ctgttaagca 20
<210> 337
   <211> 20
   <212> DNA
   <213> Homo sapiens
<400> 337
   tgttggtcgg agtcagaaat 20
<210> 338
   <211> 20
   <212> DNA
   <213> Homo sapiens
<400> 338
   gtaaaagagg ttgggatgcc 20
<210> 339
   <211> 20
   <212> DNA
   <213> Homo sapiens
<400> 339
   cgttggacat ggatcatacc 20
<210> 340
   <211> 22
   <212> DNA
   <213> Homo sapiens
<400> 340
   tcacaacaag ggaaatagcc ta 22
<210> 341
   <211> 20
   <212> DNA
   <213> Homo sapiens
<400> 341
   tagtcaggta aacaacgcct 20
<210> 342
   <211> 21
   <212> DNA
   <213> Homo sapiens
<400> 342
   tctgtttctt gtttggctga g 21
<210> 343
   <211> 20
   <212> DNA
   <213> Homo sapiens
<400> 343
   acataggtca cacaaagggt 20
<210> 344
   <211> 20
   <212> DNA
   <213> Homo sapiens
<400> 344
   gtgtgacact tttctgcctt 20
<210> 345
   <211> 21
   <212> DNA
   <213> Homo sapiens
<400> 345
   ctggaaatag aaggcctttg c 21
<210> 346
   <211> 20
   <212> DNA
   <213> Homo sapiens
<400> 346
   tcttggtctg ggaataagcc 20
<210> 347
   <211> 20
   <212> DNA
   <213> Homo sapiens
<400> 347
   tgcccctatg aacaacagaa 20
<210> 348
   <211> 20
   <212> DNA
   <213> Homo sapiens
<400> 348
   tgaacgtctt gcttacccac 20
<210> 349
   <211> 19
   <212> DNA
   <213> Homo sapiens
<400> 349
   gaaggaaggc agaggtcaa 19
<210> 350
   <211> 20
   <212> DNA
   <213> Homo sapiens
<400> 350
   cttccacaaa gtcctgcaac 20
<210> 351
   <211> 20
   <212> DNA
   <213> Homo sapiens
<400> 351
   atgtgaacca ttgagaggca 20
<210> 352
   <211> 20
   <212> DNA
   <213> Homo sapiens
<400> 352
   aagagaaact accctggcaa 20
<210> 353
   <211> 20
   <212> DNA
   <213> Homo sapiens
<400> 353
   gcatgtagtt cagttcaggc 20
<210> 354
   <211> 20
   <212> DNA
   <213> Homo sapiens
<400> 354
   atgaaatgta atggggtgcg 20
<210> 355
   <211> 20
   <212> DNA
   <213> Homo sapiens
<400> 355
   ttttggcagt gatgaccttg 20
<210> 356
   <211> 20
   <212> DNA
   <213> Homo sapiens
<400> 356
   ttcttggctt ttctgaccct 20
<210> 357
   <211> 21
   <212> DNA
   <213> Homo sapiens
<400> 357
   ttgagaaaga ccccaacaga a 21
<210> 358
   <211> 22
   <212> DNA
   <213> Homo sapiens
<400> 358
   gaaaataaca cagtagggat gc 22
<210> 359
   <211> 20
   <212> DNA
   <213> Homo sapiens
<400> 359
   gaatggagag gcagttttca 20
<210> 360
   <211> 20
   <212> DNA
   <213> Homo sapiens
<400> 360
   cacccttttc ctgttttgca 20
<210> 361
   <211> 21
   <212> DNA
   <213> Homo sapiens
<400> 361
   agaagaaact tgcagtgttg g 21
<210> 362
   <211> 21
   <212> DNA
   <213> Homo sapiens
<400> 362
   tgcagcatta ttctttctgg g 21
<210> 363
   <211> 21
   <212> DNA
   <213> Homo sapiens
<400> 363
   acacacatat tagggaacag c 21
<210> 364
   <211> 20
   <212> DNA
   <213> Homo sapiens
<400> 364
   gagtgtaggt gcttgggtat 20
<210> 365
   <211> 20
   <212> DNA
   <213> Homo sapiens
<400> 365
   ccttagaatc ctagcgcctt 20
<210> 366
   <211> 20
   <212> DNA
   <213> Homo sapiens
<400> 366
   tcatgaggtt gccagtgttt 20
<210> 367
   <211> 20
   <212> DNA
   <213> Homo sapiens
<400> 367
   ccccatacat catcacatgc 20
<210> 368
   <211> 20
   <212> DNA
   <213> Homo sapiens
<400> 368
   gggtaatgct ttcttgggga 20
<210> 369
   <211> 20
   <212> DNA
   <213> Homo sapiens
<400> 369
   agttgagaag ggaaggcaag 20
<210> 370
   <211> 21
   <212> DNA
   <213> Homo sapiens
<400> 370
   ctcctggtgg cttatttttg a 21
<210> 371
   <211> 22
   <212> DNA
   <213> Homo sapiens
<400> 371
   tctggatttt ggctactcat ga 22
<210> 372
   <211> 20
   <212> DNA
   <213> Homo sapiens
<400> 372
   gtagtcctcc tttgcccttc 20
<210> 373
   <211> 20
   <212> DNA
   <213> Homo sapiens
<400> 373
   gacatgcaca gatcgaaacc 20
<210> 374
   <211> 20
   <212> DNA
   <213> Homo sapiens
<400> 374
   cgcatttgac aacagggatc 20
<210> 375
   <211> 20
   <212> DNA
   <213> Homo sapiens
<400> 375
   aagccacctg ttctctctca 20
<210> 376
   <211> 20
   <212> DNA
   <213> Homo sapiens
<400> 376
   attccaacca ttccgacacc 20
<210> 377
   <211> 20
   <212> DNA
   <213> Homo sapiens
<400> 377
   aaagaaaatg gtgaacgtgc 20
<210> 378
   <211> 20
   <212> DNA
   <213> Homo sapiens
<400> 378
   tgatcagggc tttagaggtc 20
<210> 379
   <211> 20
   <212> DNA
   <213> Homo sapiens
<400> 379
   actccctatt gttctcccct 20
<210> 380
   <211> 20
   <212> DNA
   <213> Homo sapiens
<400> 380
   ctccttgaca gatgtgaccc 20
<210> 381
   <211> 20
   <212> DNA
   <213> Homo sapiens
<400> 381
   ttttggagtc tgagccacaa 20
<210> 382
   <211> 19
   <212> DNA
   <213> Homo sapiens
<400> 382
   ttgaagtccc gttgctgat 19
<210> 383
   <211> 20
   <212> DNA
   <213> Homo sapiens
<400> 383
   ttggggtcag ttctaacagt 20
<210> 384
   <211> 20
   <212> DNA
   <213> Homo sapiens
<400> 384
   ttttcaccac ctcttccctc 20
<210> 385
   <211> 20
   <212> DNA
   <213> Homo sapiens
<400> 385
   acctgaccac aagctttaca 20
<210> 386
   <211> 20
   <212> DNA
   <213> Homo sapiens
<400> 386
   cacatattgg cgcacagtac 20
<210> 387
   <211> 20
   <212> DNA
   <213> Homo sapiens
<400> 387
   gccatgcacc gatgaaaaat 20
<210> 388
   <211> 20
   <212> DNA
   <213> Homo sapiens
<400> 388
   ttgtgaggag atttctgggc 20
<210> 389
   <211> 20
   <212> DNA
   <213> Homo sapiens
<400> 389
   cacactaaga gcactgggaa 20
<210> 390
   <211> 20
   <212> DNA
   <213> Homo sapiens
<400> 390
   atgacctagc acatcttccc 20
<210> 391
   <211> 20
   <212> DNA
   <213> Homo sapiens
<400> 391
   acattttccc cattccatgc 20
<210> 392
   <211> 20
   <212> DNA
   <213> Homo sapiens
<400> 392
   gtctgtcaac cacactttgc 20
<210> 393
   <211> 20
   <212> DNA
   <213> Homo sapiens
<400> 393
   ctgtctctcc ttttgccaaa 20
<210> 394
   <211> 20
   <212> DNA
   <213> Homo sapiens
<400> 394
   agtaacctgc gactctcagt 20
<210> 395
   <211> 20
   <212> DNA
   <213> Homo sapiens
<400> 395
   tagcatttaa ggagtgggct 20
<210> 396
   <211> 20
   <212> DNA
   <213> Homo sapiens
<400> 396
   ggcctcctca gtgatttgaa 20
<210> 397
   <211> 20
   <212> DNA
   <213> Homo sapiens
<400> 397
   ctttctttgc ctcccctgta 20
<210> 398
   <211> 20
   <212> DNA
   <213> Homo sapiens
<400> 398
   acttccattt gtgtcaacgg 20
<210> 399
   <211> 20
   <212> DNA
   <213> Homo sapiens
<400> 399
   tcttgctttg ggttagaggg 20
<210> 400
   <211> 20
   <212> DNA
   <213> Homo sapiens
<400> 400
   aaccacacct ccacaagaaa 20
<210> 401
   <211> 20
   <212> DNA
   <213> Homo sapiens
<400> 401
   caagatatga gggaggggaa 20
<210> 402
   <211> 20
   <212> DNA
   <213> Homo sapiens
<400> 402
   tctaacctgg gccctttctt 20
<210> 403
   <211> 20
   <212> DNA
   <213> Homo sapiens
<400> 403
   tgcccttcca gaactgtaaa 20
<210> 404
   <211> 20
   <212> DNA
   <213> Homo sapiens
<400> 404
   gccaggtcac ttaacaaagc 20
<210> 405
   <211> 19
   <212> DNA
   <213> Homo sapiens
<400> 405
   caataaggcg ccaagttcg 19
<210> 406
   <211> 20
   <212> DNA
   <213> Homo sapiens
<400> 406
   gtcatcaggg gagcaaatgt 20
<210> 407
   <211> 21
   <212> DNA
   <213> Homo sapiens
<400> 407
   aacatgcaat ccctggaatt c 21
<210> 408
   <211> 21
   <212> DNA
   <213> Homo sapiens
<400> 408
   agttgtttca ggacaggatc t 21
<210> 409
   <211> 21
   <212> DNA
   <213> Homo sapiens
<400> 409
   tcctcctgcc tttaataagc t 21
<210> 410
   <211> 21
   <212> DNA
   <213> Homo sapiens
<400> 410
   ttacaaggca tctgacagga a 21
<210> 411
   <211> 21
   <212> DNA
   <213> Homo sapiens
<400> 411
   ttactggtag gtttgagcac a 21
<210> 412
   <211> 22
   <212> DNA
   <213> Homo sapiens
<400> 412
   gagctacgtt ctttctcatc ac 22
<210> 413
   <211> 21
   <212> DNA
   <213> Homo sapiens
<400> 413
   caccaattaa agtgtgctgc a 21
<210> 414
   <211> 21
   <212> DNA
   <213> Homo sapiens
<400> 414
   tcccttccaa agtgccttat a 21
<210> 415
   <211> 21
   <212> DNA
   <213> Homo sapiens
<400> 415
   tggttggttt gggatacttg a 21
<210> 416
   <211> 21
   <212> DNA
   <213> Homo sapiens
<400> 416
   gaacccaaat cgatcatgca t 21
<210> 417
   <211> 21
   <212> DNA
   <213> Homo sapiens
<400> 417
   agagttaaac gtgcaatgtg g 21
<210> 418
   <211> 22
   <212> DNA
   <213> Homo sapiens
<400> 418
   gttcgttggt catagttgtt gt 22
<210> 419
   <211> 21
   <212> DNA
   <213> Homo sapiens
<400> 419
   agtttagcca aaggattcag c 21
<210> 420
   <211> 22
   <212> DNA
   <213> Homo sapiens
<400> 420
   tcaaccattt agaaccacct tg 22
<210> 421
   <211> 21
   <212> DNA
   <213> Homo sapiens
<400> 421
   aatgtccact ttagcggaga g 21
<210> 422
   <211> 22
   <212> DNA
   <213> Homo sapiens
<400> 422
   ggagtattct gttcatgttg gg 22
<210> 423
   <211> 22
   <212> DNA
   <213> Homo sapiens
<400> 423
   tttctagaat tgaggaaggg ca 22
<210> 424
   <211> 21
   <212> DNA
   <213> Homo sapiens
<400> 424
   aggataagac gaggcatcaa t 21
<210> 425
   <211> 22
   <212> DNA
   <213> Homo sapiens
<400> 425
   ttctgtgttg acatgtacct ct 22
<210> 426
   <211> 21
   <212> DNA
   <213> Homo sapiens
<400> 426
   tccaacattt ctctctgtcc c 21
<210> 427
   <211> 20
   <212> DNA
   <213> Homo sapiens
<400> 427
   ataacgtgta ctcctcagcc 20
<210> 428
   <211> 21
   <212> DNA
   <213> Homo sapiens
<400> 428
   gcacttggag gatgtaaaga c 21
<210> 429
   <211> 21
   <212> DNA
   <213> Homo sapiens
<400> 429
   tgtatgcttt aggacccagt t 21
<210> 430
   <211> 22
   <212> DNA
   <213> Homo sapiens
<400> 430
   aactccacag gaatctttct ga 22
<210> 431
   <211> 20
   <212> DNA
   <213> Homo sapiens
<400> 431
   catttctcct gggaccgaat 20
<210> 432
   <211> 21
   <212> DNA
   <213> Homo sapiens
<400> 432
   ttctgaagct gacgaaattc c 21
<210> 433
   <211> 21
   <212> DNA
   <213> Homo sapiens
<400> 433
   gttgcttagt ccttgcttca c 21
<210> 434
   <211> 21
   <212> DNA
   <213> Homo sapiens
<400> 434
   aggtcattgg tctgcagtta t 21
<210> 435
   <211> 21
   <212> DNA
   <213> Homo sapiens
<400> 435
   agcatttaga gaacagcagt c 21
<210> 436
   <211> 21
   <212> DNA
   <213> Homo sapiens
<400> 436
   gtgtaagaag tggttgggtt t 21
<210> 437
   <211> 22
   <212> DNA
   <213> Homo sapiens
<400> 437
   aaaggcagag cagtgtattt ag 22
<210> 438
   <211> 22
   <212> DNA
   <213> Homo sapiens
<400> 438
   agacaagaga acaatcaggt ga 22
<210> 439
   <211> 21
   <212> DNA
   <213> Homo sapiens
<400> 439
   caaagaagct ctaggacagg a 21
<210> 440
   <211> 21
   <212> DNA
   <213> Homo sapiens
<400> 440
   tggtggagga aatcaatgtt g 21
<210> 441
   <211> 20
   <212> DNA
   <213> Homo sapiens
<400> 441
   cacaagggag gaaacgttct 20
<210> 442
   <211> 21
   <212> DNA
   <213> Homo sapiens
<400> 442
   tgagatttag tgccagctag a 21
<210> 443
   <211> 21
   <212> DNA
   <213> Homo sapiens
<400> 443
   aggattagtt tggctcctca g 21
<210> 444
   <211> 21
   <212> DNA
   <213> Homo sapiens
<400> 444
   cctgcactat ttcctcaaag c 21
<210> 445
   <211> 21
   <212> DNA
   <213> Homo sapiens
<400> 445
   caatttcctt ctcactgagc c 21
<210> 446
   <211> 21
   <212> DNA
   <213> Homo sapiens
<400> 446
   acgcttccca aatctatctg g 21
<210> 447
   <211> 22
   <212> DNA
   <213> Homo sapiens
<400> 447
   cttatttgtg tgcccaatac ca 22
<210> 448
   <211> 21
   <212> DNA
   <213> Homo sapiens
<400> 448
   ttgcagcagg aacaccataa a 21
<210> 449
   <211> 21
   <212> DNA
   <213> Homo sapiens
<400> 449
   ctgttcatgt tgctaccaca g 21
<210> 450
   <211> 21
   <212> DNA
   <213> Homo sapiens
<400> 450
   ctgccttaga ttcactttcg g 21
<210> 451
   <211> 21
   <212> DNA
   <213> Homo sapiens
<400> 451
   ccatctgtga ggtcttcttt g 21
<210> 452
   <211> 21
   <212> DNA
   <213> Homo sapiens
<400> 452
   tccttggttg tgtatttagc c 21
<210> 453
   <211> 20
   <212> DNA
   <213> Homo sapiens
<400> 453
   agactcaact cacattggcc 20
<210> 454
   <211> 20
   <212> DNA
   <213> Homo sapiens
<400> 454
   ggtctgactc tgtggtttgg 20
<210> 455
   <211> 21
   <212> DNA
   <213> Homo sapiens
<400> 455
   tttcatttca tcctgcccat g 21
<210> 456
   <211> 21
   <212> DNA
   <213> Homo sapiens
<400> 456
   ttctgttatt cgccatcagt c 21
<210> 457
   <211> 20
   <212> DNA
   <213> Homo sapiens
<400> 457
   gaattgggaa cttgggaagc 20
<210> 458
   <211> 21
   <212> DNA
   <213> Homo sapiens
<400> 458
   gaactttgga gaggacagtg t 21
<210> 459
   <211> 21
   <212> DNA
   <213> Homo sapiens
<400> 459
   aactgtcatg tgtgtctgct a 21
<210> 460
   <211> 21
   <212> DNA
   <213> Homo sapiens
<400> 460
   tgattccttc cacctaccaa a 21
<210> 461
   <211> 21
   <212> DNA
   <213> Homo sapiens
<400> 461
   gccaaggtcc attatctcaa g 21
<210> 462
   <211> 21
   <212> DNA
   <213> Homo sapiens
<400> 462
   gaacctgcat tgtcattctc t 21
<210> 463
   <211> 22
   <212> DNA
   <213> Homo sapiens
<400> 463
   aagaacatca acaaactcca gg 22
<210> 464
   <211> 21
   <212> DNA
   <213> Homo sapiens
<400> 464
   tcaattctct ttcacacgtg c 21
<210> 465
   <211> 21
   <212> DNA
   <213> Homo sapiens
<400> 465
   aggtgctgga tcttgaattc a 21
<210> 466
   <211> 21
   <212> DNA
   <213> Homo sapiens
<400> 466
   tccctctacc cgaatctctt a 21
<210> 467
   <211> 21
   <212> DNA
   <213> Homo sapiens
<400> 467
   tgggtttaaa ggacactagc a 21
<210> 468
   <211> 21
   <212> DNA
   <213> Homo sapiens
<400> 468
   cgaaggtcac acagtttagt c 21
<210> 469
   <211> 21
   <212> DNA
   <213> Homo sapiens
<400> 469
   atgtgccttg ttgattgatg g 21
<210> 470
   <211> 21
   <212> DNA
   <213> Homo sapiens
<400> 470
   agatggtatg tcacaaagca c 21
<210> 471
   <211> 21
   <212> DNA
   <213> Homo sapiens
<400> 471
   acactttgga gagcttcaga t 21
<210> 472
   <211> 22
   <212> DNA
   <213> Homo sapiens
<400> 472
   gtgcccagaa ttatttgtgt ct 22
<210> 473
   <211> 21
   <212> DNA
   <213> Homo sapiens
<400> 473
   gctctcttgt ggaaacgatt a 21
<210> 474
   <211> 21
   <212> DNA
   <213> Homo sapiens
<400> 474
   agtttgtttc tctggcctac t 21
<210> 475
   <211> 21
   <212> DNA
   <213> Homo sapiens
<400> 475
   accacctcaa agatttcatg g 21
<210> 476
   <211> 22
   <212> DNA
   <213> Homo sapiens
<400> 476
   gtcttcatct atttcgtgag cc 22
<210> 477
   <211> 21
   <212> DNA
   <213> Homo sapiens
<400> 477
   tcatcccaga ttcagaatgc c 21
<210> 478
   <211> 21
   <212> DNA
   <213> Homo sapiens
<400> 478
   cgttcaatga agtcccttgt c 21
<210> 479
   <211> 21
   <212> DNA
   <213> Homo sapiens
<400> 479
   ttcacaagaa ctctgctgga t 21
<210> 480
   <211> 21
   <212> DNA
   <213> Homo sapiens
<400> 480
   gaagccttct agtgggacta a 21
<210> 481
   <211> 21
   <212> DNA
   <213> Homo sapiens
<400> 481
   gttagggtca tgggtcactt t 21
<210> 482
   <211> 21
   <212> DNA
   <213> Homo sapiens
<400> 482
   gcctttgtag agtggacttc t 21
<210> 483
   <211> 21
   <212> DNA
   <213> Homo sapiens
<400> 483
   ggcttcttga tactgctttc c 21
<210> 484
   <211> 21
   <212> DNA
   <213> Homo sapiens
<400> 484
   aggtgtgcaa tactcaagga a 21
<210> 485
   <211> 21
   <212> DNA
   <213> Homo sapiens
<400> 485
   aaggtcttag gagtgaggac a 21
<210> 486
   <211> 22
   <212> DNA
   <213> Homo sapiens
<400> 486
   tcgtgctatt tcagtcagat ct 22
<210> 487
   <211> 21
   <212> DNA
   <213> Homo sapiens
<400> 487
   tgtccagccg taacatttca t 21
<210> 488
   <211> 21
   <212> DNA
   <213> Homo sapiens
<400> 488
   aatggacatc tttcaggtct g 21
<210> 489
   <211> 21
   <212> DNA
   <213> Homo sapiens
<400> 489
   tcaaattggg atcgcattag g 21
<210> 490
   <211> 22
   <212> DNA
   <213> Homo sapiens
<400> 490
   atgcctgggt ttattcatct tg 22
<210> 491
   <211> 21
   <212> DNA
   <213> Homo sapiens
<400> 491
   ggagaagttt gggtttgatc c 21
<210> 492
   <211> 21
   <212> DNA
   <213> Homo sapiens
<400> 492
   tgataggagc catcagttct t 21
<210> 493
   <211> 21
   <212> DNA
   <213> Homo sapiens
<400> 493
   agcagatgtt gttagctttc c 21
<210> 494
   <211> 21
   <212> DNA
   <213> Homo sapiens
<400> 494
   ttctctgtca cttccatgag g 21
<210> 495
   <211> 22
   <212> DNA
   <213> Homo sapiens
<400> 495
   ccagtaactt attctgccag ag 22
<210> 496
   <211> 21
   <212> DNA
   <213> Homo sapiens
<400> 496
   tgagagacaa gctgcattac a 21
<210> 497
   <211> 22
   <212> DNA
   <213> Homo sapiens
<400> 497
   gcacagaaat tacagttcat gg 22
<210> 498
   <211> 21
   <212> DNA
   <213> Homo sapiens
<400> 498
   gctctcgtat ctgacagtga a 21
<210> 499
   <211> 21
   <212> DNA
   <213> Homo sapiens
<400> 499
   caggcatctt ggtttgtagt g 21
<210> 500
   <211> 21
   <212> DNA
   <213> Homo sapiens
<400> 500
   cgtgatgaac agtgatgact t 21
<210> 501
   <211> 21
   <212> DNA
   <213> Homo sapiens
<400> 501
   cgccatttgt tctcctattc a 21
<210> 502
   <211> 21
   <212> DNA
   <213> Homo sapiens
<400> 502
   ttcgttagct actgggtact c 21
<210> 503
   <211> 21
   <212> DNA
   <213> Homo sapiens
<400> 503
   ccacccttta cacctatcca a 21
<210> 504
   <211> 21
   <212> DNA
   <213> Homo sapiens
<400> 504
   agagtgcaca aaggagaaga c 21
<210> 505
   <211> 22
   <212> DNA
   <213> Homo sapiens
<400> 505
   tctactgtgt caaagcagat tg 22
<210> 506
   <211> 21
   <212> DNA
   <213> Homo sapiens
<400> 506
   ttcttcctag ccttcctttc c 21
<210> 507
   <211> 21
   <212> DNA
   <213> Homo sapiens
<400> 507
   tctctggctg tgcagtaaat t 21
<210> 508
   <211> 21
   <212> DNA
   <213> Homo sapiens
<400> 508
   aaactcccag ctttaatccc t 21
<210> 509
   <211> 21
   <212> DNA
   <213> Homo sapiens
<400> 509
   aagaatgggt gagttgggtt c 21
<210> 510
   <211> 21
   <212> DNA
   <213> Homo sapiens
<400> 510
   ggaaactgaa ttgccaagtc t 21
<210> 511
   <211> 20
   <212> DNA
   <213> Homo sapiens
<400> 511
   ctcccaactt ttatgcagcc 20
<210> 512
   <211> 21
   <212> DNA
   <213> Homo sapiens
<400> 512
   gctcagggaa tatcttggga a 21
<210> 513
   <211> 21
   <212> DNA
   <213> Homo sapiens
<400> 513
   acattcagca agtaggaagg a 21
<210> 514
   <211> 20
   <212> DNA
   <213> Homo sapiens
<400> 514
   cccagaagag cagtaacaac 20
<210> 515
   <211> 20
   <212> DNA
   <213> Homo sapiens
<400> 515
   gaaaaagggg gataggcatt 20
<210> 516
   <211> 21
   <212> DNA
   <213> Homo sapiens
<400> 516
   cccaacaact gcaataaaag g 21
<210> 517
   <211> 20
   <212> DNA
   <213> Homo sapiens
<400> 517
   accgaaattg cttgctctta 20
<210> 518
   <211> 20
   <212> DNA
   <213> Homo sapiens
<400> 518
   ccttagtgtg acaggacagg 20
<210> 519
   <211> 20
   <212> DNA
   <213> Homo sapiens
<400> 519
   ccaagacaac taggccaatg 20
<210> 520
   <211> 21
   <212> DNA
   <213> Homo sapiens
<400> 520
   gaagagatga tgcaaaagag c 21
<210> 521
   <211> 20
   <212> DNA
   <213> Homo sapiens
<400> 521
   tccaagcaag ggatctcttc 20
<210> 522
   <211> 20
   <212> DNA
   <213> Homo sapiens
<400> 522
   gaatggtcag ggaagggttt 20
<210> 523
   <211> 20
   <212> DNA
   <213> Homo sapiens
<400> 523
   agtcttcagc catcttcctg 20
<210> 524
   <211> 21
   <212> DNA
   <213> Homo sapiens
<400> 524
   gcatttccag gctttacaag t 21
<210> 525
   <211> 20
   <212> DNA
   <213> Homo sapiens
<400> 525
   ctctctctcc ctggtcagat 20
<210> 526
   <211> 20
   <212> DNA
   <213> Homo sapiens
<400> 526
   cagcaattct caggctcaga 20
<210> 527
   <211> 21
   <212> DNA
   <213> Homo sapiens
<400> 527
   tctgaaacaa agcctcctta g 21
<210> 528
   <211> 21
   <212> DNA
   <213> Homo sapiens
<400> 528
   aggataaggt ttcccatgct c 21
<210> 529
   <211> 21
   <212> DNA
   <213> Homo sapiens
<400> 529
   atgtatctga aggagctctg c 21
<210> 530
   <211> 20
   <212> DNA
   <213> Homo sapiens
<400> 530
   ggccttcatc acaaacaaca 20
<210> 531
   <211> 20
   <212> DNA
   <213> Homo sapiens
<400> 531
   tgtgtcccat ctacaaagcc 20
<210> 532
   <211> 20
   <212> DNA
   <213> Homo sapiens
<400> 532
   tccttgaact ctttccaagc 20
<210> 533
   <211> 20
   <212> DNA
   <213> Homo sapiens
<400> 533
   tgttttcctg tccaagtcca 20
<210> 534
   <211> 19
   <212> DNA
   <213> Homo sapiens
<400> 534
   cagcatccat cgctcgaaa 19
<210> 535
   <211> 20
   <212> DNA
   <213> Homo sapiens
<400> 535
   ggtattggtg ggggaaatga 20
<210> 536
   <211> 24
   <212> DNA
   <213> Homo sapiens
<400> 536
   gcactgctgt aaaagatcta tgag 24
<210> 537
   <211> 19
   <212> DNA
   <213> Homo sapiens
<400> 537
   actcaaaggc acatttcgc 19
<210> 538
   <211> 22
   <212> DNA
   <213> Homo sapiens
<400> 538
   attctattcc gatcacagcc tt 22
<210> 539
   <211> 20
   <212> DNA
   <213> Homo sapiens
<400> 539
   ctacctgaca aatggagctt 20
<210> 540
   <211> 21
   <212> DNA
   <213> Homo sapiens
<400> 540
   gaaatggcca tgtgtactga g 21
<210> 541
   <211> 20
   <212> DNA
   <213> Homo sapiens
<400> 541
   gaagcctctc aagctacaag 20
<210> 542
   <211> 22
   <212> DNA
   <213> Homo sapiens
<400> 542
   gaatgagatt agggagcaaa gt 22
<210> 543
   <211> 20
   <212> DNA
   <213> Homo sapiens
<400> 543
   ggaagtaaga agagtgctgc 20
<210> 544
   <211> 20
   <212> DNA
   <213> Homo sapiens
<400> 544
   gctcctgatt gaagaagtgt 20
<210> 545
   <211> 19
   <212> DNA
   <213> Homo sapiens
<400> 545
   ctgctccttt gtctcctgt 19
<210> 546
   <211> 21
   <212> DNA
   <213> Homo sapiens
<400> 546
   taaggtgaga gtgtgaggaa g 21
<210> 547
   <211> 21
   <212> DNA
   <213> Homo sapiens
<400> 547
   ccaggggaac atttactcag a 21
<210> 548
   <211> 20
   <212> DNA
   <213> Homo sapiens
<400> 548
   agggtacatg taaggcagct 20
<210> 549
   <211> 20
   <212> DNA
   <213> Homo sapiens
<400> 549
   ttctgtggca ttgtgtcttg 20
<210> 550
   <211> 20
   <212> DNA
   <213> Homo sapiens
<400> 550
   ccccaaattt accccactct 20
<210> 551
   <211> 20
   <212> DNA
   <213> Homo sapiens
<400> 551
   atccctagca ctttcaggac 20
<210> 552
   <211> 20
   <212> DNA
   <213> Homo sapiens
<400> 552
   ttaccaatcc atccagcctg 20
<210> 553
   <211> 20
   <212> DNA
   <213> Homo sapiens
<400> 553
   agaagctaaa caggttgccc 20
<210> 554
   <211> 20
   <212> DNA
   <213> Homo sapiens
<400> 554
   cataagagca cagccaagat 20
<210> 555
   <211> 20
   <212> DNA
   <213> Homo sapiens
<400> 555
   tgtcctgcca cttttacatc 20
<210> 556
   <211> 21
   <212> DNA
   <213> Homo sapiens
<400> 556
   gagctaagca gaacctaagg a 21
<210> 557
   <211> 21
   <212> DNA
   <213> Homo sapiens
<400> 557
   ttaagcaaca ggaacctacc c 21
<210> 558
   <211> 21
   <212> DNA
   <213> Homo sapiens
<400> 558
   gccaagggta atcatagcaa c 21
<210> 559
   <211> 21
   <212> DNA
   <213> Homo sapiens
<400> 559
   tcataatgaa acccttgctg c 21
<210> 560
   <211> 21
   <212> DNA
   <213> Homo sapiens
<400> 560
   aatcctagtg catgagactc c 21
<210> 561
   <211> 21
   <212> DNA
   <213> Homo sapiens
<400> 561
   tactgcctgc atcattacca c 21
<210> 562
   <211> 21
   <212> DNA
   <213> Homo sapiens
<400> 562
   attaacaatg aggagccagg t 21
<210> 563
   <211> 21
   <212> DNA
   <213> Homo sapiens
<400> 563
   gagagatggt tgagaaatgc c 21
<210> 564
   <211> 21
   <212> DNA
   <213> Homo sapiens
<400> 564
   actacaacca ccaattacag c 21
<210> 565
   <211> 21
   <212> DNA
   <213> Homo sapiens
<400> 565
   agtgacattt ccaagggctt t 21
<210> 566
   <211> 21
   <212> DNA
   <213> Homo sapiens
<400> 566
   ggagatggga agacgattag a 21
<210> 567
   <211> 22
   <212> DNA
   <213> Homo sapiens
<400> 567
   agtctttcag tcttacatgg gt 22
<210> 568
   <211> 21
   <212> DNA
   <213> Homo sapiens
<400> 568
   tagtttctgt gatcctggca g 21
<210> 569
   <211> 20
   <212> DNA
   <213> Homo sapiens
<400> 569
   tttctcccag ctgttcctag 20
<210> 570
   <211> 21
   <212> DNA
   <213> Homo sapiens
<400> 570
   tacggaactt cgaatcaact c 21
<210> 571
   <211> 21
   <212> DNA
   <213> Homo sapiens
<400> 571
   tcaagtcacc ctcattgtag g 21
<210> 572
   <211> 21
   <212> DNA
   <213> Homo sapiens
<400> 572
   tgtaacgtgg atgtgagatt g 21
<210> 573
   <211> 21
   <212> DNA
   <213> Homo sapiens
<400> 573
   ttgagctaag tctgcatcac t 21
<210> 574
   <211> 21
   <212> DNA
   <213> Homo sapiens
<400> 574
   ctagagaaag caacgcctaa g 21
<210> 575
   <211> 21
   <212> DNA
   <213> Homo sapiens
<400> 575
   agcaccttcc atagcttctt t 21
<210> 576
   <211> 21
   <212> DNA
   <213> Homo sapiens
<400> 576
   gtgtcttctg atggccaaat g 21
<210> 577
   <211> 21
   <212> DNA
   <213> Homo sapiens
<400> 577
   aatcaggtga aaggtacctc c 21
<210> 578
   <211> 21
   <212> DNA
   <213> Homo sapiens
<400> 578
   ccttcacaat tcagggaaca a 21
<210> 579
   <211> 21
   <212> DNA
   <213> Homo sapiens
<400> 579
   tggacccagt tctatgcaat t 21
<210> 580
   <211> 21
   <212> DNA
   <213> Homo sapiens
<400> 580
   aatgaccctt acaactccga a 21
<210> 581
   <211> 21
   <212> DNA
   <213> Homo sapiens
<400> 581
   tttgggaagt gattgtgaag g 21
<210> 582
   <211> 21
   <212> DNA
   <213> Homo sapiens
<400> 582
   tcagctgcat agaccttgtt t 21
<210> 583
   <211> 21
   <212> DNA
   <213> Homo sapiens
<400> 583
   tcatttgttc tcattacggg c 21
<210> 584
   <211> 20
   <212> DNA
   <213> Homo sapiens
<400> 584
   ttctacctgg gttctcttgg 20
<210> 585
   <211> 21
   <212> DNA
   <213> Homo sapiens
<400> 585
   taacttcctg agcacacatc a 21
<210> 586
   <211> 21
   <212> DNA
   <213> Homo sapiens
<400> 586
   gggtaaattc aggaatgcac a 21
<210> 587
   <211> 21
   <212> DNA
   <213> Homo sapiens
<400> 587
   atttcttctg gtgagtttgc g 21
<210> 588
   <211> 21
   <212> DNA
   <213> Homo sapiens
<400> 588
   cctcttcctg acatgttgtt g 21
<210> 589
   <211> 21
   <212> DNA
   <213> Homo sapiens
<400> 589
   gttggtgcca gattgtaact t 21
<210> 590
   <211> 21
   <212> DNA
   <213> Homo sapiens
<400> 590
   gcctggctca ataatagtcc t 21
<210> 591
   <211> 21
   <212> DNA
   <213> Homo sapiens
<400> 591
   atgttgcctt ctctacgttt g 21
<210> 592
   <211> 20
   <212> DNA
   <213> Homo sapiens
<400> 592
   gagtggagcg ctacctttat 20
<210> 593
   <211> 20
   <212> DNA
   <213> Homo sapiens
<400> 593
   agaatgggaa atgggaagga 20
<210> 594
   <211> 20
   <212> DNA
   <213> Homo sapiens
<400> 594
   cacaatacat gggctgcttt 20
<210> 595
   <211> 21
   <212> DNA
   <213> Homo sapiens
<400> 595
   ccactccaac tctgctttta c 21
<210> 596
   <211> 21
   <212> DNA
   <213> Homo sapiens
<400> 596
   gatgtggatt gtctttgttg c 21
<210> 597
   <211> 22
   <212> DNA
   <213> Homo sapiens
<400> 597
   agcctttcat tgcacatttc ag 22
<210> 598
   <211> 20
   <212> DNA
   <213> Homo sapiens
<400> 598
   tcccaccaca agaccaattt 20
<210> 599
   <211> 21
   <212> DNA
   <213> Homo sapiens
<400> 599
   gaagataggt ggtggagttc a 21
<210> 600
   <211> 21
   <212> DNA
   <213> Homo sapiens
<400> 600
   ctgtttgaat gaagttggct g 21
<210> 601
   <211> 21
   <212> DNA
   <213> Homo sapiens
<400> 601
   tatacatggg tgggatttgt c 21
<210> 602
   <211> 21
   <212> DNA
   <213> Homo sapiens
<400> 602
   tttgtgatgg accatctaac c 21
<210> 603
   <211> 20
   <212> DNA
   <213> Homo sapiens
<400> 603
   gaagatctgg tgtcccacta 20
<210> 604
   <211> 20
   <212> DNA
   <213> Homo sapiens
<400> 604
   atctgaagat ctccgtggta 20
<210> 605
   <211> 21
   <212> DNA
   <213> Homo sapiens
<400> 605
   atgtatgaac catttcctgc t 21
<210> 606
   <211> 22
   <212> DNA
   <213> Homo sapiens
<400> 606
   agcacagaat tgaatgaagg aa 22
<210> 607
   <211> 20
   <212> DNA
   <213> Homo sapiens
<400> 607
   gcatgagtaa ggctgaagtg 20
<210> 608
   <211> 24
   <212> DNA
   <213> Homo sapiens
<400> 608
   aaaggcttat attgcttttg aatc 24
<210> 609
   <211> 20
   <212> DNA
   <213> Homo sapiens
<400> 609
   ttgctgttgt tgggatcaag 20
<210> 610
   <211> 20
   <212> DNA
   <213> Homo sapiens
<400> 610
   gcttctgcat ccacctatct 20
<210> 611
   <211> 20
   <212> DNA
   <213> Homo sapiens
<400> 611
   aaaagctgcc taaaatgcca 20
<210> 612
   <211> 20
   <212> DNA
   <213> Homo sapiens
<400> 612
   agtcagcaag ttagcagaaa 20
<210> 613
   <211> 20
   <212> DNA
   <213> Homo sapiens
<400> 613
   cccacattta tcccttgtcc 20
<210> 614
   <211> 20
   <212> DNA
   <213> Homo sapiens
<400> 614
   tgtctgattc catctttccc 20
<210> 615
   <211> 19
   <212> DNA
   <213> Homo sapiens
<400> 615
   cagccaccaa aacacaatg 19
<210> 616
   <211> 20
   <212> DNA
   <213> Homo sapiens
<400> 616
   aaggcctgtt ttgtgtgtag 20
<210> 617
   <211> 21
   <212> DNA
   <213> Homo sapiens
<400> 617
   acagctcaca gatctttaag c 21
<210> 618
   <211> 23
   <212> DNA
   <213> Homo sapiens
<400> 618
   ggcattataa agagatagct cca 23
<210> 619
   <211> 20
   <212> DNA
   <213> Homo sapiens
<400> 619
   tgtttttcct tgccctgtaa 20
<210> 620
   <211> 20
   <212> DNA
   <213> Homo sapiens
<400> 620
   taaccagatg aatgagggca 20
<210> 621
   <211> 20
   <212> DNA
   <213> Homo sapiens
<400> 621
   caacaagcca aaaccacatc 20
<210> 622
   <211> 20
   <212> DNA
   <213> Homo sapiens
<400> 622
   gttggttctt gaagacctga 20
<210> 623
   <211> 22
   <212> DNA
   <213> Homo sapiens
<400> 623
   tgtggtaagt agtctctaaa ga 22
<210> 624
   <211> 20
   <212> DNA
   <213> Homo sapiens
<400> 624
   accaaatttc cagatcacgg 20
<210> 625
   <211> 20
   <212> DNA
   <213> Homo sapiens
<400> 625
   aatgaatggt catggctcac 20
<210> 626
   <211> 20
   <212> DNA
   <213> Homo sapiens
<400> 626
   gccctgaagc acattaaagt 20
<210> 627
   <211> 20
   <212> DNA
   <213> Homo sapiens
<400> 627
   gcctccaggt ttatgacaac 20
<210> 628
   <211> 20
   <212> DNA
   <213> Homo sapiens
<400> 628
   cagctctatt ccccttctga 20
<210> 629
   <211> 20
   <212> DNA
   <213> Homo sapiens
<400> 629
   ccttggaagg gaaagttgat 20
<210> 630
   <211> 18
   <212> DNA
   <213> Homo sapiens
<400> 630
   gtcccaccct gctcttag 18
<210> 631
   <211> 20
   <212> DNA
   <213> Homo sapiens
<400> 631
   cagagtaaga cagtgggaca 20
<210> 632
   <211> 20
   <212> DNA
   <213> Homo sapiens
<400> 632
   aagtgtggtg cataaaggat 20
<210> 633
   <211> 20
   <212> DNA
   <213> Homo sapiens
<400> 633
   ctgtaagaag gagggtttgg 20
<210> 634
   <211> 20
   <212> DNA
   <213> Homo sapiens
<400> 634
   gaggttgatg agaggtaggg 20
<210> 635
   <211> 20
   <212> DNA
   <213> Homo sapiens
<400> 635
   agccagggat attgttgaag 20
<210> 636
   <211> 20
   <212> DNA
   <213> Homo sapiens
<400> 636
   caggtaagtg tgtgttccag 20
<210> 637
   <211> 20
   <212> DNA
   <213> Homo sapiens
<400> 637
   ttgatttcca tgcagaaggg 20
<210> 638
   <211> 20
   <212> DNA
   <213> Homo sapiens
<400> 638
   taatttggcc ttaggggttg 20
<210> 639
   <211> 21
   <212> DNA
   <213> Homo sapiens
<400> 639
   tccataaaag gtgcttaaag c 21
<210> 640
   <211> 20
   <212> DNA
   <213> Homo sapiens
<400> 640
   ttgtatcaca ccatcgtgga 20
<210> 641
   <211> 20
   <212> DNA
   <213> Homo sapiens
<400> 641
   **a**ctgctgaga acaatcatgc 20
<210> 642
   <211> 20
   <212> DNA
   <213> Homo sapiens
<400> 642
   ggtgtggaga atttgtttgc 20
<210> 643
   <211> 20
   <212> DNA
   <213> Homo sapiens
<400> 643
   tgatattagg cggtggctta 20
<210> 644
   <211> 20
   <212> DNA
   <213> Homo sapiens
<400> 644
   tctcacctaa aatctggggc 20
<210> 645
   <211> 22
   <212> DNA
   <213> Homo sapiens
<400> 645
   aggactgtga cactttatct tt 22
<210> 646
   <211> 21
   <212> DNA
   <213> Homo sapiens
<400> 646
   atgcgaggta gaaaatgaga g 21
<210> 647
   <211> 21
   <212> DNA
   <213> Homo sapiens
<400> 647
   tgtggcttta aggttctgaa g 21
<210> 648
   <211> 20
   <212> DNA
   <213> Homo sapiens
<400> 648
   tcgttgctat tctgctttga 20
<210> 649
   <211> 20
   <212> DNA
   <213> Homo sapiens
<400> 649
   tcccttttgt ggtttcttgg 20
<210> 650
   <211> 20
   <212> DNA
   <213> Homo sapiens
<400> 650
   ttgccgcact cttcattaat 20
<210> 651
   <211> 20
   <212> DNA
   <213> Homo sapiens
<400> 651
   actggcttct cctcattagt 20
<210> 652
   <211> 20
   <212> DNA
   <213> Homo sapiens
<400> 652
   actgatttgc catgtagagc 20
<210> 653
   <211> 20
   <212> DNA
   <213> Homo sapiens
<400> 653
   gaatgtcata ttgcctgcca 20
<210> 654
   <211> 20
   <212> DNA
   <213> Homo sapiens
<400> 654
   tcactcgctt agaatgttgc 20
<210> 655
   <211> 21
   <212> DNA
   <213> Homo sapiens
<400> 655
   accattaact tcctgcaaac t 21
<210> 656
   <211> 20
   <212> DNA
   <213> Homo sapiens
<400> 656
   taaagcccca taccaggatt 20
<210> 657
   <211> 20
   <212> DNA
   <213> Homo sapiens
<400> 657
   ccaaaaatca cccatatgcg 20
<210> 658
   <211> 20
   <212> DNA
   <213> Homo sapiens
<400> 658
   ataacccagg tgcttcaaag 20
<210> 659
   <211> 19
   <212> DNA
   <213> Homo sapiens
<400> 659
   tccacagcag aagtaacga 19
<210> 660
   <211> 21
   <212> DNA
   <213> Homo sapiens
<400> 660
   aggaaagcta tgaagaaagg g 21
<210> 661
   <211> 20
   <212> DNA
   <213> Homo sapiens
<400> 661
   agctccagag tgtcagtatt 20
<210> 662
   <211> 22
   <212> DNA
   <213> Homo sapiens
<400> 662
   ttgatttcca gcactgaact tt 22
<210> 663
   <211> 20
   <212> DNA
   <213> Homo sapiens
<400> 663
   tgtttgattt ttcaggctga 20
<210> 664
   <211> 20
   <212> DNA
   <213> Homo sapiens
<400> 664
   ctggtcattc ctgagtgtct 20
<210> 665
   <211> 24
   <212> DNA
   <213> Homo sapiens
<400> 665
   tcctgtttta cacttttcta actt 24
<210> 666
   <211> 20
   <212> DNA
   <213> Homo sapiens
<400> 666
   acctcaacct gttttagcac 20
<210> 667
   <211> 20
   <212> DNA
   <213> Homo sapiens
<400> 667
   cagtgtgtgt catgccaaat 20
<210> 668
   <211> 21
   <212> DNA
   <213> Homo sapiens
<400> 668
   ggtctatgtt aatcttgggc c 21
<210> 669
   <211> 21
   <212> DNA
   <213> Homo sapiens
<400> 669
   agccagtctg tatctaaagg t 21
<210> 670
   <211> 20
   <212> DNA
   <213> Homo sapiens
<400> 670
   actcttgggg tttcttcagt 20
<210> 671
   <211> 21
   <212> DNA
   <213> Homo sapiens
<400> 671
   cgaaccaaaa gcaaaatcct t 21
<210> 672
   <211> 20
   <212> DNA
   <213> Homo sapiens
<400> 672
   aatctatgga ggtcactggg 20
<210> 673
   <211> 20
   <212> DNA
   <213> Homo sapiens
<400> 673
   aggtccttgt agtttgcttg 20
<210> 674
   <211> 20
   <212> DNA
   <213> Homo sapiens
<400> 674
   gtgagagcca atagagtgtg 20
<210> 675
   <211> 20
   <212> DNA
   <213> Homo sapiens
<400> 675
   gcatggtgtg tgaaagtgat 20
<210> 676
   <211> 20
   <212> DNA
   <213> Homo sapiens
<400> 676
   atgctgcttt tgactgatgt 20
<210> 677
   <211> 20
   <212> DNA
   <213> Homo sapiens
<400> 677
   cgaagctgta ttcctgtctc 20
<210> 678
   <211> 19
   <212> DNA
   <213> Homo sapiens
<400> 678
   atggactaac tggagagcg 19
<210> 679
   <211> 20
   <212> DNA
   <213> Homo sapiens
<400> 679
   atgtcctgcc agtaaacaca 20
<210> 680
   <211> 20
   <212> DNA
   <213> Homo sapiens
<400> 680
   tgccactaaa cacctaagga 20
<210> 681
   <211> 20
   <212> DNA
   <213> Homo sapiens
<400> 681
   ttggaaattt tggggtcagg 20
<210> 682
   <211> 20
   <212> DNA
   <213> Homo sapiens
<400> 682
   ttctgacctc ccttactgag 20
<210> 683
   <211> 19
   <212> DNA
   <213> Homo sapiens
<400> 683
   tatgtgcttg cgatgtgtt 19
<210> 684
   <211> 20
   <212> DNA
   <213> Homo sapiens
<400> 684
   gaaccttagg gccagtctat 20
<210> 685
   <211> 21
   <212> DNA
   <213> Homo sapiens
<400> 685
   agaacctgat gtgttttcct c 21
<210> 686
   <211> 20
   <212> DNA
   <213> Homo sapiens
<400> 686
   atgtgagaga agcaaaaccc 20
<210> 687
   <211> 20
   <212> DNA
   <213> Homo sapiens
<400> 687
   gtagttgtct tgagggcttt 20
<210> 688
   <211> 20
   <212> DNA
   <213> Homo sapiens
<400> 688
   cctggtcaac aacatatggg 20
<210> 689
   <211> 21
   <212> DNA
   <213> Homo sapiens
<400> 689
   agaatattgc atttggccag a 21
<210> 690
   <211> 21
   <212> DNA
   <213> Homo sapiens
<400> 690
   aaagctatgc aaatagtggc a 21
<210> 691
   <211> 20
   <212> DNA
   <213> Homo sapiens
<400> 691
   agttcccaac agaggctaat 20
<210> 692
   <211> 20
   <212> DNA
   <213> Homo sapiens
<400> 692
   agttataggt gaggaagggc 20
<210> 693
   <211> 20
   <212> DNA
   <213> Homo sapiens
<400> 693
   cactgcaaaa gaaggaggtt 20
<210> 694
   <211> 21
   <212> DNA
   <213> Homo sapiens
<400> 694
   tctcaacatc gctgatctag t 21
<210> 695
   <211> 20
   <212> DNA
   <213> Homo sapiens
<400> 695
   aggtagctgg aaaaggagaa 20
<210> 696
   <211> 21
   <212> DNA
   <213> Homo sapiens
<400> 696
   tgaaattgcc cagaattgag t 21
<210> 697
   <211> 20
   <212> DNA
   <213> Homo sapiens
<400> 697
   attttcctgg acttctgaca 20
<210> 698
   <211> 21
   <212> DNA
   <213> Homo sapiens
<400> 698
   tgttattcct cttcctgtcc a 21
<210> 699
   <211> 20
   <212> DNA
   <213> Homo sapiens

<400> 699
   taaccacaca actacagctt 20
<210> 700
   <211> 20
   <212> DNA
   <213> Homo sapiens
<400> 700
   attcaaaatg gggacgagag 20
<210> 701
   <211> 19
   <212> DNA
   <213> Homo sapiens
<400> 701
   tcccagtttg ctactctgg 19
<210> 702
   <211> 24
   <212> DNA
   <213> Homo sapiens
<400> 702
   tctcattatg tgaagattgc tttc 24
<210> 703
   <211> 23
   <212> DNA
   <213> Homo sapiens
<400> 703
   ttaagattaa gcagtcttct tgg 23
<210> 704
   <211> 20
   <212> DNA
   <213> Homo sapiens
<400> 704
   caacagatct gattctgccc 20
<210> 705
   <211> 20
   <212> DNA
   <213> Homo sapiens
<400> 705
   catgtgtttc aaagttggct 20
<210> 706
   <211> 21
   <212> DNA
   <213> Homo sapiens
<400> 706
   actatagcat tagggtgagg g 21
<210> 707
   <211> 20
   <212> DNA
   <213> Homo sapiens
<400> 707
   gagatttgga catgctttca 20
<210> 708
   <211> 19
   <212> DNA
   <213> Homo sapiens
<400> 708
   cccaccacca gttgtcatc 19
<210> 709
   <211> 21
   <212> DNA
   <213> Homo sapiens
<400> 709
   gccactcact tcctagataa t 21
<210> 710
   <211> 20
   <212> DNA
   <213> Homo sapiens
<400> 710
   acagtatctc agggccttat 20
<210> 711
   <211> 21
   <212> DNA
   <213> Homo sapiens
<400> 711
   tcagtagttc ctcagatgct a 21
<210> 712
   <211> 20
   <212> DNA
   <213> Homo sapiens
<400> 712
   aatgcatgaa agtccaggaa 20
<210> 713
   <211> 21
   <212> DNA
   <213> Homo sapiens
<400> 713
   acatgcactc ttgtcttatg c 21
<210> 714
   <211> 22
   <212> DNA
   <213> Homo sapiens
<400> 714
   cacatatact ggctttctgg tc 22
<210> 715
   <211> 19
   <212> DNA
   <213> Homo sapiens
<400> 715
   gcacatgtca ttagcaggg 19
<210> 716
   <211> 20
   <212> DNA
   <213> Homo sapiens
<400> 716
   acatgctcaa ttatggagcc 20
<210> 717
   <211> 20
   <212> DNA
   <213> Homo sapiens
<400> 717
   acaaagacag gaatagggct 20
<210> 718
   <211> 24
   <212> DNA
   <213> Homo sapiens
<400> 718
   caatctgctg acttgcttct tttc 24
<210> 719
   <211> 21
   <212> DNA
   <213> Homo sapiens
<400> 719
   ctttggctca gaatcttcca a 21
<210> 720
   <211> 21
   <212> DNA
   <213> Homo sapiens
<400> 720
   tgtatgagga ccagcagtaa a 21
<210> 721
   <211> 20
   <212> DNA
   <213> Homo sapiens
<400> 721
   tcttttcccc ttgtgcatag 20
<210> 722
   <211> 20
   <212> DNA
   <213> Homo sapiens
<400> 722
   gcttagtgtg tgtgatccgt 20
<210> 723
   <211> 20
   <212> DNA
   <213> Homo sapiens
<400> 723
   actcaccttt cccaagaaga 20
<210> 724
   <211> 20
   <212> DNA
   <213> Homo sapiens
<400> 724
   tggtagtggg aagaggttga 20
<210> 725
   <211> 20
   <212> DNA
   <213> Homo sapiens
<400> 725
   gcatggagaa caaaagctga 20
<210> 726
   <211> 20
   <212> DNA
   <213> Homo sapiens
<400> 726
   tgtgagcaag aaactgaagg 20
<210> 727
   <211> 20
   <212> DNA
   <213> Homo sapiens
<400> 727
   agtatcactt gtccagctca 20
<210> 728
   <211> 20
   <212> DNA
   <213> Homo sapiens
<400> 728
   cacaggacta ggtaggcttt 20
<210> 729
   <211> 21
   <212> DNA
   <213> Homo sapiens
<400> 729
   gtgttaacag ctttcccttc a 21
<210> 730
   <211> 23
   <212> DNA
   <213> Homo sapiens
<400> 730
   tcatgtacag aaagaattag cct 23
<210> 731
   <211> 20
   <212> DNA
   <213> Homo sapiens
<400> 731
   agaggccaag tgaccaaata 20
<210> 732
   <211> 21
   <212> DNA
   <213> Homo sapiens
<400> 732
   aagccagtaa ttcatcttcc c 21
<210> 733
   <211> 21
   <212> DNA
   <213> Homo sapiens
<400> 733
   ccatgacata acacatcacc a 21
<210> 734
   <211> 20
   <212> DNA
   <213> Homo sapiens
<400> 734
   atagatttcc tcctgggctg 20
<210> 735
   <211> 20
   <212> DNA
   <213> Homo sapiens
<400> 735
   ccacctctgt acccactatc 20
<210> 736
   <211> 20
   <212> DNA
   <213> Homo sapiens
<400> 736
   ttccctggaa gatagccaat 20
<210> 737
   <211> 20
   <212> DNA
   <213> Homo sapiens
<400> 737
   tggacacgta aaagaaggtg 20
<210> 738
   <211> 20
   <212> DNA
   <213> Homo sapiens
<400> 738
   ctgagaacct tgtccaactg 20
<210> 739
   <211> 20
   <212> DNA
   <213> Homo sapiens
<400> 739
   tttcgctagt ctttgcactt 20
<210> 740
   <211> 20
   <212> DNA
   <213> Homo sapiens
<400> 740
   gcggcaataa ttgtcacaaa 20
<210> 741
   <211> 20
   <212> DNA
   <213> Homo sapiens
<400> 741
   cagaaatgtg tcaggctaca 20
<210> 742
   <211> 20
   <212> DNA
   <213> Homo sapiens
<400> 742
   aactcttcat tttgacgggg 20
<210> 743
   <211> 20
   <212> DNA
   <213> Homo sapiens
<400> 743
   tgggttgtgc tgttgtttag 20
<210> 744
   <211> 19
   <212> DNA
   <213> Homo sapiens
<400> 744
   gctcagctcc tttcatctg 19
<210> 745
   <211> 20
   <212> DNA
   <213> Homo sapiens
<400> 745
   aggacattca gcctatttgc 20
<210> 746
   <211> 20
   <212> DNA
   <213> Homo sapiens
<400> 746
   acacagtatc aaggtcaaca 20
<210> 747
   <211> 20
   <212> DNA
   <213> Homo sapiens
<400> 747
   gcagaaccac agtctatgag 20
<210> 748
   <211> 21
   <212> DNA
   <213> Homo sapiens
<400> 748
   aatgctccaa gttattccag a 21
<210> 749
   <211> 20
   <212> DNA
   <213> Homo sapiens
<400> 749
   aagagagaag cgacaaaacc 20
<210> 750
   <211> 20
   <212> DNA
   <213> Homo sapiens
<400> 750
   aatgagaagg aattgggtgc 20
<210> 751
   <211> 20
   <212> DNA
   <213> Homo sapiens
<400> 751
   aatgaagtgt tagggccatc 20
<210> 752
   <211> 21
   <212> DNA
   <213> Homo sapiens
<400> 752
   gctcatcaca gtttaaggag t 21
<210> 753
   <211> 20
   <212> DNA
   <213> Homo sapiens
<400> 753
   atgtggaagc aagagaaagg 20
<210> 754
   <211> 20
   <212> DNA
   <213> Homo sapiens
<400> 754
   tgaaaggtga agtggctttt 20
<210> 755
   <211> 20
   <212> DNA
   <213> Homo sapiens
<400> 755
   gtaagtaagg ggtcctagct 20
<210> 756
   <211> 20
   <212> DNA
   <213> Homo sapiens
<400> 756
   tgtgacttcc atgaaactgg 20
<210> 757
   <211> 20
   <212> DNA
   <213> Homo sapiens
<400> 757
   gctgacaaac taaccttcca 20
<210> 758
   <211> 20
   <212> DNA
   <213> Homo sapiens
<400> 758
   aaaacctccc aaaacagact 20
<210> 759
   <211> 20
   <212> DNA
   <213> Homo sapiens
<400> 759
   agtttagtgg ccacgtgaaa 20
<210> 760
   <211> 20
   <212> DNA
   <213> Homo sapiens
<400> 760
   ggcagaagtt tcaattccct 20
<210> 761
   <211> 20
   <212> DNA
   <213> Homo sapiens
<400> 761
   aaagcctctg tttgcacttt 20
<210> 762
   <211> 20
   <212> DNA
   <213> Homo sapiens
<400> 762
   tcagtcagct tcttgagtca 20
<210> 763
   <211> 20
   <212> DNA
   <213> Homo sapiens
<400> 763
   tgtttcattt gggtcatgga 20
<210> 764
   <211> 21
   <212> DNA
   <213> Homo sapiens
<400> 764
   tacagcacta ggatcactct g 21
<210> 765
   <211> 20
   <212> DNA
   <213> Homo sapiens
<400> 765
   atgcatgttt cttgcaaagg 20
<210> 766
   <211> 20
   <212> DNA
   <213> Homo sapiens
<400> 766
   gtaggattca gggcatttca 20
<210> 767
   <211> 19
   <212> DNA
   <213> Homo sapiens
<400> 767
   aactggaact gagcgtgag 19
<210> 768
   <211> 20
   <212> DNA
   <213> Homo sapiens
<400> 768
   tgtatgcagt tacctccaga 20
<210> 769
   <211> 20
   <212> DNA
   <213> Homo sapiens
<400> 769
   ggatgtatac cagacccctt 20
<210> 770
   <211> 20
   <212> DNA
   <213> Homo sapiens
<400> 770
   cgaaagatgt tagcacctca 20
<210> 771
   <211> 20
   <212> DNA
   <213> Homo sapiens
<400> 771
   aattggcttt gcagtgtttc 20
<210> 772
   <211> 20
   <212> DNA
   <213> Homo sapiens
<400> 772
   gcttgctggt aggaggtata 20
<210> 773
   <211> 20
   <212> DNA
   <213> Homo sapiens
<400> 773
   ctggaaacgg aaggaagttg 20
<210> 774
   <211> 20
   <212> DNA
   <213> Homo sapiens
<400> 774
   ctgtgttcag taagtggctg 20
<210> 775
   <211> 20
   <212> DNA
   <213> Homo sapiens
<400> 775
   gacagtgaag tgtgatcgtt 20
<210> 776
   <211> 20
   <212> DNA
   <213> Homo sapiens
<400> 776
   tggtgatgct gttttggaaa 20
<210> 777
   <211> 22
   <212> DNA
   <213> Homo sapiens
<400> 777
   ttctttcagg cagaagaaat ga 22
<210> 778
   <211> 24
   <212> DNA
   <213> Homo sapiens
<400> 778
   aagttccatt actgtattga aaat 24
<210> 779
   <211> 20
   <212> DNA
   <213> Homo sapiens
<400> 779
   ggacgtacgt gcttatttca 20
<210> 780
   <211> 21
   <212> DNA
   <213> Homo sapiens
<400> 780
   cagcctgata ttcccattga g 21
<210> 781
   <211> 20
   <212> DNA
   <213> Homo sapiens
<400> 781
   tcatgaggct gagtgagtat 20
<210> 782
   <211> 20
   <212> DNA
   <213> Homo sapiens
<400> 782
   aaagctctcc tatctccagt 20
<210> 783
   <211> 21
   <212> DNA
   <213> Homo sapiens
<400> 783
   acaaagagtc tggactatcc t 21
<210> 784
   <211> 21
   <212> DNA
   <213> Homo sapiens
<400> 784
   tgcctaaaaa tacccaaagc t 21
<210> 785
   <211> 20
   <212> DNA
   <213> Homo sapiens
<400> 785
   ccaagacact cactccaaag 20
<210> 786
   <211> 20
   <212> DNA
   <213> Homo sapiens
<400> 786
   ccaggaaaag gagcagtttt 20
<210> 787
   <211> 20
   <212> DNA
   <213> Homo sapiens
<400> 787
   gttcaactct ttctgcgaac 20
<210> 788
   <211> 20
   <212> DNA
   <213> Homo sapiens
<400> 788
   ccacttcttc tgtttccaac 20
<210> 789
   <211> 19
   <212> DNA
   <213> Homo sapiens
<400> 789
   gggaagggca tgctaatca 19
<210> 790
   <211> 20
   <212> DNA
   <213> Homo sapiens
<400> 790
   tgttcaatca cctctccatc 20
<210> 791
   <211> 20
   <212> DNA
   <213> Homo sapiens
<400> 791
   agaggagagg ctaagctttg 20
<210> 792
   <211> 20
   <212> DNA
   <213> Homo sapiens
<400> 792
   acaagagagg aagctgtcag 20
<210> 793
   <211> 20
   <212> DNA
   <213> Homo sapiens
<400> 793
   agcccctttc tccttattct 20
<210> 794
   <211> 20
   <212> DNA
   <213> Homo sapiens
<400> 794
   agggaagcag gattttaacg 20
<210> 795
   <211> 20
   <212> DNA
   <213> Homo sapiens
<400> 795
   aagtttcaca atacccaggt 20
<210> 796
   <211> 20
   <212> DNA
   <213> Homo sapiens
<400> 796
   tcgcagaatg gacaagtact 20
<210> 797
   <211> 20
   <212> DNA
   <213> Homo sapiens
<400> 797
   ggctcccaga ttttgatcat 20
<210> 798
   <211> 20
   <212> DNA
   <213> Homo sapiens
<400> 798
   cctagaactg caaaacacct 20
<210> 799
   <211> 21
   <212> DNA
   <213> Homo sapiens
<400> 799
   ttccattatt ttctcaccgg c 21
<210> 800
   <211> 20
   <212> DNA
   <213> Homo sapiens
<400> 800
   cagtccaaca aagaggtcac 20
<210> 801
   <211> 20
   <212> DNA
   <213> Homo sapiens
<400> 801
   ttgtgtgtgt tgaagcctag 20
<210> 802
   <211> 20
   <212> DNA
   <213> Homo sapiens
<400> 802
   agtgggggta ggaagaaaaa 20
<210> 803
   <211> 20
   <212> DNA
   <213> Homo sapiens
<400> 803
   gttccaacaa tgtaaggcac 20
<210> 804
   <211> 20
   <212> DNA
   <213> Homo sapiens
<400> 804
   ggaaccctct atggtcaaag 20
<210> 805
   <211> 20
   <212> DNA
   <213> Homo sapiens
<400> 805
   attgctgtgt agttccttga 20
<210> 806
   <211> 20
   <212> DNA
   <213> Homo sapiens
<400> 806
   tttcttgcca ccattctgac 20
<210> 807
   <211> 20
   <212> DNA
   <213> Homo sapiens
<400> 807
   cacacgttct aaccaagtgc 20
<210> 808
   <211> 21
   <212> DNA
   <213> Homo sapiens
<400> 808
   tagccacatc ttaacagacc t 21
<210> 809
   <211> 20
   <212> DNA
   <213> Homo sapiens
<400> 809
   attcctgagg gtgacatgaa 20
<210> 810
   <211> 20
   <212> DNA
   <213> Homo sapiens
<400> 810
   cctgccccat caacttaaaa 20
<210> 811
   <211> 18
   <212> DNA
   <213> Homo sapiens
<400> 811
   ctgggtgcag aggatctc 18
<210> 812
   <211> 22
   <212> DNA
   <213> Homo sapiens
<400> 812
   ttgagttgaa ctttgcttta ga 22
<210> 813
   <211> 20
   <212> DNA
   <213> Homo sapiens
<400> 813
   gaactaggag acactgggtt 20
<210> 814
   <211> 21
   <212> DNA
   <213> Homo sapiens
<400> 814
   caagaatagc taactggtgc t 21
<210> 815
   <211> 21
   <212> DNA
   <213> Homo sapiens
<400> 815
   agttacacac tgaatcatgg g 21
<210> 816
   <211> 20
   <212> DNA
   <213> Homo sapiens
<400> 816
   cccatgtggc ttcactaata 20
<210> 817
   <211> 20
   <212> DNA
   <213> Homo sapiens
<400> 817
   gattgtggtc acgtggagag 20
<210> 818
   <211> 20
   <212> DNA
   <213> Homo sapiens
<400> 818
   cagatccagg tattcggaga 20
<210> 819
   <211> 20
   <212> DNA
   <213> Homo sapiens
<400> 819
   acaacaacaa ccattaccca 20
<210> 820
   <211> 20
   <212> DNA
   <213> Homo sapiens
<400> 820
   tggccatagt actgcttgta 20
<210> 821
   <211> 20
   <212> DNA
   <213> Homo sapiens
<400> 821
   tgtctcactg ttgggaactt 20
<210> 822
   <211> 20
   <212> DNA
   <213> Homo sapiens
<400> 822
   tttgtctgta tcctatgccc 20
<210> 823
   <211> 20
   <212> DNA
   <213> Homo sapiens
<400> 823
   gctgacaaaa ttggatccca 20
<210> 824
   <211> 20
   <212> DNA
   <213> Homo sapiens
<400> 824
   aaaatcctcc tgagtcctct 20
<210> 825
   <211> 20
   <212> DNA
   <213> Homo sapiens
<400> 825
   ttcactgggc tcttcagcta 20
<210> 826
   <211> 20
   <212> DNA
   <213> Homo sapiens
<400> 826
   aaagggcagg agttaggtaa 20
<210> 827
   <211> 20
   <212> DNA
   <213> Homo sapiens
<400> 827
   aggcataaga aaccaggttg 20
<210> 828
   <211> 20
   <212> DNA
   <213> Homo sapiens
<400> 828
   gtagttcggt ccaatgtcag 20
<210> 829
   <211> 20
   <212> DNA
   <213> Homo sapiens
<400> 829
   gatggtcaca attgcaggtt 20
<210> 830
   <211> 20
   <212> DNA
   <213> Homo sapiens
<400> 830
   gccaaagatc tcaattgcca 20
<210> 831
   <211> 20
   <212> DNA
   <213> Homo sapiens
<400> 831
   gaccaagact gtctctcctt 20
<210> 832
   <211> 22
   <212> DNA
   <213> Homo sapiens
<400> 832
   taatggtcaa atccctctca aa 22
<210> 833
   <211> 20
   <212> DNA
   <213> Homo sapiens
<400> 833
   catgtaggct gaagactcct 20
<210> 834
   <211> 20
   <212> DNA
   <213> Homo sapiens
<400> 834
   tttctctcca aactggttgc 20
<210> 835
   <211> 21
   <212> DNA
   <213> Homo sapiens
<400> 835
   tacaggcaag aaatagtgtc t 21
<210> 836
   <211> 20
   <212> DNA
   <213> Homo sapiens
<400> 836
   ttcagcaaga atggggattc 20
<210> 837
   <211> 20
   <212> DNA
   <213> Homo sapiens
<400> 837
   caaagagaga gccatcacag 20
<210> 838
   <211> 21
   <212> DNA
   <213> Homo sapiens
<400> 838
   tgagaacact gctatttctg c 21
<210> 839
   <211> 18
   <212> DNA
   <213> Homo sapiens
<400> 839
   gcatggtcag gacattgg 18
<210> 840
   <211> 20
   <212> DNA
   <213> Homo sapiens
<400> 840
   gattgaatca ggagggaagc 20
<210> 841
   <211> 23
   <212> DNA
   <213> Homo sapiens
<400> 841
   agcttaaatg atgaagtgct ttc 23
<210> 842
   <211> 20
   <212> DNA
   <213> Homo sapiens
<400> 842
   ttctcctacg tatcttggca 20
<210> 843
   <211> 20
   <212> DNA
   <213> Homo sapiens
<400> 843
   acctgggaca taaccttgat 20
<210> 844
   <211> 20
   <212> DNA
   <213> Homo sapiens
<400> 844
   ccattttcct actgcgtgtc 20
<210> 845
   <211> 20
   <212> DNA
   <213> Homo sapiens
<400> 845
   gaacatacca aacccactgg 20
<210> 846
   <211> 20
   <212> DNA
   <213> Homo sapiens
<400> 846
   acccaatgat gtacagttcc 20
<210> 847
   <211> 22
   <212> DNA
   <213> Homo sapiens
<400> 847
   acctattcga cttgaaactc ag 22
<210> 848
   <211> 20
   <212> DNA
   <213> Homo sapiens
<400> 848
   atttctgcac aactgttcca 20
<210> 849
   <211> 20
   <212> DNA
   <213> Homo sapiens
<400> 849
   gctgtaatgt gactaaccct 20
<210> 850
   <211> 20
   <212> DNA
   <213> Homo sapiens
<400> 850
   tttcccgagg ttcacagata 20
<210> 851
   <211> 20
   <212> DNA
   <213> Homo sapiens
<400> 851
   gaacttgtgt gacccaaaac 20
<210> 852
   <211> 20
   <212> DNA
   <213> Homo sapiens
<400> 852
   cattgcactg tgatgtcatg 20
<210> 853
   <211> 20
   <212> DNA
   <213> Homo sapiens
<400> 853
   gcactggaaa ttgacatcac 20
<210> 854
   <211> 20
   <212> DNA
   <213> Homo sapiens
<400> 854
   gggagaggct gaaagaagaa 20
<210> 855
   <211> 20
   <212> DNA
   <213> Homo sapiens
<400> 855
   tgtatcactt cctcatgcca 20
<210> 856
   <211> 20
   <212> DNA
   <213> Homo sapiens
<400> 856
   ccaagagttt cctgtttcca 20
<210> 857
   <211> 22
   <212> DNA
   <213> Homo sapiens
<400> 857
   atgacacata catccattta ca 22
<210> 858
   <211> 20
   <212> DNA
   <213> Homo sapiens
<400> 858
   ctcaaactgc ccagtgattt 20
<210> 859
   <211> 20
   <212> DNA
   <213> Homo sapiens
<400> 859
   tcctagcttg ccaaagaaat 20
<210> 860
   <211> 20
   <212> DNA
   <213> Homo sapiens
<400> 860
   cctcctctcc aggcatttta 20
<210> 861
   <211> 20
   <212> DNA
   <213> Homo sapiens
<400> 861
   aacagagtag cacagagagt 20
<210> 862
   <211> 20
   <212> DNA
   <213> Homo sapiens
<400> 862
   ttcagagaga cagacagcat 20
<210> 863
   <211> 20
   <212> DNA
   <213> Homo sapiens
<400> 863
   agaggaaaat cacaagcagt 20
<210> 864
   <211> 20
   <212> DNA
   <213> Homo sapiens
<400> 864
   tttgaaaacc caacagacct 20
<210> 865
   <211> 20
   <212> DNA
   <213> Homo sapiens
<400> 865
   aatccacaca ccaacagagg 20
<210> 866
   <211> 21
   <212> DNA
   <213> Homo sapiens
<400> 866
   tgttcacatc tgttggtttg c 21
<210> 867
   <211> 21
   <212> DNA
   <213> Homo sapiens
<400> 867
   gttactcggt gggtgatatt t 21
<210> 868
   <211> 21
   <212> DNA
   <213> Homo sapiens
<400> 868
   aagaaacacc agcatcagtt c 21
<210> 869
   <211> 21
   <212> DNA
   <213> Homo sapiens
<400> 869
   aggtttagag gtgagtgaac a 21
<210> 870
   <211> 22
   <212> DNA
   <213> Homo sapiens
<400> 870
   agaaacttca ctgtcttcca ct 22
<210> 871
   <211> 21
   <212> DNA
   <213> Homo sapiens
<400> 871
   tgtgatggac attggtacct g 21
<210> 872
   <211> 21
   <212> DNA
   <213> Homo sapiens
<400> 872
   atatcatctg cctgtcccaa c 21
<210> 873
   <211> 21
   <212> DNA
   <213> Homo sapiens
<400> 873
   gagtgctctg tgtttgtttc a 21
<210> 874
   <211> 22
   <212> DNA
   <213> Homo sapiens
<400> 874
   tcagtggtga gctcttgaat at 22
<210> 875
   <211> 21
   <212> DNA
   <213> Homo sapiens
<400> 875
   actctctctt cacacatgca a 21
<210> 876
   <211> 21
   <212> DNA
   <213> Homo sapiens
<400> 876
   gtgttgaagt cagtaaagcc t 21
<210> 877
   <211> 21
   <212> DNA
   <213> Homo sapiens
<400> 877
   tgctttcaca tggcactaga t 21
<210> 878
   <211> 21
   <212> DNA
   <213> Homo sapiens
<400> 878
   ggagagagaa atcccaactg a 21
<210> 879
   <211> 21
   <212> DNA
   <213> Homo sapiens
<400> 879
   gggagatgtc aacactaggt c 21
<210> 880
   <211> 20
   <212> DNA
   <213> Homo sapiens
<400> 880
   atatgacatg gtggctctcc 20
<210> 881
   <211> 21
   <212> DNA
   <213> Homo sapiens
<400> 881
   aacatagagc catgggaggt a 21
<210> 882
   <211> 21
   <212> DNA
   <213> Homo sapiens
<400> 882
   gatagccttc aaatcatgcc t 21
<210> 883
   <211> 21
   <212> DNA
   <213> Homo sapiens
<400> 883
   gaaagcgggt gaacaacaat a 21
<210> 884
   <211> 21
   <212> DNA
   <213> Homo sapiens
<400> 884
   gatagagagc acaaagagca t 21
<210> 885
   <211> 21
   <212> DNA
   <213> Homo sapiens
<400> 885
   aacaagagga ataggagcca g 21
<210> 886
   <211> 21
   <212> DNA
   <213> Homo sapiens
<400> 886
   tgggtgctga tagtaacaaa g 21
<210> 887
   <211> 21
   <212> DNA
   <213> Homo sapiens
<400> 887
   actattgaac tgttggcttc g 21
<210> 888
   <211> 21
   <212> DNA
   <213> Homo sapiens
<400> 888
   tccactaaag agcaaccaaa c 21
<210> 889
   <211> 21
   <212> DNA
   <213> Homo sapiens
<400> 889
   tgaagtggtc agtaacaatg g 21
<210> 890
   <211> 21
   <212> DNA
   <213> Homo sapiens
<400> 890
   atttcagagc tcctttgtcc t 21
<210> 891
   <211> 21
   <212> DNA
   <213> Homo sapiens
<400> 891
   ggcaaagaaa tctggtgttc a 21
<210> 892
   <211> 21
   <212> DNA
   <213> Homo sapiens
<400> 892
   ttgctggttg ataggcattt g 21
<210> 893
   <211> 21
   <212> DNA
   <213> Homo sapiens
<400> 893
   attcccgcaa ttgtgagatt c 21
<210> 894
   <211> 22
   <212> DNA
   <213> Homo sapiens
<400> 894
   aaagaggtac agaactcaga cc 22
<210> 895
   <211> 21
   <212> DNA
   <213> Homo sapiens
<400> 895
   gtcttcatga acgttgccaa t 21
<210> 896
   <211> 21
   <212> DNA
   <213> Homo sapiens
<400> 896
   cttctcaggg ctctttgtgt a 21
<210> 897
   <211> 21
   <212> DNA
   <213> Homo sapiens
<400> 897
   gcaacagaaa ccaagattcc t 21
<210> 898
   <211> 22
   <212> DNA
   <213> Homo sapiens
<400> 898
   tcattgtcta cctcaaagag ca 22
<210> 899
   <211> 21
   <212> DNA
   <213> Homo sapiens
<400> 899
   ctctgaagga acaaaggatg g 21
<210> 900
   <211> 21
   <212> DNA
   <213> Homo sapiens
<400> 900
   cattagaatg cggtggtttc a 21
<210> 901
   <211> 21
   <212> DNA
   <213> Homo sapiens
<400> 901
   gatgtctggg ctgaggttta a 21
<210> 902
   <211> 21
   <212> DNA
   <213> Homo sapiens
<400> 902
   ttcctcttga agatgcactg g 21
<210> 903
   <211> 21
   <212> DNA
   <213> Homo sapiens
<400> 903
   ccagcatgtg aggaattgaa c 21
<210> 904
   <211> 21
   <212> DNA
   <213> Homo sapiens
<400> 904
   cccacttagt catccacaca t 21
<210> 905
   <211> 21
   <212> DNA
   <213> Homo sapiens
<400> 905
   gtttcacaca ccagaagaga g 21
<210> 906
   <211> 21
   <212> DNA
   <213> Homo sapiens
<400> 906
   ccatacacct gctctgacat t 21
<210> 907
   <211> 21
   <212> DNA
   <213> Homo sapiens
<400> 907
   atcagtaaca gtcccattgc t 21
<210> 908
   <211> 21
   <212> DNA
   <213> Homo sapiens
<400> 908
   catgaggcat ttgatccatg g 21
<210> 909
   <211> 21
   <212> DNA
   <213> Homo sapiens
<400> 909
   accctgtttc actgaacaac t 21
<210> 910
   <211> 21
   <212> DNA
   <213> Homo sapiens
<400> 910
   ccctgtaatg agagcgttat t 21
<210> 911
   <211> 22
   <212> DNA
   <213> Homo sapiens
<400> 911
   gagagaatgg gttaaatctg cc 22
<210> 912
   <211> 21
   <212> DNA
   <213> Homo sapiens
<400> 912
   ctgcctgact tagccttaaa t 21
<210> 913
   <211> 21
   <212> DNA
   <213> Homo sapiens
<400> 913
   aaccagaatg ttactagccc a 21
<210> 914
   <211> 21
   <212> DNA
   <213> Homo sapiens
<400> 914
   caatcctgtg tgtttagtgg a 21
<210> 915
   <211> 20
   <212> DNA
   <213> Homo sapiens
<400> 915
   tgtgggaagc attgactctt 20
<210> 916
   <211> 21
   <212> DNA
   <213> Homo sapiens
<400> 916
   tcctgtgaga aatggagctt t 21
<210> 917
   <211> 22
   <212> DNA
   <213> Homo sapiens
<400> 917
   gttgccaagc ttaaatacct gt 22
<210> 918
   <211> 21
   <212> DNA
   <213> Homo sapiens
<400> 918
   ccacaacaac ataaacactg c 21
<210> 919
   <211> 21
   <212> DNA
   <213> Homo sapiens
<400> 919
   tcttccaggc atattcattg c 21
<210> 920
   <211> 21
   <212> DNA
   <213> Homo sapiens
<400> 920
   atccattctc tctacttggg a 21
<210> 921
   <211> 22
   <212> DNA
   <213> Homo sapiens
<400> 921
   ggctagaggg tgattataag ct 22
<210> 922
   <211> 21
   <212> DNA
   <213> Homo sapiens
<400> 922
   tactcatccc gatttcttcc c 21
<210> 923
   <211> 21
   <212> DNA
   <213> Homo sapiens
<400> 923
   ttctctttct cttctgggca g 21
<210> 924
   <211> 21
   <212> DNA
   <213> Homo sapiens
<400> 924
   ttgtgagact caaggccatt t 21
<210> 925
   <211> 21
   <212> DNA
   <213> Homo sapiens
<400> 925
   ttggtagaga gaggccattt g 21
<210> 926
   <211> 21
   <212> DNA
   <213> Homo sapiens
<400> 926
   ggaggaagct cttgaagaca t 21
<210> 927
   <211> 21
   <212> DNA
   <213> Homo sapiens
<400> 927
   agcatcttcc gtttaactcc a 21
<210> 928
   <211> 20
   <212> DNA
   <213> Homo sapiens
<400> 928
   ctgcctgcca agtatgttct 20
<210> 929
   <211> 21
   <212> DNA
   <213> Homo sapiens
<400> 929
   ctactccttg tgtcattggc t 21
<210> 930
   <211> 21
   <212> DNA
   <213> Homo sapiens
<400> 930
   tgattctgag acacgtgctt a 21
<210> 931
   <211> 21
   <212> DNA
   <213> Homo sapiens
<400> 931
   cttgaggacc tttcatgctt g 21
<210> 932
   <211> 21
   <212> DNA
   <213> Homo sapiens
<400> 932
   ttgtgatttc aggtaggagg g 21
<210> 933
   <211> 21
   <212> DNA
   <213> Homo sapiens
<400> 933
   ttccctcaga gacagtatcc t 21
<210> 934
   <211> 20
   <212> DNA
   <213> Homo sapiens
<400> 934
   aaaggaggtc tggctttgaa 20
<210> 935
   <211> 21
   <212> DNA
   <213> Homo sapiens
<400> 935
   gcataaacct ggactgtgaa a 21
<210> 936
   <211> 20
   <212> DNA
   <213> Homo sapiens
<400> 936
   cactcagcga ttctcctcac 20
<210> 937
   <211> 21
   <212> DNA
   <213> Homo sapiens
<400> 937
   aaagccagac acagactagt t 21
<210> 938
   <211> 20
   <212> DNA
   <213> Homo sapiens
<400> 938
   attcctggga ccacaagcat 20
<210> 939
   <211> 20
   <212> DNA
   <213> Homo sapiens
<400> 939
   tattgcctca tgtggttgtg 20
<210> 940
   <211> 22
   <212> DNA
   <213> Homo sapiens
<400> 940
   gtgttgactt gaaaggaatc ac 22
<210> 941
   <211> 21
   <212> DNA
   <213> Homo sapiens
<400> 941
   caggttagga atgacagtgg g 21
<210> 942
   <211> 21
   <212> DNA
   <213> Homo sapiens
<400> 942
   ttaggttacc cagggacgtt a 21
<210> 943
   <211> 21
   <212> DNA
   <213> Homo sapiens
<400> 943
   cggtttgctt tctgaacaac a 21
<210> 944
   <211> 21
   <212> DNA
   <213> Homo sapiens
<400> 944
   catttgggac cctttgaaac t 21
<210> 945
   <211> 21
   <212> DNA
   <213> Homo sapiens
<400> 945
   ggttatctct gggcaaagtt c 21
<210> 946
   <211> 21
   <212> DNA
   <213> Homo sapiens
<400> 946
   tagagagcag agaacaaacc c 21
<210> 947
   <211> 21
   <212> DNA
   <213> Homo sapiens
<400> 947
   ataagggcat ttggagggaa a 21
<210> 948
   <211> 21
   <212> DNA
   <213> Homo sapiens
<400> 948
   aatgcacact tagacaccac a 21
<210> 949
   <211> 21
   <212> DNA
   <213> Homo sapiens
<400> 949
   ggattgctac ccaggagata a 21
<210> 950
   <211> 21
   <212> DNA
   <213> Homo sapiens
<400> 950
   agaggttctg tgtatgagtg t 21
<210> 951
   <211> 21
   <212> DNA
   <213> Homo sapiens
<400> 951
   tagagaattg tacgctggac a 21
<210> 952
   <211> 21
   <212> DNA
   <213> Homo sapiens
<400> 952
   tctggagaaa tgcacaagag a 21
<210> 953
   <211> 21
   <212> DNA
   <213> Homo sapiens
<400> 953
   tgggttagaa catggtgctt a 21
<210> 954
   <211> 21
   <212> DNA
   <213> Homo sapiens
<400> 954
   atcgcatcac acccttacta t 21
<210> 955
   <211> 21
   <212> DNA
   <213> Homo sapiens
<400> 955
   ggaaatttag cttgacatgg c 21
<210> 956
   <211> 22
   <212> DNA
   <213> Homo sapiens
<400> 956
   tttgtaaatc cacagtgcct ac 22
<210> 957
   <211> 21
   <212> DNA
   <213> Homo sapiens
<400> 957
   ggtactggag agcatagaag a 21
<210> 958
   <211> 21
   <212> DNA
   <213> Homo sapiens
<400> 958
   aaacaagcta tcttcaggca g 21
<210> 959
   <211> 21
   <212> DNA
   <213> Homo sapiens
<400> 959
   cgaacaatca gagactcgac t 21
<210> 960
   <211> 21
   <212> DNA
   <213> Homo sapiens
<400> 960
   ctggttgaca atctgcaagt t 21
<210> 961
   <211> 21
   <212> DNA
   <213> Homo sapiens
<400> 961
   accatccaag tcgtcttcat a 21
<210> 962
   <211> 21
   <212> DNA
   <213> Homo sapiens
<400> 962
   atagctacgc ataccctgta g 21
<210> 963
   <211> 21
   <212> DNA
   <213> Homo sapiens
<400> 963
   agcagaagaa acagtaaggc a 21
<210> 964
   <211> 20
   <212> DNA
   <213> Homo sapiens
<400> 964
   acccagggac ctatttgttc 20
<210> 965
   <211> 21
   <212> DNA
   <213> Homo sapiens
<400> 965
   caagggctca ggtcttcatt a 21
<210> 966
   <211> 21
   <212> DNA
   <213> Homo sapiens
<400> 966
   tcactgtgac ttggagacta a 21
<210> 967
   <211> 21
   <212> DNA
   <213> Homo sapiens
<400> 967
   tgctctgctt cactgtgatt a 21
<210> 968
   <211> 22
   <212> DNA
   <213> Homo sapiens
<400> 968
   gatgaatgac taatagccca cg 22
<210> 969
   <211> 21
   <212> DNA
   <213> Homo sapiens
<400> 969
   ccatgtttag tttggtgctg t 21
<210> 970
   <211> 20
   <212> DNA
   <213> Homo sapiens
<400> 970
   agccaagtga ggtgctaaat 20
<210> 971
   <211> 21
   <212> DNA
   <213> Homo sapiens
<400> 971
   tcagggagaa atgatgtcac c 21
<210> 972
   <211> 21
   <212> DNA
   <213> Homo sapiens
<400> 972
   cagtagctgg caagaatcat c 21
<210> 973
   <211> 21
   <212> DNA
   <213> Homo sapiens
<400> 973
   caacactgct agaattccca a 21
<210> 974
   <211> 21
   <212> DNA
   <213> Homo sapiens
<400> 974
   cctgagaagc acctgattgt a 21
<210> 975
   <211> 20
   <212> DNA
   <213> Homo sapiens
<400> 975
   tgttgtcaga aatcccagga 20
<210> 976
   <211> 21
   <212> DNA
   <213> Homo sapiens
<400> 976
   aactggagcc atataacgat g 21
<210> 977
   <211> 21
   <212> DNA
   <213> Homo sapiens
<400> 977
   gaaatggtgc cctattgttg a 21
<210> 978
   <211> 22
   <212> DNA
   <213> Homo sapiens
<400> 978
   tcttacatgc agtcatactc ct 22
<210> 979
   <211> 22
   <212> DNA
   <213> Homo sapiens
<400> 979
   gtccctcagt aacaccatct ta 22
<210> 980
   <211> 21
   <212> DNA
   <213> Homo sapiens
<400> 980
   gaagcaagag gatcaggcaa t 21
<210> 981
   <211> 21
   <212> DNA
   <213> Homo sapiens
<400> 981
   agtgtttcag aggcttgaaa g 21
<210> 982
   <211> 21
   <212> DNA
   <213> Homo sapiens
<400> 982
   atccggcatc ctttaaactc t 21
<210> 983
   <211> 21
   <212> DNA
   <213> Homo sapiens
<400> 983
   aggctaggaa gaaatgggaa a 21
<210> 984
   <211> 21
   <212> DNA
   <213> Homo sapiens
<400> 984
   acacatatgc tctgtctctc a 21
<210> 985
   <211> 21
   <212> DNA
   <213> Homo sapiens
<400> 985
   agttagttat cacctcgtcc c 21
<210> 986
   <211> 22
   <212> DNA
   <213> Homo sapiens
<400> 986
   tgtgtatttc cctctagttg ca 22
<210> 987
   <211> 21
   <212> DNA
   <213> Homo sapiens
<400> 987
   agcctctttc tacatcgttc g 21
<210> 988
   <211> 21
   <212> DNA
   <213> Homo sapiens
<400> 988
   ttacctgtgc agaagagtga c 21
<210> 989
   <211> 21
   <212> DNA
   <213> Homo sapiens
<400> 989
   tcttgtgttc tagcgtgttt g 21
<210> 990
   <211> 21
   <212> DNA
   <213> Homo sapiens
<400> 990
   tgtggttagt cagaaatgtg g 21
<210> 991
   <211> 21
   <212> DNA
   <213> Homo sapiens
<400> 991
   gggtcctgat gagtctttgt c 21
<210> 992
   <211> 21
   <212> DNA
   <213> Homo sapiens
<400> 992
   tggtgccttt gtttattcag c 21
<210> 993
   <211> 21
   <212> DNA
   <213> Homo sapiens
<400> 993
   tgttatgtgc cagggtttaa c 21
<210> 994
   <211> 21
   <212> DNA
   <213> Homo sapiens
<400> 994
   attggttcca gatacagtcg a 21
<210> 995
   <211> 21
   <212> DNA
   <213> Homo sapiens
<400> 995
   ggaaggaagt acagcatgga t 21
<210> 996
   <211> 21
   <212> DNA
   <213> Homo sapiens
<400> 996
   ccttattgaa gctgaccatg c 21
<210> 997
   <211> 21
   <212> DNA
   <213> Homo sapiens
<400> 997
   cagtgggaaa tgtgcttaca t 21
<210> 998
   <211> 21
   <212> DNA
   <213> Homo sapiens
<400> 998
   aaagggccta tattcaccag a 21
<210> 999
   <211> 22
   <212> DNA
   <213> Homo sapiens
<400> 999
   agagccattt aagactctct gt 22
<210> 1000
   <211> 21
   <212> DNA
   <213> Homo sapiens
<400> 1000
   taccttgttc tctgcctcaa t 21
<210> 1001
   <211> 21
   <212> DNA
   <213> Homo sapiens
<400> 1001
   tttagaagga tgtggacagg g 21
<210> 1002
   <211> 20
   <212> DNA
   <213> Homo sapiens
<400> 1002
   agcaggacat ggacttcaaa 20
<210> 1003
   <211> 21
   <212> DNA
   <213> Homo sapiens
<400> 1003
   gcggctcttg tttctgaaat c 21
<210> 1004
   <211> 21
   <212> DNA
   <213> Homo sapiens
<400> 1004
   acaggtagga gttcagagac a 21
<210> 1005
   <211> 22
   <212> DNA
   <213> Homo sapiens
<400> 1005
   tttcctattc tgctcttctg ct 22
<210> 1006
   <211> 21
   <212> DNA
   <213> Homo sapiens
<400> 1006
   aaatgctgct cagggttaga g 21
<210> 1007
   <211> 21
   <212> DNA
   <213> Homo sapiens
<400> 1007
   caactttact ctgcacagct c 21
<210> 1008
   <211> 22
   <212> DNA
   <213> Homo sapiens
<400> 1008
   cagtgccact acaaagaaat ca 22
<210> 1009
   <211> 21
   <212> DNA
   <213> Homo sapiens
<400> 1009
   agaagcagag gttggatatg g 21
<210> 1010
   <211> 21
   <212> DNA
   <213> Homo sapiens
<400> 1010
   ccacaatccc atagtcacca t 21
<210> 1011
   <211> 21
   <212> DNA
   <213> Homo sapiens
<400> 1011
   gctggttctt gttgctgata a 21
<210> 1012
   <211> 21
   <212> DNA
   <213> Homo sapiens
<400> 1012
   tccctcacga cttatgtttg a 21
<210> 1013
   <211> 21
   <212> DNA
   <213> Homo sapiens
<400> 1013
   ctaccttctc cagtgcacta t 21
<210> 1014
   <211> 21
   <212> DNA
   <213> Homo sapiens
<400> 1014
   ggacctctct ttgaaatgga c 21
<210> 1015
   <211> 21
   <212> DNA
   <213> Homo sapiens
<400> 1015
   tgggttgtgt ttctctgact t 21
<210> 1016
   <211> 21
   <212> DNA
   <213> Homo sapiens
<400> 1016
   tacaagcctc ctttaaccct t 21
<210> 1017
   <211> 21
   <212> DNA
   <213> Homo sapiens
<400> 1017
   aaagggtttg atacagttgg g 21
<210> 1018
   <211> 22
   <212> DNA
   <213> Homo sapiens
<400> 1018
   aggtattgga gagcaagaaa ga 22
<210> 1019
   <211> 21
   <212> DNA
   <213> Homo sapiens
<400> 1019
   gcaaactgga gctaaagtca t 21
<210> 1020
   <211> 21
   <212> DNA
   <213> Homo sapiens
<400> 1020
   ttggttgcta gctctcaaat g 21
<210> 1021
   <211> 21
   <212> DNA
   <213> Homo sapiens
<400> 1021
   ttcaatgccc ttacttctcc t 21
<210> 1022
   <211> 20
   <212> DNA
   <213> Homo sapiens
<400> 1022
   gcttgaggcg catatgattg 20
<210> 1023
   <211> 20
   <212> DNA
   <213> Homo sapiens
<400> 1023
   catatggtgc ttgttctggg 20
<210> 1024
   <211> 21
   <212> DNA
   <213> Homo sapiens
<400> 1024
   caaactcacc acccttcatt c 21
<210> 1025
   <211> 21
   <212> DNA
   <213> Homo sapiens
<400> 1025
   ccttagccct gcaaataaca c 21
<210> 1026
   <211> 20
   <212> DNA
   <213> Homo sapiens
<400> 1026
   tcacagatac ggacaagctc 20
<210> 1027
   <211> 21
   <212> DNA
   <213> Homo sapiens
<400> 1027
   gccacaggta tcaatcactt c 21
<210> 1028
   <211> 21
   <212> DNA
   <213> Homo sapiens
<400> 1028
   gcttctgtgg cactaatcaa g 21
<210> 1029
   <211> 22
   <212> DNA
   <213> Homo sapiens
<400> 1029
   gccttattga cttactggac tg 22
<210> 1030
   <211> 21
   <212> DNA
   <213> Homo sapiens
<400> 1030
   gtcagggatt agaggcagaa c 21
<210> 1031
   <211> 22
   <212> DNA
   <213> Homo sapiens
<400> 1031
   caatcacttg gtcagatagt gt 22
<210> 1032
   <211> 22
   <212> DNA
   <213> Homo sapiens
<400> 1032
   agtggagagg ttgagtatag tg 22
<210> 1033
   <211> 21
   <212> DNA
   <213> Homo sapiens
<400> 1033
   tgagaaggga ggaagaatgt g 21
<210> 1034
   <211> 21
   <212> DNA
   <213> Homo sapiens
<400> 1034
   aaggtcaaac tctccattcc a 21
<210> 1035
   <211> 21
   <212> DNA
   <213> Homo sapiens
<400> 1035
   ggagatgtct ttgccctgat t 21
<210> 1036
   <211> 21
   <212> DNA
   <213> Homo sapiens
<400> 1036
   ggtcctcgtt tgtccttaag a 21
<210> 1037
   <211> 21
   <212> DNA
   <213> Homo sapiens
<400> 1037
   agtattgctt tgagggctct a 21
<210> 1038
   <211> 22
   <212> DNA
   <213> Homo sapiens
<400> 1038
   agctatcatg taagtcactc cc 22
<210> 1039
   <211> 21
   <212> DNA
   <213> Homo sapiens
<400> 1039
   aggaaattca gtacctcagc t 21
<210> 1040
   <211> 22
   <212> DNA
   <213> Homo sapiens
<400> 1040
   caaccttctc attgttgaag ct 22
<210> 1041
   <211> 20
   <212> DNA
   <213> Homo sapiens
<400> 1041
   ggtgactttg ctttcccaag 20
<210> 1042
   <211> 20
   <212> DNA
   <213> Homo sapiens
<400> 1042
   gctgacaaac ggagggagag 20
<210> 1043
   <211> 21
   <212> DNA
   <213> Homo sapiens
<400> 1043
   tgttcttcat caggcacaat g 21
<210> 1044
   <211> 21
   <212> DNA
   <213> Homo sapiens
<400> 1044
   accatttgtg tgatccagaa c 21
<210> 1045
   <211> 20
   <212> DNA
   <213> Homo sapiens
<400> 1045
   cttgtcttga gtgcggtaca 20
<210> 1046
   <211> 22
   <212> DNA
   <213> Homo sapiens
<400> 1046
   gcctctcagt ttcctcttat ag 22
<210> 1047
   <211> 24
   <212> DNA
   <213> Homo sapiens
<400> 1047
   cctagatcag tgcagagaat ttag 24
<210> 1048
   <211> 22
   <212> DNA
   <213> Homo sapiens
<400> 1048
   ctttagtttg gaggcctcat tc 22
<210> 1049
   <211> 21
   <212> DNA
   <213> Homo sapiens
<400> 1049
   ctgacatcga tggaattctg g 21
<210> 1050
   <211> 21
   <212> DNA
   <213> Homo sapiens
<400> 1050
   acactgcacg aatggaagat c 21
<210> 1051
   <211> 21
   <212> DNA
   <213> Homo sapiens
<400> 1051
   ataaacttgg tgctcagtgg t 21
<210> 1052
   <211> 21
   <212> DNA
   <213> Homo sapiens
<400> 1052
   acaagcatta cagaattcgg c 21
<210> 1053
   <211> 24
   <212> DNA
   <213> Homo sapiens
<400> 1053
   actgacagat tctcacctat atca 24
<210> 1054
   <211> 21
   <212> DNA
   <213> Homo sapiens
<400> 1054
   ggatcaaagc cactctagac t 21
<210> 1055
   <211> 22
   <212> DNA
   <213> Homo sapiens
<400> 1055
   ttgagatggc atcaagttca ag 22
<210> 1056
   <211> 22
   <212> DNA
   <213> Homo sapiens
<400> 1056
   actggattca tgcgttatca ag 22
<210> 1057
   <211> 21
   <212> DNA
   <213> Homo sapiens
<400> 1057
   tattcttgtg tggaccctgt g 21
<210> 1058
   <211> 22
   <212> DNA
   <213> Homo sapiens
<400> 1058
   tgatattgca tgaaagtccc tg 22
<210> 1059
   <211> 20
   <212> DNA
   <213> Homo sapiens
<400> 1059
   tcacacatga ggagtagaca 20
<210> 1060
   <211> 20
   <212> DNA
   <213> Homo sapiens
<400> 1060
   cacagcagga gacatgagaa 20
<210> 1061
   <211> 20
   <212> DNA
   <213> Homo sapiens
<400> 1061
   tttcctcctg gcttgatcac 20
<210> 1062
   <211> 20
   <212> DNA
   <213> Homo sapiens
<400> 1062
   caaaggagag aagtgaccca 20
<210> 1063
   <211> 20
   <212> DNA
   <213> Homo sapiens
<400> 1063
   ctacgagtga aacagagtgc 20
<210> 1064
   <211> 20
   <212> DNA
   <213> Homo sapiens
<400> 1064
   agactaaaag cctccaagcc 20
<210> 1065
   <211> 20
   <212> DNA
   <213> Homo sapiens
<400> 1065
   tagaatatgt cacccagccc 20
<210> 1066
   <211> 20
   <212> DNA
   <213> Homo sapiens
<400> 1066
   acagaatcat cccatagcca 20
<210> 1067
   <211> 21
   <212> DNA
   <213> Homo sapiens
<400> 1067
   cgccattctg tgcttaattt g 21
<210> 1068
   <211> 21
   <212> DNA
   <213> Homo sapiens
<400> 1068
   gtgaagcaag agaaagcaag a 21
<210> 1069
   <211> 20
   <212> DNA
   <213> Homo sapiens
<400> 1069
   tgattcggct gcaggttatt 20
<210> 1070
   <211> 20
   <212> DNA
   <213> Homo sapiens
<400> 1070
   ctggcttcaa atgcatctga 20
<210> 1071
   <211> 20
   <212> DNA
   <213> Homo sapiens
<400> 1071
   actgggaaat tggaattcgc 20
<210> 1072
   <211> 20
   <212> DNA
   <213> Homo sapiens
<400> 1072
   ggtaaaactg cctggaaact 20
<210> 1073
   <211> 20
   <212> DNA
   <213> Homo sapiens
<400> 1073
   agaccacggg ctctatctat 20
<210> 1074
   <211> 22
   <212> DNA
   <213> Homo sapiens
<400> 1074
   catcttgctt attggcttac ga 22
<210> 1075
   <211> 20
   <212> DNA
   <213> Homo sapiens
<400> 1075
   gaggtagagg cagtgtcttg 20
<210> 1076
   <211> 20
   <212> DNA
   <213> Homo sapiens
<400> 1076
   tagtccttga actccctggt 20
<210> 1077
   <211> 20
   <212> DNA
   <213> Homo sapiens
<400> 1077
   ccagcttagc gtctgttttt 20
<210> 1078
   <211> 21
   <212> DNA
   <213> Homo sapiens
<400> 1078
   gaccacaact atcaagagca c 21
<210> 1079
   <211> 20
   <212> DNA
   <213> Homo sapiens
<400> 1079
   ccaaagaaag gttgaagccc 20
<210> 1080
   <211> 21
   <212> DNA
   <213> Homo sapiens
<400> 1080
   aataatgtgc actgtgatgg c 21
<210> 1081
   <211> 20
   <212> DNA
   <213> Homo sapiens
<400> 1081
   tttaagggtc tgatggttgc 20
<210> 1082
   <211> 20
   <212> DNA
   <213> Homo sapiens
<400> 1082
   gaaaatgccc atcgtctcaa 20
<210> 1083
   <211> 20
   <212> DNA
   <213> Homo sapiens
<400> 1083
   attccttgtc tttccccctc 20
<210> 1084
   <211> 22
   <212> DNA
   <213> Homo sapiens
<400> 1084
   tcatttctct agcccaaaga tg 22
<210> 1085
   <211> 20
   <212> DNA
   <213> Homo sapiens
<400> 1085
   ttggtttgtt gacttcagcc 20
<210> 1086
   <211> 20
   <212> DNA
   <213> Homo sapiens
<400> 1086
   gctcagtgac agttgggatt 20
<210> 1087
   <211> 22
   <212> DNA
   <213> Homo sapiens
<400> 1087
   gtcttgtctc tcttcttcca ct 22
<210> 1088
   <211> 20
   <212> DNA
   <213> Homo sapiens
<400> 1088
   tttagagcag gtggaaacga 20
<210> 1089
   <211> 21
   <212> DNA
   <213> Homo sapiens
<400> 1089
   cttgggtttt tatcggttgc t 21
<210> 1090
   <211> 20
   <212> DNA
   <213> Homo sapiens
<400> 1090
   gttgcctgga ttgctctaaa 20
<210> 1091
   <211> 20
   <212> DNA
   <213> Homo sapiens
<400> 1091
   aaatcacgac gtaggaaacc 20
<210> 1092
   <211> 20
   <212> DNA
   <213> Homo sapiens
<400> 1092
   tcaccttgga gcaggtcata 20
<210> 1093
   <211> 20
   <212> DNA
   <213> Homo sapiens
<400> 1093
   cgaagaaggt ctgggagatg 20
<210> 1094
   <211> 21
   <212> DNA
   <213> Homo sapiens
<400> 1094
   caccattgtt tcatcaggac t 21
<210> 1095
   <211> 21
   <212> DNA
   <213> Homo sapiens
<400> 1095
   ttgcatcatc agctcacata c 21
<210> 1096
   <211> 20
   <212> DNA
   <213> Homo sapiens
<400> 1096
   agacctggaa aatgatgggt 20
<210> 1097
   <211> 20
   <212> DNA
   <213> Homo sapiens
<400> 1097
   cctggaagtg tgtaacaagc 20
<210> 1098
   <211> 21
   <212> DNA
   <213> Homo sapiens
<400> 1098
   ctccctagca aaaacttctc a 21
<210> 1099
   <211> 20
   <212> DNA
   <213> Homo sapiens
<400> 1099
   tgccttattc actgtgcaac 20
<210> 1100
   <211> 20
   <212> DNA
   <213> Homo sapiens
<400> 1100
   tgatggccta gtgagtttcc 20
<210> 1101
   <211> 20
   <212> DNA
   <213> Homo sapiens
<400> 1101
   tgcaatgtaa caaaagcgtg 20
<210> 1102
   <211> 20
   <212> DNA
   <213> Homo sapiens
<400> 1102
   gccacatttg ctttcacaca 20
<210> 1103
   <211> 19
   <212> DNA
   <213> Homo sapiens
<400> 1103
   acgtcattgg gttcatggc 19
<210> 1104
   <211> 20
   <212> DNA
   <213> Homo sapiens
<400> 1104
   attttacccc cttaggcacc 20
<210> 1105
   <211> 20
   <212> DNA
   <213> Homo sapiens
<400> 1105
   tcttctacac agcccttcag 20
<210> 1106
   <211> 20
   <212> DNA
   <213> Homo sapiens
<400> 1106
   attgggatcg tcagcatcaa 20
<210> 1107
   <211> 20
   <212> DNA
   <213> Homo sapiens
<400> 1107
   tgattgtctt gtccactggt 20
<210> 1108
   <211> 20
   <212> DNA
   <213> Homo sapiens
<400> 1108
   acatcacact tcatgccctt 20
<210> 1109
   <211> 20
   <212> DNA
   <213> Homo sapiens
<400> 1109
   atgaaggcat taggagggag 20
<210> 1110
   <211> 20
   <212> DNA
   <213> Homo sapiens
<400> 1110
   aaagtggaga agtggcagat 20
<210> 1111
   <211> 22
   <212> DNA
   <213> Homo sapiens
<400> 1111
   ccttaggatt ctgagaggtg ag 22
<210> 1112
   <211> 20
   <212> DNA
   <213> Homo sapiens
<400> 1112
   agggcttctg attgatttgc 20
<210> 1113
   <211> 20
   <212> DNA
   <213> Homo sapiens
<400> 1113
   cagggtagtc gggatttctc 20
<210> 1114
   <211> 20
   <212> DNA
   <213> Homo sapiens
<400> 1114
   cccggtaatg atctacagca 20
<210> 1115
   <211> 19
   <212> DNA
   <213> Homo sapiens
<400> 1115
   aacggcactt ggttcacta 19
<210> 1116
   <211> 20
   <212> DNA
   <213> Homo sapiens
<400> 1116
   actctgaact cctcctcctg 20
<210> 1117
   <211> 20
   <212> DNA
   <213> Homo sapiens
<400> 1117
   tttgctcaca cacaagacac 20
<210> 1118
   <211> 20
   <212> DNA
   <213> Homo sapiens
<400> 1118
   acgactgcat ccttttcatg 20
<210> 1119
   <211> 22
   <212> DNA
   <213> Homo sapiens
<400> 1119
   ccatcatagc ctacaaatac cc 22
<210> 1120
   <211> 22
   <212> DNA
   <213> Homo sapiens
<400> 1120
   agagagttga aaatatcccc ca 22
<210> 1121
   <211> 20
   <212> DNA
   <213> Homo sapiens
<400> 1121
   actttcttga acaccccagt 20
<210> 1122
   <211> 20
   <212> DNA
   <213> Homo sapiens
<400> 1122
   tgcctgtgtc ctacttttcc 20
<210> 1123
   <211> 20
   <212> DNA
   <213> Homo sapiens
<400> 1123
   tgactgccca agaatgtaca 20
<210> 1124
   <211> 20
   <212> DNA
   <213> Homo sapiens
<400> 1124
   ccgaacacgc tgtatgtatt 20
<210> 1125
   <211> 20
   <212> DNA
   <213> Homo sapiens
<400> 1125
   ggaagggaat tgaagcacag 20
<210> 1126
   <211> 20
   <212> DNA
   <213> Homo sapiens
<400> 1126
   tcatcctatc caccaacctg 20
<210> 1127
   <211> 20
   <212> DNA
   <213> Homo sapiens
<400> 1127
   aatgaactgg ccctgactta 20
<210> 1128
   <211> 20
   <212> DNA
   <213> Homo sapiens
<400> 1128
   taagatacca taccgcagct 20
<210> 1129
   <211> 21
   <212> DNA
   <213> Homo sapiens
<400> 1129
   tcagcgttca tggtaccaat a 21
<210> 1130
   <211> 21
   <212> DNA
   <213> Homo sapiens
<400> 1130
   gtttcccaac caacaaacaa g 21
<210> 1131
   <211> 20
   <212> DNA
   <213> Homo sapiens
<400> 1131
   tctatcggga tggagagtga 20
<210> 1132
   <211> 20
   <212> DNA
   <213> Homo sapiens
<400> 1132
   gttcagacag gtggactagg 20
<210> 1133
   <211> 20
   <212> DNA
   <213> Homo sapiens
<400> 1133
   aagcaacctg ggaaattgtg 20
<210> 1134
   <211> 20
   <212> DNA
   <213> Homo sapiens
<400> 1134
   gggttaaggt tgctgggtta 20
<210> 1135
   <211> 21
   <212> DNA
   <213> Homo sapiens
<400> 1135
   gtgaaatgtg gttgtagtgc a 21
<210> 1136
   <211> 20
   <212> DNA
   <213> Homo sapiens
<400> 1136
   gtgactgcct tgcttcattt 20
<210> 1137
   <211> 20
   <212> DNA
   <213> Homo sapiens
<400> 1137
   cagttctgga gccttctact 20
<210> 1138
   <211> 20
   <212> DNA
   <213> Homo sapiens
<400> 1138
   ttagggcagg atgtacagaa 20
<210> 1139
   <211> 20
   <212> DNA
   <213> Homo sapiens
<400> 1139
   gctggtgacc ttcattcaag 20
<210> 1140
   <211> 19
   <212> DNA
   <213> Homo sapiens
<400> 1140
   taaaaccatg ttcggggca 19
<210> 1141
   <211> 21
   <212> DNA
   <213> Homo sapiens
<400> 1141
   ctacctgtcc gtttccctta c 21
<210> 1142
   <211> 21
   <212> DNA
   <213> Homo sapiens
<400> 1142
   gacaaagatg actggaggtg a 21
<210> 1143
   <211> 21
   <212> DNA
   <213> Homo sapiens
<400> 1143
   gggtggatgg tgagatatgt g 21
<210> 1144
   <211> 21
   <212> DNA
   <213> Homo sapiens
<400> 1144
   cttcaaacct gatccatgtg c 21
<210> 1145
   <211> 21
   <212> DNA
   <213> Homo sapiens
<400> 1145
   gcagaaacag catgaatctc c 21
<210> 1146
   <211> 21
   <212> DNA
   <213> Homo sapiens
<400> 1146
   tgacttcaaa catcccatcc a 21
<210> 1147
   <211> 21
   <212> DNA
   <213> Homo sapiens
<400> 1147
   gtgggatgag ttctagagga a 21
<210> 1148
   <211> 21
   <212> DNA
   <213> Homo sapiens
<400> 1148
   agtcacacac atacacacag t 21
<210> 1149
   <211> 21
   <212> DNA
   <213> Homo sapiens
<400> 1149
   acgacttccc tgtgtaactt a 21
<210> 1150
   <211> 21
   <212> DNA
   <213> Homo sapiens
<400> 1150
   ccttctcttg tctctagtgc c 21
<210> 1151
   <211> 21
   <212> DNA
   <213> Homo sapiens
<400> 1151
   ctggaaatac acacacacct g 21
<210> 1152
   <211> 21
   <212> DNA
   <213> Homo sapiens
<400> 1152
   atttgtaaac cacccacttc g 21
<210> 1153
   <211> 21
   <212> DNA
   <213> Homo sapiens
<400> 1153
   tcactgtgct gacaaatcct a 21
<210> 1154
   <211> 21
   <212> DNA
   <213> Homo sapiens
<400> 1154
   cccatcatca tcccttcaga c 21
<210> 1155
   <211> 21
   <212> DNA
   <213> Homo sapiens
<400> 1155
   tcatcacttt atcctcccag t 21
<210> 1156
   <211> 21
   <212> DNA
   <213> Homo sapiens
<400> 1156
   atgggcacag gtaaagagtt t 21
<210> 1157
   <211> 21
   <212> DNA
   <213> Homo sapiens
<400> 1157
   tttgtaagct gagtgtgagg t 21
<210> 1158
   <211> 21
   <212> DNA
   <213> Homo sapiens
<400> 1158
   ttactgtttg aatgccagct c 21
<210> 1159
   <211> 21
   <212> DNA
   <213> Homo sapiens
<400> 1159
   tcccttctcc catcacaatt c 21
<210> 1160
   <211> 21
   <212> DNA
   <213> Homo sapiens
<400> 1160
   gaagactgca tgtgtgtcct a 21
<210> 1161
   <211> 21
   <212> DNA
   <213> Homo sapiens
<400> 1161
   ttctcactct caactgaacc a 21
<210> 1162
   <211> 22
   <212> DNA
   <213> Homo sapiens
<400> 1162
   tcagggagct tctaattaag ga 22
<210> 1163
   <211> 21
   <212> DNA
   <213> Homo sapiens
<400> 1163
   aagatgatcc caggcttaag g 21
<210> 1164
   <211> 21
   <212> DNA
   <213> Homo sapiens
<400> 1164
   gttgaggttt gctgatcttg g 21
<210> 1165
   <211> 21
   <212> DNA
   <213> Homo sapiens
<400> 1165
   caaagataga ttcgcacacc a 21
<210> 1166
   <211> 21
   <212> DNA
   <213> Homo sapiens
<400> 1166
   gagctggaca aattaaatgg c 21
<210> 1167
   <211> 20
   <212> DNA
   <213> Homo sapiens
<400> 1167
   ccagtgcatt tggtttgaca 20
<210> 1168
   <211> 21
   <212> DNA
   <213> Homo sapiens
<400> 1168
   tatgtgaatc ctctgtgtgg c 21
<210> 1169
   <211> 21
   <212> DNA
   <213> Homo sapiens
<400> 1169
   agcttctctc tcattctgct t 21
<210> 1170
   <211> 21
   <212> DNA
   <213> Homo sapiens
<400> 1170
   gactcccgat ttcatttgct g 21
<210> 1171
   <211> 22
   <212> DNA
   <213> Homo sapiens
<400> 1171
   tcccaatcgt tgtgaaacat ac 22
<210> 1172
   <211> 21
   <212> DNA
   <213> Homo sapiens
<400> 1172
   tctttacagg aagttgggac c 21
<210> 1173
   <211> 21
   <212> DNA
   <213> Homo sapiens
<400> 1173
   taagttcaga tcagggagca g 21
<210> 1174
   <211> 22
   <212> DNA
   <213> Homo sapiens
<400> 1174
   gttgaaagtc ttacagaacg ct 22
<210> 1175
   <211> 21
   <212> DNA
   <213> Homo sapiens
<400> 1175
   caacataggc acattgtcct c 21
<210> 1176
   <211> 21
   <212> DNA
   <213> Homo sapiens
<400> 1176
   gtcaggcctc ataactctct t 21
<210> 1177
   <211> 21
   <212> DNA
   <213> Homo sapiens
<400> 1177
   gttgtgtggc tttccttatc a 21
<210> 1178
   <211> 21
   <212> DNA
   <213> Homo sapiens
<400> 1178
   cctgcagctc tgtgtaaatt t 21
<210> 1179
   <211> 21
   <212> DNA
   <213> Homo sapiens
<400> 1179
   ctttggccag ttctttctct c 21
<210> 1180
   <211> 20
   <212> DNA
   <213> Homo sapiens
<400> 1180
   gtggcccagc attatttgtt 20
<210> 1181
   <211> 21
   <212> DNA
   <213> Homo sapiens
<400> 1181
   tcttggtgtg acttgctaac a 21
<210> 1182
   <211> 21
   <212> DNA
   <213> Homo sapiens
<400> 1182
   tttctggctg agataagacc c 21
<210> 1183
   <211> 21
   <212> DNA
   <213> Homo sapiens
<400> 1183
   acaattccgt ggtatacagc t 21
<210> 1184
   <211> 21
   <212> DNA
   <213> Homo sapiens
<400> 1184
   tgattgtgcc ctaaccaaac t 21
<210> 1185
   <211> 21
   <212> DNA
   <213> Homo sapiens
<400> 1185
   tgtcgtatcc tgctgtttag a 21
<210> 1186
   <211> 21
   <212> DNA
   <213> Homo sapiens
<400> 1186
   tcaggagtaa agtcaggacc t 21
<210> 1187
   <211> 21
   <212> DNA
   <213> Homo sapiens
<400> 1187
   tgagctgatt tactgtgaca c 21
<210> 1188
   <211> 21
   <212> DNA
   <213> Homo sapiens
<400> 1188
   tagcaccttg acttcaggat t 21
<210> 1189
   <211> 21
   <212> DNA
   <213> Homo sapiens
<400> 1189
   tgaggttgga aagggtcaat t 21
<210> 1190
   <211> 21
   <212> DNA
   <213> Homo sapiens
<400> 1190
   cagctttcct tcctcttctc t 21
<210> 1191
   <211> 21
   <212> DNA
   <213> Homo sapiens
<400> 1191
   acagtgagag gaaagaacag c 21
<210> 1192
   <211> 21
   <212> DNA
   <213> Homo sapiens
<400> 1192
   ggaaataaat tgtgagctgg c 21
<210> 1193
   <211> 21
   <212> DNA
   <213> Homo sapiens
<400> 1193
   agacagccct tcaatccata c 21
<210> 1194
   <211> 21
   <212> DNA
   <213> Homo sapiens
<400> 1194
   cctacatccc ttcctccttt c 21
<210> 1195
   <211> 21
   <212> DNA
   <213> Homo sapiens
<400> 1195
   tcacccatct tccaattagc t 21
<210> 1196
   <211> 22
   <212> DNA
   <213> Homo sapiens
<400> 1196
   tttctaagca caaactgaca cc 22
<210> 1197
   <211> 21
   <212> DNA
   <213> Homo sapiens
<400> 1197
   gatgtttgca ctggagggat a 21
<210> 1198
   <211> 21
   <212> DNA
   <213> Homo sapiens
<400> 1198
   gaagactaaa tgttggccga a 21
<210> 1199
   <211> 21
   <212> DNA
   <213> Homo sapiens
<400> 1199
   gtagagagag ggaggatcac a 21
<210> 1200
   <211> 21
   <212> DNA
   <213> Homo sapiens
<400> 1200
   cttgccatga agtttgacca g 21
<210> 1201
   <211> 21
   <212> DNA
   <213> Homo sapiens
<400> 1201
   aggatgagca tttgtaacct g 21
<210> 1202
   <211> 21
   <212> DNA
   <213> Homo sapiens
<400> 1202
   tgtcgctttc aaattaccca c 21
<210> 1203
   <211> 21
   <212> DNA
   <213> Homo sapiens
<400> 1203
   catacaagtg ctctgttagg c 21
<210> 1204
   <211> 21
   <212> DNA
   <213> Homo sapiens
<400> 1204
   aggacttgga accagaaaga c 21
<210> 1205
   <211> 21
   <212> DNA
   <213> Homo sapiens
<400> 1205
   aaagagggct gatatcgtct g 21
<210> 1206
   <211> 21
   <212> DNA
   <213> Homo sapiens
<400> 1206
   ctcactgcaa actatggaac c 21
<210> 1207
   <211> 21
   <212> DNA
   <213> Homo sapiens
<400> 1207
   tattctgccc atcttcttcc t 21
<210> 1208
   <211> 20
   <212> DNA
   <213> Homo sapiens
<400> 1208
   ggagacagcc caaacataga 20
<210> 1209
   <211> 21
   <212> DNA
   <213> Homo sapiens
<400> 1209
   agcaatggtg aagttctgga t 21
<210> 1210
   <211> 21
   <212> DNA
   <213> Homo sapiens
<400> 1210
   ctggtcagtg agagaaggga a 21
<210> 1211
   <211> 20
   <212> DNA
   <213> Homo sapiens
<400> 1211
   tttctccact ggcatgaact 20
<210> 1212
   <211> 21
   <212> DNA
   <213> Homo sapiens
<400> 1212
   catgatcaca attccaagcc a 21
<210> 1213
   <211> 21
   <212> DNA
   <213> Homo sapiens
<400> 1213
   acccagtcaa gttacagtct t 21
<210> 1214
   <211> 22
   <212> DNA
   <213> Homo sapiens
<400> 1214
   tgtaaagcat atcaagggaa cg 22
<210> 1215
   <211> 21
   <212> DNA
   <213> Homo sapiens
<400> 1215
   tgcagagata tgttcccgta t 21
<210> 1216
   <211> 21
   <212> DNA
   <213> Homo sapiens
<400> 1216
   agaagacagt acaaggaagg c 21
<210> 1217
   <211> 21
   <212> DNA
   <213> Homo sapiens
<400> 1217
   tgtttgccat ttgttctcct c 21
<210> 1218
   <211> 21
   <212> DNA
   <213> Homo sapiens
<400> 1218
   ttgaaggcaa gagaagtttg g 21
<210> 1219
   <211> 21
   <212> DNA
   <213> Homo sapiens
<400> 1219
   gccaaggaaa tgtagggaaa g 21
<210> 1220
   <211> 21
   <212> DNA
   <213> Homo sapiens
<400> 1220
   aaccttcaca cctagagaca g 21
<210> 1221
   <211> 21
   <212> DNA
   <213> Homo sapiens
<400> 1221
   gcataacagg gaaagtcacc t 21
<210> 1222
   <211> 21
   <212> DNA
   <213> Homo sapiens
<400> 1222
   aggatgttag tggtttgggt a 21
<210> 1223
   <211> 20
   <212> DNA
   <213> Homo sapiens
<400> 1223
   tctgacactg accttcaact 20
<210> 1224
   <211> 20
   <212> DNA
   <213> Homo sapiens
<400> 1224
   gaaacattgc tttccctcca 20
<210> 1225
   <211> 20
   <212> DNA
   <213> Homo sapiens
<400> 1225
   ttccacacat ctcttctccg 20
<210> 1226
   <211> 20
   <212> DNA
   <213> Homo sapiens
<400> 1226
   gggagccttg aaaacctgaa 20
<210> 1227
   <211> 19
   <212> DNA
   <213> Homo sapiens
<400> 1227
   attggtagcg ttgtcagca 19
<210> 1228
   <211> 20
   <212> DNA
   <213> Homo sapiens
<400> 1228
   ttcctgcatc ttgtagaccc 20
<210> 1229
   <211> 20
   <212> DNA
   <213> Homo sapiens
<400> 1229
   gggctaatgt tttgcttcca 20
<210> 1230
   <211> 20
   <212> DNA
   <213> Homo sapiens
<400> 1230
   tgttgattag agcttccccc 20
<210> 1231
   <211> 19
   <212> DNA
   <213> Homo sapiens
<400> 1231
   agaggttttc ttccccgtg 19
<210> 1232
   <211> 20
   <212> DNA
   <213> Homo sapiens
<400> 1232
   tagtgccctc tattgtgcct 20
<210> 1233
   <211> 20
   <212> DNA
   <213> Homo sapiens
<400> 1233
   actgctggac tttgaaatgc 20
<210> 1234
   <211> 20
   <212> DNA
   <213> Homo sapiens
<400> 1234
   caaacagtga gatgtggctg 20
<210> 1235
   <211> 21
   <212> DNA
   <213> Homo sapiens
<400> 1235
   ttgcttcctg aaaactggtt c 21
<210> 1236
   <211> 20
   <212> DNA
   <213> Homo sapiens
<400> 1236
   attccaatca cgtctctgca 20
<210> 1237
   <211> 20
   <212> DNA
   <213> Homo sapiens
<400> 1237
   acaatctcac agcctggaaa 20
<210> 1238
   <211> 20
   <212> DNA
   <213> Homo sapiens
<400> 1238
   tcagatgggt gaggttcttg 20
<210> 1239
   <211> 20
   <212> DNA
   <213> Homo sapiens
<400> 1239
   ctgctccttc cctccaatta 20
<210> 1240
   <211> 20
   <212> DNA
   <213> Homo sapiens
<400> 1240
   aaatgccagt cctgtaaagg 20
<210> 1241
   <211> 20
   <212> DNA
   <213> Homo sapiens
<400> 1241
   tgtcccattg cttaggaagt 20
<210> 1242
   <211> 20
   <212> DNA
   <213> Homo sapiens
<400> 1242
   tgtgtgatcc agagacccta 20
<210> 1243
   <211> 20
   <212> DNA
   <213> Homo sapiens
<400> 1243
   accaatgtag acttagcggg 20
<210> 1244
   <211> 20
   <212> DNA
   <213> Homo sapiens
<400> 1244
   actctcatat tgccccactt 20
<210> 1245
   <211> 20
   <212> DNA
   <213> Homo sapiens
<400> 1245
   ccagggattg atgtactggt 20
<210> 1246
   <211> 20
   <212> DNA
   <213> Homo sapiens
<400> 1246
   aattctggtc tatctggcgt 20
<210> 1247
   <211> 20
   <212> DNA
   <213> Homo sapiens
<400> 1247
   gccagccctt ttcacatatt 20
<210> 1248
   <211> 20
   <212> DNA
   <213> Homo sapiens
<400> 1248
   aactacacca tcccctgttt 20
<210> 1249
   <211> 20
   <212> DNA
   <213> Homo sapiens
<400> 1249
   ccatttcaaa catgctggtc 20
<210> 1250
   <211> 22
   <212> DNA
   <213> Homo sapiens
<400> 1250
   agattataag aaggcaggga ac 22
<210> 1251
   <211> 22
   <212> DNA
   <213> Homo sapiens
<400> 1251
   gtagagggct taaaacatgt cc 22
<210> 1252
   <211> 21
   <212> DNA
   <213> Homo sapiens
<400> 1252
   acaagaacac agtcgttaag c 21
<210> 1253
   <211> 20
   <212> DNA
   <213> Homo sapiens
<400> 1253
   tctctccttc actcccttca 20
<210> 1254
   <211> 20
   <212> DNA
   <213> Homo sapiens
<400> 1254
   tgtccacccc tctttgattg 20
<210> 1255
   <211> 20
   <212> DNA
   <213> Homo sapiens
<400> 1255
   tttagcttct cctgcctttg 20
<210> 1256
   <211> 20
   <212> DNA
   <213> Homo sapiens
<400> 1256
   agaagcaatt caccaggtca 20
<210> 1257
   <211> 20
   <212> DNA
   <213> Homo sapiens
<400> 1257
   tggagtcaga agtgtgtgtt 20
<210> 1258
   <211> 20
   <212> DNA
   <213> Homo sapiens
<400> 1258
   tctggtgtca aagcttaggg 20
<210> 1259
   <211> 20
   <212> DNA
   <213> Homo sapiens
<400> 1259
   tgccgatgat gtgtgttttg 20
<210> 1260
   <211> 20
   <212> DNA
   <213> Homo sapiens
<400> 1260
   tgtcccttcc taatcccaaa 20
<210> 1261
   <211> 20
   <212> DNA
   <213> Homo sapiens
<400> 1261
   aggatgttta agttgcagca 20
<210> 1262
   <211> 20
   <212> DNA
   <213> Homo sapiens
<400> 1262
   tatgcagttt taccccctcc 20
<210> 1263
   <211> 20
   <212> DNA
   <213> Homo sapiens
<400> 1263
   ttctgtgtgg tctcctcttg 20
<210> 1264
   <211> 20
   <212> DNA
   <213> Homo sapiens
<400> 1264
   atggagggac aagtgagaca 20
<210> 1265
   <211> 20
   <212> DNA
   <213> Homo sapiens
<400> 1265
   ggggaacatg gagctgtaaa 20
<210> 1266
   <211> 20
   <212> DNA
   <213> Homo sapiens
<400> 1266
   ggacccccta ccacatttac 20
<210> 1267
   <211> 21
   <212> DNA
   <213> Homo sapiens
<400> 1267
   agatggagaa atgtgcagag a 21
<210> 1268
   <211> 19
   <212> DNA
   <213> Homo sapiens
<400> 1268
   atgactgcat ccaagagca 19
<210> 1269
   <211> 20
   <212> DNA
   <213> Homo sapiens
<400> 1269
   cagaatttcc aggcagttgt 20
<210> 1270
   <211> 21
   <212> DNA
   <213> Homo sapiens
<400> 1270
   gaatccagaa gctcagtcct t 21
<210> 1271
   <211> 20
   <212> DNA
   <213> Homo sapiens
<400> 1271
   agccctggaa tcttgacatt 20
<210> 1272
   <211> 19
   <212> DNA
   <213> Homo sapiens
<400> 1272
   gtgcattata cggatggcc 19
<210> 1273
   <211> 20
   <212> DNA
   <213> Homo sapiens
<400> 1273
   ggtagagggt cctgtgattc 20
<210> 1274
   <211> 20
   <212> DNA
   <213> Homo sapiens
<400> 1274
   gtaactgcta gccactgagt 20
<210> 1275
   <211> 20
   <212> DNA
   <213> Homo sapiens
<400> 1275
   gcctttttgg gaatcctagt 20
<210> 1276
   <211> 20
   <212> DNA
   <213> Homo sapiens
<400> 1276
   aagggtggaa gcacattgac 20
<210> 1277
   <211> 20
   <212> DNA
   <213> Homo sapiens
<400> 1277
   atagaggaac aagctgcaca 20
<210> 1278
   <211> 20
   <212> DNA
   <213> Homo sapiens
<400> 1278
   ctcgtccctt gcacatctta 20
<210> 1279
   <211> 20
   <212> DNA
   <213> Homo sapiens
<400> 1279
   aggtggggaa gaacaaaaca 20
<210> 1280
   <211> 20
   <212> DNA
   <213> Homo sapiens
<400> 1280
   gagagtaggt gcagggaaac 20
<210> 1281
   <211> 20
   <212> DNA
   <213> Homo sapiens
<400> 1281
   atcccgcatt cttaaccaca 20
<210> 1282
   <211> 21
   <212> DNA
   <213> Homo sapiens
<400> 1282
   gactaagcaa aagcatctcc c 21
<210> 1283
   <211> 20
   <212> DNA
   <213> Homo sapiens
<400> 1283
   ttcttacagg ctcagggtat 20
<210> 1284
   <211> 21
   <212> DNA
   <213> Homo sapiens
<400> 1284
   cccatagctt aacccctaca a 21
<210> 1285
   <211> 22
   <212> DNA
   <213> Homo sapiens
<400> 1285
   tcttcatctt actgtctagc ac 22
<210> 1286
   <211> 21
   <212> DNA
   <213> Homo sapiens
<400> 1286
   tcatacccta tccctgtgat c 21
<210> 1287
   <211> 21
   <212> DNA
   <213> Homo sapiens
<400> 1287
   tcttgccctt gatttgtttc c 21
<210> 1288
   <211> 21
   <212> DNA
   <213> Homo sapiens
<400> 1288
   gccttttatc catatgccac c 21
<210> 1289
   <211> 20
   <212> DNA
   <213> Homo sapiens
<400> 1289
   gccctcaact ttgcttttca 20
<210> 1290
   <211> 20
   <212> DNA
   <213> Homo sapiens
<400> 1290
   aaaaacctgc actgtgttcg 20
<210> 1291
   <211> 22
   <212> DNA
   <213> Homo sapiens
<400> 1291
   tttttacagc aatcttcact gc 22
<210> 1292
   <211> 22
   <212> DNA
   <213> Homo sapiens
<400> 1292
   taagccggaa tgatttgtaa gg 22
<210> 1293
   <211> 20
   <212> DNA
   <213> Homo sapiens
<400> 1293
   taccctttgg cttaacagct 20
<210> 1294
   <211> 20
   <212> DNA
   <213> Homo sapiens
<400> 1294
   agttgtcatg ttgggctcat 20
<210> 1295
   <211> 22
   <212> DNA
   <213> Homo sapiens
<400> 1295
   tgtcctaagt tacctgtctg ac 22
<210> 1296
   <211> 21
   <212> DNA
   <213> Homo sapiens
<400> 1296
   gccctggaaa gtactgtaac a 21
<210> 1297
   <211> 20
   <212> DNA
   <213> Homo sapiens
<400> 1297
   tgttacagcc aggctttcat 20
<210> 1298
   <211> 20
   <212> DNA
   <213> Homo sapiens
<400> 1298
   caacgaacac agggtttaca 20
<210> 1299
   <211> 21
   <212> DNA
   <213> Homo sapiens
<400> 1299
   ccaggactct ctcttttctt c 21
<210> 1300
   <211> 22
   <212> DNA
   <213> Homo sapiens
<400> 1300
   gacacataag accactttag gc 22
<210> 1301
   <211> 21
   <212> DNA
   <213> Homo sapiens
<400> 1301
   gatatgttct ggaggactgc t 21
<210> 1302
   <211> 20
   <212> DNA
   <213> Homo sapiens
<400> 1302
   tgattctcac aggctccttg 20
<210> 1303
   <211> 22
   <212> DNA
   <213> Homo sapiens
<400> 1303
   tgatggaagt ttctaggtca gt 22
<210> 1304
   <211> 20
   <212> DNA
   <213> Homo sapiens
<400> 1304
   aacactcttg ctccctatgt 20
<210> 1305
   <211> 21
   <212> DNA
   <213> Homo sapiens
<400> 1305
   tttctctccc agcttgatct t 21
<210> 1306
   <211> 20
   <212> DNA
   <213> Homo sapiens
<400> 1306
   ctgtgcagag acgaactaag 20
<210> 1307
   <211> 19
   <212> DNA
   <213> Homo sapiens
<400> 1307
   ctctcggagc aaagacctt 19
<210> 1308
   <211> 20
   <212> DNA
   <213> Homo sapiens
<400> 1308
   acctcataag tacgcccatc 20
<210> 1309
   <211> 20
   <212> DNA
   <213> Homo sapiens
<400> 1309
   tgatcttcct ttgctcctgt 20
<210> 1310
   <211> 20
   <212> DNA
   <213> Homo sapiens
<400> 1310
   atgacgacga tgttggagag 20
<210> 1311
   <211> 20
   <212> DNA
   <213> Homo sapiens
<400> 1311
   tccttcagca agcctctttt 20
<210> 1312
   <211> 20
   <212> DNA
   <213> Homo sapiens
<400> 1312
   tgagggtgat aacctgtgag 20
<210> 1313
   <211> 20
   <212> DNA
   <213> Homo sapiens
<400> 1313
   ggcttgaagt ttgtctgtga 20
<210> 1314
   <211> 20
   <212> DNA
   <213> Homo sapiens
<400> 1314
   ggattttcac attgctcagc 20
<210> 1315
   <211> 21
   <212> DNA
   <213> Homo sapiens
<400> 1315
   tagcatgaga gtgaactgag g 21
<210> 1316
   <211> 20
   <212> DNA
   <213> Homo sapiens
<400> 1316
   cagccacata gcccatatct 20
<210> 1317
   <211> 20
   <212> DNA
   <213> Homo sapiens
<400> 1317
   tgcccatgag tctacttgtg 20
<210> 1318
   <211> 20
   <212> DNA
   <213> Homo sapiens
<400> 1318
   aagtggactg agggacaatt 20
<210> 1319
   <211> 22
   <212> DNA
   <213> Homo sapiens
<400> 1319
   tcagactgag cattaaatca cc 22
<210> 1320
   <211> 20
   <212> DNA
   <213> Homo sapiens
<400> 1320
   taggtctgga agaatgccag 20
<210> 1321
   <211> 20
   <212> DNA
   <213> Homo sapiens
<400> 1321
   agtatcttgg gcttgtgaca 20
<210> 1322
   <211> 20
   <212> DNA
   <213> Homo sapiens
<400> 1322
   gctccacttc cagtctttct 20
<210> 1323
   <211> 20
   <212> DNA
   <213> Homo sapiens
<400> 1323
   ttggaatagt gagcctccct 20
<210> 1324
   <211> 20
   <212> DNA
   <213> Homo sapiens
<400> 1324
   tctggggctc tttgtctttg 20
<210> 1325
   <211> 20
   <212> DNA
   <213> Homo sapiens
<400> 1325
   atttttcctc ccctgtagct 20
<210> 1326
   <211> 20
   <212> DNA
   <213> Homo sapiens
<400> 1326
   ctgggcacac tgtattacca 20
<210> 1327
   <211> 20
   <212> DNA
   <213> Homo sapiens
<400> 1327
   caccacgttt ctaatgcaga 20
<210> 1328
   <211> 20
   <212> DNA
   <213> Homo sapiens
<400> 1328
   gagttgaaaa aggtccacgc 20
<210> 1329
   <211> 20
   <212> DNA
   <213> Homo sapiens
<400> 1329
   cagaggaaag acacagtgct 20
<210> 1330
   <211> 20
   <212> DNA
   <213> Homo sapiens
<400> 1330
   tcttggtttt gaggctgtca 20
<210> 1331
   <211> 20
   <212> DNA
   <213> Homo sapiens
<400> 1331
   atcagcctaa ttctccccac 20
<210> 1332
   <211> 20
   <212> DNA
   <213> Homo sapiens
<400> 1332
   atcccccatc atccatactc 20
<210> 1333
   <211> 20
   <212> DNA
   <213> Homo sapiens
<400> 1333
   catagctagg cctgtgagtg 20
<210> 1334
   <211> 19
   <212> DNA
   <213> Homo sapiens
<400> 1334
   tcagcttgct ccttctctg 19
<210> 1335
   <211> 20
   <212> DNA
   <213> Homo sapiens
<400> 1335
   ctaggtcctc agcagtgttt 20
<210> 1336
   <211> 21
   <212> DNA
   <213> Homo sapiens
<400> 1336
   tcccagagtt aacaataccc c 21
<210> 1337
   <211> 20
   <212> DNA
   <213> Homo sapiens
<400> 1337
   ccatttgcac tgccgatttc 20
<210> 1338
   <211> 21
   <212> DNA
   <213> Homo sapiens
<400> 1338
   actagtccca aaagcctaca c 21
<210> 1339
   <211> 20
   <212> DNA
   <213> Homo sapiens
<400> 1339
   aacagcagcg tcagaataac 20
<210> 1340
   <211> 20
   <212> DNA
   <213> Homo sapiens
<400> 1340
   aggtctttac gggaaggaaa 20
<210> 1341
   <211> 20
   <212> DNA
   <213> Homo sapiens
<400> 1341
   tgagggagaa gtttggtagg 20
<210> 1342
   <211> 20
   <212> DNA
   <213> Homo sapiens
<400> 1342
   ccctgtctaa agagccatgt 20
<210> 1343
   <211> 20
   <212> DNA
   <213> Homo sapiens
<400> 1343
   gtttggagtt tcgatgcctt 20
<210> 1344
   <211> 20
   <212> DNA
   <213> Homo sapiens
<400> 1344
   atgttttggt cctgggagaa 20
<210> 1345
   <211> 20
   <212> DNA
   <213> Homo sapiens
<400> 1345
   ggaagtggtt agggcagatt 20
<210> 1346
   <211> 21
   <212> DNA
   <213> Homo sapiens
<400> 1346
   agcccagtaa agataagagg c 21
<210> 1347
   <211> 20
   <212> DNA
   <213> Homo sapiens
<400> 1347
   cccttccctt tcatccaaga 20
<210> 1348
   <211> 20
   <212> DNA
   <213> Homo sapiens
<400> 1348
   ctgaaacctt ctccttagcc 20
<210> 1349
   <211> 20
   <212> DNA
   <213> Homo sapiens
<400> 1349
   attatccaac ctgacctgca 20
<210> 1350
   <211> 19
   <212> DNA
   <213> Homo sapiens
<400> 1350
   aggtgcaaag ctgttcatg 19
<210> 1351
   <211> 20
   <212> DNA
   <213> Homo sapiens
<400> 1351
   aatgccaaga ttgtccttca 20
<210> 1352
   <211> 19
   <212> DNA
   <213> Homo sapiens
<400> 1352
   tgcgaaacct cagtgatca 19
<210> 1353
   <211> 20
   <212> DNA
   <213> Homo sapiens
<400> 1353
   cacttccttc agcacacttt 20
<210> 1354
   <211> 20
   <212> DNA
   <213> Homo sapiens
<400> 1354
   ccggctctct atgaaagtga 20
<210> 1355
   <211> 20
   <212> DNA
   <213> Homo sapiens
<400> 1355
   acttgtctgt ctgcctgttt 20
<210> 1356
   <211> 19
   <212> DNA
   <213> Homo sapiens
<400> 1356
   ctgatgcgct gaaaaccaa 19
<210> 1357
   <211> 20
   <212> DNA
   <213> Homo sapiens
<400> 1357
   ttccatgtgt tcttcctccc 20
<210> 1358
   <211> 20
   <212> DNA
   <213> Homo sapiens
<400> 1358
   agcagtaggg ttaacaggag 20
<210> 1359
   <211> 19
   <212> DNA
   <213> Homo sapiens
<400> 1359
   gcagcaatgt ttcggtgta 19
<210> 1360
   <211> 20
   <212> DNA
   <213> Homo sapiens
<400> 1360
   tactaatggc tgggggtaac 20
<210> 1361
   <211> 20
   <212> DNA
   <213> Homo sapiens
<400> 1361
   ctgatgaggc taaaggacca 20
<210> 1362
   <211> 20
   <212> DNA
   <213> Homo sapiens
<400> 1362
   cagagttctc catcccagac 20
<210> 1363
   <211> 20
   <212> DNA
   <213> Homo sapiens
<400> 1363
   ctgagttcct ccttttgcct 20
<210> 1364
   <211> 20
   <212> DNA
   <213> Homo sapiens
<400> 1364
   gcaaaaggtg gtgttagctg 20
<210> 1365
   <211> 20
   <212> DNA
   <213> Homo sapiens
<400> 1365
   atccacatcc catgcctaag 20
<210> 1366
   <211> 20
   <212> DNA
   <213> Homo sapiens
<400> 1366
   tctattcctt tggcacctcc 20
<210> 1367
   <211> 20
   <212> DNA
   <213> Homo sapiens
<400> 1367
   aggaaaggag agctttgtcc 20
<210> 1368
   <211> 20
   <212> DNA
   <213> Homo sapiens
<400> 1368
   cctttcagct tccaagtcct 20
<210> 1369
   <211> 21
   <212> DNA
   <213> Homo sapiens
<400> 1369
   gctctcttcc tcccactaaa a 21
<210> 1370
   <211> 20
   <212> DNA
   <213> Homo sapiens
<400> 1370
   actgcctgtg ttttcttcct 20
<210> 1371
   <211> 20
   <212> DNA
   <213> Homo sapiens
<400> 1371
   aaagcaattt cttccccagc 20
<210> 1372
   <211> 20
   <212> DNA
   <213> Homo sapiens
<400> 1372
   tgtctgttgc cattccttct 20
<210> 1373
   <211> 20
   <212> DNA
   <213> Homo sapiens
<400> 1373
   tgactgtgac ttgtgctttc 20
<210> 1374
   <211> 20
   <212> DNA
   <213> Homo sapiens
<400> 1374
   gaattacatt tccctgggcg 20
<210> 1375
   <211> 20
   <212> DNA
   <213> Homo sapiens
<400> 1375
   tgaaaccgtc ttccttgtct 20
<210> 1376
   <211> 20
   <212> DNA
   <213> Homo sapiens
<400> 1376
   tttaaagcag agcaggacct 20
<210> 1377
   <211> 20
   <212> DNA
   <213> Homo sapiens
<400> 1377
   tttatgacac acagagcagc 20
<210> 1378
   <211> 20
   <212> DNA
   <213> Homo sapiens
<400> 1378
   atgtgtttga ccctttccct 20
<210> 1379
   <211> 21
   <212> DNA
   <213> Homo sapiens
<400> 1379
   atcctgaagt tgttccacat c 21
<210> 1380
   <211> 21
   <212> DNA
   <213> Homo sapiens
<400> 1380
   gaccctgctt tgttactagg a 21
<210> 1381
   <211> 20
   <212> DNA
   <213> Homo sapiens
<400> 1381
   tggacatgga catttcaacg 20
<210> 1382
   <211> 20
   <212> DNA
   <213> Homo sapiens
<400> 1382
   aaaaatgctt ccacttgcct 20
<210> 1383
   <211> 20
   <212> DNA
   <213> Homo sapiens
<400> 1383
   gcacctccaa caacattcaa 20
<210> 1384
   <211> 18
   <212> DNA
   <213> Homo sapiens
<400> 1384
   ttggaaatgg ggctggag 18
<210> 1385
   <211> 21
   <212> DNA
   <213> Homo sapiens
<400> 1385
   actggtctat tgggggaaaa t 21
<210> 1386
   <211> 20
   <212> DNA
   <213> Homo sapiens
<400> 1386
   agtgttagga aagcagagtg 20
<210> 1387
   <211> 20
   <212> DNA
   <213> Homo sapiens
<400> 1387
   tggacagggt ttcacaagat 20
<210> 1388
   <211> 20
   <212> DNA
   <213> Homo sapiens
<400> 1388
   ctcctctcca tctttccagg 20
<210> 1389
   <211> 21
   <212> DNA
   <213> Homo sapiens
<400> 1389
   ataggctgac ttccacatct c 21
<210> 1390
   <211> 20
   <212> DNA
   <213> Homo sapiens
<400> 1390
   tgtgggggtc aattctaacg 20
<210> 1391
   <211> 20
   <212> DNA
   <213> Homo sapiens
<400> 1391
   ccaacgggta gtggtagatt 20
<210> 1392
   <211> 20
   <212> DNA
   <213> Homo sapiens
<400> 1392
   cttaccccac ttcttcctga 20
<210> 1393
   <211> 19
   <212> DNA
   <213> Homo sapiens
<400> 1393
   cctgtcacaa ctgcctttg 19
<210> 1394
   <211> 20
   <212> DNA
   <213> Homo sapiens
<400> 1394
   tttgccattt tgtgatgcca 20
<210> 1395
   <211> 20
   <212> DNA
   <213> Homo sapiens
<400> 1395
   ggagtttcag gttggcagaa 20
<210> 1396
   <211> 20
   <212> DNA
   <213> Homo sapiens
<400> 1396
   acagcttgct tcaaactaca 20
<210> 1397
   <211> 20
   <212> DNA
   <213> Homo sapiens
<400> 1397
   ttgaaggggc aaaatacagc 20
<210> 1398
   <211> 20
   <212> DNA
   <213> Homo sapiens
<400> 1398
   gccccaaatt gtaacaaagc 20
<210> 1399
   <211> 20
   <212> DNA
   <213> Homo sapiens

<400> 1399
   tgacgaagac tccaacacaa 20
<210> 1400
   <211> 20
   <212> DNA
   <213> Homo sapiens
<400> 1400
   aagtcaggga aatgaagctg 20
<210> 1401
   <211> 20
   <212> DNA
   <213> Homo sapiens
<400> 1401
   gtaacacagt gctccttctc 20
<210> 1402
   <211> 20
   <212> DNA
   <213> Homo sapiens
<400> 1402
   ggccccaatt agctgatttc 20
<210> 1403
   <211> 20
   <212> DNA
   <213> Homo sapiens
<400> 1403
   ctgagcaggg aaaaatccag 20
<210> 1404
   <211> 20
   <212> DNA
   <213> Homo sapiens
<400> 1404
   acaaaggatt caggtgcagt 20
<210> 1405
   <211> 20
   <212> DNA
   <213> Homo sapiens
<400> 1405
   acaggttttg ctcttcagga 20
<210> 1406
   <211> 21
   <212> DNA
   <213> Homo sapiens
<400> 1406
   ggcaagtttg tctggttcat t 21
<210> 1407
   <211> 20
   <212> DNA
   <213> Homo sapiens
<400> 1407
   ccatctgcat ctgtctcctt 20
<210> 1408
   <211> 19
   <212> DNA
   <213> Homo sapiens
<400> 1408
   gctcctctcc ttctccctt 19
<210> 1409
   <211> 20
   <212> DNA
   <213> Homo sapiens
<400> 1409
   tgtataaggg caatcgtggt 20
<210> 1410
   <211> 20
   <212> DNA
   <213> Homo sapiens
<400> 1410
   aaggaaccag gtcagacaag 20
<210> 1411
   <211> 21
   <212> DNA
   <213> Homo sapiens
<400> 1411
   gttagaaggc aaacatcatg c 21
<210> 1412
   <211> 20
   <212> DNA
   <213> Homo sapiens
<400> 1412
   tgcagtcata ggaaaaggct 20
<210> 1413
   <211> 19
   <212> DNA
   <213> Homo sapiens
<400> 1413
   gcagtcagaa tggtttggc 19
<210> 1414
   <211> 21
   <212> DNA
   <213> Homo sapiens
<400> 1414
   agacattggt ttggttggtt c 21
<210> 1415
   <211> 20
   <212> DNA
   <213> Homo sapiens
<400> 1415
   acacaaatga aagcccgtac 20
<210> 1416
   <211> 20
   <212> DNA
   <213> Homo sapiens
<400> 1416
   ggtctgctgt ttctctttgc 20
<210> 1417
   <211> 20
   <212> DNA
   <213> Homo sapiens
<400> 1417
   agagccttac caagctgaag 20
<210> 1418
   <211> 20
   <212> DNA
   <213> Homo sapiens
<400> 1418
   gggatggtta cttagtgggg 20
<210> 1419
   <211> 20
   <212> DNA
   <213> Homo sapiens
<400> 1419
   ttacactcgc cttccaaaca 20
<210> 1420
   <211> 20
   <212> DNA
   <213> Homo sapiens
<400> 1420
   gagagaggag gagttggaag 20
<210> 1421
   <211> 20
   <212> DNA
   <213> Homo sapiens
<400> 1421
   atccacgaca tccaaaatca 20
<210> 1422
   <211> 20
   <212> DNA
   <213> Homo sapiens
<400> 1422
   ttgtgcaaga agaaacctgc 20
<210> 1423
   <211> 20
   <212> DNA
   <213> Homo sapiens
<400> 1423
   ggccttgcat aaaccacatt 20
<210> 1424
   <211> 20
   <212> DNA
   <213> Homo sapiens
<400> 1424
   tttgtaattg gtcctcgcct 20
<210> 1425
   <211> 20
   <212> DNA
   <213> Homo sapiens
<400> 1425
   caagtatttc atggcgctcc 20
<210> 1426
   <211> 21
   <212> DNA
   <213> Homo sapiens
<400> 1426
   gtcaacagta tcagcttcca a 21
<210> 1427
   <211> 20
   <212> DNA
   <213> Homo sapiens
<400> 1427
   tgggcttctt tttcattccg 20
<210> 1428
   <211> 20
   <212> DNA
   <213> Homo sapiens
<400> 1428
   tcaacaagct ctctgttcac 20
<210> 1429
   <211> 20
   <212> DNA
   <213> Homo sapiens
<400> 1429
   gtgcaaacag tgacctcaat 20
<210> 1430
   <211> 20
   <212> DNA
   <213> Homo sapiens
<400> 1430
   gctgggctgc tttaatttct 20
<210> 1431
   <211> 20
   <212> DNA
   <213> Homo sapiens
<400> 1431
   ggaattgtgg ggtcaaatgg 20
<210> 1432
   <211> 21
   <212> DNA
   <213> Homo sapiens
<400> 1432
   ctagtgcttc tacctccaga c 21
<210> 1433
   <211> 20
   <212> DNA
   <213> Homo sapiens
<400> 1433
   aaccacacac taacagggaa 20
<210> 1434
   <211> 20
   <212> DNA
   <213> Homo sapiens
<400> 1434
   tctagtttgc cctctttccc 20
<210> 1435
   <211> 20
   <212> DNA
   <213> Homo sapiens
<400> 1435
   cgaattgctt ccttgctctg 20
<210> 1436
   <211> 22
   <212> DNA
   <213> Homo sapiens
<400> 1436
   tctatcacag caggaaatca ct 22
<210> 1437
   <211> 20
   <212> DNA
   <213> Homo sapiens
<400> 1437
   tcttcgtgtt tctctagccc 20
<210> 1438
   <211> 20
   <212> DNA
   <213> Homo sapiens
<400> 1438
   ttgaagagct aaagggggag 20
<210> 1439
   <211> 20
   <212> DNA
   <213> Homo sapiens
<400> 1439
   tgtcaccgta ctacctaagc 20
<210> 1440
   <211> 19
   <212> DNA
   <213> Homo sapiens
<400> 1440
   agtacgctcc tttgcagag 19
<210> 1441
   <211> 20
   <212> DNA
   <213> Homo sapiens
<400> 1441
   ttacggggac acaaaatggt 20
<210> 1442
   <211> 19
   <212> DNA
   <213> Homo sapiens
<400> 1442
   ctgtgctttg cccttgaag 19
<210> 1443
   <211> 20
   <212> DNA
   <213> Homo sapiens
<400> 1443
   aagtcaaccc atatgccact 20
<210> 1444
   <211> 20
   <212> DNA
   <213> Homo sapiens
<400> 1444
   acattcaggc tgtcacacat 20
<210> 1445
   <211> 23
   <212> DNA
   <213> Homo sapiens
<400> 1445
   tcatgtcact agttttataa ggc 23
<210> 1446
   <211> 20
   <212> DNA
   <213> Homo sapiens
<400> 1446
   agtagtgagg ctccaaagtg 20
<210> 1447
   <211> 20
   <212> DNA
   <213> Homo sapiens
<400> 1447
   tgggaggagt ttgctgttta 20
<210> 1448
   <211> 20
   <212> DNA
   <213> Homo sapiens
<400> 1448
   ttctaagcct gtgactgaca 20
<210> 1449
   <211> 20
   <212> DNA
   <213> Homo sapiens
<400> 1449
   tgaacctgac tttccttggg 20
<210> 1450
   <211> 20
   <212> DNA
   <213> Homo sapiens
<400> 1450
   gccattctat catctcggga 20
<210> 1451
   <211> 19
   <212> DNA
   <213> Homo sapiens
<400> 1451
   agtctctccc tgaaaccca 19
<210> 1452
   <211> 20
   <212> DNA
   <213> Homo sapiens
<400> 1452
   aagagttggc ttggagttga 20
<210> 1453
   <211> 21
   <212> DNA
   <213> Homo sapiens
<400> 1453
   cccacaatta tgaaaggagg t 21
<210> 1454
   <211> 20
   <212> DNA
   <213> Homo sapiens
<400> 1454
   ttgaccagga caaatgagga 20
<210> 1455
   <211> 20
   <212> DNA
   <213> Homo sapiens
<400> 1455
   cactttgttg gtctgggtca 20
<210> 1456
   <211> 20
   <212> DNA
   <213> Homo sapiens
<400> 1456
   gggactctag gtggggttaa 20
<210> 1457
   <211> 20
   <212> DNA
   <213> Homo sapiens
<400> 1457
   gtttatgcct tgggattgcc 20
<210> 1458
   <211> 20
   <212> DNA
   <213> Homo sapiens
<400> 1458
   gtgtggtaag gatgctagga 20
<210> 1459
   <211> 20
   <212> DNA
   <213> Homo sapiens
<400> 1459
   gaaagtgact cctccctgac 20
<210> 1460
   <211> 20
   <212> DNA
   <213> Homo sapiens
<400> 1460
   ccaggttctg ttctctgtca 20
<210> 1461
   <211> 20
   <212> DNA
   <213> Homo sapiens
<400> 1461
   gtgagacatg gttgctgttc 20
<210> 1462
   <211> 20
   <212> DNA
   <213> Homo sapiens
<400> 1462
   ctttctcctg ctccacctat 20
<210> 1463
   <211> 20
   <212> DNA
   <213> Homo sapiens
<400> 1463
   ctatgtgtgt tccaacccga 20
<210> 1464
   <211> 20
   <212> DNA
   <213> Homo sapiens
<400> 1464
   gggaccttct aaccatgtgt 20
<210> 1465
   <211> 20
   <212> DNA
   <213> Homo sapiens
<400> 1465
   cgggattttg aaaaggcaga 20
<210> 1466
   <211> 20
   <212> DNA
   <213> Homo sapiens
<400> 1466
   gtgttgtctc tcagctcctc 20
<210> 1467
   <211> 20
   <212> DNA
   <213> Homo sapiens
<400> 1467
   gttctctggt taaggccctt 20
<210> 1468
   <211> 20
   <212> DNA
   <213> Homo sapiens
<400> 1468
   cctcttcacc tataagcccc 20
<210> 1469
   <211> 20
   <212> DNA
   <213> Homo sapiens
<400> 1469
   taggggacag taagccagat 20
<210> 1470
   <211> 20
   <212> DNA
   <213> Homo sapiens
<400> 1470
   acgatggacc tctgttgaac 20
<210> 1471
   <211> 21
   <212> DNA
   <213> Homo sapiens
<400> 1471
   attcccatcc atccatcact c 21
<210> 1472
   <211> 20
   <212> DNA
   <213> Homo sapiens
<400> 1472
   tcgtttttgg atggtggttg 20
<210> 1473
   <211> 21
   <212> DNA
   <213> Homo sapiens
<400> 1473
   gaagttcctc cagtagactc a 21
<210> 1474
   <211> 20
   <212> DNA
   <213> Homo sapiens
<400> 1474
   ttgtttgagt ctgggaggaa 20
<210> 1475
   <211> 20
   <212> DNA
   <213> Homo sapiens
<400> 1475
   aatactgtga gactgccacc 20
<210> 1476
   <211> 20
   <212> DNA
   <213> Homo sapiens
<400> 1476
   cagtcacgga aagtaccctc 20
<210> 1477
   <211> 20
   <212> DNA
   <213> Homo sapiens
<400> 1477
   accatgtttc cctctgtcac 20
<210> 1478
   <211> 20
   <212> DNA
   <213> Homo sapiens
<400> 1478
   ttaccaaggg acaggatgga 20
<210> 1479
   <211> 20
   <212> DNA
   <213> Homo sapiens
<400> 1479
   ccctagaggt caaggtatgg 20
<210> 1480
   <211> 20
   <212> DNA
   <213> Homo sapiens
<400> 1480
   ataggccctg tgtgttagtt 20
<210> 1481
   <211> 20
   <212> DNA
   <213> Homo sapiens
<400> 1481
   agaaagtccc ctccatttct 20
<210> 1482
   <211> 20
   <212> DNA
   <213> Homo sapiens
<400> 1482
   gccaatgcca aagtcagtta 20
<210> 1483
   <211> 20
   <212> DNA
   <213> Homo sapiens
<400> 1483
   gaggacgagt tgaacaaagc 20
<210> 1484
   <211> 21
   <212> DNA
   <213> Homo sapiens
<400> 1484
   atactggtct caaggtagca c 21
<210> 1485
   <211> 22
   <212> DNA
   <213> Homo sapiens
<400> 1485
   accaagtgaa gctgagttaa tg 22
<210> 1486
   <211> 20
   <212> DNA
   <213> Homo sapiens
<400> 1486
   cccagataca ctcctgcttc 20
<210> 1487
   <211> 20
   <212> DNA
   <213> Homo sapiens
<400> 1487
   caaacatgag agggggagaa 20
<210> 1488
   <211> 20
   <212> DNA
   <213> Homo sapiens
<400> 1488
   aaacacagca atgaggaagg 20
<210> 1489
   <211> 20
   <212> DNA
   <213> Homo sapiens
<400> 1489
   gatactcccc tgtgttgctt 20
<210> 1490
   <211> 20
   <212> DNA
   <213> Homo sapiens
<400> 1490
   gctcttacta ggatggcagg 20
<210> 1491
   <211> 20
   <212> DNA
   <213> Homo sapiens
<400> 1491
   gtttccagca gcaatccttt 20
<210> 1492
   <211> 19
   <212> DNA
   <213> Homo sapiens
<400> 1492
   ctgtgcagaa gggttagct 19
<210> 1493
   <211> 20
   <212> DNA
   <213> Homo sapiens
<400> 1493
   aaacccctgc tacccaaaat 20
<210> 1494
   <211> 20
   <212> DNA
   <213> Homo sapiens
<400> 1494
   aatgcccaga tgctgttttc 20
<210> 1495
   <211> 21
   <212> DNA
   <213> Homo sapiens
<400> 1495
   gagtggttgt tctctccaga t 21
<210> 1496
   <211> 20
   <212> DNA
   <213> Homo sapiens
<400> 1496
   cagtctagaa gctcacccag 20
<210> 1497
   <211> 20
   <212> DNA
   <213> Homo sapiens
<400> 1497
   tctcctctac ccctacactg 20
<210> 1498
   <211> 20
   <212> DNA
   <213> Homo sapiens
<400> 1498
   ggtgtaaatg tggcctctcc 20
<210> 1499
   <211> 20
   <212> DNA
   <213> Homo sapiens
<400> 1499
   acaaagctac aaactctggc 20
<210> 1500
   <211> 20
   <212> DNA
   <213> Homo sapiens
<400> 1500
   tttcactggg agactgatgc 20
<210> 1501
   <211> 20
   <212> DNA
   <213> Homo sapiens
<400> 1501
   ctgttctgtt cctgaggcta 20
<210> 1502
   <211> 21
   <212> DNA
   <213> Homo sapiens
<400> 1502
   tgtgtatcca ttgcctcatc t 21
<210> 1503
   <211> 20
   <212> DNA
   <213> Homo sapiens
<400> 1503
   gaagagggtg tgtgtaggac 20
<210> 1504
   <211> 20
   <212> DNA
   <213> Homo sapiens
<400> 1504
   tggttgctct tcctagttcc 20
<210> 1505
   <211> 20
   <212> DNA
   <213> Homo sapiens
<400> 1505
   caatgtggag gaagctcttg 20
<210> 1506
   <211> 20
   <212> DNA
   <213> Homo sapiens
<400> 1506
   ttgcacaccc aatatgctac 20
<210> 1507
   <211> 20
   <212> DNA
   <213> Homo sapiens
<400> 1507
   tccaaggttt ctctagcgac 20
<210> 1508
   <211> 20
   <212> DNA
   <213> Homo sapiens
<400> 1508
   tcgctattct ccttgccata 20
<210> 1509
   <211> 20
   <212> DNA
   <213> Homo sapiens
<400> 1509
   aacagcctct ttccttagca 20
<210> 1510
   <211> 20
   <212> DNA
   <213> Homo sapiens
<400> 1510
   tttttggctc agtgggatgt 20
<210> 1511
   <211> 21
   <212> DNA
   <213> Homo sapiens
<400> 1511
   ctgttcattc ttcttcaggg c 21
<210> 1512
   <211> 20
   <212> DNA
   <213> Homo sapiens
<400> 1512
   ttcccgagcc cataaactac 20
<210> 1513
   <211> 20
   <212> DNA
   <213> Homo sapiens
<400> 1513
   tgctcagatt tcagcttcct 20
<210> 1514
   <211> 20
   <212> DNA
   <213> Homo sapiens
<400> 1514
   gtcagcgatg tggatgtcta 20
<210> 1515
   <211> 20
   <212> DNA
   <213> Homo sapiens
<400> 1515
   aactgactcc atgacctgtg 20
<210> 1516
   <211> 20
   <212> DNA
   <213> Homo sapiens
<400> 1516
   gaagctgcta cttggtgaac 20
<210> 1517
   <211> 20
   <212> DNA
   <213> Homo sapiens
<400> 1517
   ttttcctcct gttctgttgc 20
<210> 1518
   <211> 20
   <212> DNA
   <213> Homo sapiens
<400> 1518
   ttctcaaatg caaccactcc 20
<210> 1519
   <211> 20
   <212> DNA
   <213> Homo sapiens
<400> 1519
   ctggcccttc aatttcatgc 20
<210> 1520
   <211> 21
   <212> DNA
   <213> Homo sapiens
<400> 1520
   ccagcagtac cgatatcaga g 21
<210> 1521
   <211> 20
   <212> DNA
   <213> Homo sapiens
<400> 1521
   cagaaggcag gagatggatt 20
<210> 1522
   <211> 20
   <212> DNA
   <213> Homo sapiens
<400> 1522
   aagcaaccat tttcctgagc 20
<210> 1523
   <211> 20
   <212> DNA
   <213> Homo sapiens
<400> 1523
   tcacccttca tctacccact 20
<210> 1524
   <211> 20
   <212> DNA
   <213> Homo sapiens
<400> 1524
   aagctggtga ccttctacag 20
<210> 1525
   <211> 20
   <212> DNA
   <213> Homo sapiens
<400> 1525
   aaaattctgg ttggggagga 20
<210> 1526
   <211> 20
   <212> DNA
   <213> Homo sapiens
<400> 1526
   ccatacctca tctgctctgt 20
<210> 1527
   <211> 20
   <212> DNA
   <213> Homo sapiens
<400> 1527
   ggctgtccct gaactacttt 20
<210> 1528
   <211> 20
   <212> DNA
   <213> Homo sapiens
<400> 1528
   cttggcttaa actctgctcc 20
<210> 1529
   <211> 20
   <212> DNA
   <213> Homo sapiens
<400> 1529
   gacttgaaca caccctcaga 20
<210> 1530
   <211> 20
   <212> DNA
   <213> Homo sapiens
<400> 1530
   aggagaagag accattgcag 20
<210> 1531
   <211> 20
   <212> DNA
   <213> Homo sapiens
<400> 1531
   ttagctaagt ctgtgcggag 20
<210> 1532
   <211> 19
   <212> DNA
   <213> Homo sapiens
<400> 1532
   gaagcaacac tgtacacgc 19
<210> 1533
   <211> 20
   <212> DNA
   <213> Homo sapiens
<400> 1533
   tctgataaag gctggctcat 20
<210> 1534
   <211> 20
   <212> DNA
   <213> Homo sapiens
<400> 1534
   taaaacagtg ccgctacttc 20
<210> 1535
   <211> 20
   <212> DNA
   <213> Homo sapiens
<400> 1535
   agtgctatga gtcttggtcc 20
<210> 1536
<400> 1536
   000
<210> 1537
   <211> 20
   <212> DNA
   <213> Homo sapiens
<400> 1537
   gcagagaaat gggttaaggg 20
<210> 1538
   <211> 20
   <212> DNA
   <213> Homo sapiens
<400> 1538
   ggtagaggtg ggttatctgt 20
<210> 1539
   <211> 19
   <212> DNA
   <213> Homo sapiens
<400> 1539
   ctgtaaatct ccgggggtg 19
<210> 1540
   <211> 20
   <212> DNA
   <213> Homo sapiens
<400> 1540
   ggcgagaatg gagagagaaa 20
<210> 1541
   <211> 20
   <212> DNA
   <213> Homo sapiens
<400> 1541
   tcccaagcca ggattctttt 20
<210> 1542
   <211> 20
   <212> DNA
   <213> Homo sapiens
<400> 1542
   gaacaagtac aaccgtgcag 20
<210> 1543
   <211> 20
   <212> DNA
   <213> Homo sapiens
<400> 1543
   acaaatgccc catatcaacc 20
<210> 1544
   <211> 20
   <212> DNA
   <213> Homo sapiens
<400> 1544
   ggaaagaggc ctggagtaat 20
<210> 1545
   <211> 20
   <212> DNA
   <213> Homo sapiens
<400> 1545
   tttccactgg atgtcgtcat 20
<210> 1546
   <211> 20
   <212> DNA
   <213> Homo sapiens
<400> 1546
   aggacaaagt ttcagcctct 20
<210> 1547
   <211> 21
   <212> DNA
   <213> Homo sapiens
<400> 1547
   actcaggaca cgacttcata c 21
<210> 1548
   <211> 20
   <212> DNA
   <213> Homo sapiens
<400> 1548
   acatctttgg ctcactggtt 20
<210> 1549
   <211> 20
   <212> DNA
   <213> Homo sapiens
<400> 1549
   tcacagtggg cttcattcag 20
<210> 1550
   <211> 22
   <212> DNA
   <213> Homo sapiens
<400> 1550
   agctggaatc tatgtaggat gg 22
<210> 1551
   <211> 20
   <212> DNA
   <213> Homo sapiens
<400> 1551
   ctgcggaagg atctagtctt 20
<210> 1552
   <211> 22
   <212> DNA
   <213> Homo sapiens
<400> 1552
   tgagaagtat tcagcatttc cc 22
<210> 1553
   <211> 19
   <212> DNA
   <213> Homo sapiens
<400> 1553
   cactcacgga cttttaggc 19
<210> 1554
   <211> 18
   <212> DNA
   <213> Homo sapiens
<400> 1554
   aaaaaaaaaa aaaaaaaa 18
<210> 1555
   <211> 20
   <212> DNA
   <213> Homo sapiens
<400> 1555
   tcacacgcca ggttattaca 20
<210> 1556
   <211> 20
   <212> DNA
   <213> Homo sapiens
<400> 1556
   aagtgggttt gcagtttgga 20
<210> 1557
   <211> 20
   <212> DNA
   <213> Homo sapiens
<400> 1557
   atgtacgtgt gtgtccatgt 20
<210> 1558
   <211> 20
   <212> DNA
   <213> Homo sapiens
<400> 1558
   aggcgggttg gtcaataata 20
<210> 1559
   <211> 20
   <212> DNA
   <213> Homo sapiens
<400> 1559
   ccacaaatcc catcaacaca 20
<210> 1560
   <211> 20
   <212> DNA
   <213> Homo sapiens
<400> 1560
   tttcacagta acatcggcac 20
<210> 1561
   <211> 20
   <212> DNA
   <213> Homo sapiens
<400> 1561
   ctgacagcct gcatttgatt 20
<210> 1562
   <211> 21
   <212> DNA
   <213> Homo sapiens
<400> 1562
   tttcctggag taaagcgatc t 21
<210> 1563
   <211> 20
   <212> DNA
   <213> Homo sapiens
<400> 1563
   cccacaatca cccatctcta 20
<210> 1564
   <211> 20
   <212> DNA
   <213> Homo sapiens
<400> 1564
   tatctcactc cacagcttcc 20
<210> 1565
   <211> 20
   <212> DNA
   <213> Homo sapiens
<400> 1565
   acaggtagtt tggtggtgtc 20
<210> 1566
   <211> 20
   <212> DNA
   <213> Homo sapiens
<400> 1566
   gctgttgaat gccagaactt 20
<210> 1567
   <211> 20
   <212> DNA
   <213> Homo sapiens
<400> 1567
   atagggtcgg ttttggtctg 20
<210> 1568
   <211> 20
   <212> DNA
   <213> Homo sapiens
<400> 1568
   catcatccct gtcattccca 20
<210> 1569
   <211> 20
   <212> DNA
   <213> Homo sapiens
<400> 1569
   gtgggctaag aaaacacctc 20
<210> 1570
   <211> 20
   <212> DNA
   <213> Homo sapiens
<400> 1570
   attgtggttt gtggcatgtg 20
<210> 1571
   <211> 20
   <212> DNA
   <213> Homo sapiens
<400> 1571
   ccagattcag cctgtattcc 20
<210> 1572
   <211> 20
   <212> DNA
   <213> Homo sapiens
<400> 1572
   gcccatggaa gtaaacagtc 20
<210> 1573
   <211> 22
   <212> DNA
   <213> Homo sapiens
<400> 1573
   aggtttgaca taatagtgct gc 22
<210> 1574
   <211> 20
   <212> DNA
   <213> Homo sapiens
<400> 1574
   aatcctttcc ccactcactg 20
<210> 1575
   <211> 20
   <212> DNA
   <213> Homo sapiens
<400> 1575
   cacaaagcag ttccatgtcc 20
<210> 1576
   <211> 20
   <212> DNA
   <213> Homo sapiens
<400> 1576
   ggacaatttc tcacttgcca 20
<210> 1577
   <211> 20
   <212> DNA
   <213> Homo sapiens
<400> 1577
   aaggggtgtt gttagatgct 20
<210> 1578
   <211> 20
   <212> DNA
   <213> Homo sapiens
<400> 1578
   gcatcacaca cagcagatac 20
<210> 1579
   <211> 20
   <212> DNA
   <213> Homo sapiens
<400> 1579
   aaaatgtccg tcccagatga 20
<210> 1580
   <211> 20
   <212> DNA
   <213> Homo sapiens
<400> 1580
   gggggaaaat gtgttgtgtt 20
<210> 1581
   <211> 20
   <212> DNA
   <213> Homo sapiens
<400> 1581
   aactgttagc ttctccaccc 20
<210> 1582
   <211> 20
   <212> DNA
   <213> Homo sapiens
<400> 1582
   cgagtgtagg ttccggttta 20
<210> 1583
   <211> 20
   <212> DNA
   <213> Homo sapiens
<400> 1583
   agaatgccca tttcaggagt 20
<210> 1584
   <211> 21
   <212> DNA
   <213> Homo sapiens
<400> 1584
   atatgtggtt tgaggtcagc t 21
<210> 1585
   <211> 19
   <212> DNA
   <213> Homo sapiens
<400> 1585
   ggctttggtc acatggaga 19
<210> 1586
   <211> 20
   <212> DNA
   <213> Homo sapiens
<400> 1586
   tgaggcaaga ttcagtgact 20
<210> 1587
   <211> 20
   <212> DNA
   <213> Homo sapiens
<400> 1587
   acttcatctt gacagcagct 20
<210> 1588
   <211> 22
   <212> DNA
   <213> Homo sapiens
<400> 1588
   cacttcctca tgatgttttg ga 22
<210> 1589
   <211> 20
   <212> DNA
   <213> Homo sapiens
<400> 1589
   ggccagccca cttatttttg 20
<210> 1590
   <211> 20
   <212> DNA
   <213> Homo sapiens
<400> 1590
   ctcagggtgg agtttcaaac 20
<210> 1591
   <211> 20
   <212> DNA
   <213> Homo sapiens
<400> 1591
   gatctttctt cccctcctcc 20
<210> 1592
   <211> 21
   <212> DNA
   <213> Homo sapiens
<400> 1592
   tgtacatcca ccacttgttt g 21
<210> 1593
   <211> 20
   <212> DNA
   <213> Homo sapiens
<400> 1593
   agcggtagta agaaggcaaa 20
<210> 1594
   <211> 20
   <212> DNA
   <213> Homo sapiens
<400> 1594
   agacaggtga ccattttccc 20
<210> 1595
   <211> 20
   <212> DNA
   <213> Homo sapiens
<400> 1595
   tgtggaactt ttgagccaga 20
<210> 1596
   <211> 21
   <212> DNA
   <213> Homo sapiens
<400> 1596
   cataacgaaa tagggccttc c 21
<210> 1597
   <211> 21
   <212> DNA
   <213> Homo sapiens
<400> 1597
   atttctgcct cttctcttcc c 21
<210> 1598
   <211> 20
   <212> DNA
   <213> Homo sapiens
<400> 1598
   tcatcaagtc acctctccac 20
<210> 1599
   <211> 20
   <212> DNA
   <213> Homo sapiens
<400> 1599
   tggaaaacta gacagcagcc 20
<210> 1600
   <211> 20
   <212> DNA
   <213> Homo sapiens
<400> 1600
   ggaaagggga aaaggtgaca 20
<210> 1601
   <211> 20
   <212> DNA
   <213> Homo sapiens
<400> 1601
   ctgtcctctg tcccacataa 20
<210> 1602
   <211> 20
   <212> DNA
   <213> Homo sapiens
<400> 1602
   tttctgagtc cattccccat 20
<210> 1603
   <211> 20
   <212> DNA
   <213> Homo sapiens
<400> 1603
   ggccatcctg atatcttcca 20
<210> 1604
   <211> 20
   <212> DNA
   <213> Homo sapiens
<400> 1604
   cagagagatg cagaggttca 20
<210> 1605
   <211> 20
   <212> DNA
   <213> Homo sapiens
<400> 1605
   tgtgtgtgag ctagctgaat 20
<210> 1606
   <211> 20
   <212> DNA
   <213> Homo sapiens
<400> 1606
   ggtttcccat cctaccacat 20
<210> 1607
   <211> 20
   <212> DNA
   <213> Homo sapiens
<400> 1607
   ggtgcttttg ttgccttact 20
<210> 1608
   <211> 20
   <212> DNA
   <213> Homo sapiens
<400> 1608
   actagaaagc agggtacagt 20
<210> 1609
   <211> 20
   <212> DNA
   <213> Homo sapiens
<400> 1609
   gctttttcca acttctgctg 20
<210> 1610
   <211> 20
   <212> DNA
   <213> Homo sapiens
<400> 1610
   caacagacaa gtcacctcct 20
<210> 1611
   <211> 20
   <212> DNA
   <213> Homo sapiens
<400> 1611
   ggttcttcct ggacttcaaa 20
<210> 1612
   <211> 21
   <212> DNA
   <213> Homo sapiens
<400> 1612
   gaaagcagta gtttcaggtg t 21
<210> 1613
   <211> 20
   <212> DNA
   <213> Homo sapiens
<400> 1613
   tgaaagactc tgttgccatg 20
<210> 1614
   <211> 20
   <212> DNA
   <213> Homo sapiens
<400> 1614
   cactacacgc tcagaacaaa 20
<210> 1615
   <211> 20
   <212> DNA
   <213> Homo sapiens
<400> 1615
   aaggcaagca ataatgaggc 20
<210> 1616
   <211> 21
   <212> DNA
   <213> Homo sapiens
<400> 1616
   ggacagtctg tgaaaattgc t 21
<210> 1617
   <211> 20
   <212> DNA
   <213> Homo sapiens
<400> 1617
   acaaacaacc cttaatgccc 20
<210> 1618
   <211> 20
   <212> DNA
   <213> Homo sapiens
<400> 1618
   tgaaacagtg aatccgcaat 20
<210> 1619
   <211> 21
   <212> DNA
   <213> Homo sapiens
<400> 1619
   aagggaaatg tggatgcagt a 21
<210> 1620
   <211> 19
   <212> DNA
   <213> Homo sapiens
<400> 1620
   ttgaagggaa gcggaaagt 19
<210> 1621
   <211> 21
   <212> DNA
   <213> Homo sapiens
<400> 1621
   atttccagct aatgatgctc c 21
<210> 1622
   <211> 20
   <212> DNA
   <213> Homo sapiens
<400> 1622
   gctcacttac gcattaacca 20
<210> 1623
   <211> 20
   <212> DNA
   <213> Homo sapiens
<400> 1623
   acaggcaaaa ttcagttgga 20
<210> 1624
   <211> 20
   <212> DNA
   <213> Homo sapiens
<400> 1624
   aggctgaatc acgtcaaaac 20
<210> 1625
   <211> 19
   <212> DNA
   <213> Homo sapiens
<400> 1625
   cctcgttcac atttgacgc 19
<210> 1626
   <211> 20
   <212> DNA
   <213> Homo sapiens
<400> 1626
   tgtctgggtt caactgtttg 20
<210> 1627
   <211> 20
   <212> DNA
   <213> Homo sapiens
<400> 1627
   attttgcatg cctgttgaga 20
<210> 1628
   <211> 20
   <212> DNA
   <213> Homo sapiens
<400> 1628
   gctctcctca aaacccaagt 20
<210> 1629
   <211> 20
   <212> DNA
   <213> Homo sapiens
<400> 1629
   cttaatgagg gggcacaaag 20
<210> 1630
   <211> 20
   <212> DNA
   <213> Homo sapiens
<400> 1630
   ttttcgtcca gtcttccacc 20
<210> 1631
   <211> 20
   <212> DNA
   <213> Homo sapiens
<400> 1631
   tacaggaccg tcagtgagag 20
<210> 1632
   <211> 20
   <212> DNA
   <213> Homo sapiens
<400> 1632
   ttagctactg acgcttcacc 20
<210> 1633
   <211> 20
   <212> DNA
   <213> Homo sapiens
<400> 1633
   gcacagacaa catgctagtt 20
<210> 1634
   <211> 20
   <212> DNA
   <213> Homo sapiens
<400> 1634
   ttagtctgtt cactggcaca 20
<210> 1635
   <211> 19
   <212> DNA
   <213> Homo sapiens
<400> 1635
   cttatcagca gggcacagt 19
<210> 1636
   <211> 20
   <212> DNA
   <213> Homo sapiens
<400> 1636
   ttgcatcaaa caaagccaca 20
<210> 1637
   <211> 20
   <212> DNA
   <213> Homo sapiens
<400> 1637
   gagagacaag tcaccccttc 20
<210> 1638
   <211> 20
   <212> DNA
   <213> Homo sapiens
<400> 1638
   agtcaactac aaatggggga 20
<210> 1639
   <211> 20
   <212> DNA
   <213> Homo sapiens
<400> 1639
   gtgccaaaat caacgaaagc 20
<210> 1640
   <211> 21
   <212> DNA
   <213> Homo sapiens
<400> 1640
   aaggagggag tacaaagtga g 21
<210> 1641
   <211> 20
   <212> DNA
   <213> Homo sapiens
<400> 1641
   atcacatttt cagcacgagg 20
<210> 1642
   <211> 20
   <212> DNA
   <213> Homo sapiens
<400> 1642
   cagggaggga tgatttggaa 20
<210> 1643
   <211> 20
   <212> DNA
   <213> Homo sapiens
<400> 1643
   agaactgaga ggggagcata 20
<210> 1644
   <211> 20
   <212> DNA
   <213> Homo sapiens
<400> 1644
   cagtcaccaa caaaggcttt 20
<210> 1645
   <211> 20
   <212> DNA
   <213> Homo sapiens
<400> 1645
   tccccatctc cctaactcat 20
<210> 1646
   <211> 20
   <212> DNA
   <213> Homo sapiens
<400> 1646
   tttttctgct gcatccaagg 20
<210> 1647
   <211> 20
   <212> DNA
   <213> Homo sapiens
<400> 1647
   actgtacgcc atgaaaaaca 20
<210> 1648
   <211> 20
   <212> DNA
   <213> Homo sapiens
<400> 1648
   ggcaaatcaa gtgagctgac 20
<210> 1649
   <211> 20
   <212> DNA
   <213> Homo sapiens
<400> 1649
   cgtgggtgga gaatttcaca 20
<210> 1650
   <211> 20
   <212> DNA
   <213> Homo sapiens
<400> 1650
   acttaggtca gttgcttggt 20
<210> 1651
   <211> 20
   <212> DNA
   <213> Homo sapiens
<400> 1651
   gacttcatca gcacgtactt 20
<210> 1652
   <211> 20
   <212> DNA
   <213> Homo sapiens
<400> 1652
   tgcaggcaaa attagcatgg 20
<210> 1653
   <211> 20
   <212> DNA
   <213> Homo sapiens
<400> 1653
   gacttatctg ctttcacccc 20
<210> 1654
   <211> 20
   <212> DNA
   <213> Homo sapiens
<400> 1654
   ccccatgaac ctaagaccat 20
<210> 1655
   <211> 20
   <212> DNA
   <213> Homo sapiens
<400> 1655
   tctggacatg tctttgcgta 20
<210> 1656
   <211> 20
   <212> DNA
   <213> Homo sapiens
<400> 1656
   ctccaggaca tctcagcaat 20
<210> 1657
   <211> 20
   <212> DNA
   <213> Homo sapiens
<400> 1657
   cctctcgtgt gggaaatgta 20
<210> 1658
   <211> 20
   <212> DNA
   <213> Homo sapiens
<400> 1658
   tatctctggc tacctcctgt 20
<210> 1659
   <211> 19
   <212> DNA
   <213> Homo sapiens
<400> 1659
   ccccatccct gtaccaaag 19
<210> 1660
   <211> 20
   <212> DNA
   <213> Homo sapiens
<400> 1660
   ctgcagagat attccatggc 20
<210> 1661
   <211> 20
   <212> DNA
   <213> Homo sapiens
<400> 1661
   aaagctgggt tcttaggctt 20
<210> 1662
   <211> 20
   <212> DNA
   <213> Homo sapiens
<400> 1662
   gctgttttag gggcacattt 20
<210> 1663
   <211> 20
   <212> DNA
   <213> Homo sapiens
<400> 1663
   aagaggcaat gtggaggtta 20
<210> 1664
   <211> 21
   <212> DNA
   <213> Homo sapiens
<400> 1664
   gcccaaacaa tctgcctttt a 21
<210> 1665
   <211> 20
   <212> DNA
   <213> Homo sapiens
<400> 1665
   tggcttcaaa taactgggct 20
<210> 1666
   <211> 20
   <212> DNA
   <213> Homo sapiens
<400> 1666
   agagcacaca gaacagaact 20
<210> 1667
   <211> 20
   <212> DNA
   <213> Homo sapiens
<400> 1667
   ctggtggatt tctcgtcaga 20
<210> 1668
   <211> 20
   <212> DNA
   <213> Homo sapiens
<400> 1668
   taggtgtttg tgtgaggctt 20
<210> 1669
   <211> 20
   <212> DNA
   <213> Homo sapiens
<400> 1669
   gcctcactgc tcctatcttt 20
<210> 1670
   <211> 19
   <212> DNA
   <213> Homo sapiens
<400> 1670
   ctctagcagc tgttcctcc 19
<210> 1671
   <211> 20
   <212> DNA
   <213> Homo sapiens
<400> 1671
   cgtcgtatct ctggctttgt 20
<210> 1672
   <211> 20
   <212> DNA
   <213> Homo sapiens
<400> 1672
   gtccccaacc tcatctttca 20
<210> 1673
   <211> 20
   <212> DNA
   <213> Homo sapiens
<400> 1673
   tcttcaaaga tggctgcaaa 20
<210> 1674
   <211> 21
   <212> DNA
   <213> Homo sapiens
<400> 1674
   agtataacca gatagccgtg c 21
<210> 1675
   <211> 20
   <212> DNA
   <213> Homo sapiens
<400> 1675
   tgtcctcagg gcaataaagt 20
<210> 1676
   <211> 20
   <212> DNA
   <213> Homo sapiens
<400> 1676
   tttgctgctt gaagtggaac 20
<210> 1677
   <211> 20
   <212> DNA
   <213> Homo sapiens
<400> 1677
   gctgcaatga cctgatttct 20
<210> 1678
   <211> 20
   <212> DNA
   <213> Homo sapiens
<400> 1678
   tccctctctc ctccaaatga 20
<210> 1679
   <211> 21
   <212> DNA
   <213> Homo sapiens
<400> 1679
   tgccacagta ggtataggtt g 21
<210> 1680
   <211> 20
   <212> DNA
   <213> Homo sapiens
<400> 1680
   ccctcgccct aaagaaacta 20
<210> 1681
   <211> 20
   <212> DNA
   <213> Homo sapiens
<400> 1681
   cccctgaatc cctacctcat 20
<210> 1682
   <211> 21
   <212> DNA
   <213> Homo sapiens
<400> 1682
   tgttgtacaa gtgagccatt c 21
<210> 1683
   <211> 21
   <212> DNA
   <213> Homo sapiens
<400> 1683
   accaagcaat caactcactc t 21
<210> 1684
   <211> 21
   <212> DNA
   <213> Homo sapiens
<400> 1684
   gcccaatttg tctagccaat a 21
<210> 1685
   <211> 21
   <212> DNA
   <213> Homo sapiens
<400> 1685
   ctactgatcc caaagaaggc a 21
<210> 1686
   <211> 21
   <212> DNA
   <213> Homo sapiens
<400> 1686
   gtgaaaggtt ctatctgcca a 21
<210> 1687
   <211> 21
   <212> DNA
   <213> Homo sapiens
<400> 1687
   ggcataccga gcatacatag a 21
<210> 1688
   <211> 21
   <212> DNA
   <213> Homo sapiens
<400> 1688
   cacctgtttc accaaatcac t 21
<210> 1689
   <211> 21
   <212> DNA
   <213> Homo sapiens
<400> 1689
   tctcatgctc tgacagacaa g 21
<210> 1690
   <211> 21
   <212> DNA
   <213> Homo sapiens
<400> 1690
   ttgtcctgtt tctcttgtga c 21
<210> 1691
   <211> 21
   <212> DNA
   <213> Homo sapiens
<400> 1691
   cacaggactg catgcctatt a 21
<210> 1692
   <211> 21
   <212> DNA
   <213> Homo sapiens
<400> 1692
   gactctctca gcatcgagtt t 21
<210> 1693
   <211> 21
   <212> DNA
   <213> Homo sapiens
<400> 1693
   ctgatggaag ggcattatcc a 21
<210> 1694
   <211> 22
   <212> DNA
   <213> Homo sapiens
<400> 1694
   ttctggttcc ataaatccat gc 22
<210> 1695
   <211> 22
   <212> DNA
   <213> Homo sapiens
<400> 1695
   gggattattg ttggctactg ag 22
<210> 1696
   <211> 21
   <212> DNA
   <213> Homo sapiens
<400> 1696
   aattggtgac ctagggatca g 21
<210> 1697
   <211> 21
   <212> DNA
   <213> Homo sapiens
<400> 1697
   gggtttggta agggagaatg a 21
<210> 1698
   <211> 23
   <212> DNA
   <213> Homo sapiens
<400> 1698
   aagacatccc agttatgcat tgt 23
<210> 1699
   <211> 21
   <212> DNA
   <213> Homo sapiens
<400> 1699
   agatgggagg gagattagac a 21
<210> 1700
   <211> 21
   <212> DNA
   <213> Homo sapiens
<400> 1700
   gagtagcaac aacacatgga g 21
<210> 1701
   <211> 21
   <212> DNA
   <213> Homo sapiens
<400> 1701
   ttctccttca ttagccacac a 21
<210> 1702
   <211> 21
   <212> DNA
   <213> Homo sapiens
<400> 1702
   agtctgcact gtactcttct g 21
<210> 1703
   <211> 21
   <212> DNA
   <213> Homo sapiens
<400> 1703
   acaaatggtt catgatggtg g 21
<210> 1704
   <211> 21
   <212> DNA
   <213> Homo sapiens
<400> 1704
   ggtactcacg tttcagtttc c 21
<210> 1705
   <211> 21
   <212> DNA
   <213> Homo sapiens
<400> 1705
   gttcatttct acagtccagg c 21
<210> 1706
   <211> 21
   <212> DNA
   <213> Homo sapiens
<400> 1706
   ctctcactgt gctgcttaaa g 21
<210> 1707
   <211> 22
   <212> DNA
   <213> Homo sapiens
<400> 1707
   caaagaattc cacagagatg gg 22
<210> 1708
   <211> 21
   <212> DNA
   <213> Homo sapiens
<400> 1708
   tgagctacag acaagattgc a 21
<210> 1709
   <211> 21
   <212> DNA
   <213> Homo sapiens
<400> 1709
   tcacatggga tcgacatatg c 21
<210> 1710
   <211> 21
   <212> DNA
   <213> Homo sapiens
<400> 1710
   ccacacaatt tcctggctat g 21
<210> 1711
   <211> 21
   <212> DNA
   <213> Homo sapiens
<400> 1711
   tcttgcttct ggagagttct t 21
<210> 1712
   <211> 21
   <212> DNA
   <213> Homo sapiens
<400> 1712
   aggttatgca gacttcagga a 21
<210> 1713
   <211> 21
   <212> DNA
   <213> Homo sapiens
<400> 1713
   aagccaattc tgcctctcta g 21
<210> 1714
   <211> 21
   <212> DNA
   <213> Homo sapiens
<400> 1714
   tcccttcctt attctggcaa c 21
<210> 1715
   <211> 21
   <212> DNA
   <213> Homo sapiens
<400> 1715
   gcttcaaaca ctctaaaggg c 21
<210> 1716
   <211> 21
   <212> DNA
   <213> Homo sapiens
<400> 1716
   tgccattaat gagaagtgct g 21
<210> 1717
   <211> 22
   <212> DNA
   <213> Homo sapiens
<400> 1717
   tggcaatcct gttaaacaac tc 22
<210> 1718
   <211> 22
   <212> DNA
   <213> Homo sapiens
<400> 1718
   cccgaacatt gataacagaa ga 22
<210> 1719
   <211> 22
   <212> DNA
   <213> Homo sapiens
<400> 1719
   gcaactcagg aaagactaca tc 22
<210> 1720
   <211> 22
   <212> DNA
   <213> Homo sapiens
<400> 1720
   ttaagaaagt acccatcctc cc 22
<210> 1721
   <211> 21
   <212> DNA
   <213> Homo sapiens
<400> 1721
   gtcatgcctt acaacttagc a 21
<210> 1722
   <211> 21
   <212> DNA
   <213> Homo sapiens
<400> 1722
   aatctttgcc aaggtatgag c 21
<210> 1723
   <211> 21
   <212> DNA
   <213> Homo sapiens
<400> 1723
   gccacttata cctccagaca t 21
<210> 1724
   <211> 20
   <212> DNA
   <213> Homo sapiens
<400> 1724
   ccacaatcct gaatgccatg 20
<210> 1725
   <211> 21
   <212> DNA
   <213> Homo sapiens
<400> 1725
   ctgcaaggta caacacaagt c 21
<210> 1726
   <211> 21
   <212> DNA
   <213> Homo sapiens
<400> 1726
   atctctgtgc cagcaagtat t 21
<210> 1727
   <211> 21
   <212> DNA
   <213> Homo sapiens
<400> 1727
   attgggaaac tgtcactgat g 21
<210> 1728
   <211> 21
   <212> DNA
   <213> Homo sapiens
<400> 1728
   gccctaatag agaagcaaag c 21
<210> 1729
   <211> 21
   <212> DNA
   <213> Homo sapiens
<400> 1729
   gggagccaat cagatagaag t 21
<210> 1730
   <211> 22
   <212> DNA
   <213> Homo sapiens
<400> 1730
   gggaagttgg gctatttaat gc 22
<210> 1731
   <211> 21
   <212> DNA
   <213> Homo sapiens
<400> 1731
   aactgtgtag agcgaccaaa t 21
<210> 1732
   <211> 21
   <212> DNA
   <213> Homo sapiens
<400> 1732
   cagtaaggcc atggtctaga t 21
<210> 1733
   <211> 21
   <212> DNA
   <213> Homo sapiens
<400> 1733
   ctcagaacat ttgcaccttc t 21
<210> 1734
   <211> 21
   <212> DNA
   <213> Homo sapiens
<400> 1734
   acctgataca atggagcatg t 21
<210> 1735
   <211> 21
   <212> DNA
   <213> Homo sapiens
<400> 1735
   gggacctaaa ctcctttgga a 21
<210> 1736
   <211> 21
   <212> DNA
   <213> Homo sapiens
<400> 1736
   tctgcagtgg tgttatctag t 21
<210> 1737
   <211> 20
   <212> DNA
   <213> Homo sapiens
<400> 1737
   cacttaagtt tccacgccag 20
<210> 1738
   <211> 21
   <212> DNA
   <213> Homo sapiens
<400> 1738
   gtaatggcga gaggttaaag c 21
<210> 1739
   <211> 22
   <212> DNA
   <213> Homo sapiens
<400> 1739
   tttaggtatc gaagttgggt ca 22
<210> 1740
   <211> 22
   <212> DNA
   <213> Homo sapiens
<400> 1740
   gataagtttg gaagctgcat ca 22
<210> 1741
   <211> 21
   <212> DNA
   <213> Homo sapiens
<400> 1741
   aagctctgcc attgacttta c 21
<210> 1742
   <211> 20
   <212> DNA
   <213> Homo sapiens
<400> 1742
   ccctacagaa ccgaggaatc 20
<210> 1743
   <211> 21
   <212> DNA
   <213> Homo sapiens
<400> 1743
   tcaatctttg catacacagc c 21
<210> 1744
   <211> 22
   <212> DNA
   <213> Homo sapiens
<400> 1744
   gtaggtttac atggacagat gc 22
<210> 1745
   <211> 21
   <212> DNA
   <213> Homo sapiens
<400> 1745
   tcttctgttt agtgctgtgg t 21
<210> 1746
   <211> 21
   <212> DNA
   <213> Homo sapiens
<400> 1746
   caagttcatt tcttccctgc a 21
<210> 1747
   <211> 21
   <212> DNA
   <213> Homo sapiens
<400> 1747
   tgcaggaata catggtagac a 21
<210> 1748
   <211> 21
   <212> DNA
   <213> Homo sapiens
<400> 1748
   cataggcctt catgtctctc a 21
<210> 1749
   <211> 21
   <212> DNA
   <213> Homo sapiens
<400> 1749
   tatgagggtg cactaacaga t 21
<210> 1750
   <211> 21
   <212> DNA
   <213> Homo sapiens
<400> 1750
   ggtgctacta ctggtgtatg t 21
<210> 1751
   <211> 21
   <212> DNA
   <213> Homo sapiens
<400> 1751
   gccatgcaat atcaaatccc a 21
<210> 1752
   <211> 21
   <212> DNA
   <213> Homo sapiens
<400> 1752
   gaaaccaaag actagtgcag c 21
<210> 1753
   <211> 21
   <212> DNA
   <213> Homo sapiens
<400> 1753
   atgactaaca ctctgccaag t 21
<210> 1754
   <211> 21
   <212> DNA
   <213> Homo sapiens
<400> 1754
   aacaaatgca tcccagacag a 21
<210> 1755
   <211> 21
   <212> DNA
   <213> Homo sapiens
<400> 1755
   gcagagagga gtatgtggta t 21
<210> 1756
   <211> 21
   <212> DNA
   <213> Homo sapiens
<400> 1756
   atgggtcatt ctacgaagca a 21
<210> 1757
   <211> 22
   <212> DNA
   <213> Homo sapiens
<400> 1757
   tggtgtgtgt ggtgataatt ag 22
<210> 1758
   <211> 21
   <212> DNA
   <213> Homo sapiens
<400> 1758
   agtgaccctc tgaataacct g 21
<210> 1759
   <211> 21
   <212> DNA
   <213> Homo sapiens
<400> 1759
   tcagcaagta aacctgagac c 21
<210> 1760
   <211> 20
   <212> DNA
   <213> Homo sapiens
<400> 1760
   gtaccttcac cctccagatc 20
<210> 1761
   <211> 21
   <212> DNA
   <213> Homo sapiens
<400> 1761
   tcatcaaatt gcccactcct a 21
<210> 1762
   <211> 21
   <212> DNA
   <213> Homo sapiens
<400> 1762
   catcagactg tcttgccttt c 21
<210> 1763
   <211> 21
   <212> DNA
   <213> Homo sapiens
<400> 1763
   cattcttgct gacatttccc a 21
<210> 1764
   <211> 22
   <212> DNA
   <213> Homo sapiens
<400> 1764
   gaagatcagg gtattgctga aa 22
<210> 1765
   <211> 21
   <212> DNA
   <213> Homo sapiens
<400> 1765
   tttgtaggtc attcagcctc c 21
<210> 1766
   <211> 22
   <212> DNA
   <213> Homo sapiens
<400> 1766
   tggctaggat tcacttagga aa 22
<210> 1767
   <211> 21
   <212> DNA
   <213> Homo sapiens
<400> 1767
   gcaaacaggg tgaattatgc t 21
<210> 1768
   <211> 21
   <212> DNA
   <213> Homo sapiens
<400> 1768
   agaagtctgg gaaacgaaga g 21
<210> 1769
   <211> 21
   <212> DNA
   <213> Homo sapiens
<400> 1769
   cagatgctcc attactaggt g 21
<210> 1770
   <211> 21
   <212> DNA
   <213> Homo sapiens
<400> 1770
   cacatggaga aaggtgaatc a 21
<210> 1771
   <211> 20
   <212> DNA
   <213> Homo sapiens
<400> 1771
   tcccatccaa tactgccttc 20
<210> 1772
   <211> 21
   <212> DNA
   <213> Homo sapiens
<400> 1772
   tctggctcaa aggatcacat g 21
<210> 1773
   <211> 21
   <212> DNA
   <213> Homo sapiens
<400> 1773
   aagatcccat tgaccctgaa t 21
<210> 1774
   <211> 21
   <212> DNA
   <213> Homo sapiens
<400> 1774
   ccacaggctc tctagaacta a 21
<210> 1775
   <211> 21
   <212> DNA
   <213> Homo sapiens
<400> 1775
   ttgagagggt ttacaaggtc c 21
<210> 1776
   <211> 22
   <212> DNA
   <213> Homo sapiens
<400> 1776
   cttctcctac tctgcattct ca 22
<210> 1777
   <211> 21
   <212> DNA
   <213> Homo sapiens
<400> 1777
   gacattagtg gattcaggcc a 21
<210> 1778
   <211> 21
   <212> DNA
   <213> Homo sapiens
<400> 1778
   gctgaagtgg aggcaattaa c 21
<210> 1779
   <211> 21
   <212> DNA
   <213> Homo sapiens
<400> 1779
   tataactgtt gagtctgccc a 21
<210> 1780
   <211> 21
   <212> DNA
   <213> Homo sapiens
<400> 1780
   actgagctta cattcatgca c 21
<210> 1781
   <211> 21
   <212> DNA
   <213> Homo sapiens
<400> 1781
   gccactttct ctgcaaagaa t 21
<210> 1782
   <211> 21
   <212> DNA
   <213> Homo sapiens
<400> 1782
   acttcctacg gactcaaatc t 21
<210> 1783
   <211> 21
   <212> DNA
   <213> Homo sapiens
<400> 1783
   ggctctcatt acaattggct g 21
<210> 1784
   <211> 21
   <212> DNA
   <213> Homo sapiens
<400> 1784
   attgctttca gtggtggatt g 21
<210> 1785
   <211> 21
   <212> DNA
   <213> Homo sapiens
<400> 1785
   ggaatgaaac agaggagtcc c 21
<210> 1786
   <211> 21
   <212> DNA
   <213> Homo sapiens
<400> 1786
   aagatgctag aaacccacaa g 21
<210> 1787
   <211> 20
   <212> DNA
   <213> Homo sapiens
<400> 1787
   aatgggtggg ttacagagag 20
<210> 1788
   <211> 20
   <212> DNA
   <213> Homo sapiens
<400> 1788
   gcatttggac atgaacaagc 20
<210> 1789
   <211> 20
   <212> DNA
   <213> Homo sapiens
<400> 1789
   cattggtggg tggatagctg 20
<210> 1790
   <211> 20
   <212> DNA
   <213> Homo sapiens
<400> 1790
   accaggagga gaaaagcaaa 20
<210> 1791
   <211> 20
   <212> DNA
   <213> Homo sapiens
<400> 1791
   cggaaaacaa accctgaagt 20
<210> 1792
   <211> 20
   <212> DNA
   <213> Homo sapiens
<400> 1792
   acctgcatat tgagccatac 20
<210> 1793
   <211> 21
   <212> DNA
   <213> Homo sapiens
<400> 1793
   tgttctttca cttttagccc c 21
<210> 1794
   <211> 21
   <212> DNA
   <213> Homo sapiens
<400> 1794
   tttctagaac cctcagcaac t 21
<210> 1795
   <211> 21
   <212> DNA
   <213> Homo sapiens
<400> 1795
   cccccaacag tttttagtgg t 21
<210> 1796
   <211> 20
   <212> DNA
   <213> Homo sapiens
<400> 1796
   aaggcaaaac actccctttt 20
<210> 1797
   <211> 22
   <212> DNA
   <213> Homo sapiens
<400> 1797
   cagtgattgc ctctagaaaa gg 22
<210> 1798
   <211> 20
   <212> DNA
   <213> Homo sapiens
<400> 1798
   ctaagcagat tgaagcagct 20
<210> 1799
   <211> 20
   <212> DNA
   <213> Homo sapiens
<400> 1799
   tgtccccagg cttaagaatc 20
<210> 1800
   <211> 20
   <212> DNA
   <213> Homo sapiens
<400> 1800
   agccagaata agcaactgtc 20
<210> 1801
   <211> 20
   <212> DNA
   <213> Homo sapiens
<400> 1801
   tttctcctat cccagcttgc 20
<210> 1802
   <211> 21
   <212> DNA
   <213> Homo sapiens
<400> 1802
   agtacagagg ataacaaggg t 21
<210> 1803
   <211> 20
   <212> DNA
   <213> Homo sapiens
<400> 1803
   ctcccatcag taccctctct 20
<210> 1804
   <211> 21
   <212> DNA
   <213> Homo sapiens
<400> 1804
   ggaatgccta aaccatactg t 21
<210> 1805
   <211> 22
   <212> DNA
   <213> Homo sapiens
<400> 1805
   agtggtattt caatgctcta cc 22
<210> 1806
   <211> 19
   <212> DNA
   <213> Homo sapiens
<400> 1806
   gcagtacatc gtcctggaa 19
<210> 1807
   <211> 21
   <212> DNA
   <213> Homo sapiens
<400> 1807
   cctgagcgag aagaaatttg t 21
<210> 1808
   <211> 21
   <212> DNA
   <213> Homo sapiens
<400> 1808
   ggaactgaac aagaagtgga g 21
<210> 1809
   <211> 20
   <212> DNA
   <213> Homo sapiens
<400> 1809
   acctactctt attccgcact 20
<210> 1810
   <211> 20
   <212> DNA
   <213> Homo sapiens
<400> 1810
   tgacagcctc tctcttcaat 20
<210> 1811
   <211> 18
   <212> DNA
   <213> Homo sapiens
<400> 1811
   aaaaaaaaaa aaaaaaaa 18
<210> 1812
   <211> 20
   <212> DNA
   <213> Homo sapiens
<400> 1812
   gctgtttttc tgtgtgcttc 20
<210> 1813
   <211> 20
   <212> DNA
   <213> Homo sapiens
<400> 1813
   atgtatttcc tttagcgccc 20
<210> 1814
   <211> 20
   <212> DNA
   <213> Homo sapiens
<400> 1814
   aattttgcaa gacttccggt 20
<210> 1815
   <211> 20
   <212> DNA
   <213> Homo sapiens
<400> 1815
   tcccagttgt gaacatttgc 20
<210> 1816
   <211> 21
   <212> DNA
   <213> Homo sapiens
<400> 1816
   atgggttttt gcacagatga c 21
<210> 1817
   <211> 19
   <212> DNA
   <213> Homo sapiens
<400> 1817
   gggagtcaga aggaggtca 19
<210> 1818
   <211> 21
   <212> DNA
   <213> Homo sapiens
<400> 1818
   cctctttttg catgaacctg a 21
<210> 1819
   <211> 20
   <212> DNA
   <213> Homo sapiens
<400> 1819
   ccttaccctt tccactcaga 20
<210> 1820
   <211> 21
   <212> DNA
   <213> Homo sapiens
<400> 1820
   gactggtata atcttgccgt g 21
<210> 1821
   <211> 21
   <212> DNA
   <213> Homo sapiens
<400> 1821
   tgtctgcatc ttgatctctg g 21
<210> 1822
   <211> 19
   <212> DNA
   <213> Homo sapiens
<400> 1822
   ggttccacag catttgagc 19
<210> 1823
   <211> 20
   <212> DNA
   <213> Homo sapiens
<400> 1823
   aggagccctt aactatggtg 20
<210> 1824
   <211> 20
   <212> DNA
   <213> Homo sapiens
<400> 1824
   tggcttgggt attgcagata 20
<210> 1825
   <211> 20
   <212> DNA
   <213> Homo sapiens
<400> 1825
   ggggacagta gaagatgagt 20
<210> 1826
   <211> 20
   <212> DNA
   <213> Homo sapiens
<400> 1826
   agttgtttct ggacggactt 20
<210> 1827
   <211> 21
   <212> DNA
   <213> Homo sapiens
<400> 1827
   agaatttccc tgtccatacc a 21
<210> 1828
   <211> 20
   <212> DNA
   <213> Homo sapiens
<400> 1828
   agtgagtgaa cttgccatca 20
<210> 1829
   <211> 20
   <212> DNA
   <213> Homo sapiens
<400> 1829
   ggcttttctc tgcactgatt 20
<210> 1830
   <211> 21
   <212> DNA
   <213> Homo sapiens
<400> 1830
   gcaaaggaaa caggctaact a 21
<210> 1831
   <211> 21
   <212> DNA
   <213> Homo sapiens
<400> 1831
   atggcagctg aatcgatatc t 21
<210> 1832
   <211> 21
   <212> DNA
   <213> Homo sapiens
<400> 1832
   taggtgatgg gcaaattctc a 21
<210> 1833
   <211> 22
   <212> DNA
   <213> Homo sapiens
<400> 1833
   gaaatgcctt cccacttaca at 22
<210> 1834
   <211> 21
   <212> DNA
   <213> Homo sapiens
<400> 1834
   attggcaaat gtacctgaag c 21
<210> 1835
   <211> 21
   <212> DNA
   <213> Homo sapiens
<400> 1835
   caatgattgc tcttgtgcca a 21
<210> 1836
   <211> 21
   <212> DNA
   <213> Homo sapiens
<400> 1836
   agctgtcctc ctctccatat a 21
<210> 1837
   <211> 21
   <212> DNA
   <213> Homo sapiens
<400> 1837
   ggcgttcaag ttactcgatt g 21
<210> 1838
   <211> 21
   <212> DNA
   <213> Homo sapiens
<400> 1838
   tagtgactag ctttggagag t 21
<210> 1839
   <211> 22
   <212> DNA
   <213> Homo sapiens
<400> 1839
   agccaatgat cccttatgac tt 22
<210> 1840
   <211> 21
   <212> DNA
   <213> Homo sapiens
<400> 1840
   aactaaactg gtaggcaatc g 21
<210> 1841
   <211> 21
   <212> DNA
   <213> Homo sapiens
<400> 1841
   caatcagacc acaggaagga a 21
<210> 1842
   <211> 21
   <212> DNA
   <213> Homo sapiens
<400> 1842
   gatgtgttta ttgcctgtgg t 21
<210> 1843
   <211> 21
   <212> DNA
   <213> Homo sapiens
<400> 1843
   gctcctttca tagtttcagg g 21
<210> 1844
   <211> 22
   <212> DNA
   <213> Homo sapiens
<400> 1844
   acacactgca gttctcacta ta 22
<210> 1845
   <211> 21
   <212> DNA
   <213> Homo sapiens
<400> 1845
   tgaatcaagt gacatgacag c 21
<210> 1846
   <211> 21
   <212> DNA
   <213> Homo sapiens
<400> 1846
   ttcttacagt cctcagcact t 21
<210> 1847
   <211> 21
   <212> DNA
   <213> Homo sapiens
<400> 1847
   ccactaggct gcactaatgt a 21
<210> 1848
   <211> 22
   <212> DNA
   <213> Homo sapiens
<400> 1848
   cactagcttc tgtaactgtg tg 22
<210> 1849
   <211> 21
   <212> DNA
   <213> Homo sapiens
<400> 1849
   gtctcacact gctcatttcc a 21
<210> 1850
   <211> 21
   <212> DNA
   <213> Homo sapiens
<400> 1850
   cactagggtt catcagctgt t 21
<210> 1851
   <211> 21
   <212> DNA
   <213> Homo sapiens
<400> 1851
   caaccaacat tagagtgacc c 21
<210> 1852
   <211> 21
   <212> DNA
   <213> Homo sapiens
<400> 1852
   gctggttgag agagagagag a 21
<210> 1853
   <211> 21
   <212> DNA
   <213> Homo sapiens
<400> 1853
   acaccagccg aatacagatt t 21
<210> 1854
   <211> 21
   <212> DNA
   <213> Homo sapiens
<400> 1854
   tgcttcatac ctttctgctt c 21
<210> 1855
   <211> 21
   <212> DNA
   <213> Homo sapiens
<400> 1855
   atttcccatg cctttcaact c 21
<210> 1856
   <211> 21
   <212> DNA
   <213> Homo sapiens
<400> 1856
   tctctcagta ggcgtcttta a 21
<210> 1857
   <211> 20
   <212> DNA
   <213> Homo sapiens
<400> 1857
   tgactgctac gctagacttg 20
<210> 1858
   <211> 20
   <212> DNA
   <213> Homo sapiens
<400> 1858
   ggatagagga aacccaggtg 20
<210> 1859
   <211> 21
   <212> DNA
   <213> Homo sapiens
<400> 1859
   ggctctttca aagtatccag g 21
<210> 1860
   <211> 22
   <212> DNA
   <213> Homo sapiens
<400> 1860
   agactttctt tgttgccttc ag 22
<210> 1861
   <211> 20
   <212> DNA
   <213> Homo sapiens
<400> 1861
   gcctcaacaa ttcagtccac 20
<210> 1862
   <211> 22
   <212> DNA
   <213> Homo sapiens
<400> 1862
   cataagttgc tggaagagaa ca 22
<210> 1863
   <211> 21
   <212> DNA
   <213> Homo sapiens
<400> 1863
   tcgactgctt taagtgaagg a 21
<210> 1864
   <211> 22
   <212> DNA
   <213> Homo sapiens
<400> 1864
   accagtgatt agtgtttctc ct 22
<210> 1865
   <211> 21
   <212> DNA
   <213> Homo sapiens
<400> 1865
   caagacacac acaaacacac a 21
<210> 1866
   <211> 21
   <212> DNA
   <213> Homo sapiens
<400> 1866
   ggtaagagtt gccctaatgt c 21
<210> 1867
   <211> 20
   <212> DNA
   <213> Homo sapiens
<400> 1867
   gcttggcttc tcacaaatgt 20
<210> 1868
   <211> 20
   <212> DNA
   <213> Homo sapiens
<400> 1868
   gtttgccagt agaaatggga 20
<210> 1869
   <211> 20
   <212> DNA
   <213> Homo sapiens
<400> 1869
   gtaaagtgtt cagaggacgg 20
<210> 1870
   <211> 21
   <212> DNA
   <213> Homo sapiens
<400> 1870
   actagggaga agaattggca g 21
<210> 1871
   <211> 20
   <212> DNA
   <213> Homo sapiens
<400> 1871
   ccagttcatt ccagcttcca 20
<210> 1872
   <211> 22
   <212> DNA
   <213> Homo sapiens
<400> 1872
   gatcgccaac ctgttttata ag 22
<210> 1873
   <211> 21
   <212> DNA
   <213> Homo sapiens
<400> 1873
   gctgtcaaag tggagataac c 21
<210> 1874
   <211> 21
   <212> DNA
   <213> Homo sapiens
<400> 1874
   aacgcttcca tccacctaat t 21
<210> 1875
   <211> 21
   <212> DNA
   <213> Homo sapiens
<400> 1875
   acctgcctgt cttaccatta a 21
<210> 1876
   <211> 23
   <212> DNA
   <213> Homo sapiens
<400> 1876
   gatactttcc tttctccaga tct 23
<210> 1877
   <211> 20
   <212> DNA
   <213> Homo sapiens
<400> 1877
   ggccgcagtt tttgatttag 20
<210> 1878
   <211> 19
   <212> DNA
   <213> Homo sapiens
<400> 1878
   tgggaaggac ggtttgtta 19
<210> 1879
   <211> 20
   <212> DNA
   <213> Homo sapiens
<400> 1879
   tgaatcatgc tgtggagaac 20
<210> 1880
   <211> 20
   <212> DNA
   <213> Homo sapiens
<400> 1880
   atagaagagg tacccagcaa 20
<210> 1881
   <211> 20
   <212> DNA
   <213> Homo sapiens
<400> 1881
   ccctgactat gctaagttgc 20
<210> 1882
   <211> 20
   <212> DNA
   <213> Homo sapiens
<400> 1882
   tgcaagcaaa atgaaaccag 20
<210> 1883
   <211> 18
   <212> DNA
   <213> Homo sapiens
<400> 1883
   aaaaaaaaaa aaaaaaaa 18
<210> 1884
   <211> 19
   <212> DNA
   <213> Homo sapiens
<400> 1884
   cagctgctcc ttctttagc 19
<210> 1885
   <211> 20
   <212> DNA
   <213> Homo sapiens
<400> 1885
   tatactgcca aaggtgacct 20
<210> 1886
   <211> 21
   <212> DNA
   <213> Homo sapiens
<400> 1886
   gaagcataat gagaacctcc a 21
<210> 1887
   <211> 20
   <212> DNA
   <213> Homo sapiens
<400> 1887
   aacccaactt gcagacaatc 20
<210> 1888
   <211> 20
   <212> DNA
   <213> Homo sapiens
<400> 1888
   atctgtttgc tgtgtcagaa 20
<210> 1889
   <211> 23
   <212> DNA
   <213> Homo sapiens
<400> 1889
   tccagatata ttcaaaaggg aga 23
<210> 1890
   <211> 20
   <212> DNA
   <213> Homo sapiens
<400> 1890
   aggtttttat caaagcccca 20
<210> 1891
   <211> 21
   <212> DNA
   <213> Homo sapiens
<400> 1891
   gggacttgat gttctaagca a 21
<210> 1892
   <211> 20
   <212> DNA
   <213> Homo sapiens
<400> 1892
   cctacatgat acgcacagtc 20
<210> 1893
   <211> 18
   <212> DNA
   <213> Homo sapiens
<400> 1893
   aaaaaaaaaa aaaaaaaa 18
<210> 1894
   <211> 20
   <212> DNA
   <213> Homo sapiens
<400> 1894
   gtttttctct acccagcaca 20
<210> 1895
   <211> 21
   <212> DNA
   <213> Homo sapiens
<400> 1895
   ctctaatttg ccaccctctt t 21
<210> 1896
   <211> 20
   <212> DNA
   <213> Homo sapiens
<400> 1896
   gaacgctaaa gcttttccca 20
<210> 1897
   <211> 20
   <212> DNA
   <213> Homo sapiens
<400> 1897
   tctttctcct gccaagtaga 20
<210> 1898
   <211> 20
   <212> DNA
   <213> Homo sapiens
<400> 1898
   ataacactgt ccttctgggc 20
<210> 1899
   <211> 20
   <212> DNA
   <213> Homo sapiens
<400> 1899
   gagcctactc tctgatacga 20
<210> 1900
   <211> 21
   <212> DNA
   <213> Homo sapiens
<400> 1900
   ccagttgttc acttctctga t 21
<210> 1901
   <211> 20
   <212> DNA
   <213> Homo sapiens
<400> 1901
   gccttaaaac caaactgtgt 20
<210> 1902
   <211> 20
   <212> DNA
   <213> Homo sapiens
<400> 1902
   ctggacttca atcacccaag 20
<210> 1903
   <211> 20
   <212> DNA
   <213> Homo sapiens
<400> 1903
   tgaacaaatt gctgtgctga 20
<210> 1904
   <211> 20
   <212> DNA
   <213> Homo sapiens
<400> 1904
   ccccctaaat gaaagtggtc 20
<210> 1905
   <211> 18
   <212> DNA
   <213> Homo sapiens
<400> 1905
   aaaaaaaaaa aaaaaaaa 18
<210> 1906
   <211> 20
   <212> DNA
   <213> Homo sapiens
<400> 1906
   tgagtgcttg gaattttgca 20
<210> 1907
   <211> 20
   <212> DNA
   <213> Homo sapiens
<400> 1907
   ggttggctac ttcatggtac 20
<210> 1908
   <211> 20
   <212> DNA
   <213> Homo sapiens
<400> 1908
   tcactgcatt cctagaacct 20
<210> 1909
   <211> 20
   <212> DNA
   <213> Homo sapiens
<400> 1909
   ataactcagg caaaatgggg 20
<210> 1910
   <211> 20
   <212> DNA
   <213> Homo sapiens
<400> 1910
   ccttctgctc tcactttacg 20
<210> 1911
   <211> 20
   <212> DNA
   <213> Homo sapiens
<400> 1911
   ggatactagc agaggtggag 20
<210> 1912
   <211> 21
   <212> DNA
   <213> Homo sapiens
<400> 1912
   tgtacacaat atgccaggaa c 21
<210> 1913
   <211> 20
   <212> DNA
   <213> Homo sapiens
<400> 1913
   gcacttgagt tatgggactt 20
<210> 1914
   <211> 23
   <212> DNA
   <213> Homo sapiens
<400> 1914
   tgtgatatgt agtgtgtatc agt 23
<210> 1915
   <211> 20
   <212> DNA
   <213> Homo sapiens
<400> 1915
   gaatgtgttc aaaggagggt 20
<210> 1916
   <211> 20
   <212> DNA
   <213> Homo sapiens
<400> 1916
   gatgaagggg attacgggaa 20
<210> 1917
   <211> 20
   <212> DNA
   <213> Homo sapiens
<400> 1917
   tttaggaagc agcacaagaa 20
<210> 1918
   <211> 18
   <212> DNA
   <213> Homo sapiens
<400> 1918
   gagggtgctg gggttatc 18
<210> 1919
   <211> 20
   <212> DNA
   <213> Homo sapiens
<400> 1919
   cccgaaagca cttacctttt 20
<210> 1920
   <211> 20
   <212> DNA
   <213> Homo sapiens
<400> 1920
   ttcatgagct gcaatgtgtt 20
<210> 1921
   <211> 20
   <212> DNA
   <213> Homo sapiens
<400> 1921
   tgctatgaaa agagggacca 20
<210> 1922
   <211> 20
   <212> DNA
   <213> Homo sapiens
<400> 1922
   gtcctggatc tacacgtgaa 20
<210> 1923
   <211> 20
   <212> DNA
   <213> Homo sapiens
<400> 1923
   gtaaatctgt gtgccagcaa 20
<210> 1924
   <211> 20
   <212> DNA
   <213> Homo sapiens
<400> 1924
   taaaagaggc gtgtggaaaa 20
<210> 1925
   <211> 20
   <212> DNA
   <213> Homo sapiens
<400> 1925
   ccagtagctt gtgatgtgta 20
<210> 1926
   <211> 20
   <212> DNA
   <213> Homo sapiens
<400> 1926
   tacaggcctc tgaaagatga 20
<210> 1927
   <211> 20
   <212> DNA
   <213> Homo sapiens
<400> 1927
   agagcatgct agacgtcttt 20
<210> 1928
   <211> 20
   <212> DNA
   <213> Homo sapiens
<400> 1928
   ctgggctgta attaaggctc 20
<210> 1929
   <211> 20
   <212> DNA
   <213> Homo sapiens
<400> 1929
   aacacctgca cactttgaaa 20
<210> 1930
   <211> 20
   <212> DNA
   <213> Homo sapiens
<400> 1930
   aatgaacttt gtgggctgaa 20
<210> 1931
   <211> 20
   <212> DNA
   <213> Homo sapiens
<400> 1931
   ccacactctc actggttcta 20
<210> 1932
   <211> 20
   <212> DNA
   <213> Homo sapiens
<400> 1932
   cacagtggat acctcaggaa 20
<210> 1933
   <211> 20
   <212> DNA
   <213> Homo sapiens
<400> 1933
   ccaacacagg aggaactttt 20
<210> 1934
   <211> 22
   <212> DNA
   <213> Homo sapiens
<400> 1934
   gagaaaagcc aacaaaaatg tg 22
<210> 1935
   <211> 22
   <212> DNA
   <213> Homo sapiens
<400> 1935
   tgtcattact agaagcacct tt 22
<210> 1936
   <211> 23
   <212> DNA
   <213> Homo sapiens
<400> 1936
   tggtgtgagt tcaggagggt tta 23
<210> 1937
   <211> 20
   <212> DNA
   <213> Homo sapiens
<400> 1937
   accatttctg acagaacaga 20
<210> 1938
   <211> 20
   <212> DNA
   <213> Homo sapiens
<400> 1938
   tgatgcttaa acacatgcct 20
<210> 1939
   <211> 20
   <212> DNA
   <213> Homo sapiens
<400> 1939
   aagggatatg cagcttgttc 20
<210> 1940
   <211> 18
   <212> DNA
   <213> Homo sapiens
<400> 1940
   aaaaaaaaaa aaaaaaaa 18
<210> 1941
   <211> 22
   <212> DNA
   <213> Homo sapiens
<400> 1941
   gttgtcattc aaatgtcacc ac 22
<210> 1942
   <211> 19
   <212> DNA
   <213> Homo sapiens
<400> 1942
   ctcagattcc agagccctc 19
<210> 1943
   <211> 20
   <212> DNA
   <213> Homo sapiens
<400> 1943
   acattgctag cagcttttgt 20
<210> 1944
   <211> 21
   <212> DNA
   <213> Homo sapiens
<400> 1944
   acttgccaag aacagtatct g 21
<210> 1945
   <211> 20
   <212> DNA
   <213> Homo sapiens
<400> 1945
   ttccttcttc caggtgaaca 20
<210> 1946
   <211> 20
   <212> DNA
   <213> Homo sapiens
<400> 1946
   tgtttctcct tcatctggtc 20
<210> 1947
   <211> 20
   <212> DNA
   <213> Homo sapiens
<400> 1947
   tgtcaccttg cagatacagg 20
<210> 1948
   <211> 20
   <212> DNA
   <213> Homo sapiens
<400> 1948
   attgttcaca gggtcaagtc 20
<210> 1949
   <211> 20
   <212> DNA
   <213> Homo sapiens
<400> 1949
   atgagccagg agaatcatca 20
<210> 1950
   <211> 20
   <212> DNA
   <213> Homo sapiens
<400> 1950
   catcatttca aaggggctca 20
<210> 1951
   <211> 20
   <212> DNA
   <213> Homo sapiens
<400> 1951
   tcttctctaa cacccactcc 20
<210> 1952
   <211> 20
   <212> DNA
   <213> Homo sapiens
<400> 1952
   acccttacca aagtagcatc 20
<210> 1953
   <211> 20
   <212> DNA
   <213> Homo sapiens
<400> 1953
   atcacgacgc cttgtttatt 20
<210> 1954
   <211> 20
   <212> DNA
   <213> Homo sapiens
<400> 1954
   actttcctgc caacaatctc 20
<210> 1955
   <211> 20
   <212> DNA
   <213> Homo sapiens
<400> 1955
   ttgatagttg catctgggga 20
<210> 1956
   <211> 22
   <212> DNA
   <213> Homo sapiens
<400> 1956
   tttgttaact aacgtgattc ca 22
<210> 1957
   <211> 20
   <212> DNA
   <213> Homo sapiens
<400> 1957
   cccactctcc atgtgttctt 20
<210> 1958
   <211> 20
   <212> DNA
   <213> Homo sapiens
<400> 1958
   tcctgcttca actcaatacg 20
<210> 1959
   <211> 20
   <212> DNA
   <213> Homo sapiens
<400> 1959
   acccctcatt ttcgtatgtc 20
<210> 1960
   <211> 20
   <212> DNA
   <213> Homo sapiens
<400> 1960
   acagttatgg aggaattgcg 20
<210> 1961
   <211> 22
   <212> DNA
   <213> Homo sapiens
<400> 1961
   tcaaacctct tttatctgtc cc 22
<210> 1962
   <211> 20
   <212> DNA
   <213> Homo sapiens
<400> 1962
   atttcacgta acactctggt 20
<210> 1963
   <211> 21
   <212> DNA
   <213> Homo sapiens
<400> 1963
   ctctggcaaa actttctgga t 21
<210> 1964
   <211> 19
   <212> DNA
   <213> Homo sapiens
<400> 1964
   gcttctcatg ctcacactg 19
<210> 1965
   <211> 21
   <212> DNA
   <213> Homo sapiens
<400> 1965
   aatatgactt gcccttttga a 21
<210> 1966
   <211> 19
   <212> DNA
   <213> Homo sapiens
<400> 1966
   tcacagccag ttacacaga 19
<210> 1967
   <211> 20
   <212> DNA
   <213> Homo sapiens
<400> 1967
   ctacagtgca gaagagtccc 20
<210> 1968
   <211> 20
   <212> DNA
   <213> Homo sapiens
<400> 1968
   ttggtttcat gtggctttga 20
<210> 1969
   <211> 20
   <212> DNA
   <213> Homo sapiens
<400> 1969
   tccttctccc caactttctt 20
<210> 1970
   <211> 21
   <212> DNA
   <213> Homo sapiens
<400> 1970
   ctttagattc cagggctctt g 21
<210> 1971
   <211> 20
   <212> DNA
   <213> Homo sapiens
<400> 1971
   tgaaagcgtg aaaatcagct 20
<210> 1972
   <211> 20
   <212> DNA
   <213> Homo sapiens
<400> 1972
   actcgatccc taggtaatgt 20
<210> 1973
   <211> 20
   <212> DNA
   <213> Homo sapiens
<400> 1973
   acaagtgggt agggatgttc 20
<210> 1974
   <211> 20
   <212> DNA
   <213> Homo sapiens
<400> 1974
   gcacaagttt tcagggaatg 20
<210> 1975
   <211> 20
   <212> DNA
   <213> Homo sapiens
<400> 1975
   gtggaaagtc tcgtcagaat 20
<210> 1976
   <211> 20
   <212> DNA
   <213> Homo sapiens
<400> 1976
   tccacctctg agcataacat 20
<210> 1977
   <211> 18
   <212> DNA
   <213> Homo sapiens
<400> 1977
   aaaaaaaaaa aaaaaaaa 18
<210> 1978
   <211> 18
   <212> DNA
   <213> Homo sapiens
<400> 1978
   aaaaaaaaaa aaaaaaaa 18
<210> 1979
   <211> 20
   <212> DNA
   <213> Homo sapiens
<400> 1979
   ccatcccact tctccagata 20
<210> 1980
   <211> 20
   <212> DNA
   <213> Homo sapiens
<400> 1980
   ccaccttcct gcttaaagaa 20
<210> 1981
   <211> 20
   <212> DNA
   <213> Homo sapiens
<400> 1981
   ctccttactt gcactgagtt 20
<210> 1982
   <211> 20
   <212> DNA
   <213> Homo sapiens
<400> 1982
   gggaaaactt acgggaactt 20
<210> 1983
   <211> 22
   <212> DNA
   <213> Homo sapiens
<400> 1983
   tagaaatgtt tagggtgcat ga 22
<210> 1984
   <211> 20
   <212> DNA
   <213> Homo sapiens
<400> 1984
   tacccctctt ttccatctgc 20
<210> 1985
   <211> 20
   <212> DNA
   <213> Homo sapiens
<400> 1985
   gcattttgac aggaaagtgg 20
<210> 1986
   <211> 20
   <212> DNA
   <213> Homo sapiens
<400> 1986
   tagggccaca gtttctcaat 20
<210> 1987
   <211> 20
   <212> DNA
   <213> Homo sapiens
<400> 1987
   gaatgagaaa tctggcagga 20
<210> 1988
   <211> 20
   <212> DNA
   <213> Homo sapiens
<400> 1988
   tcagatgcct gatgaccata 20
<210> 1989
   <211> 20
   <212> DNA
   <213> Homo sapiens
<400> 1989
   aatatgcagt gggtaggagc 20
<210> 1990
   <211> 20
   <212> DNA
   <213> Homo sapiens
<400> 1990
   gacaacagct gacttccatt 20
<210> 1991
   <211> 20
   <212> DNA
   <213> Homo sapiens
<400> 1991
   cactctctgg aacaaacaca 20
<210> 1992
   <211> 20
   <212> DNA
   <213> Homo sapiens
<400> 1992
   gttttgaggc ggtttcatga 20
<210> 1993
   <211> 18
   <212> DNA
   <213> Homo sapiens
<400> 1993
   aaaaaaaaaa aaaaaaaa 18
<210> 1994
   <211> 20
   <212> DNA
   <213> Homo sapiens
<400> 1994
   gaacacctca aagttgctca 20
<210> 1995
   <211> 20
   <212> DNA
   <213> Homo sapiens
<400> 1995
   aagaagcaag gacaaggatg 20
<210> 1996
   <211> 20
   <212> DNA
   <213> Homo sapiens
<400> 1996
   gaaacaacca ccacaacaaa 20
<210> 1997
   <211> 20
   <212> DNA
   <213> Homo sapiens
<400> 1997
   ccaccatgtt tacaccgttt 20
<210> 1998
   <211> 20
   <212> DNA
   <213> Homo sapiens
<400> 1998
   tttacccatg aattgctgca 20
<210> 1999
   <211> 20
   <212> DNA
   <213> Homo sapiens
<400> 1999
   agtttgcatt tgttcaggga 20
<210> 2000
   <211> 20
   <212> DNA
   <213> Homo sapiens
<400> 2000
   ttcggatggc tttgattgtc 20
<210> 2001
   <211> 21
   <212> DNA
   <213> Homo sapiens
<400> 2001
   cattgtaaat taaacggcct c 21
<210> 2002
   <211> 20
   <212> DNA
   <213> Homo sapiens
<400> 2002
   ctaatgcaag ctgcttctct 20
<210> 2003
   <211> 20
   <212> DNA
   <213> Homo sapiens
<400> 2003
   tgtctagtgg taatctgggg 20
<210> 2004
   <211> 20
   <212> DNA
   <213> Homo sapiens
<400> 2004
   tctggacagt ggagttgaaa 20
<210> 2005
   <211> 18
   <212> DNA
   <213> Homo sapiens
<400> 2005
   aaaaaaaaaa aaaaaaaa 18
<210> 2006
   <211> 20
   <212> DNA
   <213> Homo sapiens
<400> 2006
   aggagagaca taactggtct 20
<210> 2007
   <211> 20
   <212> DNA
   <213> Homo sapiens
<400> 2007
   attgcaatgt ctgtggatgt 20
<210> 2008
   <211> 19
   <212> DNA
   <213> Homo sapiens
<400> 2008
   aagaagttga ggtagcacg 19
<210> 2009
   <211> 20
   <212> DNA
   <213> Homo sapiens
<400> 2009
   agtgtccttt cctccagttc 20
<210> 2010
   <211> 21
   <212> DNA
   <213> Homo sapiens
<400> 2010
   ttgaacctga aaggaactgt g 21
<210> 2011
   <211> 20
   <212> DNA
   <213> Homo sapiens
<400> 2011
   gctcaatcac ctgttccctt 20
<210> 2012
   <211> 21
   <212> DNA
   <213> Homo sapiens
<400> 2012
   cattgaagct cactctaagg g 21
<210> 2013
   <211> 20
   <212> DNA
   <213> Homo sapiens
<400> 2013
   ctaaagtttg ggttaggggt 20
<210> 2014
   <211> 20
   <212> DNA
   <213> Homo sapiens
<400> 2014
   tgccttacat tttctgtggg 20
<210> 2015
   <211> 20
   <212> DNA
   <213> Homo sapiens
<400> 2015
   gccataagat ttccccactc 20
<210> 2016
   <211> 21
   <212> DNA
   <213> Homo sapiens
<400> 2016
   acaaagcaag aggatgaaac a 21
<210> 2017
   <211> 20
   <212> DNA
   <213> Homo sapiens
<400> 2017
   caaagcacca tcaacactta 20
<210> 2018
   <211> 20
   <212> DNA
   <213> Homo sapiens
<400> 2018
   cactgcaact cctagaatga 20
<210> 2019
   <211> 20
   <212> DNA
   <213> Homo sapiens
<400> 2019
   tgtggggaaa ttgctgttta 20
<210> 2020
   <211> 20
   <212> DNA
   <213> Homo sapiens
<400> 2020
   gtatgggcag ctgtaacttg 20
<210> 2021
   <211> 20
   <212> DNA
   <213> Homo sapiens
<400> 2021
   tcacaagcca ctgaaaatgt 20
<210> 2022
   <211> 21
   <212> DNA
   <213> Homo sapiens
<400> 2022
   tgtatgttaa gctagccaac a 21
<210> 2023
   <211> 20
   <212> DNA
   <213> Homo sapiens
<400> 2023
   tgtgttattg aactttgcca 20
<210> 2024
   <211> 20
   <212> DNA
   <213> Homo sapiens
<400> 2024
   ttcatcccta cctcatcacc 20
<210> 2025
   <211> 21
   <212> DNA
   <213> Homo sapiens
<400> 2025
   agatcacttt tggctgtaac c 21
<210> 2026
   <211> 20
   <212> DNA
   <213> Homo sapiens
<400> 2026
   caagtgacaa tctcagccaa 20
<210> 2027
   <211> 20
   <212> DNA
   <213> Homo sapiens
<400> 2027
   gcattttcag atggttccct 20
<210> 2028
   <211> 20
   <212> DNA
   <213> Homo sapiens
<400> 2028
   atggagagtt tgagtggagt 20
<210> 2029
   <211> 22
   <212> DNA
   <213> Homo sapiens
<400> 2029
   ctgccactgg gtttatagaa aa 22
<210> 2030
   <211> 20
   <212> DNA
   <213> Homo sapiens
<400> 2030
   acacctttac tcctgtggat 20
<210> 2031
   <211> 20
   <212> DNA
   <213> Homo sapiens
<400> 2031
   gatgtagggc cttatccaca 20
<210> 2032
   <211> 20
   <212> DNA
   <213> Homo sapiens
<400> 2032
   agcttggtga tcttcaaaca 20
<210> 2033
   <211> 20
   <212> DNA
   <213> Homo sapiens
<400> 2033
   tgaaatggtg ggtaatgctc 20
<210> 2034
   <211> 20
   <212> DNA
   <213> Homo sapiens
<400> 2034
   catgcaagtt cacgaggtta 20
<210> 2035
   <211> 20
   <212> DNA
   <213> Homo sapiens
<400> 2035
   agtctgagga agaagcaact 20
<210> 2036
   <211> 20
   <212> DNA
   <213> Homo sapiens
<400> 2036
   gagtccaatc ttttcccaca 20
<210> 2037
   <211> 20
   <212> DNA
   <213> Homo sapiens
<400> 2037
   tccactgcgt tcttatcctt 20
<210> 2038
   <211> 20
   <212> DNA
   <213> Homo sapiens
<400> 2038
   ggacagaaca gctacaaagg 20
<210> 2039
   <211> 20
   <212> DNA
   <213> Homo sapiens
<400> 2039
   ctggcttgtg aattagaggg 20
<210> 2040
   <211> 19
   <212> DNA
   <213> Homo sapiens
<400> 2040
   gtaagatggt gggcaggat 19
<210> 2041
   <211> 20
   <212> DNA
   <213> Homo sapiens
<400> 2041
   agaagggctc aaaacacatc 20
<210> 2042
   <211> 21
   <212> DNA
   <213> Homo sapiens
<400> 2042
   tcatgtaggc tttctgattt t 21
<210> 2043
   <211> 20
   <212> DNA
   <213> Homo sapiens
<400> 2043
   tgtagctctt gacctagcaa 20
<210> 2044
   <211> 21
   <212> DNA
   <213> Homo sapiens
<400> 2044
   taagttcacg gtgaagtcaa c 21
<210> 2045
   <211> 20
   <212> DNA
   <213> Homo sapiens
<400> 2045
   gtggcttcaa ctaactggac 20
<210> 2046
   <211> 20
   <212> DNA
   <213> Homo sapiens
<400> 2046
   acccgtaagt gtttgagtga 20
<210> 2047
   <211> 20
   <212> DNA
   <213> Homo sapiens
<400> 2047
   tgtagaagta gggtttgcgt 20
<210> 2048
   <211> 20
   <212> DNA
   <213> Homo sapiens
<400> 2048
   caggggacat ttgaagatgg 20
<210> 2049
   <211> 20
   <212> DNA
   <213> Homo sapiens
<400> 2049
   gaaattgtgc agtgaaagca 20
<210> 2050
   <211> 21
   <212> DNA
   <213> Homo sapiens
<400> 2050
   atgtaagaag tgcgttgctt a 21
<210> 2051
   <211> 21
   <212> DNA
   <213> Homo sapiens
<400> 2051
   agatctggaa tctgagactc c 21
<210> 2052
   <211> 22
   <212> DNA
   <213> Homo sapiens
<400> 2052
   tataggataa ggtcaagcag gt 22
<210> 2053
   <211> 18
   <212> DNA
   <213> Homo sapiens
<400> 2053
   aaaaaaaaaa aaaaaaaa 18
<210> 2054
   <211> 21
   <212> DNA
   <213> Homo sapiens
<400> 2054
   tcagtgaatg aggaatcatg c 21
<210> 2055
   <211> 20
   <212> DNA
   <213> Homo sapiens
<400> 2055
   ttctcaggag taccacaagc 20
<210> 2056
   <211> 20
   <212> DNA
   <213> Homo sapiens
<400> 2056
   ggatgaaaca cagaaccatg 20
<210> 2057
   <211> 20
   <212> DNA
   <213> Homo sapiens
<400> 2057
   cagatccctt tcattttgca 20
<210> 2058
   <211> 20
   <212> DNA
   <213> Homo sapiens
<400> 2058
   ggaaaagtgc tcaattaggc 20
<210> 2059
   <211> 20
   <212> DNA
   <213> Homo sapiens
<400> 2059
   ctggcagtta tagtcaccaa 20
<210> 2060
   <211> 20
   <212> DNA
   <213> Homo sapiens
<400> 2060
   aggaagtcct tatgatgcca 20
<210> 2061
   <211> 20
   <212> DNA
   <213> Homo sapiens
<400> 2061
   gctggtgaag ttggagtttt 20
<210> 2062
   <211> 20
   <212> DNA
   <213> Homo sapiens
<400> 2062
   gctgggttta agccacatat 20
<210> 2063
   <211> 20
   <212> DNA
   <213> Homo sapiens
<400> 2063
   tgtagcctaa atagcagcct 20
<210> 2064
   <211> 23
   <212> DNA
   <213> Homo sapiens
<400> 2064
   aggaattgct tttattttaa cca 23
<210> 2065
   <211> 20
   <212> DNA
   <213> Homo sapiens
<400> 2065
   gctgctttca ggtttttgtg 20
<210> 2066
   <211> 20
   <212> DNA
   <213> Homo sapiens
<400> 2066
   atggtgcaga aaagagcaat 20
<210> 2067
   <211> 20
   <212> DNA
   <213> Homo sapiens
<400> 2067
   gtgtggtgcc attttctttc 20
<210> 2068
   <211> 20
   <212> DNA
   <213> Homo sapiens
<400> 2068
   gaacaatact ttctccccgg 20
<210> 2069
   <211> 20
   <212> DNA
   <213> Homo sapiens
<400> 2069
   gaaatggcca tgctaggaat 20
<210> 2070
   <211> 20
   <212> DNA
   <213> Homo sapiens
<400> 2070
   acttccagta acatggatgc 20
<210> 2071
   <211> 20
   <212> DNA
   <213> Homo sapiens
<400> 2071
   gaaacaccca gacttgtagc 20
<210> 2072
   <211> 20
   <212> DNA
   <213> Homo sapiens
<400> 2072
   ttaaatcttt gtgtgcgtgt 20
<210> 2073
   <211> 20
   <212> DNA
   <213> Homo sapiens
<400> 2073
   cctccaggaa ctttgttcag 20
<210> 2074
   <211> 20
   <212> DNA
   <213> Homo sapiens
<400> 2074
   aaaaaccttc acaaacccca 20
<210> 2075
   <211> 20
   <212> DNA
   <213> Homo sapiens
<400> 2075
   gagtggatat tgtctcgctg 20
<210> 2076
   <211> 20
   <212> DNA
   <213> Homo sapiens
<400> 2076
   actagcccac taatgttgct 20
<210> 2077
   <211> 22
   <212> DNA
   <213> Homo sapiens
<400> 2077
   cgattatcag aacagatgag gt 22
<210> 2078
   <211> 20
   <212> DNA
   <213> Homo sapiens
<400> 2078
   attgcacagc tgaaaatcct 20
<210> 2079
   <211> 20
   <212> DNA
   <213> Homo sapiens
<400> 2079
   ggcacactga ccgtatttat 20
<210> 2080
   <211> 20
   <212> DNA
   <213> Homo sapiens
<400> 2080
   tggtagtggg tcaggaattt 20
<210> 2081
   <211> 20
   <212> DNA
   <213> Homo sapiens
<400> 2081
   tctcttgaaa agaaaggcgg 20
<210> 2082
   <211> 20
   <212> DNA
   <213> Homo sapiens
<400> 2082
   tgaattacac agcaaagccc 20
<210> 2083
   <211> 20
   <212> DNA
   <213> Homo sapiens
<400> 2083
   ctgccaatgg gatcgaattt 20
<210> 2084
   <211> 20
   <212> DNA
   <213> Homo sapiens
<400> 2084
   gagtgacgct gttcattctt 20
<210> 2085
   <211> 20
   <212> DNA
   <213> Homo sapiens
<400> 2085
   gatagtaacc gggtgtagca 20
<210> 2086
   <211> 18
   <212> DNA
   <213> Homo sapiens
<400> 2086
   aaaaaaaaaa aaaaaaaa 18
<210> 2087
   <211> 23
   <212> DNA
   <213> Homo sapiens
<400> 2087
   tctaataagg gattgatgga gtt 23
<210> 2088
   <211> 20
   <212> DNA
   <213> Homo sapiens
<400> 2088
   gggagatttc ctgcttgtag 20
<210> 2089
   <211> 20
   <212> DNA
   <213> Homo sapiens
<400> 2089
   aatccatgca gcttctctct 20
<210> 2090
   <211> 20
   <212> DNA
   <213> Homo sapiens
<400> 2090
   tgcattgtct ctggtttgaa 20
<210> 2091
   <211> 20
   <212> DNA
   <213> Homo sapiens
<400> 2091
   tcctgaatgc atccttaacc 20
<210> 2092
   <211> 20
   <212> DNA
   <213> Homo sapiens
<400> 2092
   tgggaaaggt gagaaggatt 20
<210> 2093
   <211> 20
   <212> DNA
   <213> Homo sapiens
<400> 2093
   ttcgggaccc atacctaaaa 20
<210> 2094
   <211> 20
   <212> DNA
   <213> Homo sapiens
<400> 2094
   tcttttctgg acccacatga 20
<210> 2095
   <211> 22
   <212> DNA
   <213> Homo sapiens
<400> 2095
   gacctctaca tctgtatctt cc 22
<210> 2096
   <211> 20
   <212> DNA
   <213> Homo sapiens
<400> 2096
   cccttcattt tctgtcccat 20
<210> 2097
   <211> 21
   <212> DNA
   <213> Homo sapiens
<400> 2097
   tcagccttga gtattagcct a 21
<210> 2098
   <211> 20
   <212> DNA
   <213> Homo sapiens
<400> 2098
   aagtttgcca tgaaggtcat 20
<210> 2099
   <211> 20
   <212> DNA
   <213> Homo sapiens
<400> 2099
   gacttctggt tgtttcctca 20

<210> 2100
   <211> 20
   <212> DNA
   <213> Homo sapiens
<400> 2100
   atgtgtctat tgccctacct 20
<210> 2101
   <211> 19
   <212> DNA
   <213> Homo sapiens
<400> 2101
   tgcgggaagt tcacatgaa 19
<210> 2102
   <211> 19
   <212> DNA
   <213> Homo sapiens
<400> 2102
   gtgaacttca ggctgctta 19
<210> 2103
   <211> 20
   <212> DNA
   <213> Homo sapiens
<400> 2103
   tggtttcgtc ccgtaaatag 20
<210> 2104
   <211> 20
   <212> DNA
   <213> Homo sapiens
<400> 2104
   aattcctttc aatgctggct 20
<210> 2105
   <211> 21
   <212> DNA
   <213> Homo sapiens
<400> 2105
   agaaagacac atatgccatg g 21
<210> 2106
   <211> 20
   <212> DNA
   <213> Homo sapiens
<400> 2106
   ttgttggtgt cagttctgaa 20
<210> 2107
   <211> 20
   <212> DNA
   <213> Homo sapiens
<400> 2107
   tgtaggaaca gattagggca 20
<210> 2108
   <211> 20
   <212> DNA
   <213> Homo sapiens
<400> 2108
   ctccaagctg atatgccatc 20
<210> 2109
   <211> 20
   <212> DNA
   <213> Homo sapiens
<400> 2109
   ccacccctca tttcttcctt 20
<210> 2110
   <211> 20
   <212> DNA
   <213> Homo sapiens
<400> 2110
   gggtccttcg ttttctgttt 20
<210> 2111
   <211> 21
   <212> DNA
   <213> Homo sapiens
<400> 2111
   taggaaacag gctaaaaggg a 21
<210> 2112
   <211> 20
   <212> DNA
   <213> Homo sapiens
<400> 2112
   tactgtgtag aaggcagtgt 20
<210> 2113
   <211> 20
   <212> DNA
   <213> Homo sapiens
<400> 2113
   gctaaggaca aagaaccact 20
<210> 2114
   <211> 18
   <212> DNA
   <213> Homo sapiens
<400> 2114
   aaaaaaaaaa aaaaaaaa 18
<210> 2115
   <211> 20
   <212> DNA
   <213> Homo sapiens
<400> 2115
   tggctaagac caggattgtt 20
<210> 2116
   <211> 18
   <212> DNA
   <213> Homo sapiens
<400> 2116
   aaaaaaaaaa aaaaaaaa 18
<210> 2117
   <211> 20
   <212> DNA
   <213> Homo sapiens
<400> 2117
   tgataggcag atcattcccc 20
<210> 2118
   <211> 20
   <212> DNA
   <213> Homo sapiens
<400> 2118
   gcaaatggca aagggaaaac 20
<210> 2119
   <211> 19
   <212> DNA
   <213> Homo sapiens
<400> 2119
   cacggctagt gctcatttt 19
<210> 2120
   <211> 21
   <212> DNA
   <213> Homo sapiens
<400> 2120
   ccgtaatacc caagtcatct g 21
<210> 2121
   <211> 22
   <212> DNA
   <213> Homo sapiens
<400> 2121
   agcaaactaa aacagcaact tc 22
<210> 2122
   <211> 19
   <212> DNA
   <213> Homo sapiens
<400> 2122
   agcctgctat cttcactgg 19
<210> 2123
   <211> 23
   <212> DNA
   <213> Homo sapiens
<400> 2123
   accacatata tagagacttt gaa 23
<210> 2124
   <211> 19
   <212> DNA
   <213> Homo sapiens
<400> 2124
   ctccagactc tgcaaggat 19
<210> 2125
   <211> 20
   <212> DNA
   <213> Homo sapiens
<400> 2125
   tttcctgttg ctcttgatca 20
<210> 2126
   <211> 20
   <212> DNA
   <213> Homo sapiens
<400> 2126
   ccacaaagat gaaggccaag 20
<210> 2127
   <211> 21
   <212> DNA
   <213> Homo sapiens
<400> 2127
   ccatacctta gttctcaggg t 21
<210> 2128
   <211> 20
   <212> DNA
   <213> Homo sapiens
<400> 2128
   gtaagagaga gctgggacaa 20
<210> 2129
   <211> 20
   <212> DNA
   <213> Homo sapiens
<400> 2129
   tgtgaccatc ctatccacaa 20
<210> 2130
   <211> 20
   <212> DNA
   <213> Homo sapiens
<400> 2130
   agaaccagtt gtacgagttc 20
<210> 2131
   <211> 20
   <212> DNA
   <213> Homo sapiens
<400> 2131
   agtcttctcc cttccttgtc 20
<210> 2132
   <211> 20
   <212> DNA
   <213> Homo sapiens
<400> 2132
   gcccttttct ctctttgacc 20
<210> 2133
   <211> 21
   <212> DNA
   <213> Homo sapiens
<400> 2133
   tcttgttcca agtattcctg g 21
<210> 2134
   <211> 20
   <212> DNA
   <213> Homo sapiens
<400> 2134
   accactttag cccatctctt 20
<210> 2135
   <211> 20
   <212> DNA
   <213> Homo sapiens
<400> 2135
   gtggtggatt attgagctgg 20
<210> 2136
   <211> 21
   <212> DNA
   <213> Homo sapiens
<400> 2136
   tgcttaatgg gatcattgac c 21
<210> 2137
   <211> 20
   <212> DNA
   <213> Homo sapiens
<400> 2137
   cccacatagt gcaaaagaca 20
<210> 2138
   <211> 20
   <212> DNA
   <213> Homo sapiens
<400> 2138
   gtatgtgtga agtagccgag 20
<210> 2139
   <211> 20
   <212> DNA
   <213> Homo sapiens
<400> 2139
   tggtcctaac tcagaccttt 20
<210> 2140
   <211> 20
   <212> DNA
   <213> Homo sapiens
<400> 2140
   aacatgaagg gaaggttgtg 20
<210> 2141
   <211> 21
   <212> DNA
   <213> Homo sapiens
<400> 2141
   tatacttcaa cttgcaggca g 21
<210> 2142
   <211> 21
   <212> DNA
   <213> Homo sapiens
<400> 2142
   gccaaagaca atgagagagt c 21
<210> 2143
   <211> 21
   <212> DNA
   <213> Homo sapiens
<400> 2143
   atttagcagc catgaccagt a 21
<210> 2144
   <211> 21
   <212> DNA
   <213> Homo sapiens
<400> 2144
   tgggccaatt cctaatccat t 21
<210> 2145
   <211> 23
   <212> DNA
   <213> Homo sapiens
<400> 2145
   cgatctcatg aataagtctg acc 23
<210> 2146
   <211> 21
   <212> DNA
   <213> Homo sapiens
<400> 2146
   tggaaagcag actaacagtg a 21
<210> 2147
   <211> 21
   <212> DNA
   <213> Homo sapiens
<400> 2147
   gggaccctat gtagagattg t 21
<210> 2148
   <211> 21
   <212> DNA
   <213> Homo sapiens
<400> 2148
   ggagcccaag gatgtattag a 21
<210> 2149
   <211> 21
   <212> DNA
   <213> Homo sapiens
<400> 2149
   ctaatagctg gttctgcaca c 21
<210> 2150
   <211> 22
   <212> DNA
   <213> Homo sapiens
<400> 2150
   gatgaaatga atgctgactc tc 22
<210> 2151
   <211> 21
   <212> DNA
   <213> Homo sapiens
<400> 2151
   aaactgaagc ttcgagaacc c 21
<210> 2152
   <211> 21
   <212> DNA
   <213> Homo sapiens
<400> 2152
   tgccgacaac tactttaggt a 21
<210> 2153
   <211> 21
   <212> DNA
   <213> Homo sapiens
<400> 2153
   tgtatccaat cacctgtcag a 21
<210> 2154
   <211> 21
   <212> DNA
   <213> Homo sapiens
<400> 2154
   cagcacctta agcagaaatc a 21
<210> 2155
   <211> 21
   <212> DNA
   <213> Homo sapiens
<400> 2155
   aagcccttca tccatttctc t 21
<210> 2156
   <211> 21
   <212> DNA
   <213> Homo sapiens
<400> 2156
   atcttggtgc catcttaagg t 21
<210> 2157
   <211> 21
   <212> DNA
   <213> Homo sapiens
<400> 2157
   agcaaaccaa tcgcaaacta g 21
<210> 2158
   <211> 22
   <212> DNA
   <213> Homo sapiens
<400> 2158
   aatcatcatc ttcatcagct cg 22
<210> 2159
   <211> 21
   <212> DNA
   <213> Homo sapiens
<400> 2159
   cctttcgcct tgcttatatg g 21
<210> 2160
   <211> 21
   <212> DNA
   <213> Homo sapiens
<400> 2160
   actactcaac agcctaccaa a 21
<210> 2161
   <211> 21
   <212> DNA
   <213> Homo sapiens
<400> 2161
   tggagctggg aactttaatg t 21
<210> 2162
   <211> 21
   <212> DNA
   <213> Homo sapiens
<400> 2162
   gaagagagag agaatgcgtg t 21
<210> 2163
   <211> 21
   <212> DNA
   <213> Homo sapiens
<400> 2163
   gacatggata ttctggtgcc a 21
<210> 2164
   <211> 21
   <212> DNA
   <213> Homo sapiens
<400> 2164
   ctgttgcaag atgacccaaa t 21
<210> 2165
   <211> 22
   <212> DNA
   <213> Homo sapiens
<400> 2165
   agatacacac acgttcacaa ac 22
<210> 2166
   <211> 21
   <212> DNA
   <213> Homo sapiens
<400> 2166
   gatggcaatg cttgataacg a 21
<210> 2167
   <211> 22
   <212> DNA
   <213> Homo sapiens
<400> 2167
   ttccatgaag ttcctcaaga ct 22
<210> 2168
   <211> 21
   <212> DNA
   <213> Homo sapiens
<400> 2168
   ccacccacat accctgaaat t 21
<210> 2169
   <211> 20
   <212> DNA
   <213> Homo sapiens
<400> 2169
   gtaacacacg gatgctgaag 20
<210> 2170
   <211> 21
   <212> DNA
   <213> Homo sapiens
<400> 2170
   tggacacgag gctatttgta g 21
<210> 2171
   <211> 21
   <212> DNA
   <213> Homo sapiens
<400> 2171
   ggcttaagaa ggagagtggt t 21
<210> 2172
   <211> 21
   <212> DNA
   <213> Homo sapiens
<400> 2172
   tagtttcctt tggccttctc c 21
<210> 2173
   <211> 21
   <212> DNA
   <213> Homo sapiens
<400> 2173
   ctagggttcc cagttcacaa a 21
<210> 2174
   <211> 21
   <212> DNA
   <213> Homo sapiens
<400> 2174
   gaaacaacat tgagggcatt g 21
<210> 2175
   <211> 21
   <212> DNA
   <213> Homo sapiens
<400> 2175
   gagctctttg aagtagaagc a 21
<210> 2176
   <211> 20
   <212> DNA
   <213> Homo sapiens
<400> 2176
   ccattccaac aaagcttccg 20
<210> 2177
   <211> 21
   <212> DNA
   <213> Homo sapiens
<400> 2177
   ctacttgccc tattgtgtcg a 21
<210> 2178
   <211> 21
   <212> DNA
   <213> Homo sapiens
<400> 2178
   ccagggtgtt tgaaggtaga a 21
<210> 2179
   <211> 21
   <212> DNA
   <213> Homo sapiens
<400> 2179
   ctggaggtaa gaaggaatgc a 21
<210> 2180
   <211> 20
   <212> DNA
   <213> Homo sapiens
<400> 2180
   atgtgggact ctttgctctc 20
<210> 2181
   <211> 21
   <212> DNA
   <213> Homo sapiens
<400> 2181
   atgaatacag ctttgcatgg c 21
<210> 2182
   <211> 21
   <212> DNA
   <213> Homo sapiens
<400> 2182
   gttaaagcat tcacagccct c 21
<210> 2183
   <211> 21
   <212> DNA
   <213> Homo sapiens
<400> 2183
   ctgggacttg tctatcctcc t 21
<210> 2184
   <211> 21
   <212> DNA
   <213> Homo sapiens
<400> 2184
   atgtctgtcc aagtgaacag t 21
<210> 2185
   <211> 21
   <212> DNA
   <213> Homo sapiens
<400> 2185
   aaagtatcca gacccagaac c 21
<210> 2186
   <211> 21
   <212> DNA
   <213> Homo sapiens
<400> 2186
   ataggccagc actccaaata a 21
<210> 2187
   <211> 21
   <212> DNA
   <213> Homo sapiens
<400> 2187
   caaatcaagt cccatggtag g 21
<210> 2188
   <211> 21
   <212> DNA
   <213> Homo sapiens
<400> 2188
   ctttccgtct ttataggcag c 21
<210> 2189
   <211> 21
   <212> DNA
   <213> Homo sapiens
<400> 2189
   agaaatcgtg ttcacagcct a 21
<210> 2190
   <211> 21
   <212> DNA
   <213> Homo sapiens
<400> 2190
   ccgtaaatga agtggcttga a 21
<210> 2191
   <211> 21
   <212> DNA
   <213> Homo sapiens
<400> 2191
   cctttcttgc aaccttgaga t 21
<210> 2192
   <211> 21
   <212> DNA
   <213> Homo sapiens
<400> 2192
   ctctagatgc tcaacctcag g 21
<210> 2193
   <211> 21
   <212> DNA
   <213> Homo sapiens
<400> 2193
   aataacagtc caccagaacc a 21
<210> 2194
   <211> 21
   <212> DNA
   <213> Homo sapiens
<400> 2194
   tccaaggacc tgcaaatgtt a 21
<210> 2195
   <211> 21
   <212> DNA
   <213> Homo sapiens
<400> 2195
   aggttacatc attcacccac a 21
<210> 2196
   <211> 21
   <212> DNA
   <213> Homo sapiens
<400> 2196
   atgaagacaa tgacatctgc g 21
<210> 2197
   <211> 21
   <212> DNA
   <213> Homo sapiens
<400> 2197
   cacttgtcat ggtttaggga c 21
<210> 2198
   <211> 21
   <212> DNA
   <213> Homo sapiens
<400> 2198
   acctccacct tattgcttca a 21
<210> 2199
   <211> 21
   <212> DNA
   <213> Homo sapiens
<400> 2199
   gaattgcaaa ggatgggtag g 21
<210> 2200
   <211> 21
   <212> DNA
   <213> Homo sapiens
<400> 2200
   ctgcattgtg agtccatgta a 21
<210> 2201
   <211> 21
   <212> DNA
   <213> Homo sapiens
<400> 2201
   tcctatcttc atccctcttc c 21
<210> 2202
   <211> 21
   <212> DNA
   <213> Homo sapiens
<400> 2202
   ttccatttag cctcccatct g 21
<210> 2203
   <211> 21
   <212> DNA
   <213> Homo sapiens
<400> 2203
   tagctttatg ggccttgttc t 21
<210> 2204
   <211> 21
   <212> DNA
   <213> Homo sapiens
<400> 2204
   ccacctctca aacccagatt t 21
<210> 2205
   <211> 22
   <212> DNA
   <213> Homo sapiens
<400> 2205
   gtcagcgtat ttgggattga at 22
<210> 2206
   <211> 21
   <212> DNA
   <213> Homo sapiens
<400> 2206
   aaatctgcca cccatttctt c 21
<210> 2207
   <211> 21
   <212> DNA
   <213> Homo sapiens
<400> 2207
   tattctgagt tctacccagg t 21
<210> 2208
   <211> 21
   <212> DNA
   <213> Homo sapiens
<400> 2208
   tacatgagac ccagaaacag a 21
<210> 2209
   <211> 21
   <212> DNA
   <213> Homo sapiens
<400> 2209
   cttcttggca gactatcagg a 21
<210> 2210
   <211> 21
   <212> DNA
   <213> Homo sapiens
<400> 2210
   aagctgctaa atctgtaggg a 21
<210> 2211
   <211> 21
   <212> DNA
   <213> Homo sapiens
<400> 2211
   ctgaccagac ctgttgacta a 21
<210> 2212
   <211> 22
   <212> DNA
   <213> Homo sapiens
<400> 2212
   tgatatgttc agtttgccta cc 22
<210> 2213
   <211> 20
   <212> DNA
   <213> Homo sapiens
<400> 2213
   agcttgagtt tcttgctggg 20
<210> 2214
   <211> 21
   <212> DNA
   <213> Homo sapiens
<400> 2214
   ttcccgcaaa gtagaagcta t 21
<210> 2215
   <211> 21
   <212> DNA
   <213> Homo sapiens
<400> 2215
   aaacgcatac aaacaggaga c 21
<210> 2216
   <211> 21
   <212> DNA
   <213> Homo sapiens
<400> 2216
   cttctaaacc catcacctgc t 21
<210> 2217
   <211> 21
   <212> DNA
   <213> Homo sapiens
<400> 2217
   cacccaaact cacaggtaca a 21
<210> 2218
   <211> 21
   <212> DNA
   <213> Homo sapiens
<400> 2218
   tgaattctga gatcgagagc c 21
<210> 2219
   <211> 21
   <212> DNA
   <213> Homo sapiens
<400> 2219
   agattaactg ttgcctcact g 21
<210> 2220
   <211> 20
   <212> DNA
   <213> Homo sapiens
<400> 2220
   caagacagtg cattccatgg 20
<210> 2221
   <211> 21
   <212> DNA
   <213> Homo sapiens
<400> 2221
   ccaaggaaag agttgagaag g 21
<210> 2222
   <211> 21
   <212> DNA
   <213> Homo sapiens
<400> 2222
   tgagtgcagt cgataaggaa g 21
<210> 2223
   <211> 21
   <212> DNA
   <213> Homo sapiens
<400> 2223
   aacaccgaga aagagagaga g 21
<210> 2224
   <211> 21
   <212> DNA
   <213> Homo sapiens
<400> 2224
   tgtactgctt tcgtcttatg c 21
<210> 2225
   <211> 21
   <212> DNA
   <213> Homo sapiens
<400> 2225
   catattcgca ctgtatagcc g 21
<210> 2226
   <211> 21
   <212> DNA
   <213> Homo sapiens
<400> 2226
   ctgatggatt ctctggtgtg a 21
<210> 2227
   <211> 21
   <212> DNA
   <213> Homo sapiens
<400> 2227
   tcaaggagaa gagagagggt a 21
<210> 2228
   <211> 21
   <212> DNA
   <213> Homo sapiens
<400> 2228
   tctcccaaag cagacaaaga c 21
<210> 2229
   <211> 21
   <212> DNA
   <213> Homo sapiens
<400> 2229
   agtcagttgt tacgtgcaaa g 21
<210> 2230
   <211> 21
   <212> DNA
   <213> Homo sapiens
<400> 2230
   aaaggtttgt tcatcctccc t 21
<210> 2231
   <211> 21
   <212> DNA
   <213> Homo sapiens
<400> 2231
   acagagcacg caatatagga a 21
<210> 2232
   <211> 21
   <212> DNA
   <213> Homo sapiens
<400> 2232
   ctgcattcac ccatgtactt t 21
<210> 2233
   <211> 21
   <212> DNA
   <213> Homo sapiens
<400> 2233
   tccagccata ccatgtctat c 21
<210> 2234
   <211> 21
   <212> DNA
   <213> Homo sapiens
<400> 2234
   ctcactaggg aagaacagca g 21
<210> 2235
   <211> 22
   <212> DNA
   <213> Homo sapiens
<400> 2235
   tgctgggtct gagtgttata aa 22
<210> 2236
   <211> 21
   <212> DNA
   <213> Homo sapiens
<400> 2236
   gttgtgtgaa tggtgctgtt a 21
<210> 2237
   <211> 21
   <212> DNA
   <213> Homo sapiens
<400> 2237
   ggcccagaag actcttgtaa t 21
<210> 2238
   <211> 21
   <212> DNA
   <213> Homo sapiens
<400> 2238
   cgacctacat cagctaatgg t 21
<210> 2239
   <211> 22
   <212> DNA
   <213> Homo sapiens
<400> 2239
   aagggaagaa taacaatggt gc 22
<210> 2240
   <211> 21
   <212> DNA
   <213> Homo sapiens
<400> 2240
   atgggagtat gggagtagga a 21
<210> 2241
   <211> 20
   <212> DNA
   <213> Homo sapiens
<400> 2241
   ttggtggctt gcagagattt 20
<210> 2242
   <211> 22
   <212> DNA
   <213> Homo sapiens
<400> 2242
   tcacacgatc atcatactca ca 22
<210> 2243
   <211> 21
   <212> DNA
   <213> Homo sapiens
<400> 2243
   ggtagcagat gactagacga t 21
<210> 2244
   <211> 21
   <212> DNA
   <213> Homo sapiens
<400> 2244
   acgcctctgt catttcctaa c 21
<210> 2245
   <211> 21
   <212> DNA
   <213> Homo sapiens
<400> 2245
   cagttgactc aatggtgcaa t 21
<210> 2246
   <211> 21
   <212> DNA
   <213> Homo sapiens
<400> 2246
   ataggttaca gattgccacg t 21
<210> 2247
   <211> 21
   <212> DNA
   <213> Homo sapiens
<400> 2247
   gatgctgcta tcaaaggaac a 21
<210> 2248
   <211> 21
   <212> DNA
   <213> Homo sapiens
<400> 2248
   aagacaaaga gatggaaggc a 21
<210> 2249
   <211> 21
   <212> DNA
   <213> Homo sapiens
<400> 2249
   aataccctct tcccttcctc a 21
<210> 2250
   <211> 22
   <212> DNA
   <213> Homo sapiens
<400> 2250
   ccaccgtcaa tatttatcag ct 22
<210> 2251
   <211> 21
   <212> DNA
   <213> Homo sapiens
<400> 2251
   gcctcagtcc aaatcttaga t 21
<210> 2252
   <211> 22
   <212> DNA
   <213> Homo sapiens
<400> 2252
   ccttaggatt ctcaaagagt gt 22
<210> 2253
   <211> 22
   <212> DNA
   <213> Homo sapiens
<400> 2253
   gcagtacaga ttcttgaaca gt 22
<210> 2254
   <211> 22
   <212> DNA
   <213> Homo sapiens
<400> 2254
   agaggattag atgtcttgct gt 22
<210> 2255
   <211> 21
   <212> DNA
   <213> Homo sapiens
<400> 2255
   tactgcaggc aattcaggta a 21
<210> 2256
   <211> 21
   <212> DNA
   <213> Homo sapiens
<400> 2256
   gaagaggtcc agtaagtgag g 21
<210> 2257
   <211> 21
   <212> DNA
   <213> Homo sapiens
<400> 2257
   ttgtgagtcc ttgtctcctt g 21
<210> 2258
   <211> 21
   <212> DNA
   <213> Homo sapiens
<400> 2258
   gacgactaag acattgcatc a 21
<210> 2259
   <211> 21
   <212> DNA
   <213> Homo sapiens
<400> 2259
   agaagtctct ctccgttgtt t 21
<210> 2260
   <211> 21
   <212> DNA
   <213> Homo sapiens
<400> 2260
   cttatgtgca tcaactgtgc t 21
<210> 2261
   <211> 21
   <212> DNA
   <213> Homo sapiens
<400> 2261
   agtgtctctc agaatcagga c 21
<210> 2262
   <211> 21
   <212> DNA
   <213> Homo sapiens
<400> 2262
   tttatttccc tacgcaaagc c 21
<210> 2263
   <211> 21
   <212> DNA
   <213> Homo sapiens
<400> 2263
   agcctttgat gactgagttg a 21
<210> 2264
   <211> 21
   <212> DNA
   <213> Homo sapiens
<400> 2264
   ttggtttcta ttctgcactg c 21
<210> 2265
   <211> 21
   <212> DNA
   <213> Homo sapiens
<400> 2265
   ttagagcttg ctagtatcgg g 21
<210> 2266
   <211> 21
   <212> DNA
   <213> Homo sapiens
<400> 2266
   gaaatcccaa actgcctgaa a 21
<210> 2267
   <211> 21
   <212> DNA
   <213> Homo sapiens
<400> 2267
   atccttcttg tgaaccttcc t 21
<210> 2268
   <211> 22
   <212> DNA
   <213> Homo sapiens
<400> 2268
   accagatgca tgtgattaaa gg 22
<210> 2269
   <211> 21
   <212> DNA
   <213> Homo sapiens
<400> 2269
   gtgtactcta ggctactgtc a 21
<210> 2270
   <211> 21
   <212> DNA
   <213> Homo sapiens
<400> 2270
   gtcagcagca agtaaaggtt c 21
<210> 2271
   <211> 21
   <212> DNA
   <213> Homo sapiens
<400> 2271
   catctagtca agggttccac a 21
<210> 2272
   <211> 21
   <212> DNA
   <213> Homo sapiens
<400> 2272
   caagtcatgc tccaaactgt t 21
<210> 2273
   <211> 21
   <212> DNA
   <213> Homo sapiens
<400> 2273
   aggacttagg acaacagaga a 21
<210> 2274
   <211> 21
   <212> DNA
   <213> Homo sapiens
<400> 2274
   tgcccaacac catctctaat a 21
<210> 2275
   <211> 21
   <212> DNA
   <213> Homo sapiens
<400> 2275
   gccttcatca ctcagaactt c 21
<210> 2276
   <211> 21
   <212> DNA
   <213> Homo sapiens
<400> 2276
   agggtatcta ttctccggac a 21
<210> 2277
   <211> 21
   <212> DNA
   <213> Homo sapiens
<400> 2277
   atttgcccaa gtaagttcca c 21
<210> 2278
   <211> 21
   <212> DNA
   <213> Homo sapiens
<400> 2278
   tggaagtaca tgggatgcat t 21
<210> 2279
   <211> 21
   <212> DNA
   <213> Homo sapiens
<400> 2279
   cttgaagagt tccaatgcca a 21
<210> 2280
   <211> 21
   <212> DNA
   <213> Homo sapiens
<400> 2280
   cgctgctgtt taaatcgatc a 21
<210> 2281
   <211> 21
   <212> DNA
   <213> Homo sapiens
<400> 2281
   gctctgcttt gctcaaattc t 21
<210> 2282
   <211> 22
   <212> DNA
   <213> Homo sapiens
<400> 2282
   tgagctccag aattagatgt gt 22
<210> 2283
   <211> 22
   <212> DNA
   <213> Homo sapiens
<400> 2283
   aacacctcct ttctcactac ag 22
<210> 2284
   <211> 20
   <212> DNA
   <213> Homo sapiens
<400> 2284
   acaaacttca ttcaccgcag 20
<210> 2285
   <211> 20
   <212> DNA
   <213> Homo sapiens
<400> 2285
   aactacgcca cccaactaaa 20
<210> 2286
   <211> 21
   <212> DNA
   <213> Homo sapiens
<400> 2286
   gcttcagtgt aaccatgact c 21
<210> 2287
   <211> 22
   <212> DNA
   <213> Homo sapiens
<400> 2287
   tgcacaatta agctacttct cc 22
<210> 2288
   <211> 21
   <212> DNA
   <213> Homo sapiens
<400> 2288
   caacaccaaa cttgcctgaa t 21
<210> 2289
   <211> 21
   <212> DNA
   <213> Homo sapiens
<400> 2289
   tccacaattt ctacagcaac c 21
<210> 2290
   <211> 21
   <212> DNA
   <213> Homo sapiens
<400> 2290
   gtgtcatttg attggtgctc t 21
<210> 2291
   <211> 22
   <212> DNA
   <213> Homo sapiens
<400> 2291
   gggtgtttca gtaggttagg at 22
<210> 2292
   <211> 21
   <212> DNA
   <213> Homo sapiens
<400> 2292
   tgggattcta atgtctggtg c 21
<210> 2293
   <211> 18
   <212> DNA
   <213> Homo sapiens
<400> 2293
   agaggtggtt ggttggtt 18
<210> 2294
   <211> 21
   <212> DNA
   <213> Homo sapiens
<400> 2294
   gggcatcctg tctgaaatat g 21
<210> 2295
   <211> 21
   <212> DNA
   <213> Homo sapiens
<400> 2295
   aagaagcgca gatacagtac a 21
<210> 2296
   <211> 21
   <212> DNA
   <213> Homo sapiens
<400> 2296
   cacagcaagt ttgaacctag t 21
<210> 2297
   <211> 21
   <212> DNA
   <213> Homo sapiens
<400> 2297
   agtgcaaatg atgacctgtt g 21
<210> 2298
   <211> 21
   <212> DNA
   <213> Homo sapiens
<400> 2298
   gaactgttgc atgagaggta c 21
<210> 2299
   <211> 21
   <212> DNA
   <213> Homo sapiens
<400> 2299
   ctttgtcctt ctctgttgtg t 21
<210> 2300
   <211> 22
   <212> DNA
   <213> Homo sapiens
<400> 2300
   tttcttctag agtccagagg tg 22
<210> 2301
   <211> 21
   <212> DNA
   <213> Homo sapiens
<400> 2301
   attctcttct ctcttccagc c 21
<210> 2302
   <211> 21
   <212> DNA
   <213> Homo sapiens
<400> 2302
   tatggctttg ctaccttgtc a 21
<210> 2303
   <211> 21
   <212> DNA
   <213> Homo sapiens
<400> 2303
   ctatttctct ggctcttgac c 21
<210> 2304
   <211> 21
   <212> DNA
   <213> Homo sapiens
<400> 2304
   ttcttaaacc tctgtgtggc t 21
<210> 2305
   <211> 21
   <212> DNA
   <213> Homo sapiens
<400> 2305
   tggacaaaca agaactgggt a 21
<210> 2306
   <211> 21
   <212> DNA
   <213> Homo sapiens
<400> 2306
   ccatgatcac tgaaaccaac a 21
<210> 2307
   <211> 21
   <212> DNA
   <213> Homo sapiens
<400> 2307
   ccatatgccc tgctctttaa g 21
<210> 2308
   <211> 21
   <212> DNA
   <213> Homo sapiens
<400> 2308
   gggtggacaa agcaattcaa a 21
<210> 2309
   <211> 22
   <212> DNA
   <213> Homo sapiens
<400> 2309
   cagcattcaa ttcatccttg tg 22
<210> 2310
   <211> 21
   <212> DNA
   <213> Homo sapiens
<400> 2310
   aagtagaagc aagccctgaa t 21
<210> 2311
   <211> 22
   <212> DNA
   <213> Homo sapiens
<400> 2311
   gtgtggtagg gatgagaatt at 22
<210> 2312
   <211> 20
   <212> DNA
   <213> Homo sapiens
<400> 2312
   tcaggtaagc ttccctccac 20
<210> 2313
   <211> 21
   <212> DNA
   <213> Homo sapiens
<400> 2313
   actggttgta gaaaggacct c 21
<210> 2314
   <211> 21
   <212> DNA
   <213> Homo sapiens
<400> 2314
   agtaagaggc cagaagtcag a 21
<210> 2315
   <211> 21
   <212> DNA
   <213> Homo sapiens
<400> 2315
   gcagtgcagg cctatatata g 21
<210> 2316
   <211> 21
   <212> DNA
   <213> Homo sapiens
<400> 2316
   aaatctctga gtcggccata a 21
<210> 2317
   <211> 20
   <212> DNA
   <213> Homo sapiens
<400> 2317
   aggcatggca aacttacttg 20
<210> 2318
   <211> 21
   <212> DNA
   <213> Homo sapiens
<400> 2318
   catttcactt tcgaggatgg t 21
<210> 2319
   <211> 21
   <212> DNA
   <213> Homo sapiens
<400> 2319
   gtcagactaa agtgaggacc a 21
<210> 2320
   <211> 21
   <212> DNA
   <213> Homo sapiens
<400> 2320
   ccagccctac ctaaagtgaa t 21
<210> 2321
   <211> 21
   <212> DNA
   <213> Homo sapiens
<400> 2321
   ccctttcaca agactcttct c 21
<210> 2322
   <211> 18
   <212> DNA
   <213> Homo sapiens
<400> 2322
   aaaaaaaaaa aaaaaaaa 18
<210> 2323
   <211> 22
   <212> DNA
   <213> Homo sapiens
<400> 2323
   ccaaatgtag aacaggatca gc 22
<210> 2324
   <211> 20
   <212> DNA
   <213> Homo sapiens
<400> 2324
   tagcagtagg tgtggctttc 20
<210> 2325
   <211> 21
   <212> DNA
   <213> Homo sapiens
<400> 2325
   ttggattctc ttggttgtga g 21
<210> 2326
   <211> 22
   <212> DNA
   <213> Homo sapiens
<400> 2326
   caacgctttg gtatagtttg tg 22
<210> 2327
   <211> 21
   <212> DNA
   <213> Homo sapiens
<400> 2327
   ccaggtgcca tcgttaaaga a 21
<210> 2328
   <211> 18
   <212> DNA
   <213> Homo sapiens
<400> 2328
   aaaaaaaaaa aaaaaaaa 18
<210> 2329
   <211> 22
   <212> DNA
   <213> Homo sapiens
<400> 2329
   ggataagtca actaccatgg tt 22
<210> 2330
   <211> 21
   <212> DNA
   <213> Homo sapiens
<400> 2330
   aatggaatta ctcagctgtg g 21
<210> 2331
   <211> 21
   <212> DNA
   <213> Homo sapiens
<400> 2331
   ggcagaaact gatagagact g 21
<210> 2332
   <211> 21
   <212> DNA
   <213> Homo sapiens
<400> 2332
   accatgttct gagtacctct t 21
<210> 2333
   <211> 23
   <212> DNA
   <213> Homo sapiens
<400> 2333
   agtcctgaat caatgtctaa cac 23
<210> 2334
   <211> 20
   <212> DNA
   <213> Homo sapiens
<400> 2334
   tggtccctgt gctttgatat 20
<210> 2335
   <211> 20
   <212> DNA
   <213> Homo sapiens
<400> 2335
   tggcttttct ttcctcggta 20
<210> 2336
   <211> 20
   <212> DNA
   <213> Homo sapiens
<400> 2336
   ccaaggctgc tttaattcca 20
<210> 2337
   <211> 20
   <212> DNA
   <213> Homo sapiens
<400> 2337
   caaactatcg ctgaggacct 20
<210> 2338
   <211> 20
   <212> DNA
   <213> Homo sapiens
<400> 2338
   gtctgctgcc attgagttat 20
<210> 2339
   <211> 20
   <212> DNA
   <213> Homo sapiens
<400> 2339
   gctcaccctc tcttctctct 20
<210> 2340
   <211> 20
   <212> DNA
   <213> Homo sapiens
<400> 2340
   acctgtttct cccagttaca 20
<210> 2341
   <211> 20
   <212> DNA
   <213> Homo sapiens
<400> 2341
   gcccatgaaa gagaaaccag 20
<210> 2342
   <211> 20
   <212> DNA
   <213> Homo sapiens
<400> 2342
   ctccatcagt gcagaagtcc 20
<210> 2343
   <211> 20
   <212> DNA
   <213> Homo sapiens
<400> 2343
   acagtcagca gccctaaaat 20
<210> 2344
   <211> 20
   <212> DNA
   <213> Homo sapiens
<400> 2344
   gcctccttca cataatgcag 20
<210> 2345
   <211> 20
   <212> DNA
   <213> Homo sapiens
<400> 2345
   ttgtcaacag agagtcagct 20
<210> 2346
   <211> 20
   <212> DNA
   <213> Homo sapiens
<400> 2346
   ctgaaatggt ctgggagtct 20
<210> 2347
   <211> 20
   <212> DNA
   <213> Homo sapiens
<400> 2347
   tcaaagacag agtgagtgga 20
<210> 2348
   <211> 21
   <212> DNA
   <213> Homo sapiens
<400> 2348
   tttgggggtt acacttcata g 21
<210> 2349
   <211> 21
   <212> DNA
   <213> Homo sapiens
<400> 2349
   agccagcaga ataataccag g 21
<210> 2350
   <211> 20
   <212> DNA
   <213> Homo sapiens
<400> 2350
   acctcatctt ttgtcagcct 20
<210> 2351
   <211> 20
   <212> DNA
   <213> Homo sapiens
<400> 2351
   acagttccat aggcaggttt 20
<210> 2352
   <211> 21
   <212> DNA
   <213> Homo sapiens
<400> 2352
   gcagttccag atccaatatg c 21
<210> 2353
   <211> 20
   <212> DNA
   <213> Homo sapiens
<400> 2353
   tttgggaaag atgggagagc 20
<210> 2354
   <211> 21
   <212> DNA
   <213> Homo sapiens
<400> 2354
   ttgcaggtaa ggtacagaag a 21
<210> 2355
   <211> 19
   <212> DNA
   <213> Homo sapiens
<400> 2355
   cttcagctgc atcttgagc 19
<210> 2356
   <211> 20
   <212> DNA
   <213> Homo sapiens
<400> 2356
   ggcacttcaa aaacaaaccc 20
<210> 2357
   <211> 20
   <212> DNA
   <213> Homo sapiens
<400> 2357
   agggttttat ggtctcctgg 20
<210> 2358
   <211> 21
   <212> DNA
   <213> Homo sapiens
<400> 2358
   tcaacacgga gaactgaaaa c 21
<210> 2359
   <211> 22
   <212> DNA
   <213> Homo sapiens
<400> 2359
   tccgtgtaaa tgaacaaagc ac 22
<210> 2360
   <211> 20
   <212> DNA
   <213> Homo sapiens
<400> 2360
   ttcagggaat ggtttgcatt 20
<210> 2361
   <211> 20
   <212> DNA
   <213> Homo sapiens
<400> 2361
   gagatgccat tcccaaaagg 20
<210> 2362
   <211> 20
   <212> DNA
   <213> Homo sapiens
<400> 2362
   ccctaccata gtgccagatg 20
<210> 2363
   <211> 20
   <212> DNA
   <213> Homo sapiens
<400> 2363
   cagctttcag tgacagagga 20
<210> 2364
   <211> 20
   <212> DNA
   <213> Homo sapiens
<400> 2364
   ccaaaggcag atgagtgttt 20
<210> 2365
   <211> 20
   <212> DNA
   <213> Homo sapiens
<400> 2365
   gcctgggata gaaatgggaa 20
<210> 2366
   <211> 20
   <212> DNA
   <213> Homo sapiens
<400> 2366
   ggaaaggaaa ggaagctgtg 20
<210> 2367
   <211> 20
   <212> DNA
   <213> Homo sapiens
<400> 2367
   tcagagaggt cttgctgaag 20
<210> 2368
   <211> 21
   <212> DNA
   <213> Homo sapiens
<400> 2368
   tctctctgtt gcttgtttcc t 21
<210> 2369
   <211> 20
   <212> DNA
   <213> Homo sapiens
<400> 2369
   cgcatgtggt agatcatcag 20
<210> 2370
   <211> 20
   <212> DNA
   <213> Homo sapiens
<400> 2370
   tataacaccc tcacctccca 20
<210> 2371
   <211> 21
   <212> DNA
   <213> Homo sapiens
<400> 2371
   cacccaaatc accttgctat g 21
<210> 2372
   <211> 20
   <212> DNA
   <213> Homo sapiens
<400> 2372
   caactaccgt ggattccgtt 20
<210> 2373
   <211> 20
   <212> DNA
   <213> Homo sapiens
<400> 2373
   tctatcatga gtcgcttcca 20
<210> 2374
   <211> 20
   <212> DNA
   <213> Homo sapiens
<400> 2374
   tttctgtcac tttctgggct 20
<210> 2375
   <211> 21
   <212> DNA
   <213> Homo sapiens
<400> 2375
   cgtgtgtttc tagtgcattg t 21
<210> 2376
   <211> 20
   <212> DNA
   <213> Homo sapiens
<400> 2376
   gggctcagag ggaatatcag 20
<210> 2377
   <211> 20
   <212> DNA
   <213> Homo sapiens
<400> 2377
   ccgacgaatg gatgaaagac 20
<210> 2378
   <211> 20
   <212> DNA
   <213> Homo sapiens
<400> 2378
   acccaaattc catgcctact 20
<210> 2379
   <211> 20
   <212> DNA
   <213> Homo sapiens
<400> 2379
   tgtacagcag tctccagaaa 20
<210> 2380
   <211> 20
   <212> DNA
   <213> Homo sapiens
<400> 2380
   gccctcttac cctttctcat 20
<210> 2381
   <211> 21
   <212> DNA
   <213> Homo sapiens
<400> 2381
   cattcaaaga tccagaccag g 21
<210> 2382
   <211> 19
   <212> DNA
   <213> Homo sapiens
<400> 2382
   atctgtgatt gctgccctc 19
<210> 2383
   <211> 20
   <212> DNA
   <213> Homo sapiens
<400> 2383
   ctagaaactc ccaggacaga 20
<210> 2384
   <211> 20
   <212> DNA
   <213> Homo sapiens
<400> 2384
   catgtgtgga aaggattggt 20
<210> 2385
   <211> 20
   <212> DNA
   <213> Homo sapiens
<400> 2385
   tatgaatgaa ccgtggctca 20
<210> 2386
   <211> 20
   <212> DNA
   <213> Homo sapiens
<400> 2386
   aagttgagtc gtttgtccca 20
<210> 2387
   <211> 20
   <212> DNA
   <213> Homo sapiens
<400> 2387
   tagtgtttca ggagcgtgtt 20
<210> 2388
   <211> 20
   <212> DNA
   <213> Homo sapiens
<400> 2388
   ccaggacaag cagacatttt 20
<210> 2389
   <211> 21
   <212> DNA
   <213> Homo sapiens
<400> 2389
   gctgctggtg atttttgaag a 21
<210> 2390
   <211> 20
   <212> DNA
   <213> Homo sapiens
<400> 2390
   ctctgggcaa acaagaaacc 20
<210> 2391
   <211> 20
   <212> DNA
   <213> Homo sapiens
<400> 2391
   gtgtgtgttt gtggaagtgt 20
<210> 2392
   <211> 21
   <212> DNA
   <213> Homo sapiens
<400> 2392
   aaagcccaat ctctctggtt a 21
<210> 2393
   <211> 20
   <212> DNA
   <213> Homo sapiens
<400> 2393
   taggcgggct tattgtgttt 20
<210> 2394
   <211> 20
   <212> DNA
   <213> Homo sapiens
<400> 2394
   tcaatgtaaa ctgcccggag 20
<210> 2395
   <211> 21
   <212> DNA
   <213> Homo sapiens
<400> 2395
   atgggagtcg aatggtgtaa a 21
<210> 2396
   <211> 20
   <212> DNA
   <213> Homo sapiens
<400> 2396
   taacatttga gggcatggga 20
<210> 2397
   <211> 20
   <212> DNA
   <213> Homo sapiens
<400> 2397
   cagaataccc tcactgtgct 20
<210> 2398
   <211> 20
   <212> DNA
   <213> Homo sapiens
<400> 2398
   ggagataaca gcagaggtcc 20
<210> 2399
   <211> 20
   <212> DNA
   <213> Homo sapiens
<400> 2399
   tgtgggtgat atctgtgtct 20
<210> 2400
   <211> 20
   <212> DNA
   <213> Homo sapiens
<400> 2400
   gccatcctgt aactgaatgc 20
<210> 2401
   <211> 20
   <212> DNA
   <213> Homo sapiens
<400> 2401
   gtattttccc tttgccgcag 20
<210> 2402
   <211> 20
   <212> DNA
   <213> Homo sapiens
<400> 2402
   attacagcaa agaacgtggc 20
<210> 2403
   <211> 20
   <212> DNA
   <213> Homo sapiens
<400> 2403
   tctgtgtgtt ttgcattggt 20
<210> 2404
   <211> 20
   <212> DNA
   <213> Homo sapiens
<400> 2404
   gtgacagttt tccaaggcat 20
<210> 2405
   <211> 20
   <212> DNA
   <213> Homo sapiens
<400> 2405
   gcattacttt ttcgcacact 20
<210> 2406
   <211> 20
   <212> DNA
   <213> Homo sapiens
<400> 2406
   ggggattgtt ttaagcaggc 20
<210> 2407
   <211> 20
   <212> DNA
   <213> Homo sapiens
<400> 2407
   ccattgttct caccaactct 20
<210> 2408
   <211> 19
   <212> DNA
   <213> Homo sapiens
<400> 2408
   cctgaaacac aagcagcag 19
<210> 2409
   <211> 20
   <212> DNA
   <213> Homo sapiens
<400> 2409
   agctgcctat ttgattggtg 20
<210> 2410
   <211> 20
   <212> DNA
   <213> Homo sapiens
<400> 2410
   cagtacagtc agccttcctt 20
<210> 2411
   <211> 20
   <212> DNA
   <213> Homo sapiens
<400> 2411
   tgcattcaaa ctaccccaag 20
<210> 2412
   <211> 20
   <212> DNA
   <213> Homo sapiens
<400> 2412
   ccgaaaagag gcaagcaatt 20
<210> 2413
   <211> 20
   <212> DNA
   <213> Homo sapiens
<400> 2413
   gtcacctcaa cctaactcca 20
<210> 2414
   <211> 20
   <212> DNA
   <213> Homo sapiens
<400> 2414
   gcaacagtct acccgtctag 20
<210> 2415
   <211> 20
   <212> DNA
   <213> Homo sapiens
<400> 2415
   atcaatgctc tgacctcctg 20
<210> 2416
   <211> 21
   <212> DNA
   <213> Homo sapiens
<400> 2416
   atacatcagg cctccagaat t 21
<210> 2417
   <211> 22
   <212> DNA
   <213> Homo sapiens
<400> 2417
   cacatcttta gagctcaggt ga 22
<210> 2418
   <211> 21
   <212> DNA
   <213> Homo sapiens
<400> 2418
   ccatcacttc acaatccaca c 21
<210> 2419
   <211> 20
   <212> DNA
   <213> Homo sapiens
<400> 2419
   aatcctgcag tcatcttccc 20
<210> 2420
   <211> 21
   <212> DNA
   <213> Homo sapiens
<400> 2420
   caagtctggt ttgtgagaag c 21
<210> 2421
   <211> 21
   <212> DNA
   <213> Homo sapiens
<400> 2421
   gggagcttct gtagtctttg a 21
<210> 2422
   <211> 21
   <212> DNA
   <213> Homo sapiens
<400> 2422
   taaggagagc aggacttaca g 21
<210> 2423
   <211> 21
   <212> DNA
   <213> Homo sapiens
<400> 2423
   ccaacggttc atttgtcgta t 21
<210> 2424
   <211> 21
   <212> DNA
   <213> Homo sapiens
<400> 2424
   ccttgctctg ttaatgggtt t 21
<210> 2425
   <211> 21
   <212> DNA
   <213> Homo sapiens
<400> 2425
   aacaatgctt aacgggaatc c 21
<210> 2426
   <211> 21
   <212> DNA
   <213> Homo sapiens
<400> 2426
   gcagagttca tagaagggtc a 21
<210> 2427
   <211> 21
   <212> DNA
   <213> Homo sapiens
<400> 2427
   aaaccgagac gaccacctaa t 21
<210> 2428
   <211> 21
   <212> DNA
   <213> Homo sapiens
<400> 2428
   gagtgatgat gagccatgat g 21
<210> 2429
   <211> 21
   <212> DNA
   <213> Homo sapiens
<400> 2429
   ggatattgga gatagcaggc a 21
<210> 2430
   <211> 21
   <212> DNA
   <213> Homo sapiens
<400> 2430
   gtttggcact gcaactagat a 21
<210> 2431
   <211> 21
   <212> DNA
   <213> Homo sapiens
<400> 2431
   tgaaataagg gaagccacac a 21
<210> 2432
   <211> 21
   <212> DNA
   <213> Homo sapiens
<400> 2432
   tccttagtgt gccaattagc c 21
<210> 2433
   <211> 20
   <212> DNA
   <213> Homo sapiens
<400> 2433
   ttaatgtgga gagacaggcc 20
<210> 2434
   <211> 21
   <212> DNA
   <213> Homo sapiens
<400> 2434
   caatgcatct tactcaccct t 21
<210> 2435
   <211> 21
   <212> DNA
   <213> Homo sapiens
<400> 2435
   agtatggaag tgggaattgg a 21
<210> 2436
   <211> 21
   <212> DNA
   <213> Homo sapiens
<400> 2436
   ttgcttccac agaaactctt c 21
<210> 2437
   <211> 21
   <212> DNA
   <213> Homo sapiens
<400> 2437
   aaccgaccta ttccaaagtc t 21
<210> 2438
   <211> 21
   <212> DNA
   <213> Homo sapiens
<400> 2438
   tgtgaagcga atacagctca a 21
<210> 2439
   <211> 21
   <212> DNA
   <213> Homo sapiens
<400> 2439
   gttctaacta caccaggctc t 21
<210> 2440
   <211> 21
   <212> DNA
   <213> Homo sapiens
<400> 2440
   gtatgagtgt aggtgtggag g 21
<210> 2441
   <211> 21
   <212> DNA
   <213> Homo sapiens
<400> 2441
   aatctggatc tagcgaagga c 21
<210> 2442
   <211> 21
   <212> DNA
   <213> Homo sapiens
<400> 2442
   taatgagaag gcaggatgag g 21
<210> 2443
   <211> 21
   <212> DNA
   <213> Homo sapiens
<400> 2443
   aagacaactc tctaggcctc a 21
<210> 2444
   <211> 21
   <212> DNA
   <213> Homo sapiens
<400> 2444
   gtttatggtt gtccctggag a 21
<210> 2445
   <211> 21
   <212> DNA
   <213> Homo sapiens
<400> 2445
   tccaccttct gatcacacaa t 21
<210> 2446
   <211> 21
   <212> DNA
   <213> Homo sapiens
<400> 2446
   aagatcaggt accaaggcat t 21
<210> 2447
   <211> 21
   <212> DNA
   <213> Homo sapiens
<400> 2447
   atgatgtgaa gtccatggtg a 21
<210> 2448
   <211> 21
   <212> DNA
   <213> Homo sapiens
<400> 2448
   tccaaattga cttccatgag c 21
<210> 2449
   <211> 21
   <212> DNA
   <213> Homo sapiens
<400> 2449
   gtggtttcag gaatttggag g 21
<210> 2450
   <211> 22
   <212> DNA
   <213> Homo sapiens
<400> 2450
   tatttgctgc ttcattcttc cc 22
<210> 2451
   <211> 21
   <212> DNA
   <213> Homo sapiens
<400> 2451
   aagtcattac gtcccacact g 21
<210> 2452
   <211> 21
   <212> DNA
   <213> Homo sapiens
<400> 2452
   aaagtcatca gaagggtagc a 21
<210> 2453
   <211> 21
   <212> DNA
   <213> Homo sapiens
<400> 2453
   aatgatgccg aacagtgagt a 21
<210> 2454
   <211> 21
   <212> DNA
   <213> Homo sapiens
<400> 2454
   aatgtaagac agggacagag a 21
<210> 2455
   <211> 21
   <212> DNA
   <213> Homo sapiens
<400> 2455
   tgaattccac agtccagtca a 21
<210> 2456
   <211> 21
   <212> DNA
   <213> Homo sapiens
<400> 2456
   aatgccttca aagacagtga c 21
<210> 2457
   <211> 21
   <212> DNA
   <213> Homo sapiens
<400> 2457
   cacacctgca attgagatga a 21
<210> 2458
   <211> 21
   <212> DNA
   <213> Homo sapiens
<400> 2458
   gtcatgatga tgcaacagct a 21
<210> 2459
   <211> 21
   <212> DNA
   <213> Homo sapiens
<400> 2459
   tttcacggta agaggagcaa a 21
<210> 2460
   <211> 21
   <212> DNA
   <213> Homo sapiens
<400> 2460
   ttctctctag gcaggtgaac t 21
<210> 2461
   <211> 21
   <212> DNA
   <213> Homo sapiens
<400> 2461
   accctttgaa agaaccagga a 21
<210> 2462
   <211> 21
   <212> DNA
   <213> Homo sapiens
<400> 2462
   aatgagccac tgttctctag g 21
<210> 2463
   <211> 21
   <212> DNA
   <213> Homo sapiens
<400> 2463
   atttgcacat tagggcctca a 21
<210> 2464
   <211> 20
   <212> DNA
   <213> Homo sapiens
<400> 2464
   gaacttccct gcttccttct 20
<210> 2465
   <211> 21
   <212> DNA
   <213> Homo sapiens
<400> 2465
   aaatacttgg ctgtgaccat g 21
<210> 2466
   <211> 21
   <212> DNA
   <213> Homo sapiens
<400> 2466
   aagacccttg agaacttcca a 21
<210> 2467
   <211> 21
   <212> DNA
   <213> Homo sapiens
<400> 2467
   atgtgtaaag acgtcctgga a 21
<210> 2468
   <211> 21
   <212> DNA
   <213> Homo sapiens
<400> 2468
   tctatgtgga gggatttgac a 21
<210> 2469
   <211> 22
   <212> DNA
   <213> Homo sapiens
<400> 2469
   ttcctgaagt ttatggtgca ac 22
<210> 2470
   <211> 21
   <212> DNA
   <213> Homo sapiens
<400> 2470
   aaggttgaat gaggatcaag c 21
<210> 2471
   <211> 21
   <212> DNA
   <213> Homo sapiens
<400> 2471
   gagggaagaa cacaacacat g 21
<210> 2472
   <211> 21
   <212> DNA
   <213> Homo sapiens
<400> 2472
   cagactagcc tacaatcctc c 21
<210> 2473
   <211> 21
   <212> DNA
   <213> Homo sapiens
<400> 2473
   cattcaggtt cttaagggct g 21
<210> 2474
   <211> 21
   <212> DNA
   <213> Homo sapiens
<400> 2474
   gaaaggcaga cgatgaaaga g 21
<210> 2475
   <211> 21
   <212> DNA
   <213> Homo sapiens
<400> 2475
   atctccatca gcacaggaat t 21
<210> 2476
   <211> 21
   <212> DNA
   <213> Homo sapiens
<400> 2476
   gcagtgtgac atctgtgaat g 21
<210> 2477
   <211> 21
   <212> DNA
   <213> Homo sapiens
<400> 2477
   agactttcct gttcctcttc a 21
<210> 2478
   <211> 21
   <212> DNA
   <213> Homo sapiens
<400> 2478
   tttacaatga actagccagg c 21
<210> 2479
   <211> 21
   <212> DNA
   <213> Homo sapiens
<400> 2479
   taacatctgc ctgaaagctt c 21
<210> 2480
   <211> 21
   <212> DNA
   <213> Homo sapiens
<400> 2480
   ctcgtgtgtg caatttggaa t 21
<210> 2481
   <211> 21
   <212> DNA
   <213> Homo sapiens
<400> 2481
   ggagagatgg agaagacctt t 21
<210> 2482
   <211> 21
   <212> DNA
   <213> Homo sapiens
<400> 2482
   aatgtattac tgtgctcccg t 21
<210> 2483
   <211> 21
   <212> DNA
   <213> Homo sapiens
<400> 2483
   accacctgcc actgtataaa t 21
<210> 2484
   <211> 21
   <212> DNA
   <213> Homo sapiens
<400> 2484
   aattcttctt ctggtgcaag g 21
<210> 2485
   <211> 21
   <212> DNA
   <213> Homo sapiens
<400> 2485
   ttgccctttg aactgttgat c 21
<210> 2486
   <211> 21
   <212> DNA
   <213> Homo sapiens
<400> 2486
   aagcacacta aggcctgata a 21
<210> 2487
   <211> 21
   <212> DNA
   <213> Homo sapiens
<400> 2487
   tcagtatcca ttcagcatcc a 21
<210> 2488
   <211> 20
   <212> DNA
   <213> Homo sapiens
<400> 2488
   taaccgaagc ccatactctg 20
<210> 2489
   <211> 21
   <212> DNA
   <213> Homo sapiens
<400> 2489
   tccttactcc agatacccga t 21
<210> 2490
   <211> 21
   <212> DNA
   <213> Homo sapiens
<400> 2490
   tctggcttct ttcttggaga g 21
<210> 2491
   <211> 21
   <212> DNA
   <213> Homo sapiens
<400> 2491
   gcaacattca tttcatcctg c 21
<210> 2492
   <211> 21
   <212> DNA
   <213> Homo sapiens
<400> 2492
   catcacgaca tccatttcca c 21
<210> 2493
   <211> 21
   <212> DNA
   <213> Homo sapiens
<400> 2493
   gtggttatta tcggtgggtg a 21
<210> 2494
   <211> 20
   <212> DNA
   <213> Homo sapiens
<400> 2494
   ctgccattcc ttgtttccaa 20
<210> 2495
   <211> 20
   <212> DNA
   <213> Homo sapiens
<400> 2495
   atttggactt gaagcagcct 20
<210> 2496
   <211> 21
   <212> DNA
   <213> Homo sapiens
<400> 2496
   ctgtattctt tgtccaccac c 21
<210> 2497
   <211> 21
   <212> DNA
   <213> Homo sapiens
<400> 2497
   gacaggacac cttggattga t 21
<210> 2498
   <211> 20
   <212> DNA
   <213> Homo sapiens
<400> 2498
   gagtaattcc cccgatgcag 20
<210> 2499
   <211> 21
   <212> DNA
   <213> Homo sapiens
<400> 2499
   tgagaatcat tgagccaaac c 21
<210> 2500
   <211> 20
   <212> DNA
   <213> Homo sapiens
<400> 2500
   aattgtgagc gttagagtgc 20
<210> 2501
   <211> 21
   <212> DNA
   <213> Homo sapiens
<400> 2501
   ccagtgggtc ttaacattga g 21
<210> 2502
   <211> 20
   <212> DNA
   <213> Homo sapiens
<400> 2502
   aaaaggctag tagagggtgc 20
<210> 2503
   <211> 20
   <212> DNA
   <213> Homo sapiens
<400> 2503
   tcgctgaaga actgagacac 20
<210> 2504
   <211> 20
   <212> DNA
   <213> Homo sapiens
<400> 2504
   gggaagtgat tggagagagg 20
<210> 2505
   <211> 20
   <212> DNA
   <213> Homo sapiens
<400> 2505
   ccgcttggta tagagtgctg 20
<210> 2506
   <211> 20
   <212> DNA
   <213> Homo sapiens
<400> 2506
   ttgaggttgt caggaaagct 20
<210> 2507
   <211> 20
   <212> DNA
   <213> Homo sapiens
<400> 2507
   tcttgggaaa gggtcattct 20
<210> 2508
   <211> 19
   <212> DNA
   <213> Homo sapiens
<400> 2508
   tctcttccat gcactccac 19
<210> 2509
   <211> 19
   <212> DNA
   <213> Homo sapiens
<400> 2509
   gatctgaccc tctgctcac 19
<210> 2510
   <211> 20
   <212> DNA
   <213> Homo sapiens
<400> 2510
   cctgaaagat gcatggttgg 20
<210> 2511
   <211> 20
   <212> DNA
   <213> Homo sapiens
<400> 2511
   gccacagaac aaccagattc 20
<210> 2512
   <211> 20
   <212> DNA
   <213> Homo sapiens
<400> 2512
   tggggttaag agctcaagag 20
<210> 2513
   <211> 20
   <212> DNA
   <213> Homo sapiens
<400> 2513
   agtgtggatg acttctgcaa 20
<210> 2514
   <211> 20
   <212> DNA
   <213> Homo sapiens
<400> 2514
   gccccattat cctcctttgt 20
<210> 2515
   <211> 21
   <212> DNA
   <213> Homo sapiens
<400> 2515
   caaatcagac ccactaagca c 21
<210> 2516
   <211> 20
   <212> DNA
   <213> Homo sapiens
<400> 2516
   tgttttggac tgcttcactc 20
<210> 2517
   <211> 21
   <212> DNA
   <213> Homo sapiens
<400> 2517
   attatcatgc tcactcctcc a 21
<210> 2518
   <211> 19
   <212> DNA
   <213> Homo sapiens
<400> 2518
   gacatagcac gggagagta 19
<210> 2519
   <211> 20
   <212> DNA
   <213> Homo sapiens
<400> 2519
   gctagtggcg ttttaggaaa 20
<210> 2520
   <211> 19
   <212> DNA
   <213> Homo sapiens
<400> 2520
   tcgcttggaa gtcatagcc 19
<210> 2521
   <211> 23
   <212> DNA
   <213> Homo sapiens
<400> 2521
   cagagctgct ttgaagataa tcc 23
<210> 2522
   <211> 20
   <212> DNA
   <213> Homo sapiens
<400> 2522
   gaagcctgat agatgtgcct 20
<210> 2523
   <211> 20
   <212> DNA
   <213> Homo sapiens
<400> 2523
   tacctctgcc tccaattgtc 20
<210> 2524
   <211> 20
   <212> DNA
   <213> Homo sapiens
<400> 2524
   cccatgtaga caaagtgctt 20
<210> 2525
   <211> 20
   <212> DNA
   <213> Homo sapiens
<400> 2525
   ggcaggtttg tcttacagtt 20
<210> 2526
   <211> 20
   <212> DNA
   <213> Homo sapiens
<400> 2526
   tgtgagattg cattcccctt 20
<210> 2527
   <211> 20
   <212> DNA
   <213> Homo sapiens
<400> 2527
   cgggtcagag aaagatcagg 20
<210> 2528
   <211> 22
   <212> DNA
   <213> Homo sapiens
<400> 2528
   aaacatttgt aaccactccc tg 22
<210> 2529
   <211> 21
   <212> DNA
   <213> Homo sapiens
<400> 2529
   cctgtaatat gggactcctg g 21
<210> 2530
   <211> 20
   <212> DNA
   <213> Homo sapiens
<400> 2530
   ccaaaccaca cacacaaact 20
<210> 2531
   <211> 21
   <212> DNA
   <213> Homo sapiens
<400> 2531
   aggagaagga catttcacag g 21
<210> 2532
   <211> 20
   <212> DNA
   <213> Homo sapiens
<400> 2532
   aggccgataa gacaaggttc 20
<210> 2533
   <211> 19
   <212> DNA
   <213> Homo sapiens
<400> 2533
   gtttcccata gagccctgg 19
<210> 2534
   <211> 20
   <212> DNA
   <213> Homo sapiens
<400> 2534
   ctccactctc tccaaccaac 20
<210> 2535
   <211> 20
   <212> DNA
   <213> Homo sapiens
<400> 2535
   aaactgtgaa aggacgagga 20
<210> 2536
   <211> 20
   <212> DNA
   <213> Homo sapiens
<400> 2536
   tcccttgctt ttgtaccagg 20
<210> 2537
   <211> 20
   <212> DNA
   <213> Homo sapiens
<400> 2537
   catgtgtgta ctgtgcctca 20
<210> 2538
   <211> 21
   <212> DNA
   <213> Homo sapiens
<400> 2538
   tctttcactg tcactatggg g 21
<210> 2539
   <211> 20
   <212> DNA
   <213> Homo sapiens
<400> 2539
   aaatgcagct tcccaacatc 20
<210> 2540
   <211> 20
   <212> DNA
   <213> Homo sapiens
<400> 2540
   gagactttct ggaggacgaa 20
<210> 2541
   <211> 22
   <212> DNA
   <213> Homo sapiens
<400> 2541
   gaaacgtaat ttagtgactg gc 22
<210> 2542
   <211> 22
   <212> DNA
   <213> Homo sapiens
<400> 2542
   atgtgtctat tgctacctgt ga 22
<210> 2543
   <211> 20
   <212> DNA
   <213> Homo sapiens
<400> 2543
   ctccttcatt ttgctggtgg 20
<210> 2544
   <211> 20
   <212> DNA
   <213> Homo sapiens
<400> 2544
   ggtgatcatt tgtctgcaca 20
<210> 2545
   <211> 20
   <212> DNA
   <213> Homo sapiens
<400> 2545
   tgaagggatg aaggcagaag 20
<210> 2546
   <211> 20
   <212> DNA
   <213> Homo sapiens
<400> 2546
   attgtgttgt ccttccgttt 20
<210> 2547
   <211> 20
   <212> DNA
   <213> Homo sapiens
<400> 2547
   ctgaagcatc actggcattg 20
<210> 2548
   <211> 20
   <212> DNA
   <213> Homo sapiens
<400> 2548
   aagcttgtag tctgggtagc 20
<210> 2549
   <211> 22
   <212> DNA
   <213> Homo sapiens
<400> 2549
   tccctctgta ctatgtagca tg 22
<210> 2550
   <211> 20
   <212> DNA
   <213> Homo sapiens
<400> 2550
   ttcatttccc tttgttgccc 20

## Description

### Field of the Invention

The invention is in the field of biology, medicine and chemistry, more in particular in the field of molecular biology and more in particular in the field of molecular diagnostics.

### Background of the Invention

The discovery of free fetal DNA (ffDNA) in maternal circulation (Lo, Y.M. et al. (1997) Lancet 350:485-487) was a landmark towards the development of non-invasive prenatal testing for chromosomal abnormalities and has opened up new possibilities in the clinical setting. However, direct analysis of the limited amount of ffDNA in the presence of an excess of maternal DNA is a great challenge for Non-Invasive Prenatal Testing (NIPT) of chromosomal abnormalities. The implementation of next generation sequencing (NGS) technologies in the development of NIPT has revolutionized the field. In 2008, two independent groups demonstrated that NIPT of trisomy 21 could be achieved using next generation massively parallel shotgun sequencing (MPSS) (Chiu, R. W. et al.(2008) Proc. Natl. Acad. Sci. USA 105:20458-20463; Fan, H.C. et al.(2008) Proc. Natl. Acad. Sci. USA 105:16266-162710). The new era of NIPT for chromosomal abnormalities has opened new possibilities for the implementation of these technologies into clinical practice. Biotechnology companies that are partly or wholly dedicated to the development of NIPT tests have initiated large-scale clinical studies towards their implementation (Palomaki, G.E. et al. (2011) Genet. Med. 13:913-920; Ehrich, M. et al. (2011) Am. J. Obstet. Gynecol. 204:205e1-11; Chen, E.Z. et al. (2011) PLoS One 6:e21791; Sehnert, A.J. et al. (2011) Clin. Chem. 57:1042-1049; Palomaki, G.E. et al. (2012); Genet. Med. 14:296-305; Bianchi, D.W. et al. (2012) Obstet. Gynecol. 119:890-901; Zimmerman, B. et al. (2012) Prenat. Diag. 32:1233-1241; Nicolaides, K.H. et al. (2013) Prenat. Diagn. 33:575-579; Sparks, A.B. et al. (2012) Prenat. Diagn. 32:3-9)

Initial NIPT approaches used massively parallel shotgun sequencing (MPSS) NGS methodologies (see e.g., US Patent No. 7,888,017; US Patent No. 8,008,018; US Patent No. 8,195,415; US Patent No. 8,296,076; US Patent No. 8,682,594; US Patent Publication 20110201507; US Patent Publication 20120270739). Thus, these approaches are whole genome-based, in which the entire maternal sample containing both maternal DNA and free fetal DNA is subjected to amplification, sequencing and analysis.

More recently, targeted-based NGS approaches for NIPT, in which only specific sequences of interest are sequenced, have been developed. For example, a targeted NIPT approach using TArget Capture Sequences (TACS) for identifying fetal chromosomal abnormalities using a maternal blood sample has been described (PCT Publication WO 2016/189388; US Patent Publication 2016/0340733; Koumbaris, G. et al. (2016) Clinical chemistry, 62(6), pp.848-855).

Such targeted approaches require significantly less sequencing than the MPSS approaches, since sequencing is only performed on specific loci on the target sequence of interest rather than across the whole genome. Additional methodologies for NGS-based approaches are still needed, in particular approaches that can target specific sequences of interest, thereby greatly reducing the amount of sequencing needed as compared to whole genome-based approaches, as well as increasing the read-depth of regions of interest, thus enabling detection of low signal to noise ratio regions. In particular, additional methodologies are still needed that allow for genetic aberrations present in diminutive amounts in a sample can be reliably detected. For example, additional methodologies are still needed that allow for analysis of DNA samples that contain predominantly fetal or embryonic DNA, since such samples contain only diminutive amounts of fetal or embryonic DNA.

### Summary of the Invention

This invention provides improved methods for enriching targeted genomic regions of interest to be analyzed by multiplexed parallel sequencing, wherein the DNA sample used in the method contains predominantly or only fetal/embryonic DNA. Accordingly, the methods allow for analysis of very small starting amounts of fetal or embryonic DNA. The methods of the disclosure can be used in the analysis of fetal or embryonic DNA samples, e.g., for the presence of genetic abnormalities, for example for purposes of IVF Pre-implantation Genetic Screening (PGS) and Diagnosis (PGD). The methods of the invention utilize a pool of TArget Capture Sequences (TACS) designed such that the sequences within the pool have features that optimize the efficiency, specificity and accuracy of genetic assessment. In one embodiment, the pool of TACS comprises member sequences whose binding encompasses all chromosomes within the human genome (chromosomes 1-22, X and Y), thereby allowing for evaluation of the entire human genome in a single fetal/embryonic DNA sample.

Accordingly, in one aspect, the invention pertains to a method of testing for risk of a genetic abnormality in a DNA sample comprising predominantly fetal or embryonic DNA and comprising genomic sequences of interest, the method comprising:a) preparing a sequencing library from the DNA sample comprising predominantly fetal or embryonic DNA;b) hybridizing the sequencing library to a pool of double-stranded TArget Capture Sequences (TACS), wherein the pool of TACS comprises a plurality of TACS families directed to different genomic sequences of interest comprising a genetic abnormality, wherein each TACS family comprises a plurality of member sequences, wherein each member sequence binds to the same genomic sequence of interest but has different start and/or stop positions with respect to a reference coordinate system for the genomic sequence of interest, wherein the start and/or stop positions for the member sequences within a TACS family, with respect to a reference coordinate system for the genomic sequence of interest are staggered by 5 to 10 base pairs, and further wherein:i) each member sequence within the pool of TACS is between 150-260 base pairs in length, each member sequence having a 5' end and a 3' end; andii) each member sequence binds to the same genomic sequence of interest at least 50 base pairs away, on both the 5' end and the 3' end, from regions harboring Copy Number Variations (CNVs), Segmental duplications or repetitive DNA elements; andiii) the GC content of the pool of TACS is between 19% and 80%, as determined by calculating the GC content of each member within the pool of TACS;c) isolating members of the sequencing library that bind to the pool of TACS to obtain an enriched library;d) amplifying and sequencing the enriched library;e) aligning, the enriched library to a reference genome thereby obtaining read-depth information and allelic counts; andf) performing statistical analysis on the enriched library sequences to thereby determine risk of a genetic abnormality in the DNA sample.

In various embodiments, the DNA sample is from, for example, a pre-implantation embryo, intact trophoblasts collected from a maternal Papanicolaou smear or a fetal cell found in maternal plasma. In one embodiment, the DNA sample is obtained directly from fetal or embryonic tissue. In certain embodiments, the DNA sample is obtained directly from fetal tissue, or amniotic fluid, or chorionic villi, or medium where products of conception were grown.

In one embodiment, the pool of TACS comprises members that bind to chromosomes 1-22, X and Y of the human genome.

In certain embodiments, the GC content of the pool of TACS is between 19% and 80% or is between 19% and 46%. Alternative % ranges for the GC content of the pool of TACS are described herein.

In one embodiment, the pool of TACS comprises a plurality of TACS families, wherein each member of a TACS family binds to the same target sequence of interest but with different start and/or stop positions on the sequence with respect to a reference coordinate system (i.e., binding of TACS family members to the target sequence is staggered) to thereby enrich for target sequences of interest, followed by massive parallel sequencing and statistical analysis of the enriched population. The use of families of TACS with the TACS pool that bind to each target sequence of interest, as compared to use of a single TACS within the TACS pool that binds to each target sequence of interest, significantly increases enrichment for the target sequences of interest, as evidenced by a greater than 50% average increase in read-depth for the family of TACS versus a single TACS.

Accordingly, in one embodiment, the pool of TACS comprises a plurality of TACS families directed to different genomic sequences of interest, wherein each TACS family comprises a plurality of member sequences, wherein each member sequence binds to the same genomic sequence of interest but has different start and/or stop positions with respect to a reference coordinate system for the genomic sequence of interest.

In certain embodiments, each TACS family comprises at least 3 member sequences or at least 5 member sequences. Alternative numbers of member sequences in each TACS family are described herein. In one embodiment, the pool of TACS comprises at least 50 different TACS families. Alternative numbers of different TACS families within the pool of TACS are described herein. In certain embodiments, the start and/or stop positions for the member sequences within a TACS family, with respect to a reference coordinate system for the genomic sequence of interest, are staggered by 5 to 10 base pairs.

In one embodiment, the genomic abnormality is a chromosomal aneuploidy. In other embodiments, the genomic abnormality is a structural abnormality, including but not limited to copy number changes including microdeletions and microduplications, insertions, deletions, translocations, inversions and small-size mutations including point mutations and mutational signatures.

In one embodiment, the pool of TACS is fixed to a solid support. For example, in one embodiment, the TACS are biotinylated and are bound to streptavidin-coated magnetic beads.

In one embodiment, amplification of the enriched library is performed in the presence of blocking sequences that inhibit amplification of wild-type sequences.

In one embodiment, members of the sequencing library that bind to the pool of TACS are partially complementary to the TACS.

In one embodiment, the statistical analysis comprises a segmentation algorithm, for example, likelihood-based segmentation, segmentation using small overlapping windows, segmentation using parallel pairwise testing, and combinations thereof. In one embodiment, the statistical analysis comprises a score-based classification system. In on embodiment, sequencing of the enriched library provides a read-depth for the genomic sequences of interest and read-depths for reference loci and the statistical analysis comprises applying an algorithm that tests sequentially the read-depth of the loci of from the genomic sequences of interest against the read-depth of the reference loci, the algorithm comprising steps for: (a) removal of inadequately sequenced loci; (b) GC-content bias alleviation; and (c) ploidy status determination. In one embodiment, GC-content bias is alleviated by grouping together loci of matching GC content. In one embodiment, sequencing of the enriched library provides the number and size of sequenced fragments for TACS-specific coordinates and the statistical analysis comprises applying an algorithm that tests sequentially the fragment-size proportion for the genomic sequence of interest against the fragment-size proportion of the reference loci, the algorithm comprising steps for: (a) removal of fragment-size outliers; (b) fragment-size proportion calculation; and (c) ploidy status determination.

### Brief Description of the Figures

The patent or application file contains at least one drawing executed in color. Copies of this patent or patent application publication with color drawing(s) will be provided by the Office upon request and payment of the necessary fee.
Figure 1 is a schematic diagram of multiplexed parallel analysis of targeted genomic regions for non-invasive prenatal testing using TArget Capture Sequences (TACS).
Figure 2 is a listing of exemplary chromosomal regions for amplifying TACS that bind to for example chromosomes 13, 18, 21 or X. A more extensive list is shown in Table 1 below. The TACS in Table 1 are those preferred herein.
Figure 3 is a schematic diagram of TACS-based enrichment of a sequence of interest (bold line) using a single TACS (left) versus TACS-based enrichment using a family of TACS (right).
Figures 4A-4B are graphs showing enrichment using families of TACS versus a single TACS, as illustrated by increase in the average read-depth. Figure 4A shows loci enriched using a family of TACS (red dots) as compared to loci enriched using a single TACS (blue dots), with different target sequences shown on the X-axis and the fold change in read-depth shown on the Y-axis. Figure 4B is a bar graph illustrating the average fold-increase in read-depth (54.7%) using a family of TACS (right) versus a single TACS (left).

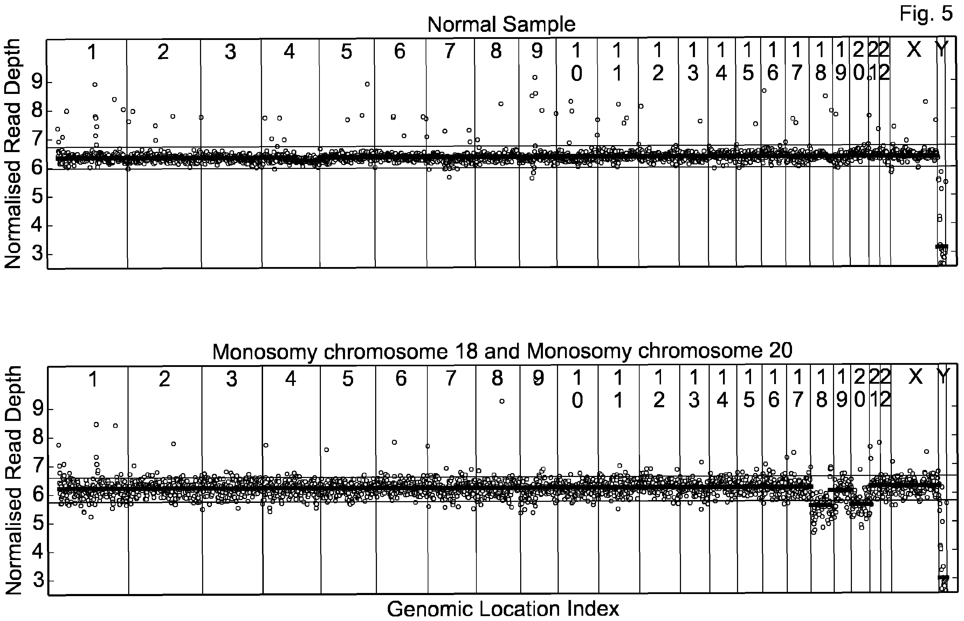

Figure 5 is a graph of results from fetal DNA samples that underwent ploidy status determination using likelihood-based segmentation analysis and whole-genome sequencing data. The horizontal blue line indicates the average read-depth of each chromosome. The red lines indicate threshold intervals of expected diploids. Data above the top red line is classified as more than diploid and data below the red line is classified as less than diploid. The top panel illustrates the results of a euploid female sample (i.e., a female fetus with diploid X chromosome, no Y chromosome, and without any ploidy abnormalities present). The bottom panel illustrates the results of a female aneuploid sample (i.e., a female fetus with diploid X chromosome and no Y chromosome) with monosomy 18 and monosomy 20. Values on the y-axis are log of read-depth.
Figure 6 is a graph of results from fetal DNA samples that underwent ploidy status determination by whole genome sequencing, followed by segmentation analysis using small overlapping windows analysis. The horizontal blue line indicates the average read-depth of each chromosome.
The red lines indicate threshold intervals of expected diploids. The top panel illustrates the results of a euploid male sample (i.e., a male fetus with a single copy of X and Y chromosomes and
without any ploidy abnormalities present). The bottom panel illustrates the results of an aneuploid male sample (i.e., a male fetus with a single copy of X and Y chromosomes) and with aneuploidies on chromosomes 13 and 19 (trisomy 13 and mosaicism on chromosome 19). Values are log of read-depth.

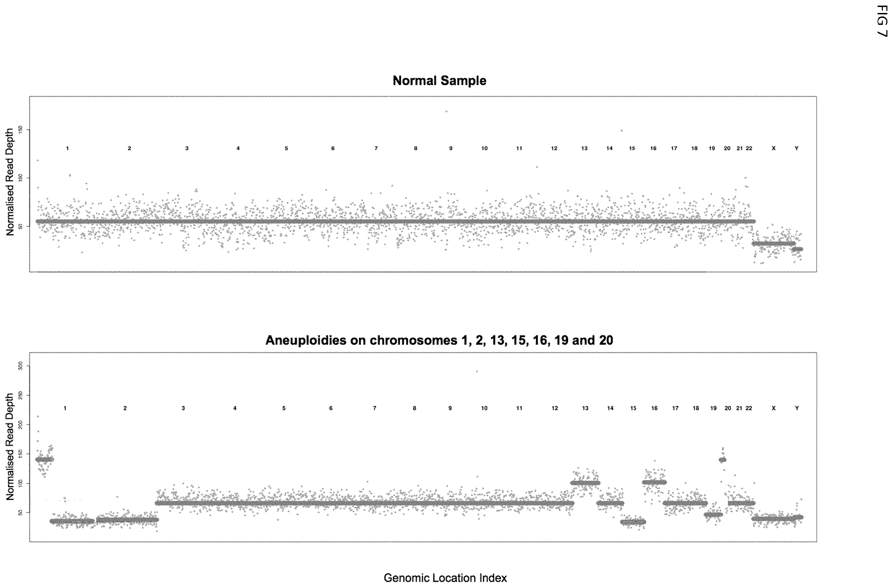

Figure 7 is a graph of results from fetal DNA samples that underwent ploidy status determination by whole genome sequencing, followed by segmentation analysis using parallel pairwise testing.
The top panel illustrates the results of a normal (euploid) sample and the bottom panel illustrates the results of an aneuploidy sample with aneuploidies on chromosomes 1, 2, 13, 15, 16, 19, and 20.

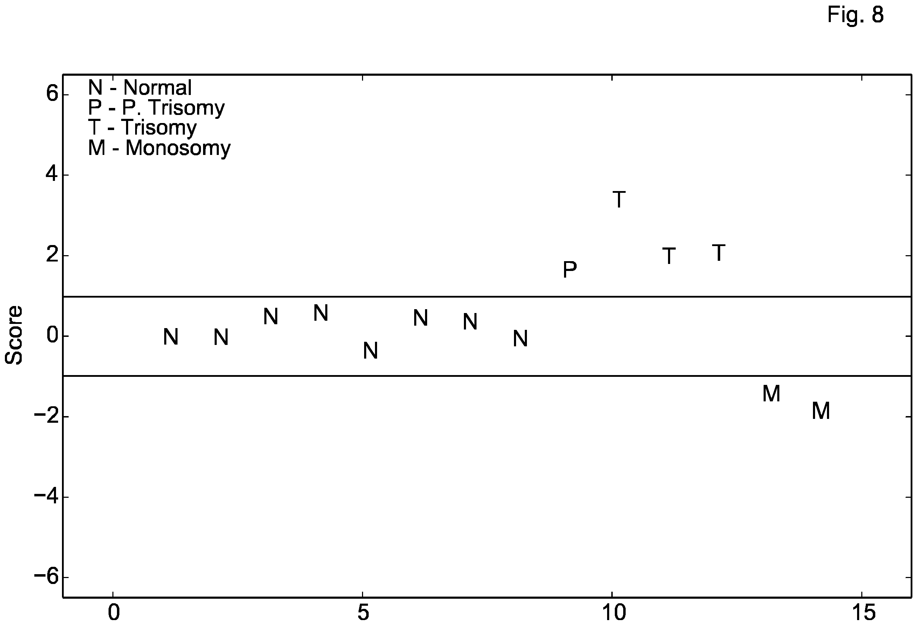

Figure 8 is a graph depicting results from fetal DNA samples that underwent ploidy status determination using TACS-based enrichment, followed by a score-based classification. As per the key, samples plotted with N indicate normal ploidy status, the sample plotted with P illustrates partial trisomy, the samples plotted with T indicate trisomy and the samples plotted with M indicate monosomy.

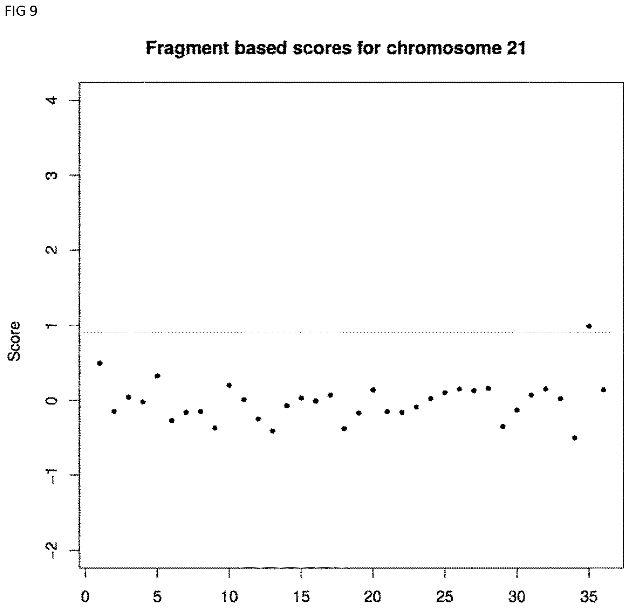

Figure 9 is a dot plot graph showing results of a fragments-based test for detecting increased numbers of smaller-size fragments in a mixed sample. An abnormal, aneuploid sample, with an estimated fetal fraction of 2.8%, was correctly detected using this method. The black dots are individual samples. The x-axis shows the sample index. The y-axis shows the score result of the fragments-size based method. A score result greater than the threshold shown by the grey line indicates a deviation from the expected size of fragments illustrating the presence of aneuploidy.

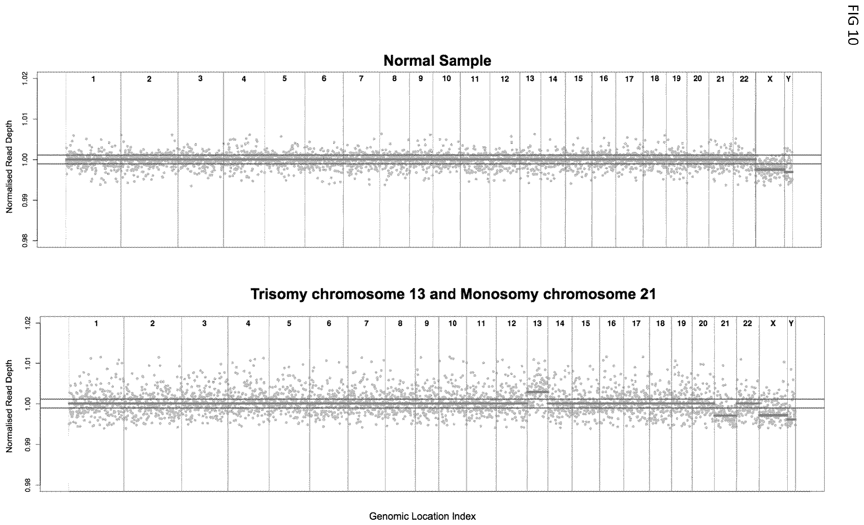

Figure 10 is a graph of results from fetal DNA samples that underwent ploidy status determination using likelihood-based segmentation analysis and TACS-based enrichment whole genome sequencing data. The horizontal blue line indicates the average read-depth of each chromosome.
The red lines indicate threshold intervals of expected diploids. Data above the top red line is classified as more than diploid and data below the red line is classified as less than diploid. The top panel illustrates the results of a euploid male sample (i.e., a male fetus with one copy of chromosome X chromosome and one copy of chromosome Y, and without any ploidy abnormalities present). The bottom panel illustrates the results of a male aneuploid sample with trisomy 13 and monosomy 21. Values on the y-axis are log-based transformations of read-depth.

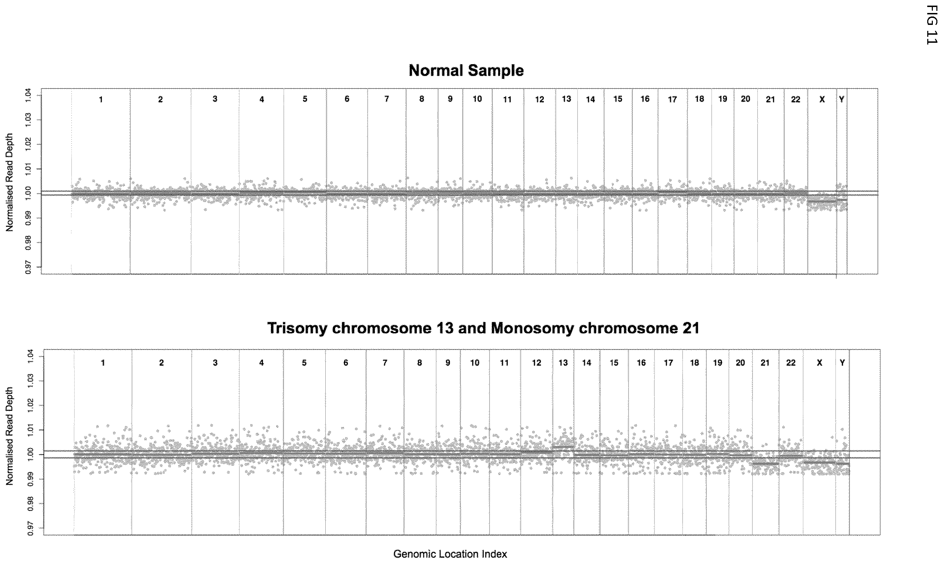

Figure 11 is a graph of results from fetal DNA samples that underwent ploidy status determination using likelihood-based segmentation analysis and TACS-based enrichment data. The horizontal blue line indicates the average read-depth of each chromosome. The red lines indicate threshold intervals of expected diploids. Data above the top red line is classified as more than diploid and data below the red line is classified as less than diploid. The top panel illustrates the results of a euploid male sample (i.e., a male fetus with one copy of chromosome X chromosome and one copy of chromosome Y, and without any ploidy abnormalities present). The bottom panel illustrates the results of a male aneuploid sample with trisomy 13 and monosomy 21. Values on the y-axis are log-based transformations of read-depth.

**Table 1 shows exemplary and preferred TACS positions. The corresponding sequences are depicted in the sequence protocol.**

| Ch. | Start | Stop | GC content | | Ch. | Start | Stop | GC content |
|---|---|---|---|---|---|---|---|---|
| chr1 | 1321966 | 1322216 | 0,348 | | chr10 | 9058150 | 9058400 | 0,3 |
| chr1 | 2223227 | 2223477 | 0,348 | | chr10 | 9876196 | 9876446 | 0,348 |
| chr1 | 3047692 | 3047942 | 0,348 | | chr10 | 10677282 | 10677532 | 0,348 |
| chr1 | 4134402 | 4134652 | 0,348 | | chr10 | 11492282 | 11492532 | 0,348 |
| chr1 | 5007713 | 5007963 | 0,348 | | chr10 | 12401604 | 12401854 | 0,348 |
| chr1 | 5865510 | 5865760 | 0,348 | | chr10 | 13224650 | 13224900 | 0,348 |
| chr1 | 6714342 | 6714592 | 0,348 | | chr10 | 14043964 | 14044214 | 0,348 |
| chr1 | 7651255 | 7651505 | 0,348 | | chr10 | 14853423 | 14853673 | 0,348 |
| chr1 | 8470924 | 8471174 | 0,348 | | chr10 | 15658199 | 15658449 | 0,316 |
| chr1 | 9407377 | 9407627 | 0,324 | | chr10 | 16622416 | 16622666 | 0,344 |
| chr1 | 10209181 | 10209431 | 0,296 | | chr10 | 17516150 | 17516400 | 0,276 |
| chr1 | 11076652 | 11076902 | 0,348 | | chr10 | 18901507 | 18901757 | 0,336 |
| chr1 | 12295996 | 12296246 | 0,348 | | chr10 | 19710316 | 19710566 | 0,344 |
| chr1 | 13923467 | 13923717 | 0,348 | | chr10 | 20513351 | 20513601 | 0,348 |
| chr1 | 14770392 | 14770642 | 0,348 | | chr10 | 21313889 | 21314139 | 0,348 |
| chr1 | 15578046 | 15578296 | 0,348 | | chr10 | 22142078 | 22142328 | 0,348 |
| chr1 | 16593363 | 16593613 | 0,348 | | chr10 | 22944374 | 22944624 | 0,348 |
| chr1 | 17424880 | 17425130 | 0,348 | | chr10 | 25115150 | 25115400 | 0,34 |
| chr1 | 18298306 | 18298556 | 0,348 | | chr10 | 25924260 | 25924510 | 0,348 |
| chr1 | 19423315 | 19423565 | 0,348 | | chr10 | 26730024 | 26730274 | 0,3 |
| chr1 | 20230997 | 20231247 | 0,348 | | chr10 | 27655856 | 27656106 | 0,348 |
| chr1 | 21064982 | 21065232 | 0,348 | | chr10 | 28491351 | 28491601 | 0,348 |
| chr1 | 22007055 | 22007305 | 0,348 | | chr10 | 29660163 | 29660413 | 0,348 |
| chr1 | 22807861 | 22808111 | 0,344 | | chr10 | 30460737 | 30460987 | 0,332 |
| chr1 | 23611830 | 23612080 | 0,348 | | chr10 | 31312134 | 31312384 | 0,312 |
| chr1 | 24692851 | 24693101 | 0,348 | | chr10 | 32164168 | 32164418 | 0,348 |
| chr1 | 25500621 | 25500871 | 0,348 | | chr10 | 33112517 | 33112767 | 0,308 |
| chr1 | 26321343 | 26321593 | 0,348 | | chr10 | 34810150 | 34810400 | 0,296 |
| chr1 | 27450716 | 27450966 | 0,348 | | chr10 | 35813150 | 35813400 | 0,34 |
| chr1 | 28296472 | 28296722 | 0,296 | | chr10 | 37514244 | 37514494 | 0,288 |
| chr1 | 29098007 | 29098257 | 0,348 | | chr10 | 38417233 | 38417483 | 0,348 |
| chr1 | 30034947 | 30035197 | 0,348 | | chr10 | 42889111 | 42889361 | 0,34 |
| chr1 | 30884476 | 30884726 | 0,348 | | chr10 | 43849286 | 43849536 | 0,348 |
| chr1 | 31759697 | 31759947 | 0,348 | | chr10 | 44650414 | 44650664 | 0,348 |
| chr1 | 32646478 | 32646728 | 0,348 | | chr10 | 45497370 | 45497620 | 0,32 |
| chr1 | 33479257 | 33479507 | 0,348 | | chr10 | 49404717 | 49404967 | 0,348 |
| chr1 | 34305150 | 34305400 | 0,348 | | chr10 | 50210193 | 50210443 | 0,348 |
| chr1 | 35132601 | 35132851 | 0,348 | | chr10 | 51026129 | 51026379 | 0,348 |
| chr1 | 35939215 | 35939465 | 0,348 | | chr10 | 52010556 | 52010806 | 0,348 |
| chr1 | 36744730 | 36744980 | 0,336 | | chr10 | 53059445 | 53059695 | 0,348 |
| chr1 | 37623596 | 37623846 | 0,348 | | chr10 | 54008212 | 54008462 | 0,304 |
| chr1 | 38444825 | 38445075 | 0,348 | | chr10 | 55079406 | 55079656 | 0,328 |
| chr1 | 39248090 | 39248340 | 0,348 | | chr10 | 55884413 | 55884663 | 0,348 |
| chr1 | 40135959 | 40136209 | 0,348 | | chr10 | 57175168 | 57175418 | 0,304 |
| chr1 | 41158448 | 41158698 | 0,348 | | chr10 | 58259360 | 58259610 | 0,32 |
| chr1 | 42642199 | 42642449 | 0,348 | | chr10 | 59429265 | 59429515 | 0,348 |
| chr1 | 43546530 | 43546780 | 0,308 | | chr10 | 60330265 | 60330515 | 0,348 |
| chr1 | 44429847 | 44430097 | 0,348 | | chr10 | 61209233 | 61209483 | 0,348 |
| chr1 | 45307055 | 45307305 | 0,348 | | chr10 | 62192170 | 62192420 | 0,348 |
| chr1 | 46108116 | 46108366 | 0,348 | | chr10 | 63359159 | 63359409 | 0,348 |
| chr1 | 47100462 | 47100712 | 0,324 | | chr10 | 64382591 | 64382841 | 0,332 |
| chr1 | 48012499 | 48012749 | 0,348 | | chr10 | 65188150 | 65188400 | 0,336 |
| chr1 | 48821604 | 48821854 | 0,268 | | chr10 | 65996966 | 65997216 | 0,348 |
| chr1 | 49632073 | 49632323 | 0,336 | | chr10 | 66856797 | 66857047 | 0,34 |
| chr1 | 50440111 | 50440361 | 0,332 | | chr10 | 67661286 | 67661536 | 0,348 |
| chr1 | 51241903 | 51242153 | 0,312 | | chr10 | 68465570 | 68465820 | 0,348 |
| chr1 | 52076744 | 52076994 | 0,348 | | chr10 | 69265603 | 69265853 | 0,284 |
| chr1 | 53710264 | 53710514 | 0,348 | | chr10 | 70087603 | 70087853 | 0,284 |
| chr1 | 54512550 | 54512800 | 0,344 | | chr10 | 70891648 | 70891898 | 0,312 |
| chr1 | 55394792 | 55395042 | 0,348 | | chr10 | 71718353 | 71718603 | 0,348 |
| chr1 | 56384481 | 56384731 | 0,348 | | chr10 | 72578751 | 72579001 | 0,344 |
| chr1 | 57349269 | 57349519 | 0,348 | | chr10 | 73736422 | 73736672 | 0,348 |
| chr1 | 58229509 | 58229759 | 0,348 | | chr10 | 74541034 | 74541284 | 0,348 |
| chr1 | 59040876 | 59041126 | 0,348 | | chr10 | 75344307 | 75344557 | 0,296 |
| chr1 | 59858357 | 59858607 | 0,304 | | chr10 | 76146051 | 76146301 | 0,288 |
| chr1 | 60930291 | 60930541 | 0,348 | | chr10 | 76988866 | 76989116 | 0,348 |
| chr1 | 62103549 | 62103799 | 0,304 | | chr10 | 78429430 | 78429680 | 0,348 |
| chr1 | 62916429 | 62916679 | 0,348 | | chr10 | 79322018 | 79322268 | 0,348 |
| chr1 | 64067557 | 64067807 | 0,348 | | chr10 | 80218152 | 80218402 | 0,348 |
| chr1 | 64969248 | 64969498 | 0,348 | | chr10 | 81110884 | 81111134 | 0,348 |
| chr1 | 65878461 | 65878711 | 0,348 | | chr10 | 82759514 | 82759764 | 0,348 |
| chr1 | 67063532 | 67063782 | 0,336 | | chr10 | 83909150 | 83909400 | 0,324 |
| chr1 | 67873342 | 67873592 | 0,348 | | chr10 | 84718905 | 84719155 | 0,348 |
| chr1 | 70446150 | 70446400 | 0,272 | | chr10 | 85528253 | 85528503 | 0,348 |
| chr1 | 71372533 | 71372783 | 0,348 | | chr10 | 86363170 | 86363420 | 0,348 |
| chr1 | 72327150 | 72327400 | 0,348 | | chr10 | 87163913 | 87164163 | 0,348 |
| chr1 | 73213150 | 73213400 | 0,332 | | chr10 | 87972367 | 87972617 | 0,348 |
| chr1 | 74040085 | 74040335 | 0,344 | | chr10 | 89475150 | 89475400 | 0,348 |
| chr1 | 74845564 | 74845814 | 0,348 | | chr10 | 90512150 | 90512400 | 0,328 |
| chr1 | 75862550 | 75862800 | 0,316 | | chr10 | 91327550 | 91327800 | 0,336 |
| chr1 | 76678210 | 76678460 | 0,348 | | chr10 | 92191303 | 92191553 | 0,348 |
| chr1 | 77512868 | 77513118 | 0,348 | | chr10 | 92993341 | 92993591 | 0,312 |
| chr1 | 78324741 | 78324991 | 0,284 | | chr10 | 93806630 | 93806880 | 0,304 |
| chr1 | 79622150 | 79622400 | 0,324 | | chr10 | 95371273 | 95371523 | 0,32 |
| chr1 | 79622150 | 79622400 | 0,324 | | chr10 | 96172179 | 96172429 | 0,324 |
| chr1 | 81028150 | 81028400 | 0,28 | | chr10 | 96973624 | 96973874 | 0,3 |
| chr1 | 81829490 | 81829740 | 0,3 | | chr10 | 97793018 | 97793268 | 0,34 |
| chr1 | 82631657 | 82631907 | 0,344 | | chr10 | 98599885 | 98600135 | 0,308 |
| chr1 | 83432297 | 83432547 | 0,348 | | chr10 | 99371221 | 99371471 | 0,608 |
| chr1 | 84232408 | 84232658 | 0,348 | | chr10 | 99444709 | 99444959 | 0,348 |
| chr1 | 85186300 | 85186550 | 0,348 | | chr10 | 100283039 | 100283289 | 0,336 |
| chr1 | 85987798 | 85988048 | 0,312 | | chr10 | 101099626 | 101099876 | 0,264 |
| chr1 | 86792219 | 86792469 | 0,3 | | chr10 | 101913511 | 101913761 | 0,348 |
| chr1 | 88716354 | 88716604 | 0,348 | | chr10 | 102718384 | 102718634 | 0,34 |
| chr1 | 89574150 | 89574400 | 0,344 | | chr10 | 103725234 | 103725484 | 0,348 |
| chr1 | 90818292 | 90818542 | 0,348 | | chr10 | 104591173 | 104591423 | 0,576 |
| chr1 | 91937586 | 91937836 | 0,332 | | chr10 | 104594996 | 104595246 | 0,568 |
| chr1 | 92757305 | 92757555 | 0,32 | | chr10 | 104595007 | 104595257 | 0,568 |
| chr1 | 93564210 | 93564460 | 0,328 | | chr10 | 104596713 | 104596963 | 0,532 |
| chr1 | 94366822 | 94367072 | 0,348 | | chr10 | 104596835 | 104597085 | 0,536 |
| chr1 | 94473766 | 94474015 | 0,53012 | | chr10 | 104596913 | 104597163 | 0,552 |
| chr1 | 94476259 | 94476508 | 0,566265 | | chr10 | 104816277 | 104816527 | 0,348 |
| chr1 | 94496446 | 94496695 | 0,546185 | | chr10 | 105676155 | 105676405 | 0,348 |
| chr1 | 94508204 | 94508453 | 0,554217 | | chr10 | 106811336 | 106811586 | 0,348 |
| chr1 | 94508724 | 94508973 | 0,570281 | | chr10 | 107659600 | 107659850 | 0,304 |
| chr1 | 94517145 | 94517394 | 0,369478 | | chr10 | 108512082 | 108512332 | 0,348 |
| chr1 | 94525991 | 94526240 | 0,477912 | | chr10 | 109373152 | 109373402 | 0,348 |
| chr1 | 95460410 | 95460660 | 0,336 | | chr10 | 110180327 | 110180577 | 0,348 |
| chr1 | 96550309 | 96550559 | 0,348 | | chr10 | 110981331 | 110981581 | 0,348 |
| chr1 | 97375580 | 97375830 | 0,348 | | chr10 | 112590150 | 112590400 | 0,324 |
| chr1 | 98175941 | 98176191 | 0,256 | | chr10 | 113580385 | 113580635 | 0,348 |
| chr1 | 99069150 | 99069400 | 0,3 | | chr10 | 115419322 | 115419572 | 0,348 |
| chr1 | 99919444 | 99919694 | 0,34 | | chr10 | 116388354 | 116388604 | 0,276 |
| chr1 | 100316484 | 100316734 | 0,34 | | chr10 | 117195710 | 117195960 | 0,264 |
| chr1 | 100316495 | 100316745 | 0,348 | | chr10 | 118001417 | 118001667 | 0,348 |
| chr1 | 100340820 | 100341070 | 0,344 | | chr10 | 118804036 | 118804286 | 0,312 |
| chr1 | 100346760 | 100347010 | 0,38 | | chr10 | 119604589 | 119604839 | 0,348 |
| chr1 | 100381830 | 100382080 | 0,28 | | chr10 | 121415468 | 121415718 | 0,348 |
| chr1 | 100381939 | 100382189 | 0,292 | | chr10 | 122562221 | 122562471 | 0,344 |
| chr1 | 100382143 | 100382393 | 0,356 | | chr10 | 123467150 | 123467400 | 0,324 |
| chr1 | 100881150 | 100881400 | 0,272 | | chr10 | 124501500 | 124501750 | 0,348 |
| chr1 | 101683272 | 101683522 | 0,348 | | chr10 | 125600180 | 125600430 | 0,348 |
| chr1 | 102490150 | 102490400 | 0,292 | | chr10 | 126768584 | 126768834 | 0,348 |
| chr1 | 103317424 | 103317674 | 0,348 | | chr10 | 127706472 | 127706722 | 0,348 |
| chr1 | 104122384 | 104122634 | 0,344 | | chr10 | 128534329 | 128534579 | 0,348 |
| chr1 | 104943912 | 104944162 | 0,328 | | chr10 | 129334357 | 129334607 | 0,348 |
| chr1 | 105852554 | 105852804 | 0,328 | | chr10 | 130135711 | 130135961 | 0,348 |
| chr1 | 107171238 | 107171488 | 0,32 | | chr10 | 131363498 | 131363748 | 0,348 |
| chr1 | 108028411 | 108028661 | 0,348 | | chr10 | 132216150 | 132216400 | 0,316 |
| chr1 | 108856564 | 108856814 | 0,288 | | chr10 | 133163299 | 133163549 | 0,348 |
| chr1 | 109676087 | 109676337 | 0,348 | | chr10 | 133998082 | 133998332 | 0,348 |
| chr1 | 110522602 | 110522852 | 0,348 | | chr11 | 244145 | 244395 | 0,348 |
| chr1 | 111340253 | 111340503 | 0,34 | | chr11 | 1320632 | 1320882 | 0,348 |
| chr1 | 112949435 | 112949685 | 0,332 | | chr11 | 2819685 | 2819935 | 0,348 |
| chr1 | 113770383 | 113770633 | 0,332 | | chr11 | 3630780 | 3631030 | 0,312 |
| chr1 | 114637753 | 114638003 | 0,348 | | chr11 | 4437568 | 4437818 | 0,312 |
| chr1 | 115437771 | 115438021 | 0,3 | | chr11 | 5246865 | 5247108 | 0,465021 |
| chr1 | 116573150 | 116573400 | 0,32 | | chr11 | 5247849 | 5248045 | 0,540816 |
| chr1 | 117560545 | 117560795 | 0,348 | | chr11 | 5247863 | 5248085 | 0,531532 |
| chr1 | 118572363 | 118572613 | 0,308 | | chr11 | 5247979 | 5248198 | 0,511416 |
| chr1 | 119423232 | 119423482 | 0,332 | | chr11 | 5248145 | 5248333 | 0,510638 |
| chr1 | 119957941 | 119958191 | 0,512 | | chr11 | 5261108 | 5261358 | 0,348 |
| chr1 | 119964669 | 119964919 | 0,508 | | chr11 | 6415310 | 6415560 | 0,576 |
| chr1 | 120230467 | 120230717 | 0,348 | | chr11 | 6415651 | 6415901 | 0,604 |
| chr1 | 120269395 | 120269645 | 0,58 | | chr11 | 6444321 | 6444571 | 0,348 |
| chr1 | 120277828 | 120278078 | 0,612 | | chr11 | 7612590 | 7612840 | 0,348 |
| chr1 | 120277930 | 120278180 | 0,604 | | chr11 | 8563550 | 8563800 | 0,292 |
| chr1 | 120284326 | 120284576 | 0,58 | | chr11 | 9416225 | 9416475 | 0,348 |
| chr1 | 120285389 | 120285639 | 0,636 | | chr11 | 10216982 | 10217232 | 0,324 |
| chr1 | 120286404 | 120286654 | 0,556 | | chr11 | 11061166 | 11061416 | 0,344 |
| chr1 | 144917078 | 144917328 | 0,312 | | chr11 | 12380273 | 12380523 | 0,348 |
| chr1 | 145416495 | 145416745 | 0,54 | | chr11 | 13235472 | 13235722 | 0,348 |
| chr1 | 145732849 | 145733099 | 0,348 | | chr11 | 14037966 | 14038216 | 0,268 |
| chr1 | 147385212 | 147385462 | 0,344 | | chr11 | 14864472 | 14864722 | 0,308 |
| chr1 | 149912055 | 149912305 | 0,348 | | chr11 | 15678839 | 15679089 | 0,344 |
| chr1 | 150722611 | 150722861 | 0,348 | | chr11 | 16481891 | 16482141 | 0,32 |
| chr1 | 151586322 | 151586572 | 0,348 | | chr11 | 17299219 | 17299469 | 0,312 |
| chr1 | 152399440 | 152399690 | 0,344 | | chr11 | 18110459 | 18110709 | 0,316 |
| chr1 | 153275352 | 153275602 | 0,348 | | chr11 | 18956824 | 18957074 | 0,348 |
| chr1 | 154245900 | 154246150 | 0,536 | | chr11 | 19766011 | 19766261 | 0,348 |
| chr1 | 154247516 | 154247766 | 0,464 | | chr11 | 20882553 | 20882803 | 0,348 |
| chr1 | 154812453 | 154812703 | 0,348 | | chr11 | 21696859 | 21697109 | 0,348 |
| chr1 | 155204665 | 155204914 | 0,562249 | | chr11 | 22498768 | 22499018 | 0,348 |
| chr1 | 155204957 | 155205206 | 0,574297 | | chr11 | 23301963 | 23302213 | 0,328 |
| chr1 | 155205406 | 155205571 | 0,521212 | | chr11 | 24105150 | 24105400 | 0,304 |
| chr1 | 155205538 | 155205717 | 0,564246 | | chr11 | 25703491 | 25703741 | 0,348 |
| chr1 | 155210424 | 155210673 | 0,562249 | | chr11 | 26517322 | 26517572 | 0,348 |
| chr1 | 155691410 | 155691660 | 0,348 | | chr11 | 27334775 | 27335025 | 0,332 |
| chr1 | 156691635 | 156691885 | 0,304 | | chr11 | 28364408 | 28364658 | 0,32 |
| chr1 | 157494327 | 157494577 | 0,348 | | chr11 | 29209327 | 29209577 | 0,348 |
| chr1 | 158381408 | 158381658 | 0,348 | | chr11 | 30166480 | 30166730 | 0,324 |
| chr1 | 159416150 | 159416400 | 0,348 | | chr11 | 31761546 | 31761796 | 0,348 |
| chr1 | 161320957 | 161321207 | 0,348 | | chr11 | 32622160 | 32622410 | 0,348 |
| chr1 | 162192273 | 162192523 | 0,348 | | chr11 | 33583297 | 33583547 | 0,348 |
| chr1 | 162995450 | 162995700 | 0,324 | | chr11 | 34970150 | 34970400 | 0,284 |
| chr1 | 163811190 | 163811440 | 0,348 | | chr11 | 35911282 | 35911532 | 0,348 |
| chr1 | 164673253 | 164673503 | 0,348 | | chr11 | 37092305 | 37092555 | 0,268 |
| chr1 | 165475943 | 165476193 | 0,348 | | chr11 | 37909582 | 37909832 | 0,348 |
| chr1 | 166300688 | 166300938 | 0,336 | | chr11 | 38712541 | 38712791 | 0,3 |
| chr1 | 167123296 | 167123546 | 0,336 | | chr11 | 39522829 | 39523079 | 0,348 |
| chr1 | 169063150 | 169063400 | 0,324 | | chr11 | 40714150 | 40714400 | 0,324 |
| chr1 | 170055150 | 170055400 | 0,328 | | chr11 | 41532238 | 41532488 | 0,348 |
| chr1 | 170920233 | 170920483 | 0,328 | | chr11 | 42571467 | 42571717 | 0,348 |
| chr1 | 171773161 | 171773411 | 0,348 | | chr11 | 43660553 | 43660803 | 0,336 |
| chr1 | 172673411 | 172673661 | 0,348 | | chr11 | 44580611 | 44580861 | 0,348 |
| chr1 | 173542401 | 173542651 | 0,288 | | chr11 | 45451636 | 45451886 | 0,32 |
| chr1 | 174517204 | 174517454 | 0,348 | | chr11 | 46261790 | 46262040 | 0,348 |
| chr1 | 175778380 | 175778630 | 0,332 | | chr11 | 47072439 | 47072689 | 0,348 |
| chr1 | 176580310 | 176580560 | 0,348 | | chr11 | 47879220 | 47879470 | 0,348 |
| chr1 | 177395900 | 177396150 | 0,348 | | chr11 | 49919350 | 49919600 | 0,344 |
| chr1 | 178513548 | 178513798 | 0,324 | | chr11 | 55082493 | 55082743 | 0,348 |
| chr1 | 179452150 | 179452400 | 0,344 | | chr11 | 56022150 | 56022400 | 0,336 |
| chr1 | 179521616 | 179521866 | 0,532 | | chr11 | 56842806 | 56843056 | 0,34 |
| chr1 | 179521631 | 179521881 | 0,52 | | chr11 | 57725394 | 57725644 | 0,348 |
| chr1 | 179526237 | 179526487 | 0,42 | | chr11 | 58526329 | 58526579 | 0,288 |
| chr1 | 179530337 | 179530587 | 0,376 | | chr11 | 59347634 | 59347884 | 0,34 |
| chr1 | 179544561 | 179544811 | 0,596 | | chr11 | 60158561 | 60158811 | 0,348 |
| chr1 | 180255735 | 180255985 | 0,316 | | chr11 | 61095547 | 61095797 | 0,312 |
| chr1 | 181056840 | 181057090 | 0,348 | | chr11 | 61940132 | 61940382 | 0,348 |
| chr1 | 182634465 | 182634715 | 0,348 | | chr11 | 62810381 | 62810631 | 0,348 |
| chr1 | 183811332 | 183811582 | 0,348 | | chr11 | 63633992 | 63634242 | 0,348 |
| chr1 | 184719150 | 184719400 | 0,308 | | chr11 | 64538602 | 64538852 | 0,348 |
| chr1 | 185737150 | 185737400 | 0,344 | | chr11 | 65827667 | 65827917 | 0,348 |
| chr1 | 186544294 | 186544544 | 0,336 | | chr11 | 66889179 | 66889429 | 0,348 |
| chr1 | 187345956 | 187346206 | 0,348 | | chr11 | 67837487 | 67837737 | 0,348 |
| chr1 | 188148703 | 188148953 | 0,328 | | chr11 | 68763563 | 68763813 | 0,348 |
| chr1 | 188964150 | 188964400 | 0,3 | | chr11 | 69829791 | 69830041 | 0,348 |
| chr1 | 189860180 | 189860430 | 0,312 | | chr11 | 70707409 | 70707659 | 0,348 |
| chr1 | 191057168 | 191057418 | 0,348 | | chr11 | 71743478 | 71743728 | 0,332 |
| chr1 | 191860751 | 191861001 | 0,344 | | chr11 | 72548958 | 72549208 | 0,348 |
| chr1 | 192733150 | 192733400 | 0,348 | | chr11 | 73427854 | 73428104 | 0,324 |
| chr1 | 193629150 | 193629400 | 0,268 | | chr11 | 74260390 | 74260640 | 0,32 |
| chr1 | 194870567 | 194870817 | 0,272 | | chr11 | 75308356 | 75308606 | 0,348 |
| chr1 | 195678176 | 195678426 | 0,348 | | chr11 | 76140267 | 76140517 | 0,348 |
| chr1 | 196491241 | 196491491 | 0,308 | | chr11 | 76942624 | 76942874 | 0,312 |
| chr1 | 197292396 | 197292646 | 0,312 | | chr11 | 77810095 | 77810345 | 0,348 |
| chr1 | 198093741 | 198093991 | 0,288 | | chr11 | 78638250 | 78638500 | 0,324 |
| chr1 | 199102394 | 199102644 | 0,32 | | chr11 | 79439911 | 79440161 | 0,336 |
| chr1 | 199910959 | 199911209 | 0,348 | | chr11 | 80667558 | 80667808 | 0,308 |
| chr1 | 200726178 | 200726428 | 0,336 | | chr11 | 81817150 | 81817400 | 0,3 |
| chr1 | 201594767 | 201595017 | 0,348 | | chr11 | 82620784 | 82621034 | 0,3 |
| chr1 | 202763399 | 202763649 | 0,348 | | chr11 | 83424544 | 83424794 | 0,348 |
| chr1 | 203583274 | 203583524 | 0,344 | | chr11 | 84581358 | 84581608 | 0,336 |
| chr1 | 204505599 | 204505849 | 0,348 | | chr11 | 85477260 | 85477510 | 0,348 |
| chr1 | 205323323 | 205323573 | 0,348 | | chr11 | 86731237 | 86731487 | 0,348 |
| chr1 | 206199203 | 206199453 | 0,348 | | chr11 | 87860240 | 87860490 | 0,348 |
| chr1 | 207040001 | 207040251 | 0,348 | | chr11 | 88900460 | 88900710 | 0,316 |
| chr1 | 208628219 | 208628469 | 0,348 | | chr11 | 89859383 | 89859633 | 0,348 |
| chr1 | 209429745 | 209429995 | 0,348 | | chr11 | 90678351 | 90678601 | 0,316 |
| chr1 | 211050331 | 211050581 | 0,348 | | chr11 | 91493550 | 91493800 | 0,328 |
| chr1 | 211854312 | 211854562 | 0,308 | | chr11 | 92523358 | 92523608 | 0,348 |
| chr1 | 212715103 | 212715353 | 0,332 | | chr11 | 93330704 | 93330954 | 0,348 |
| chr1 | 213681370 | 213681620 | 0,348 | | chr11 | 94966331 | 94966581 | 0,348 |
| chr1 | 214976150 | 214976400 | 0,32 | | chr11 | 95920272 | 95920522 | 0,348 |
| chr1 | 215844302 | 215844552 | 0,416 | | chr11 | 96726378 | 96726628 | 0,288 |
| chr1 | 215853595 | 215853845 | 0,416 | | chr11 | 97527417 | 97527667 | 0,324 |
| chr1 | 215992471 | 215992721 | 0,328 | | chr11 | 98344481 | 98344731 | 0,324 |
| chr1 | 216420312 | 216420562 | 0,46 | | chr11 | 99148179 | 99148429 | 0,348 |
| chr1 | 216420402 | 216420652 | 0,4 | | chr11 | 100371430 | 100371680 | 0,336 |
| chr1 | 216497437 | 216497687 | 0,328 | | chr11 | 101194937 | 101195187 | 0,348 |
| chr1 | 216792844 | 216793094 | 0,348 | | chr11 | 102003016 | 102003266 | 0,348 |
| chr1 | 217599535 | 217599785 | 0,276 | | chr11 | 102809148 | 102809398 | 0,332 |
| chr1 | 219297150 | 219297400 | 0,32 | | chr11 | 103620204 | 103620454 | 0,348 |
| chr1 | 220100279 | 220100529 | 0,348 | | chr11 | 104509150 | 104509400 | 0,328 |
| chr1 | 220903327 | 220903577 | 0,348 | | chr11 | 106310150 | 106310400 | 0,336 |
| chr1 | 222029233 | 222029483 | 0,348 | | chr11 | 107416442 | 107416692 | 0,348 |
| chr1 | 222831067 | 222831317 | 0,304 | | chr11 | 108154976 | 108155226 | 0,38 |
| chr1 | 223637903 | 223638153 | 0,348 | | chr11 | 108203436 | 108203686 | 0,34 |
| chr1 | 224462520 | 224462770 | 0,324 | | chr11 | 108419336 | 108419586 | 0,348 |
| chr1 | 225395150 | 225395400 | 0,328 | | chr11 | 109219928 | 109220178 | 0,348 |
| chr1 | 226223152 | 226223402 | 0,348 | | chr11 | 110346453 | 110346703 | 0,348 |
| chr1 | 227178529 | 227178779 | 0,348 | | chr11 | 111248566 | 111248816 | 0,348 |
| chr1 | 228644123 | 228644373 | 0,348 | | chr11 | 112099263 | 112099513 | 0,356 |
| chr1 | 229446849 | 229447099 | 0,348 | | chr11 | 112103776 | 112104026 | 0,392 |
| chr1 | 230259328 | 230259578 | 0,328 | | chr11 | 112103798 | 112104048 | 0,384 |
| chr1 | 231872599 | 231872849 | 0,332 | | chr11 | 112361216 | 112361466 | 0,348 |
| chr1 | 232812441 | 232812691 | 0,328 | | chr11 | 113186794 | 113187044 | 0,348 |
| chr1 | 233881150 | 233881400 | 0,296 | | chr11 | 114018172 | 114018422 | 0,348 |
| chr1 | 234687934 | 234688184 | 0,312 | | chr11 | 115212550 | 115212800 | 0,3 |
| chr1 | 235489202 | 235489452 | 0,324 | | chr11 | 116431362 | 116431612 | 0,348 |
| chr1 | 236335526 | 236335776 | 0,348 | | chr11 | 117338011 | 117338261 | 0,348 |
| chr1 | 237165928 | 237166178 | 0,348 | | chr11 | 118182107 | 118182357 | 0,32 |
| chr1 | 238564150 | 238564400 | 0,308 | | chr11 | 118895800 | 118896050 | 0,544 |
| chr1 | 239364391 | 239364641 | 0,3 | | chr11 | 118895869 | 118896119 | 0,552 |
| chr1 | 240522579 | 240522829 | 0,344 | | chr11 | 118895879 | 118896129 | 0,552 |
| chr1 | 242534150 | 242534400 | 0,328 | | chr11 | 119078108 | 119078358 | 0,348 |
| chr1 | 243386411 | 243386661 | 0,332 | | chr11 | 120178614 | 120178864 | 0,348 |
| chr1 | 244192638 | 244192888 | 0,348 | | chr11 | 121065150 | 121065400 | 0,316 |
| chr1 | 245000355 | 245000605 | 0,348 | | chr11 | 122640481 | 122640731 | 0,348 |
| chr1 | 245854798 | 245855048 | 0,348 | | chr11 | 123618251 | 123618501 | 0,336 |
| chr1 | 246660293 | 246660543 | 0,316 | | chr11 | 124545250 | 124545500 | 0,348 |
| chr1 | 247618340 | 247618590 | 0,348 | | chr11 | 125353052 | 125353302 | 0,348 |
| chr1 | 248428706 | 248428956 | 0,308 | | chr11 | 126357309 | 126357559 | 0,348 |
| chr2 | 65470 | 65720 | 0,348 | | chr11 | 127164952 | 127165202 | 0,348 |
| chr2 | 887693 | 887943 | 0,32 | | chr11 | 127969605 | 127969855 | 0,348 |
| chr2 | 1696872 | 1697122 | 0,348 | | chr11 | 130098150 | 130098400 | 0,332 |
| chr2 | 2498456 | 2498706 | 0,304 | | chr11 | 131496162 | 131496412 | 0,34 |
| chr2 | 3336432 | 3336682 | 0,348 | | chr11 | 132296933 | 132297183 | 0,348 |
| chr2 | 4140186 | 4140436 | 0,348 | | chr11 | 133138077 | 133138327 | 0,348 |
| chr2 | 4957104 | 4957354 | 0,348 | | chr11 | 133944178 | 133944428 | 0,336 |
| chr2 | 6772150 | 6772400 | 0,34 | | chr12 | 1010150 | 1010400 | 0,316 |
| chr2 | 7580936 | 7581186 | 0,348 | | chr12 | 2078150 | 2078400 | 0,316 |
| chr2 | 8382165 | 8382415 | 0,348 | | chr12 | 2998433 | 2998683 | 0,348 |
| chr2 | 9193965 | 9194215 | 0,348 | | chr12 | 3852150 | 3852400 | 0,312 |
| chr2 | 10008838 | 10009088 | 0,304 | | chr12 | 4652702 | 4652952 | 0,252 |
| chr2 | 10811702 | 10811952 | 0,34 | | chr12 | 5476654 | 5476904 | 0,348 |
| chr2 | 11639024 | 11639274 | 0,348 | | chr12 | 7509182 | 7509432 | 0,348 |
| chr2 | 12448608 | 12448858 | 0,336 | | chr12 | 8602743 | 8602993 | 0,344 |
| chr2 | 13475150 | 13475400 | 0,34 | | chr12 | 10532150 | 10532400 | 0,32 |
| chr2 | 14298194 | 14298444 | 0,348 | | chr12 | 11669397 | 11669647 | 0,348 |
| chr2 | 15098693 | 15098943 | 0,348 | | chr12 | 12624150 | 12624400 | 0,348 |
| chr2 | 15948834 | 15949084 | 0,344 | | chr12 | 14017296 | 14017546 | 0,348 |
| chr2 | 16749787 | 16750037 | 0,348 | | chr12 | 14825153 | 14825403 | 0,348 |
| chr2 | 17563803 | 17564053 | 0,288 | | chr12 | 15864451 | 15864701 | 0,348 |
| chr2 | 18584239 | 18584489 | 0,348 | | chr12 | 17209551 | 17209801 | 0,348 |
| chr2 | 19417426 | 19417676 | 0,348 | | chr12 | 18564576 | 18564826 | 0,348 |
| chr2 | 20234783 | 20235033 | 0,348 | | chr12 | 19368406 | 19368656 | 0,312 |
| chr2 | 21034816 | 21035066 | 0,348 | | chr12 | 20169044 | 20169294 | 0,336 |
| chr2 | 21841601 | 21841851 | 0,328 | | chr12 | 21809388 | 21809638 | 0,348 |
| chr2 | 22644838 | 22645088 | 0,348 | | chr12 | 23194150 | 23194400 | 0,324 |
| chr2 | 23466443 | 23466693 | 0,348 | | chr12 | 24378150 | 24378400 | 0,336 |
| chr2 | 24289207 | 24289457 | 0,332 | | chr12 | 25261150 | 25261400 | 0,252 |
| chr2 | 25100859 | 25101109 | 0,348 | | chr12 | 26222263 | 26222513 | 0,348 |
| chr2 | 25957531 | 25957781 | 0,348 | | chr12 | 27032150 | 27032400 | 0,296 |
| chr2 | 26782767 | 26783017 | 0,348 | | chr12 | 28065150 | 28065400 | 0,312 |
| chr2 | 27595658 | 27595908 | 0,348 | | chr12 | 28867298 | 28867548 | 0,344 |
| chr2 | 28407842 | 28408092 | 0,316 | | chr12 | 29672600 | 29672850 | 0,348 |
| chr2 | 29274893 | 29275143 | 0,348 | | chr12 | 30484504 | 30484754 | 0,348 |
| chr2 | 30090065 | 30090315 | 0,348 | | chr12 | 31354128 | 31354378 | 0,28 |
| chr2 | 30952180 | 30952430 | 0,348 | | chr12 | 32233641 | 32233891 | 0,348 |
| chr2 | 31755042 | 31755292 | 0,348 | | chr12 | 33035048 | 33035298 | 0,348 |
| chr2 | 32583549 | 32583799 | 0,348 | | chr12 | 33852725 | 33852975 | 0,316 |
| chr2 | 33391150 | 33391400 | 0,276 | | chr12 | 38762712 | 38762962 | 0,312 |
| chr2 | 34383150 | 34383400 | 0,348 | | chr12 | 39568259 | 39568509 | 0,348 |
| chr2 | 35195332 | 35195582 | 0,344 | | chr12 | 41547550 | 41547800 | 0,304 |
| chr2 | 36137213 | 36137463 | 0,348 | | chr12 | 42374550 | 42374800 | 0,324 |
| chr2 | 36943435 | 36943685 | 0,348 | | chr12 | 43286446 | 43286696 | 0,324 |
| chr2 | 37916535 | 37916785 | 0,348 | | chr12 | 44125571 | 44125821 | 0,324 |
| chr2 | 38718989 | 38719239 | 0,348 | | chr12 | 44928251 | 44928501 | 0,348 |
| chr2 | 39520447 | 39520697 | 0,252 | | chr12 | 45741587 | 45741837 | 0,28 |
| chr2 | 40715152 | 40715402 | 0,348 | | chr12 | 47018150 | 47018400 | 0,32 |
| chr2 | 41775150 | 41775400 | 0,336 | | chr12 | 47954368 | 47954618 | 0,348 |
| chr2 | 42935152 | 42935402 | 0,348 | | chr12 | 48807366 | 48807616 | 0,348 |
| chr2 | 43736701 | 43736951 | 0,288 | | chr12 | 49653664 | 49653914 | 0,348 |
| chr2 | 44201259 | 44201509 | 0,356 | | chr12 | 50552411 | 50552661 | 0,348 |
| chr2 | 45388418 | 45388668 | 0,34 | | chr12 | 51355238 | 51355488 | 0,348 |
| chr2 | 46218740 | 46218990 | 0,348 | | chr12 | 52156338 | 52156588 | 0,348 |
| chr2 | 47124807 | 47125057 | 0,328 | | chr12 | 53047852 | 53048102 | 0,348 |
| chr2 | 48209532 | 48209782 | 0,348 | | chr12 | 53867986 | 53868236 | 0,348 |
| chr2 | 49436565 | 49436815 | 0,348 | | chr12 | 54849331 | 54849581 | 0,348 |
| chr2 | 50262150 | 50262400 | 0,312 | | chr12 | 55967213 | 55967463 | 0,328 |
| chr2 | 51067246 | 51067496 | 0,304 | | chr12 | 57136531 | 57136781 | 0,324 |
| chr2 | 51923177 | 51923427 | 0,348 | | chr12 | 58191150 | 58191400 | 0,276 |
| chr2 | 52934234 | 52934484 | 0,348 | | chr12 | 59121159 | 59121409 | 0,348 |
| chr2 | 53762231 | 53762481 | 0,348 | | chr12 | 59962108 | 59962358 | 0,324 |
| chr2 | 54564438 | 54564688 | 0,348 | | chr12 | 60789979 | 60790229 | 0,252 |
| chr2 | 55380451 | 55380701 | 0,348 | | chr12 | 62345357 | 62345607 | 0,348 |
| chr2 | 56181574 | 56181824 | 0,348 | | chr12 | 63226154 | 63226404 | 0,348 |
| chr2 | 57163150 | 57163400 | 0,316 | | chr12 | 64269210 | 64269460 | 0,256 |
| chr2 | 58358268 | 58358518 | 0,34 | | chr12 | 65278150 | 65278400 | 0,308 |
| chr2 | 59360150 | 59360400 | 0,328 | | chr12 | 66088150 | 66088400 | 0,348 |
| chr2 | 60236150 | 60236400 | 0,34 | | chr12 | 67025166 | 67025416 | 0,324 |
| chr2 | 61078467 | 61078717 | 0,348 | | chr12 | 68088206 | 68088456 | 0,348 |
| chr2 | 61898047 | 61898297 | 0,348 | | chr12 | 68891849 | 68892099 | 0,348 |
| chr2 | 63027252 | 63027502 | 0,348 | | chr12 | 70321174 | 70321424 | 0,332 |
| chr2 | 64476343 | 64476593 | 0,348 | | chr12 | 71418451 | 71418701 | 0,348 |
| chr2 | 65539531 | 65539781 | 0,348 | | chr12 | 72312150 | 72312400 | 0,296 |
| chr2 | 66468258 | 66468508 | 0,348 | | chr12 | 73121298 | 73121548 | 0,268 |
| chr2 | 67310247 | 67310497 | 0,348 | | chr12 | 74229436 | 74229686 | 0,308 |
| chr2 | 68121736 | 68121986 | 0,348 | | chr12 | 75137472 | 75137722 | 0,304 |
| chr2 | 68937150 | 68937400 | 0,324 | | chr12 | 75942150 | 75942400 | 0,32 |
| chr2 | 69754384 | 69754634 | 0,348 | | chr12 | 76747550 | 76747800 | 0,312 |
| chr2 | 70609376 | 70609626 | 0,348 | | chr12 | 77549810 | 77550060 | 0,26 |
| chr2 | 71418299 | 71418549 | 0,348 | | chr12 | 79018196 | 79018446 | 0,348 |
| chr2 | 72388795 | 72389045 | 0,348 | | chr12 | 79819302 | 79819552 | 0,288 |
| chr2 | 73673243 | 73673493 | 0,348 | | chr12 | 80623713 | 80623963 | 0,332 |
| chr2 | 74477048 | 74477298 | 0,348 | | chr12 | 81432262 | 81432512 | 0,324 |
| chr2 | 75293899 | 75294149 | 0,348 | | chr12 | 82234916 | 82235166 | 0,26 |
| chr2 | 76188150 | 76188400 | 0,348 | | chr12 | 83040197 | 83040447 | 0,348 |
| chr2 | 77065379 | 77065629 | 0,288 | | chr12 | 83842541 | 83842791 | 0,348 |
| chr2 | 77963477 | 77963727 | 0,292 | | chr12 | 84644338 | 84644588 | 0,272 |
| chr2 | 79082465 | 79082715 | 0,316 | | chr12 | 86518150 | 86518400 | 0,344 |
| chr2 | 79883120 | 79883370 | 0,324 | | chr12 | 87422150 | 87422400 | 0,288 |
| chr2 | 80684819 | 80685069 | 0,348 | | chr12 | 88420350 | 88420600 | 0,348 |
| chr2 | 81668320 | 81668570 | 0,348 | | chr12 | 89430494 | 89430744 | 0,348 |
| chr2 | 82672150 | 82672400 | 0,316 | | chr12 | 90270282 | 90270532 | 0,348 |
| chr2 | 83483150 | 83483400 | 0,344 | | chr12 | 91174165 | 91174415 | 0,264 |
| chr2 | 84462272 | 84462522 | 0,348 | | chr12 | 92085151 | 92085401 | 0,348 |
| chr2 | 85281169 | 85281419 | 0,348 | | chr12 | 92947510 | 92947760 | 0,348 |
| chr2 | 86625495 | 86625745 | 0,32 | | chr12 | 93971575 | 93971825 | 0,348 |
| chr2 | 88326662 | 88326912 | 0,28 | | chr12 | 95097150 | 95097400 | 0,34 |
| chr2 | 89132432 | 89132682 | 0,348 | | chr12 | 96292457 | 96292707 | 0,336 |
| chr2 | 90105696 | 90105946 | 0,328 | | chr12 | 97672267 | 97672517 | 0,348 |
| chr2 | 95627799 | 95628049 | 0,284 | | chr12 | 98631302 | 98631552 | 0,348 |
| chr2 | 96845176 | 96845426 | 0,348 | | chr12 | 99884150 | 99884400 | 0,276 |
| chr2 | 97651219 | 97651469 | 0,348 | | chr12 | 101143158 | 101143408 | 0,348 |
| chr2 | 98452233 | 98452483 | 0,348 | | chr12 | 102015150 | 102015400 | 0,324 |
| chr2 | 99255916 | 99256166 | 0,332 | | chr12 | 103234177 | 103234426 | 0,46988 |
| chr2 | 100057041 | 100057291 | 0,348 | | chr12 | 103234235 | 103234340 | 0,504762 |
| chr2 | 100890150 | 100890400 | 0,3 | | chr12 | 103237341 | 103237591 | 0,512 |
| chr2 | 102415179 | 102415429 | 0,316 | | chr12 | 103237398 | 103237647 | 0,477912 |
| chr2 | 103622548 | 103622798 | 0,348 | | chr12 | 103237926 | 103238175 | 0,405622 |
| chr2 | 104670507 | 104670757 | 0,348 | | chr12 | 103240539 | 103240788 | 0,477912 |
| chr2 | 105567150 | 105567400 | 0,332 | | chr12 | 103245348 | 103245598 | 0,5 |
| chr2 | 106412373 | 106412623 | 0,336 | | chr12 | 103245355 | 103245604 | 0,497992 |
| chr2 | 107768153 | 107768403 | 0,348 | | chr12 | 103245396 | 103245646 | 0,456 |
| chr2 | 108612236 | 108612486 | 0,284 | | chr12 | 103246529 | 103246778 | 0,538153 |
| chr2 | 109664556 | 109664806 | 0,316 | | chr12 | 103248878 | 103249127 | 0,453815 |
| chr2 | 110464569 | 110464819 | 0,348 | | chr12 | 103248915 | 103249165 | 0,464 |
| chr2 | 111395348 | 111395598 | 0,3 | | chr12 | 103260367 | 103260616 | 0,445783 |
| chr2 | 112278329 | 112278579 | 0,348 | | chr12 | 103260384 | 103260490 | 0,45283 |
| chr2 | 113583150 | 113583400 | 0,304 | | chr12 | 103262150 | 103262400 | 0,304 |
| chr2 | 114468912 | 114469162 | 0,264 | | chr12 | 103271293 | 103271492 | 0,492462 |
| chr2 | 115268995 | 115269245 | 0,348 | | chr12 | 103288527 | 103288776 | 0,441767 |
| chr2 | 116107157 | 116107407 | 0,328 | | chr12 | 103306591 | 103306840 | 0,345382 |
| chr2 | 117369331 | 117369581 | 0,344 | | chr12 | 104098004 | 104098254 | 0,348 |
| chr2 | 118244266 | 118244516 | 0,316 | | chr12 | 106181481 | 106181731 | 0,344 |
| chr2 | 119059803 | 119060053 | 0,348 | | chr12 | 107176462 | 107176712 | 0,348 |
| chr2 | 119900354 | 119900604 | 0,348 | | chr12 | 108008885 | 108009135 | 0,348 |
| chr2 | 121044398 | 121044648 | 0,304 | | chr12 | 108830808 | 108831058 | 0,332 |
| chr2 | 122113389 | 122113639 | 0,348 | | chr12 | 109748594 | 109748844 | 0,348 |
| chr2 | 122919222 | 122919472 | 0,348 | | chr12 | 109994761 | 109995011 | 0,504 |
| chr2 | 123777443 | 123777693 | 0,348 | | chr12 | 109994805 | 109995055 | 0,48 |
| chr2 | 124919150 | 124919400 | 0,332 | | chr12 | 110555517 | 110555767 | 0,328 |
| chr2 | 126026342 | 126026592 | 0,292 | | chr12 | 111363237 | 111363487 | 0,308 |
| chr2 | 126917504 | 126917754 | 0,344 | | chr12 | 112247405 | 112247655 | 0,348 |
| chr2 | 128045375 | 128045625 | 0,336 | | chr12 | 113673150 | 113673400 | 0,292 |
| chr2 | 129682980 | 129683230 | 0,252 | | chr12 | 114525604 | 114525854 | 0,348 |
| chr2 | 130487549 | 130487799 | 0,348 | | chr12 | 115358313 | 115358563 | 0,34 |
| chr2 | 131534801 | 131535051 | 0,34 | | chr12 | 116191119 | 116191369 | 0,348 |
| chr2 | 133127584 | 133127834 | 0,348 | | chr12 | 117911938 | 117912188 | 0,312 |
| chr2 | 134661154 | 134661404 | 0,348 | | chr12 | 118730793 | 118731043 | 0,348 |
| chr2 | 135922383 | 135922633 | 0,348 | | chr12 | 119551405 | 119551655 | 0,348 |
| chr2 | 136723496 | 136723746 | 0,348 | | chr12 | 120405381 | 120405631 | 0,348 |
| chr2 | 137528425 | 137528675 | 0,344 | | chr12 | 121175612 | 121175862 | 0,628 |
| chr2 | 138373550 | 138373800 | 0,34 | | chr12 | 121176887 | 121177137 | 0,648 |
| chr2 | 139318150 | 139318400 | 0,288 | | chr12 | 121176999 | 121177249 | 0,64 |
| chr2 | 140527261 | 140527511 | 0,348 | | chr12 | 121210585 | 121210835 | 0,348 |
| chr2 | 141332198 | 141332448 | 0,312 | | chr12 | 122377840 | 122378090 | 0,252 |
| chr2 | 142149579 | 142149829 | 0,348 | | chr12 | 123239426 | 123239676 | 0,336 |
| chr2 | 142949600 | 142949850 | 0,268 | | chr12 | 124104421 | 124104671 | 0,348 |
| chr2 | 144077240 | 144077490 | 0,328 | | chr12 | 125045274 | 125045524 | 0,348 |
| chr2 | 144964208 | 144964458 | 0,348 | | chr12 | 125856241 | 125856491 | 0,348 |
| chr2 | 145817150 | 145817400 | 0,332 | | chr12 | 126656299 | 126656549 | 0,348 |
| chr2 | 146618150 | 146618400 | 0,312 | | chr12 | 128000199 | 128000449 | 0,348 |
| chr2 | 147969538 | 147969788 | 0,348 | | chr12 | 128945526 | 128945776 | 0,348 |
| chr2 | 149217150 | 149217400 | 0,304 | | chr12 | 129761193 | 129761443 | 0,348 |
| chr2 | 150017703 | 150017953 | 0,348 | | chr12 | 130586431 | 130586681 | 0,348 |
| chr2 | 150828995 | 150829245 | 0,336 | | chr12 | 131407236 | 131407486 | 0,348 |
| chr2 | 151767165 | 151767415 | 0,348 | | chr12 | 132208361 | 132208611 | 0,348 |
| chr2 | 152568463 | 152568713 | 0,348 | | chr12 | 133160778 | 133161028 | 0,348 |
| chr2 | 153683234 | 153683484 | 0,3 | | chr13 | 19509424 | 19509674 | 0,348 |
| chr2 | 154938150 | 154938400 | 0,348 | | chr13 | 20346157 | 20346407 | 0,344 |
| chr2 | 156008150 | 156008400 | 0,32 | | chr13 | 21163780 | 21164030 | 0,288 |
| chr2 | 156870242 | 156870492 | 0,348 | | chr13 | 21966517 | 21966767 | 0,332 |
| chr2 | 158163167 | 158163417 | 0,348 | | chr13 | 22769312 | 22769562 | 0,344 |
| chr2 | 159077150 | 159077400 | 0,288 | | chr13 | 23577459 | 23577709 | 0,336 |
| chr2 | 159891571 | 159891821 | 0,348 | | chr13 | 23909041 | 23909291 | 0,352 |
| chr2 | 161025175 | 161025425 | 0,348 | | chr13 | 23910386 | 23910636 | 0,392 |
| chr2 | 161831540 | 161831790 | 0,348 | | chr13 | 24381225 | 24381475 | 0,348 |
| chr2 | 162632193 | 162632443 | 0,316 | | chr13 | 25685243 | 25685493 | 0,324 |
| chr2 | 163715424 | 163715674 | 0,3 | | chr13 | 26952583 | 26952833 | 0,344 |
| chr2 | 165052569 | 165052819 | 0,348 | | chr13 | 28009207 | 28009457 | 0,348 |
| chr2 | 166288165 | 166288415 | 0,348 | | chr13 | 29040155 | 29040405 | 0,348 |
| chr2 | 167465150 | 167465400 | 0,296 | | chr13 | 30258409 | 30258659 | 0,348 |
| chr2 | 168517553 | 168517803 | 0,348 | | chr13 | 31078371 | 31078621 | 0,348 |
| chr2 | 169362170 | 169362420 | 0,348 | | chr13 | 31990150 | 31990400 | 0,328 |
| chr2 | 169780201 | 169780451 | 0,5 | | chr13 | 32794049 | 32794299 | 0,348 |
| chr2 | 169826516 | 169826766 | 0,484 | | chr13 | 33596179 | 33596429 | 0,316 |
| chr2 | 169832981 | 169833231 | 0,404 | | chr13 | 34427302 | 34427552 | 0,348 |
| chr2 | 169847204 | 169847454 | 0,444 | | chr13 | 35241150 | 35241400 | 0,328 |
| chr2 | 170163218 | 170163468 | 0,288 | | chr13 | 36047181 | 36047431 | 0,296 |
| chr2 | 171000920 | 171001170 | 0,348 | | chr13 | 36965545 | 36965795 | 0,348 |
| chr2 | 171805339 | 171805589 | 0,308 | | chr13 | 37907550 | 37907800 | 0,328 |
| chr2 | 172606368 | 172606618 | 0,3 | | chr13 | 38920435 | 38920685 | 0,348 |
| chr2 | 173421849 | 173422099 | 0,348 | | chr13 | 39771325 | 39771575 | 0,336 |
| chr2 | 174223616 | 174223866 | 0,348 | | chr13 | 40882353 | 40882603 | 0,348 |
| chr2 | 175024289 | 175024539 | 0,252 | | chr13 | 41765150 | 41765400 | 0,32 |
| chr2 | 176231150 | 176231400 | 0,328 | | chr13 | 42575249 | 42575499 | 0,344 |
| chr2 | 177265150 | 177265400 | 0,304 | | chr13 | 43379109 | 43379359 | 0,348 |
| chr2 | 178168408 | 178168658 | 0,348 | | chr13 | 44311168 | 44311418 | 0,348 |
| chr2 | 178969134 | 178969384 | 0,348 | | chr13 | 45121408 | 45121658 | 0,312 |
| chr2 | 179769150 | 179769400 | 0,344 | | chr13 | 46109150 | 46109400 | 0,332 |
| chr2 | 181084474 | 181084724 | 0,348 | | chr13 | 47732544 | 47732794 | 0,328 |
| chr2 | 181981239 | 181981489 | 0,348 | | chr13 | 48942150 | 48942400 | 0,268 |
| chr2 | 182819465 | 182819715 | 0,348 | | chr13 | 49742819 | 49743069 | 0,348 |
| chr2 | 183718150 | 183718400 | 0,3 | | chr13 | 50549888 | 50550138 | 0,332 |
| chr2 | 184593423 | 184593673 | 0,292 | | chr13 | 51354438 | 51354688 | 0,348 |
| chr2 | 185397270 | 185397520 | 0,316 | | chr13 | 52162009 | 52162259 | 0,348 |
| chr2 | 186197382 | 186197632 | 0,28 | | chr13 | 52966531 | 52966781 | 0,348 |
| chr2 | 187064150 | 187064400 | 0,284 | | chr13 | 53770557 | 53770807 | 0,344 |
| chr2 | 187967150 | 187967400 | 0,348 | | chr13 | 54575830 | 54576080 | 0,348 |
| chr2 | 188804233 | 188804483 | 0,32 | | chr13 | 55376257 | 55376507 | 0,332 |
| chr2 | 189831150 | 189831400 | 0,348 | | chr13 | 57034150 | 57034400 | 0,34 |
| chr2 | 190812502 | 190812752 | 0,328 | | chr13 | 58422150 | 58422400 | 0,28 |
| chr2 | 191629581 | 191629831 | 0,348 | | chr13 | 59588150 | 59588400 | 0,332 |
| chr2 | 192431709 | 192431959 | 0,348 | | chr13 | 60397150 | 60397400 | 0,312 |
| chr2 | 193235999 | 193236249 | 0,348 | | chr13 | 61202290 | 61202540 | 0,324 |
| chr2 | 194633277 | 194633527 | 0,3 | | chr13 | 62004633 | 62004883 | 0,272 |
| chr2 | 195731550 | 195731800 | 0,344 | | chr13 | 63351150 | 63351400 | 0,328 |
| chr2 | 196720594 | 196720844 | 0,348 | | chr13 | 64158420 | 64158670 | 0,288 |
| chr2 | 198035335 | 198035585 | 0,344 | | chr13 | 64958934 | 64959184 | 0,252 |
| chr2 | 198852416 | 198852666 | 0,308 | | chr13 | 65964404 | 65964654 | 0,316 |
| chr2 | 199715449 | 199715699 | 0,348 | | chr13 | 66764993 | 66765243 | 0,32 |
| chr2 | 200878150 | 200878400 | 0,272 | | chr13 | 67565742 | 67565992 | 0,268 |
| chr2 | 202016150 | 202016400 | 0,34 | | chr13 | 68592150 | 68592400 | 0,32 |
| chr2 | 202966480 | 202966730 | 0,348 | | chr13 | 69974172 | 69974422 | 0,26 |
| chr2 | 203783437 | 203783687 | 0,284 | | chr13 | 70774901 | 70775151 | 0,292 |
| chr2 | 204585253 | 204585503 | 0,348 | | chr13 | 71711150 | 71711400 | 0,336 |
| chr2 | 205421450 | 205421700 | 0,332 | | chr13 | 72600186 | 72600436 | 0,348 |
| chr2 | 206266431 | 206266681 | 0,348 | | chr13 | 73459222 | 73459472 | 0,324 |
| chr2 | 207571598 | 207571848 | 0,348 | | chr13 | 74546150 | 74546400 | 0,308 |
| chr2 | 208734150 | 208734400 | 0,34 | | chr13 | 75459380 | 75459630 | 0,3 |
| chr2 | 209711150 | 209711400 | 0,32 | | chr13 | 76488150 | 76488400 | 0,312 |
| chr2 | 210732509 | 210732759 | 0,328 | | chr13 | 77291150 | 77291400 | 0,332 |
| chr2 | 211568334 | 211568584 | 0,348 | | chr13 | 77574876 | 77575126 | 0,3 |
| chr2 | 212713453 | 212713703 | 0,348 | | chr13 | 77574930 | 77575180 | 0,304 |
| chr2 | 213773225 | 213773475 | 0,332 | | chr13 | 78107382 | 78107632 | 0,348 |
| chr2 | 214848150 | 214848400 | 0,296 | | chr13 | 79007255 | 79007505 | 0,348 |
| chr2 | 216079487 | 216079737 | 0,348 | | chr13 | 79813627 | 79813877 | 0,348 |
| chr2 | 216891573 | 216891823 | 0,304 | | chr13 | 80621943 | 80622193 | 0,348 |
| chr2 | 217729569 | 217729819 | 0,348 | | chr13 | 81803150 | 81803400 | 0,288 |
| chr2 | 218601613 | 218601863 | 0,344 | | chr13 | 83285173 | 83285423 | 0,348 |
| chr2 | 219412476 | 219412726 | 0,348 | | chr13 | 84093150 | 84093400 | 0,344 |
| chr2 | 219525688 | 219525938 | 0,576 | | chr13 | 85142259 | 85142509 | 0,348 |
| chr2 | 219525751 | 219526001 | 0,58 | | chr13 | 86482550 | 86482800 | 0,292 |
| chr2 | 219525833 | 219526083 | 0,532 | | chr13 | 87283355 | 87283605 | 0,252 |
| chr2 | 219525881 | 219526131 | 0,504 | | chr13 | 88107306 | 88107556 | 0,32 |
| chr2 | 219526345 | 219526595 | 0,548 | | chr13 | 88911884 | 88912134 | 0,348 |
| chr2 | 219526444 | 219526694 | 0,536 | | chr13 | 89717798 | 89718048 | 0,3 |
| chr2 | 219526446 | 219526696 | 0,536 | | chr13 | 90777553 | 90777803 | 0,348 |
| chr2 | 219527213 | 219527463 | 0,588 | | chr13 | 91581084 | 91581334 | 0,348 |
| chr2 | 219527776 | 219528026 | 0,572 | | chr13 | 92381844 | 92382094 | 0,348 |
| chr2 | 219676946 | 219677196 | 0,568 | | chr13 | 93184022 | 93184272 | 0,348 |
| chr2 | 219677019 | 219677269 | 0,552 | | chr13 | 93988458 | 93988708 | 0,348 |
| chr2 | 219677317 | 219677567 | 0,572 | | chr13 | 94864550 | 94864800 | 0,344 |
| chr2 | 219677348 | 219677598 | 0,564 | | chr13 | 95767550 | 95767800 | 0,324 |
| chr2 | 219677704 | 219677954 | 0,592 | | chr13 | 96598197 | 96598447 | 0,256 |
| chr2 | 219679007 | 219679257 | 0,54 | | chr13 | 97398760 | 97399010 | 0,284 |
| chr2 | 219679057 | 219679307 | 0,536 | | chr13 | 98206114 | 98206364 | 0,328 |
| chr2 | 219679259 | 219679509 | 0,604 | | chr13 | 99147389 | 99147639 | 0,348 |
| chr2 | 219679350 | 219679600 | 0,628 | | chr13 | 99953575 | 99953825 | 0,348 |
| chr2 | 220712330 | 220712580 | 0,348 | | chr13 | 100764123 | 100764373 | 0,312 |
| chr2 | 221621220 | 221621470 | 0,348 | | chr13 | 100925353 | 100925603 | 0,368 |
| chr2 | 222580464 | 222580714 | 0,348 | | chr13 | 101572024 | 101572274 | 0,348 |
| chr2 | 223398818 | 223399068 | 0,348 | | chr13 | 102372104 | 102372354 | 0,34 |
| chr2 | 224206872 | 224207122 | 0,32 | | chr13 | 104448504 | 104448754 | 0,348 |
| chr2 | 225175548 | 225175798 | 0,348 | | chr13 | 105811166 | 105811416 | 0,348 |
| chr2 | 226463150 | 226463400 | 0,3 | | chr13 | 106646233 | 106646483 | 0,348 |
| chr2 | 227271729 | 227271979 | 0,316 | | chr13 | 107819307 | 107819557 | 0,288 |
| chr2 | 227872066 | 227872316 | 0,536 | | chr13 | 108621182 | 108621432 | 0,348 |
| chr2 | 227872698 | 227872948 | 0,632 | | chr13 | 109437750 | 109438000 | 0,348 |
| chr2 | 227896640 | 227896890 | 0,508 | | chr13 | 110244948 | 110245198 | 0,348 |
| chr2 | 227896844 | 227897094 | 0,516 | | chr13 | 111045509 | 111045759 | 0,304 |
| chr2 | 228173604 | 228173854 | 0,42 | | chr13 | 111854189 | 111854439 | 0,348 |
| chr2 | 228720266 | 228720516 | 0,348 | | chr13 | 112657452 | 112657702 | 0,348 |
| chr2 | 229615501 | 229615751 | 0,324 | | chr13 | 113458943 | 113459193 | 0,308 |
| chr2 | 230420658 | 230420908 | 0,288 | | chr13 | 114266718 | 114266968 | 0,348 |
| chr2 | 232026193 | 232026443 | 0,348 | | chr13 | 115077515 | 115077765 | 0,348 |
| chr2 | 232832077 | 232832327 | 0,348 | | chr14 | 20331746 | 20331996 | 0,332 |
| chr2 | 233404678 | 233404928 | 0,64 | | chr14 | 20731811 | 20732061 | 0,348 |
| chr2 | 233405261 | 233405511 | 0,632 | | chr14 | 21156990 | 21157240 | 0,348 |
| chr2 | 233407577 | 233407827 | 0,612 | | chr14 | 21963671 | 21963921 | 0,348 |
| chr2 | 233722288 | 233722538 | 0,348 | | chr14 | 23243450 | 23243700 | 0,48 |
| chr2 | 234669321 | 234669571 | 0,552 | | chr14 | 23945665 | 23945915 | 0,348 |
| chr2 | 234669649 | 234669899 | 0,44 | | chr14 | 24728884 | 24729134 | 0,62 |
| chr2 | 234675613 | 234675863 | 0,372 | | chr14 | 24903163 | 24903413 | 0,312 |
| chr2 | 234676780 | 234677030 | 0,484 | | chr14 | 25341210 | 25341460 | 0,348 |
| chr2 | 235489755 | 235490005 | 0,348 | | chr14 | 26002455 | 26002705 | 0,416 |
| chr2 | 236310414 | 236310664 | 0,348 | | chr14 | 26015264 | 26015514 | 0,348 |
| chr2 | 237286150 | 237286400 | 0,312 | | chr14 | 27132386 | 27132636 | 0,348 |
| chr2 | 238626336 | 238626586 | 0,348 | | chr14 | 27988150 | 27988400 | 0,312 |
| chr2 | 239657398 | 239657648 | 0,328 | | chr14 | 28789081 | 28789331 | 0,252 |
| chr2 | 240490228 | 240490478 | 0,348 | | chr14 | 29592176 | 29592426 | 0,348 |
| chr2 | 241499243 | 241499493 | 0,348 | | chr14 | 30393187 | 30393437 | 0,284 |
| chr2 | 241808489 | 241808739 | 0,62 | | chr14 | 31195558 | 31195808 | 0,324 |
| chr2 | 241808572 | 241808822 | 0,62 | | chr14 | 31600116 | 31600366 | 0,324 |
| chr2 | 242312643 | 242312893 | 0,348 | | chr14 | 32004544 | 32004794 | 0,32 |
| chr3 | 106582 | 106832 | 0,348 | | chr14 | 32806096 | 32806346 | 0,348 |
| chr3 | 908384 | 908634 | 0,312 | | chr14 | 33607614 | 33607864 | 0,336 |
| chr3 | 1765150 | 1765400 | 0,312 | | chr14 | 34411885 | 34412135 | 0,312 |
| chr3 | 2567150 | 2567400 | 0,304 | | chr14 | 35218148 | 35218398 | 0,348 |
| chr3 | 3462150 | 3462400 | 0,316 | | chr14 | 35615486 | 35615736 | 0,324 |
| chr3 | 4337222 | 4337472 | 0,348 | | chr14 | 37299150 | 37299400 | 0,34 |
| chr3 | 5258275 | 5258525 | 0,348 | | chr14 | 38183150 | 38183400 | 0,256 |
| chr3 | 6270433 | 6270683 | 0,348 | | chr14 | 38989082 | 38989332 | 0,28 |
| chr3 | 7086564 | 7086814 | 0,348 | | chr14 | 39789090 | 39789340 | 0,252 |
| chr3 | 8132516 | 8132766 | 0,336 | | chr14 | 40589667 | 40589917 | 0,348 |
| chr3 | 8945155 | 8945405 | 0,348 | | chr14 | 40993988 | 40994238 | 0,312 |
| chr3 | 10011145 | 10011395 | 0,344 | | chr14 | 41393524 | 41393774 | 0,348 |
| chr3 | 10874086 | 10874336 | 0,348 | | chr14 | 43262940 | 43263190 | 0,312 |
| chr3 | 11859185 | 11859435 | 0,348 | | chr14 | 44065317 | 44065567 | 0,348 |
| chr3 | 12659562 | 12659812 | 0,308 | | chr14 | 44870813 | 44871063 | 0,264 |
| chr3 | 13508599 | 13508849 | 0,348 | | chr14 | 45277796 | 45278046 | 0,344 |
| chr3 | 14357844 | 14358094 | 0,348 | | chr14 | 45673230 | 45673480 | 0,284 |
| chr3 | 15686845 | 15687095 | 0,544 | | chr14 | 46798150 | 46798400 | 0,284 |
| chr3 | 15718550 | 15718800 | 0,34 | | chr14 | 48248386 | 48248636 | 0,348 |
| chr3 | 16857172 | 16857422 | 0,348 | | chr14 | 49055150 | 49055400 | 0,336 |
| chr3 | 18013150 | 18013400 | 0,328 | | chr14 | 49869097 | 49869347 | 0,328 |
| chr3 | 18993185 | 18993435 | 0,348 | | chr14 | 50693325 | 50693575 | 0,348 |
| chr3 | 19802245 | 19802495 | 0,336 | | chr14 | 51202151 | 51202401 | 0,348 |
| chr3 | 20604117 | 20604367 | 0,348 | | chr14 | 51710426 | 51710676 | 0,32 |
| chr3 | 21451150 | 21451400 | 0,324 | | chr14 | 53399150 | 53399400 | 0,292 |
| chr3 | 22286462 | 22286712 | 0,348 | | chr14 | 54325152 | 54325402 | 0,348 |
| chr3 | 23158341 | 23158591 | 0,348 | | chr14 | 55133584 | 55133834 | 0,348 |
| chr3 | 24158297 | 24158547 | 0,348 | | chr14 | 55954997 | 55955247 | 0,348 |
| chr3 | 25277173 | 25277423 | 0,348 | | chr14 | 56356037 | 56356287 | 0,348 |
| chr3 | 26446293 | 26446543 | 0,328 | | chr14 | 56757300 | 56757550 | 0,348 |
| chr3 | 27249004 | 27249254 | 0,324 | | chr14 | 57562963 | 57563213 | 0,348 |
| chr3 | 28051902 | 28052152 | 0,348 | | chr14 | 58448295 | 58448545 | 0,344 |
| chr3 | 28868402 | 28868652 | 0,348 | | chr14 | 59330150 | 59330400 | 0,316 |
| chr3 | 29670992 | 29671242 | 0,32 | | chr14 | 60137150 | 60137400 | 0,324 |
| chr3 | 30519197 | 30519447 | 0,348 | | chr14 | 61029567 | 61029817 | 0,312 |
| chr3 | 31364183 | 31364433 | 0,348 | | chr14 | 61831817 | 61832067 | 0,348 |
| chr3 | 32524425 | 32524675 | 0,348 | | chr14 | 62234974 | 62235224 | 0,348 |
| chr3 | 33428371 | 33428621 | 0,348 | | chr14 | 62648150 | 62648400 | 0,32 |
| chr3 | 34284461 | 34284711 | 0,348 | | chr14 | 63451150 | 63451400 | 0,34 |
| chr3 | 35362150 | 35362400 | 0,34 | | chr14 | 64679200 | 64679450 | 0,348 |
| chr3 | 36162197 | 36162447 | 0,348 | | chr14 | 65479421 | 65479671 | 0,348 |
| chr3 | 36969914 | 36970164 | 0,348 | | chr14 | 66291609 | 66291859 | 0,348 |
| chr3 | 37806754 | 37807004 | 0,348 | | chr14 | 66707755 | 66708005 | 0,348 |
| chr3 | 38725451 | 38725701 | 0,348 | | chr14 | 67099658 | 67099908 | 0,336 |
| chr3 | 39545431 | 39545681 | 0,348 | | chr14 | 67900455 | 67900705 | 0,328 |
| chr3 | 40346322 | 40346572 | 0,32 | | chr14 | 68191809 | 68192059 | 0,532 |
| chr3 | 41182781 | 41183031 | 0,348 | | chr14 | 68193588 | 68193838 | 0,572 |
| chr3 | 42062642 | 42062892 | 0,348 | | chr14 | 68193689 | 68193939 | 0,552 |
| chr3 | 42870181 | 42870431 | 0,348 | | chr14 | 68195776 | 68196026 | 0,584 |
| chr3 | 43679165 | 43679415 | 0,348 | | chr14 | 68706399 | 68706649 | 0,348 |
| chr3 | 44511929 | 44512179 | 0,332 | | chr14 | 69519728 | 69519978 | 0,348 |
| chr3 | 45335638 | 45335888 | 0,336 | | chr14 | 70427169 | 70427419 | 0,348 |
| chr3 | 46151421 | 46151671 | 0,348 | | chr14 | 71955192 | 71955442 | 0,324 |
| chr3 | 47066811 | 47067061 | 0,348 | | chr14 | 72369138 | 72369388 | 0,348 |
| chr3 | 47901166 | 47901416 | 0,348 | | chr14 | 72780476 | 72780726 | 0,348 |
| chr3 | 48730727 | 48730977 | 0,348 | | chr14 | 73583334 | 73583584 | 0,348 |
| chr3 | 49570998 | 49571248 | 0,348 | | chr14 | 74419912 | 74420162 | 0,348 |
| chr3 | 50643833 | 50644083 | 0,348 | | chr14 | 74947285 | 74947535 | 0,412 |
| chr3 | 51451458 | 51451708 | 0,348 | | chr14 | 74950993 | 74951243 | 0,484 |
| chr3 | 52301455 | 52301705 | 0,348 | | chr14 | 74952902 | 74953152 | 0,444 |
| chr3 | 53125457 | 53125707 | 0,332 | | chr14 | 74952982 | 74953232 | 0,472 |
| chr3 | 53930140 | 53930390 | 0,308 | | chr14 | 75231341 | 75231591 | 0,348 |
| chr3 | 54731843 | 54732093 | 0,348 | | chr14 | 76128102 | 76128352 | 0,348 |
| chr3 | 55552366 | 55552616 | 0,348 | | chr14 | 76717009 | 76717259 | 0,348 |
| chr3 | 56374796 | 56375046 | 0,344 | | chr14 | 77352577 | 77352827 | 0,348 |
| chr3 | 57175379 | 57175629 | 0,268 | | chr14 | 78858150 | 78858400 | 0,348 |
| chr3 | 58002540 | 58002790 | 0,32 | | chr14 | 79899295 | 79899545 | 0,348 |
| chr3 | 58914444 | 58914694 | 0,316 | | chr14 | 81000150 | 81000400 | 0,34 |
| chr3 | 60826550 | 60826800 | 0,28 | | chr14 | 81938150 | 81938400 | 0,332 |
| chr3 | 62217159 | 62217409 | 0,348 | | chr14 | 82739526 | 82739776 | 0,308 |
| chr3 | 63145150 | 63145400 | 0,344 | | chr14 | 83277489 | 83277739 | 0,348 |
| chr3 | 64458150 | 64458400 | 0,348 | | chr14 | 83828150 | 83828400 | 0,312 |
| chr3 | 65372154 | 65372404 | 0,348 | | chr14 | 84903229 | 84903479 | 0,348 |
| chr3 | 66316150 | 66316400 | 0,348 | | chr14 | 85751567 | 85751817 | 0,34 |
| chr3 | 67172150 | 67172400 | 0,324 | | chr14 | 86625529 | 86625779 | 0,348 |
| chr3 | 67972470 | 67972720 | 0,304 | | chr14 | 87501333 | 87501583 | 0,348 |
| chr3 | 68775683 | 68775933 | 0,348 | | chr14 | 88407748 | 88407998 | 0,368 |
| chr3 | 69819562 | 69819812 | 0,348 | | chr14 | 88413964 | 88414214 | 0,452 |
| chr3 | 70622150 | 70622400 | 0,256 | | chr14 | 88650507 | 88650757 | 0,348 |
| chr3 | 71422272 | 71422522 | 0,348 | | chr14 | 89060856 | 89061106 | 0,328 |
| chr3 | 72235107 | 72235357 | 0,336 | | chr14 | 89464609 | 89464859 | 0,348 |
| chr3 | 73035206 | 73035456 | 0,348 | | chr14 | 90271098 | 90271348 | 0,336 |
| chr3 | 73959481 | 73959731 | 0,348 | | chr14 | 91072033 | 91072283 | 0,348 |
| chr3 | 74759920 | 74760170 | 0,252 | | chr14 | 91876716 | 91876966 | 0,348 |
| chr3 | 75954621 | 75954871 | 0,284 | | chr14 | 93250166 | 93250416 | 0,348 |
| chr3 | 76763596 | 76763846 | 0,348 | | chr14 | 94052683 | 94052933 | 0,348 |
| chr3 | 77570832 | 77571082 | 0,344 | | chr14 | 94626755 | 94627005 | 0,348 |
| chr3 | 78386873 | 78387123 | 0,348 | | chr14 | 94844782 | 94845032 | 0,504 |
| chr3 | 79188557 | 79188807 | 0,348 | | chr14 | 94844822 | 94845072 | 0,544 |
| chr3 | 80261571 | 80261821 | 0,348 | | chr14 | 94849223 | 94849473 | 0,54 |
| chr3 | 81419171 | 81419421 | 0,348 | | chr14 | 94888492 | 94888742 | 0,316 |
| chr3 | 81697870 | 81698120 | 0,304 | | chr14 | 96231311 | 96231561 | 0,348 |
| chr3 | 82375279 | 82375529 | 0,348 | | chr14 | 97299482 | 97299732 | 0,312 |
| chr3 | 83179213 | 83179463 | 0,316 | | chr14 | 98110316 | 98110566 | 0,348 |
| chr3 | 83997183 | 83997433 | 0,292 | | chr14 | 98925215 | 98925465 | 0,348 |
| chr3 | 84798234 | 84798484 | 0,312 | | chr14 | 99322526 | 99322776 | 0,348 |
| chr3 | 85600581 | 85600831 | 0,328 | | chr14 | 99726035 | 99726285 | 0,348 |
| chr3 | 86406853 | 86407103 | 0,296 | | chr14 | 100695219 | 100695469 | 0,348 |
| chr3 | 87309262 | 87309512 | 0,348 | | chr14 | 101528862 | 101529112 | 0,348 |
| chr3 | 88400150 | 88400400 | 0,3 | | chr14 | 102342620 | 102342870 | 0,316 |
| chr3 | 89682150 | 89682400 | 0,3 | | chr14 | 103166071 | 103166321 | 0,348 |
| chr3 | 93653233 | 93653483 | 0,336 | | chr14 | 104199459 | 104199709 | 0,348 |
| chr3 | 94486575 | 94486825 | 0,344 | | chr14 | 104799911 | 104800161 | 0,348 |
| chr3 | 95493267 | 95493517 | 0,312 | | chr14 | 106327752 | 106328002 | 0,348 |
| chr3 | 96311242 | 96311492 | 0,276 | | chr15 | 21937192 | 21937442 | 0,348 |
| chr3 | 97326150 | 97326400 | 0,276 | | chr15 | 22815525 | 22815775 | 0,336 |
| chr3 | 98164597 | 98164847 | 0,288 | | chr15 | 23062275 | 23062525 | 0,348 |
| chr3 | 98967593 | 98967843 | 0,348 | | chr15 | 23805462 | 23805712 | 0,328 |
| chr3 | 99767959 | 99768209 | 0,34 | | chr15 | 24856221 | 24856471 | 0,348 |
| chr3 | 100568517 | 100568767 | 0,332 | | chr15 | 25256023 | 25256273 | 0,3 |
| chr3 | 102171151 | 102171401 | 0,332 | | chr15 | 25656926 | 25657176 | 0,336 |
| chr3 | 104463150 | 104463400 | 0,344 | | chr15 | 27756150 | 27756400 | 0,344 |
| chr3 | 105267545 | 105267795 | 0,348 | | chr15 | 28437259 | 28437509 | 0,348 |
| chr3 | 106008363 | 106008613 | 0,276 | | chr15 | 29143960 | 29144210 | 0,316 |
| chr3 | 106219344 | 106219594 | 0,332 | | chr15 | 29946126 | 29946376 | 0,308 |
| chr3 | 107172470 | 107172720 | 0,348 | | chr15 | 31338150 | 31338400 | 0,308 |
| chr3 | 108216183 | 108216433 | 0,304 | | chr15 | 32115541 | 32115791 | 0,348 |
| chr3 | 109044868 | 109045118 | 0,348 | | chr15 | 32928697 | 32928947 | 0,348 |
| chr3 | 110072469 | 110072719 | 0,348 | | chr15 | 33816249 | 33816499 | 0,348 |
| chr3 | 110913541 | 110913791 | 0,348 | | chr15 | 34418150 | 34418400 | 0,34 |
| chr3 | 111996421 | 111996671 | 0,348 | | chr15 | 34532737 | 34532986 | 0,457831 |
| chr3 | 113052335 | 113052585 | 0,348 | | chr15 | 34536054 | 34536236 | 0,412088 |
| chr3 | 113880193 | 113880443 | 0,348 | | chr15 | 35006335 | 35006585 | 0,348 |
| chr3 | 115100591 | 115100841 | 0,348 | | chr15 | 36298291 | 36298541 | 0,348 |
| chr3 | 116316282 | 116316532 | 0,348 | | chr15 | 36751553 | 36751803 | 0,348 |
| chr3 | 117419150 | 117419400 | 0,34 | | chr15 | 37098509 | 37098759 | 0,348 |
| chr3 | 118228268 | 118228518 | 0,348 | | chr15 | 38023487 | 38023737 | 0,348 |
| chr3 | 119031004 | 119031254 | 0,348 | | chr15 | 39315508 | 39315758 | 0,348 |
| chr3 | 119848682 | 119848932 | 0,348 | | chr15 | 39870231 | 39870481 | 0,348 |
| chr3 | 120369570 | 120369820 | 0,508 | | chr15 | 40677150 | 40677400 | 0,336 |
| chr3 | 120393624 | 120393874 | 0,488 | | chr15 | 40707528 | 40707778 | 0,552 |
| chr3 | 120394586 | 120394836 | 0,408 | | chr15 | 42054189 | 42054439 | 0,348 |
| chr3 | 121124509 | 121124759 | 0,288 | | chr15 | 42693828 | 42694078 | 0,58 |
| chr3 | 121967180 | 121967430 | 0,348 | | chr15 | 42703005 | 42703255 | 0,548 |
| chr3 | 123211159 | 123211409 | 0,348 | | chr15 | 42955175 | 42955425 | 0,348 |
| chr3 | 124011977 | 124012227 | 0,316 | | chr15 | 44096462 | 44096712 | 0,348 |
| chr3 | 124820334 | 124820584 | 0,284 | | chr15 | 44492018 | 44492268 | 0,348 |
| chr3 | 125753018 | 125753268 | 0,336 | | chr15 | 44898296 | 44898546 | 0,312 |
| chr3 | 126599445 | 126599695 | 0,348 | | chr15 | 45699464 | 45699714 | 0,348 |
| chr3 | 127455014 | 127455264 | 0,348 | | chr15 | 46502884 | 46503134 | 0,348 |
| chr3 | 129170444 | 129170694 | 0,348 | | chr15 | 47004873 | 47005123 | 0,304 |
| chr3 | 131092150 | 131092400 | 0,336 | | chr15 | 47506569 | 47506819 | 0,348 |
| chr3 | 132208201 | 132208451 | 0,328 | | chr15 | 48748150 | 48748400 | 0,336 |
| chr3 | 133852268 | 133852518 | 0,348 | | chr15 | 49239150 | 49239400 | 0,34 |
| chr3 | 135245150 | 135245400 | 0,336 | | chr15 | 49912448 | 49912698 | 0,348 |
| chr3 | 135975298 | 135975548 | 0,396 | | chr15 | 50717615 | 50717865 | 0,348 |
| chr3 | 135980686 | 135980936 | 0,432 | | chr15 | 51503082 | 51503332 | 0,444 |
| chr3 | 135980741 | 135980991 | 0,456 | | chr15 | 51503089 | 51503339 | 0,444 |
| chr3 | 136009682 | 136009932 | 0,332 | | chr15 | 51510606 | 51510856 | 0,38 |
| chr3 | 136045901 | 136046151 | 0,536 | | chr15 | 51510730 | 51510980 | 0,4 |
| chr3 | 136045956 | 136046206 | 0,536 | | chr15 | 51514421 | 51514671 | 0,452 |
| chr3 | 136046361 | 136046611 | 0,504 | | chr15 | 51528775 | 51529025 | 0,34 |
| chr3 | 136048668 | 136048918 | 0,476 | | chr15 | 51749584 | 51749834 | 0,348 |
| chr3 | 136048719 | 136048969 | 0,444 | | chr15 | 52375584 | 52375834 | 0,32 |
| chr3 | 136271150 | 136271400 | 0,272 | | chr15 | 53488150 | 53488400 | 0,28 |
| chr3 | 137101096 | 137101346 | 0,348 | | chr15 | 54303419 | 54303669 | 0,348 |
| chr3 | 138709161 | 138709411 | 0,348 | | chr15 | 55400166 | 55400416 | 0,348 |
| chr3 | 139558134 | 139558384 | 0,348 | | chr15 | 55947150 | 55947400 | 0,324 |
| chr3 | 140396238 | 140396488 | 0,348 | | chr15 | 56611250 | 56611500 | 0,332 |
| chr3 | 141198475 | 141198725 | 0,296 | | chr15 | 57552150 | 57552400 | 0,32 |
| chr3 | 142016082 | 142016332 | 0,348 | | chr15 | 58755932 | 58756182 | 0,348 |
| chr3 | 143067286 | 143067536 | 0,316 | | chr15 | 59168882 | 59169132 | 0,348 |
| chr3 | 143869968 | 143870218 | 0,348 | | chr15 | 60057234 | 60057484 | 0,296 |
| chr3 | 144671028 | 144671278 | 0,308 | | chr15 | 61399384 | 61399634 | 0,348 |
| chr3 | 145486925 | 145487175 | 0,3 | | chr15 | 62048550 | 62048800 | 0,32 |
| chr3 | 146287344 | 146287594 | 0,348 | | chr15 | 62410361 | 62410611 | 0,348 |
| chr3 | 147094525 | 147094775 | 0,348 | | chr15 | 63213584 | 63213834 | 0,348 |
| chr3 | 147896273 | 147896523 | 0,336 | | chr15 | 63624114 | 63624364 | 0,348 |
| chr3 | 148857589 | 148857839 | 0,336 | | chr15 | 64025256 | 64025506 | 0,348 |
| chr3 | 148881618 | 148881868 | 0,312 | | chr15 | 64840241 | 64840491 | 0,332 |
| chr3 | 149900583 | 149900833 | 0,348 | | chr15 | 65674276 | 65674526 | 0,34 |
| chr3 | 150645769 | 150646019 | 0,38 | | chr15 | 66097124 | 66097374 | 0,348 |
| chr3 | 150645848 | 150646098 | 0,404 | | chr15 | 66541083 | 66541333 | 0,348 |
| chr3 | 150690222 | 150690472 | 0,532 | | chr15 | 67364323 | 67364573 | 0,268 |
| chr3 | 150859582 | 150859832 | 0,348 | | chr15 | 68461235 | 68461485 | 0,348 |
| chr3 | 151668293 | 151668543 | 0,304 | | chr15 | 68501858 | 68502108 | 0,58 |
| chr3 | 152474698 | 152474948 | 0,344 | | chr15 | 68503470 | 68503720 | 0,588 |
| chr3 | 153432150 | 153432400 | 0,316 | | chr15 | 68504006 | 68504256 | 0,592 |
| chr3 | 154235025 | 154235275 | 0,344 | | chr15 | 68504067 | 68504317 | 0,604 |
| chr3 | 155041150 | 155041400 | 0,328 | | chr15 | 69405375 | 69405625 | 0,348 |
| chr3 | 155924313 | 155924563 | 0,348 | | chr15 | 69906737 | 69906987 | 0,348 |
| chr3 | 157018150 | 157018400 | 0,32 | | chr15 | 70261781 | 70262031 | 0,348 |
| chr3 | 158109466 | 158109716 | 0,348 | | chr15 | 71298502 | 71298752 | 0,316 |
| chr3 | 159249551 | 159249801 | 0,332 | | chr15 | 72129534 | 72129784 | 0,336 |
| chr3 | 160003430 | 160003680 | 0,296 | | chr15 | 72638860 | 72639056 | 0,515306 |
| chr3 | 160316406 | 160316656 | 0,348 | | chr15 | 72640258 | 72640508 | 0,544 |
| chr3 | 161438158 | 161438408 | 0,348 | | chr15 | 73399350 | 73399600 | 0,332 |
| chr3 | 162238201 | 162238451 | 0,26 | | chr15 | 74451150 | 74451400 | 0,324 |
| chr3 | 163049817 | 163050067 | 0,276 | | chr15 | 75189266 | 75189516 | 0,532 |
| chr3 | 163869741 | 163869991 | 0,34 | | chr15 | 75465113 | 75465363 | 0,348 |
| chr3 | 164670585 | 164670835 | 0,3 | | chr15 | 75869324 | 75869574 | 0,316 |
| chr3 | 165478954 | 165479204 | 0,348 | | chr15 | 76301292 | 76301542 | 0,284 |
| chr3 | 165491155 | 165491404 | 0,313253 | | chr15 | 77750576 | 77750826 | 0,344 |
| chr3 | 165548392 | 165548641 | 0,401606 | | chr15 | 78309918 | 78310168 | 0,34 |
| chr3 | 166281735 | 166281985 | 0,328 | | chr15 | 78565345 | 78565595 | 0,348 |
| chr3 | 167085940 | 167086190 | 0,308 | | chr15 | 80450387 | 80450637 | 0,508 |
| chr3 | 168459468 | 168459718 | 0,308 | | chr15 | 80465310 | 80465560 | 0,576 |
| chr3 | 169309551 | 169309801 | 0,344 | | chr15 | 80472405 | 80472655 | 0,64 |
| chr3 | 170492150 | 170492400 | 0,324 | | chr15 | 80472447 | 80472697 | 0,624 |
| chr3 | 171325476 | 171325726 | 0,312 | | chr15 | 80473260 | 80473510 | 0,536 |
| chr3 | 172228471 | 172228721 | 0,348 | | chr15 | 80730551 | 80730801 | 0,348 |
| chr3 | 173228258 | 173228508 | 0,348 | | chr15 | 80938280 | 80938530 | 0,348 |
| chr3 | 174030196 | 174030446 | 0,348 | | chr15 | 81549150 | 81549400 | 0,328 |
| chr3 | 174909327 | 174909577 | 0,348 | | chr15 | 82355082 | 82355332 | 0,348 |
| chr3 | 176159550 | 176159800 | 0,272 | | chr15 | 83783444 | 83783694 | 0,348 |
| chr3 | 177050167 | 177050417 | 0,296 | | chr15 | 84178159 | 84178409 | 0,348 |
| chr3 | 178235166 | 178235416 | 0,34 | | chr15 | 85189700 | 85189950 | 0,348 |
| chr3 | 179511583 | 179511833 | 0,348 | | chr15 | 86017842 | 86018092 | 0,284 |
| chr3 | 180752251 | 180752501 | 0,344 | | chr15 | 86446885 | 86447135 | 0,312 |
| chr3 | 181861577 | 181861827 | 0,348 | | chr15 | 86821668 | 86821918 | 0,348 |
| chr3 | 182665063 | 182665313 | 0,268 | | chr15 | 87865458 | 87865708 | 0,348 |
| chr3 | 183472150 | 183472400 | 0,348 | | chr15 | 88320038 | 88320288 | 0,348 |
| chr3 | 184526150 | 184526400 | 0,312 | | chr15 | 88810009 | 88810259 | 0,348 |
| chr3 | 185410377 | 185410627 | 0,348 | | chr15 | 89644678 | 89644928 | 0,348 |
| chr3 | 186420548 | 186420798 | 0,324 | | chr15 | 89753895 | 89754145 | 0,564 |
| chr3 | 187267150 | 187267400 | 0,348 | | chr15 | 89860637 | 89860887 | 0,532 |
| chr3 | 188173164 | 188173414 | 0,348 | | chr15 | 89862087 | 89862337 | 0,596 |
| chr3 | 189008153 | 189008403 | 0,348 | | chr15 | 89862159 | 89862409 | 0,536 |
| chr3 | 189809150 | 189809400 | 0,28 | | chr15 | 89864054 | 89864304 | 0,536 |
| chr3 | 190627116 | 190627366 | 0,348 | | chr15 | 89866532 | 89866782 | 0,572 |
| chr3 | 191447581 | 191447831 | 0,348 | | chr15 | 89868746 | 89868996 | 0,612 |
| chr3 | 192375286 | 192375536 | 0,348 | | chr15 | 89870087 | 89870337 | 0,58 |
| chr3 | 193179318 | 193179568 | 0,288 | | chr15 | 89873301 | 89873551 | 0,588 |
| chr3 | 194874250 | 194874500 | 0,348 | | chr15 | 89873358 | 89873608 | 0,6 |
| chr3 | 195777466 | 195777716 | 0,348 | | chr15 | 90992339 | 90992589 | 0,348 |
| chr3 | 196621577 | 196621827 | 0,312 | | chr15 | 91879321 | 91879571 | 0,348 |
| chr3 | 197424150 | 197424400 | 0,308 | | chr15 | 92283729 | 92283979 | 0,348 |
| chr4 | 524439 | 524689 | 0,348 | | chr15 | 92694222 | 92694472 | 0,34 |
| chr4 | 1694543 | 1694793 | 0,296 | | chr15 | 93494887 | 93495137 | 0,348 |
| chr4 | 1806047 | 1806226 | 0,653631 | | chr15 | 94516150 | 94516400 | 0,272 |
| chr4 | 2590392 | 2590642 | 0,348 | | chr15 | 94738150 | 94738400 | 0,336 |
| chr4 | 3408543 | 3408793 | 0,348 | | chr15 | 95430150 | 95430400 | 0,34 |
| chr4 | 4248009 | 4248259 | 0,34 | | chr15 | 96242150 | 96242400 | 0,34 |
| chr4 | 5073125 | 5073375 | 0,32 | | chr15 | 97236279 | 97236529 | 0,348 |
| chr4 | 5911997 | 5912247 | 0,292 | | chr15 | 97542332 | 97542582 | 0,292 |
| chr4 | 6786181 | 6786431 | 0,348 | | chr15 | 98054150 | 98054400 | 0,332 |
| chr4 | 7889150 | 7889400 | 0,284 | | chr15 | 99928187 | 99928437 | 0,348 |
| chr4 | 8768669 | 8768919 | 0,348 | | chr15 | 101013180 | 101013430 | 0,348 |
| chr4 | 11023186 | 11023436 | 0,348 | | chr15 | 101227226 | 101227476 | 0,348 |
| chr4 | 11924150 | 11924400 | 0,332 | | chr15 | 101907373 | 101907623 | 0,348 |
| chr4 | 12802731 | 12802981 | 0,348 | | chr16 | 258208 | 258458 | 0,348 |
| chr4 | 13608443 | 13608693 | 0,3 | | chr16 | 946720 | 946970 | 0,348 |
| chr4 | 14700204 | 14700454 | 0,348 | | chr16 | 1413133 | 1413383 | 0,348 |
| chr4 | 15516150 | 15516400 | 0,316 | | chr16 | 2320307 | 2320557 | 0,348 |
| chr4 | 16593239 | 16593489 | 0,348 | | chr16 | 3293218 | 3293428 | 0,52381 |
| chr4 | 17959355 | 17959605 | 0,34 | | chr16 | 3293284 | 3293534 | 0,536 |
| chr4 | 19122551 | 19122801 | 0,348 | | chr16 | 3293312 | 3293561 | 0,558233 |
| chr4 | 20409254 | 20409504 | 0,348 | | chr16 | 3293322 | 3293572 | 0,548 |
| chr4 | 21274269 | 21274519 | 0,32 | | chr16 | 3293404 | 3293654 | 0,516 |
| chr4 | 22246426 | 22246676 | 0,34 | | chr16 | 3297026 | 3297276 | 0,584 |
| chr4 | 23364150 | 23364400 | 0,348 | | chr16 | 3297026 | 3297276 | 0,584 |
| chr4 | 24165166 | 24165416 | 0,348 | | chr16 | 3304577 | 3304735 | 0,702532 |
| chr4 | 25408550 | 25408800 | 0,268 | | chr16 | 3699883 | 3700133 | 0,348 |
| chr4 | 26570550 | 26570800 | 0,292 | | chr16 | 4062366 | 4062616 | 0,348 |
| chr4 | 27713150 | 27713400 | 0,3 | | chr16 | 4883448 | 4883698 | 0,348 |
| chr4 | 28518303 | 28518553 | 0,348 | | chr16 | 6227319 | 6227569 | 0,348 |
| chr4 | 29328570 | 29328820 | 0,348 | | chr16 | 6622150 | 6622400 | 0,28 |
| chr4 | 30272440 | 30272690 | 0,32 | | chr16 | 7509405 | 7509655 | 0,348 |
| chr4 | 31367442 | 31367692 | 0,348 | | chr16 | 8312670 | 8312920 | 0,348 |
| chr4 | 32172526 | 32172776 | 0,34 | | chr16 | 8941497 | 8941747 | 0,6 |
| chr4 | 32976113 | 32976363 | 0,268 | | chr16 | 9148517 | 9148767 | 0,348 |
| chr4 | 33782576 | 33782826 | 0,34 | | chr16 | 9553912 | 9554162 | 0,348 |
| chr4 | 35566150 | 35566400 | 0,268 | | chr16 | 9952565 | 9952815 | 0,348 |
| chr4 | 36384406 | 36384656 | 0,328 | | chr16 | 10814797 | 10815047 | 0,348 |
| chr4 | 37187183 | 37187433 | 0,348 | | chr16 | 11641822 | 11642072 | 0,348 |
| chr4 | 38037150 | 38037400 | 0,34 | | chr16 | 12060400 | 12060650 | 0,348 |
| chr4 | 38857304 | 38857554 | 0,348 | | chr16 | 12521810 | 12522060 | 0,348 |
| chr4 | 39657375 | 39657625 | 0,3 | | chr16 | 13341606 | 13341856 | 0,32 |
| chr4 | 40457994 | 40458244 | 0,332 | | chr16 | 14167801 | 14168051 | 0,308 |
| chr4 | 41262121 | 41262371 | 0,348 | | chr16 | 14604714 | 14604964 | 0,348 |
| chr4 | 42799150 | 42799400 | 0,324 | | chr16 | 15052738 | 15052988 | 0,348 |
| chr4 | 43607150 | 43607400 | 0,308 | | chr16 | 15959882 | 15960132 | 0,32 |
| chr4 | 44408802 | 44409052 | 0,276 | | chr16 | 16859878 | 16860128 | 0,348 |
| chr4 | 45217030 | 45217280 | 0,32 | | chr16 | 17663171 | 17663421 | 0,336 |
| chr4 | 46213423 | 46213673 | 0,348 | | chr16 | 18119811 | 18120061 | 0,344 |
| chr4 | 47159179 | 47159429 | 0,348 | | chr16 | 18799227 | 18799477 | 0,348 |
| chr4 | 48947216 | 48947466 | 0,348 | | chr16 | 19621285 | 19621535 | 0,348 |
| chr4 | 52703125 | 52703375 | 0,348 | | chr16 | 20620997 | 20621247 | 0,288 |
| chr4 | 53519881 | 53520131 | 0,348 | | chr16 | 21600723 | 21600973 | 0,308 |
| chr4 | 54575550 | 54575800 | 0,308 | | chr16 | 21995758 | 21996008 | 0,348 |
| chr4 | 55541150 | 55541400 | 0,332 | | chr16 | 22413441 | 22413691 | 0,32 |
| chr4 | 56681150 | 56681400 | 0,268 | | chr16 | 23252457 | 23252707 | 0,348 |
| chr4 | 57640467 | 57640717 | 0,348 | | chr16 | 24097792 | 24098042 | 0,348 |
| chr4 | 58471328 | 58471578 | 0,344 | | chr16 | 24552683 | 24552933 | 0,34 |
| chr4 | 59809215 | 59809465 | 0,348 | | chr16 | 24939654 | 24939904 | 0,304 |
| chr4 | 60849558 | 60849808 | 0,32 | | chr16 | 25759791 | 25760041 | 0,348 |
| chr4 | 61651913 | 61652163 | 0,3 | | chr16 | 26644833 | 26645083 | 0,348 |
| chr4 | 62453032 | 62453282 | 0,348 | | chr16 | 26964539 | 26964789 | 0,348 |
| chr4 | 63256751 | 63257001 | 0,304 | | chr16 | 27504199 | 27504449 | 0,348 |
| chr4 | 64088143 | 64088393 | 0,256 | | chr16 | 29806827 | 29807077 | 0,348 |
| chr4 | 64899871 | 64900121 | 0,304 | | chr16 | 30764038 | 30764288 | 0,348 |
| chr4 | 65701600 | 65701850 | 0,296 | | chr16 | 31200605 | 31200855 | 0,328 |
| chr4 | 66514018 | 66514268 | 0,348 | | chr16 | 31656295 | 31656545 | 0,348 |
| chr4 | 67894302 | 67894552 | 0,348 | | chr16 | 32622254 | 32622504 | 0,348 |
| chr4 | 68776550 | 68776800 | 0,34 | | chr16 | 34199349 | 34199599 | 0,34 |
| chr4 | 69576924 | 69577174 | 0,348 | | chr16 | 35039854 | 35040104 | 0,348 |
| chr4 | 70382938 | 70383188 | 0,288 | | chr16 | 46501463 | 46501713 | 0,348 |
| chr4 | 71237341 | 71237591 | 0,348 | | chr16 | 46860612 | 46860862 | 0,324 |
| chr4 | 72411150 | 72411400 | 0,336 | | chr16 | 47307020 | 47307270 | 0,348 |
| chr4 | 73710150 | 73710400 | 0,344 | | chr16 | 48123485 | 48123735 | 0,348 |
| chr4 | 75071150 | 75071400 | 0,328 | | chr16 | 48979520 | 48979770 | 0,348 |
| chr4 | 76179362 | 76179612 | 0,348 | | chr16 | 49409741 | 49409991 | 0,34 |
| chr4 | 77033574 | 77033824 | 0,348 | | chr16 | 49854234 | 49854484 | 0,348 |
| chr4 | 77846718 | 77846968 | 0,288 | | chr16 | 50777251 | 50777501 | 0,348 |
| chr4 | 78648984 | 78649234 | 0,272 | | chr16 | 51611803 | 51612053 | 0,348 |
| chr4 | 79457938 | 79458188 | 0,344 | | chr16 | 52521397 | 52521647 | 0,344 |
| chr4 | 80278331 | 80278581 | 0,34 | | chr16 | 53001263 | 53001513 | 0,312 |
| chr4 | 81378288 | 81378538 | 0,324 | | chr16 | 53933270 | 53933520 | 0,348 |
| chr4 | 82584150 | 82584400 | 0,348 | | chr16 | 54744744 | 54744994 | 0,348 |
| chr4 | 83424150 | 83424400 | 0,284 | | chr16 | 55560218 | 55560468 | 0,336 |
| chr4 | 84768150 | 84768400 | 0,34 | | chr16 | 55979882 | 55980132 | 0,348 |
| chr4 | 86164348 | 86164598 | 0,32 | | chr16 | 56438259 | 56438509 | 0,344 |
| chr4 | 87420154 | 87420404 | 0,348 | | chr16 | 56548274 | 56548524 | 0,384 |
| chr4 | 88759560 | 88759810 | 0,348 | | chr16 | 56548356 | 56548606 | 0,44 |
| chr4 | 89613224 | 89613474 | 0,348 | | chr16 | 56903881 | 56904131 | 0,628 |
| chr4 | 90622593 | 90622843 | 0,348 | | chr16 | 57242761 | 57243011 | 0,336 |
| chr4 | 91908223 | 91908473 | 0,344 | | chr16 | 58047908 | 58048158 | 0,32 |
| chr4 | 92715215 | 92715465 | 0,348 | | chr16 | 58439726 | 58439976 | 0,348 |
| chr4 | 94023452 | 94023702 | 0,308 | | chr16 | 58854106 | 58854356 | 0,348 |
| chr4 | 95644839 | 95645089 | 0,288 | | chr16 | 59723150 | 59723400 | 0,32 |
| chr4 | 96736322 | 96736572 | 0,348 | | chr16 | 60845346 | 60845596 | 0,348 |
| chr4 | 98008150 | 98008400 | 0,316 | | chr16 | 61360230 | 61360480 | 0,252 |
| chr4 | 99308150 | 99308400 | 0,284 | | chr16 | 61758472 | 61758722 | 0,348 |
| chr4 | 100232150 | 100232400 | 0,332 | | chr16 | 62727578 | 62727828 | 0,348 |
| chr4 | 100543788 | 100544038 | 0,42 | | chr16 | 63914255 | 63914505 | 0,328 |
| chr4 | 101068550 | 101068800 | 0,344 | | chr16 | 64454577 | 64454827 | 0,268 |
| chr4 | 101875150 | 101875400 | 0,348 | | chr16 | 64980267 | 64980517 | 0,348 |
| chr4 | 102676263 | 102676513 | 0,348 | | chr16 | 65805485 | 65805735 | 0,336 |
| chr4 | 103487276 | 103487526 | 0,308 | | chr16 | 66650107 | 66650357 | 0,348 |
| chr4 | 104289731 | 104289981 | 0,296 | | chr16 | 67124434 | 67124684 | 0,348 |
| chr4 | 105095425 | 105095675 | 0,284 | | chr16 | 67597956 | 67598206 | 0,348 |
| chr4 | 105907870 | 105908120 | 0,332 | | chr16 | 68432415 | 68432665 | 0,348 |
| chr4 | 107094224 | 107094474 | 0,332 | | chr16 | 69235387 | 69235637 | 0,324 |
| chr4 | 108325550 | 108325800 | 0,332 | | chr16 | 70192752 | 70193002 | 0,348 |
| chr4 | 109267150 | 109267400 | 0,344 | | chr16 | 70597322 | 70597572 | 0,348 |
| chr4 | 110081150 | 110081400 | 0,296 | | chr16 | 71206932 | 71207182 | 0,348 |
| chr4 | 110970150 | 110970400 | 0,348 | | chr16 | 72033345 | 72033595 | 0,348 |
| chr4 | 111958216 | 111958466 | 0,348 | | chr16 | 72837496 | 72837746 | 0,348 |
| chr4 | 112823591 | 112823841 | 0,348 | | chr16 | 73239267 | 73239517 | 0,348 |
| chr4 | 113629945 | 113630195 | 0,264 | | chr16 | 73642603 | 73642853 | 0,336 |
| chr4 | 115384150 | 115384400 | 0,328 | | chr16 | 74481276 | 74481526 | 0,348 |
| chr4 | 116190298 | 116190548 | 0,348 | | chr16 | 75327074 | 75327324 | 0,328 |
| chr4 | 117009231 | 117009481 | 0,3 | | chr16 | 75766225 | 75766475 | 0,344 |
| chr4 | 117809721 | 117809971 | 0,336 | | chr16 | 76259454 | 76259704 | 0,348 |
| chr4 | 118609887 | 118610137 | 0,324 | | chr16 | 77308495 | 77308745 | 0,312 |
| chr4 | 119597337 | 119597587 | 0,348 | | chr16 | 78168150 | 78168400 | 0,348 |
| chr4 | 120411943 | 120412193 | 0,268 | | chr16 | 78721550 | 78721800 | 0,344 |
| chr4 | 121214719 | 121214969 | 0,348 | | chr16 | 78979256 | 78979506 | 0,336 |
| chr4 | 122018543 | 122018793 | 0,348 | | chr16 | 79786137 | 79786387 | 0,348 |
| chr4 | 122820118 | 122820368 | 0,332 | | chr16 | 80589959 | 80590209 | 0,348 |
| chr4 | 123622182 | 123622432 | 0,284 | | chr16 | 81009953 | 81010203 | 0,348 |
| chr4 | 123663257 | 123663507 | 0,36 | | chr16 | 81411556 | 81411806 | 0,348 |
| chr4 | 123663787 | 123664037 | 0,392 | | chr16 | 82958335 | 82958585 | 0,348 |
| chr4 | 123663985 | 123664235 | 0,408 | | chr16 | 83829502 | 83829752 | 0,348 |
| chr4 | 123664405 | 123664655 | 0,436 | | chr16 | 84166382 | 84166632 | 0,336 |
| chr4 | 124422894 | 124423144 | 0,324 | | chr16 | 85076447 | 85076697 | 0,348 |
| chr4 | 125512382 | 125512632 | 0,264 | | chr16 | 85907481 | 85907731 | 0,32 |
| chr4 | 126813261 | 126813511 | 0,348 | | chr16 | 86714059 | 86714309 | 0,324 |
| chr4 | 127615051 | 127615301 | 0,3 | | chr16 | 87736388 | 87736638 | 0,348 |
| chr4 | 128419953 | 128420203 | 0,348 | | chr16 | 88097355 | 88097605 | 0,348 |
| chr4 | 129523487 | 129523737 | 0,348 | | chr16 | 89161654 | 89161904 | 0,348 |
| chr4 | 130323522 | 130323772 | 0,348 | | chr16 | 89849148 | 89849398 | 0,484 |
| chr4 | 131125980 | 131126230 | 0,256 | | chr16 | 89849164 | 89849414 | 0,492 |
| chr4 | 131938368 | 131938618 | 0,304 | | chr16 | 89877010 | 89877260 | 0,384 |
| chr4 | 132925295 | 132925545 | 0,332 | | chr17 | 465619 | 465869 | 0,476 |
| chr4 | 133727028 | 133727278 | 0,292 | | chr17 | 559153 | 559403 | 0,348 |
| chr4 | 134535097 | 134535347 | 0,336 | | chr17 | 879453 | 879703 | 0,348 |
| chr4 | 135337052 | 135337302 | 0,284 | | chr17 | 1455714 | 1455964 | 0,348 |
| chr4 | 136137384 | 136137634 | 0,276 | | chr17 | 2287357 | 2287607 | 0,348 |
| chr4 | 136938690 | 136938940 | 0,328 | | chr17 | 2588173 | 2588423 | 0,348 |
| chr4 | 137875318 | 137875568 | 0,348 | | chr17 | 3088772 | 3089022 | 0,284 |
| chr4 | 138925335 | 138925585 | 0,348 | | chr17 | 3563424 | 3563674 | 0,576 |
| chr4 | 139726418 | 139726668 | 0,348 | | chr17 | 3916884 | 3917134 | 0,308 |
| chr4 | 140766433 | 140766683 | 0,348 | | chr17 | 5011281 | 5011531 | 0,348 |
| chr4 | 142917150 | 142917400 | 0,284 | | chr17 | 5431814 | 5432064 | 0,348 |
| chr4 | 143721319 | 143721569 | 0,344 | | chr17 | 6026560 | 6026810 | 0,348 |
| chr4 | 144616150 | 144616400 | 0,272 | | chr17 | 6830537 | 6830787 | 0,336 |
| chr4 | 145420015 | 145420265 | 0,332 | | chr17 | 7128160 | 7128410 | 0,628 |
| chr4 | 146237783 | 146238033 | 0,3 | | chr17 | 7651065 | 7651315 | 0,348 |
| chr4 | 146560230 | 146560480 | 0,436 | | chr17 | 7918695 | 7918945 | 0,604 |
| chr4 | 146560327 | 146560577 | 0,396 | | chr17 | 8263781 | 8264031 | 0,344 |
| chr4 | 146560432 | 146560682 | 0,404 | | chr17 | 8461018 | 8461268 | 0,348 |
| chr4 | 146560450 | 146560700 | 0,396 | | chr17 | 9328553 | 9328803 | 0,304 |
| chr4 | 146560519 | 146560769 | 0,372 | | chr17 | 9787971 | 9788221 | 0,348 |
| chr4 | 146560579 | 146560829 | 0,356 | | chr17 | 10223454 | 10223704 | 0,348 |
| chr4 | 146563453 | 146563703 | 0,4 | | chr17 | 11055587 | 11055837 | 0,32 |
| chr4 | 146563507 | 146563757 | 0,436 | | chr17 | 11886166 | 11886416 | 0,324 |
| chr4 | 146567088 | 146567338 | 0,38 | | chr17 | 12278449 | 12278699 | 0,308 |
| chr4 | 146567195 | 146567445 | 0,352 | | chr17 | 12691155 | 12691405 | 0,348 |
| chr4 | 146576280 | 146576530 | 0,476 | | chr17 | 14005537 | 14005787 | 0,348 |
| chr4 | 147039150 | 147039400 | 0,348 | | chr17 | 15093150 | 15093400 | 0,34 |
| chr4 | 147861172 | 147861422 | 0,348 | | chr17 | 15416497 | 15416747 | 0,336 |
| chr4 | 148947515 | 148947765 | 0,348 | | chr17 | 15901623 | 15901873 | 0,348 |
| chr4 | 149763150 | 149763400 | 0,336 | | chr17 | 16781645 | 16781895 | 0,348 |
| chr4 | 151024279 | 151024529 | 0,3 | | chr17 | 17377934 | 17378184 | 0,348 |
| chr4 | 151837404 | 151837654 | 0,308 | | chr17 | 17818108 | 17818358 | 0,348 |
| chr4 | 152647195 | 152647445 | 0,324 | | chr17 | 18764031 | 18764281 | 0,348 |
| chr4 | 154417224 | 154417474 | 0,348 | | chr17 | 19566518 | 19566768 | 0,352 |
| chr4 | 155217502 | 155217752 | 0,348 | | chr17 | 19999381 | 19999631 | 0,336 |
| chr4 | 156024046 | 156024296 | 0,348 | | chr17 | 20209152 | 20209402 | 0,336 |
| chr4 | 156824354 | 156824604 | 0,316 | | chr17 | 20829712 | 20829962 | 0,348 |
| chr4 | 157626326 | 157626576 | 0,332 | | chr17 | 22025919 | 22026169 | 0,348 |
| chr4 | 158431044 | 158431294 | 0,296 | | chr17 | 25630230 | 25630480 | 0,348 |
| chr4 | 159237626 | 159237876 | 0,348 | | chr17 | 27580360 | 27580610 | 0,276 |
| chr4 | 160045854 | 160046104 | 0,28 | | chr17 | 28417566 | 28417816 | 0,348 |
| chr4 | 160857413 | 160857663 | 0,312 | | chr17 | 28819459 | 28819709 | 0,32 |
| chr4 | 161657414 | 161657664 | 0,344 | | chr17 | 29219615 | 29219865 | 0,332 |
| chr4 | 162457547 | 162457797 | 0,304 | | chr17 | 30219281 | 30219531 | 0,348 |
| chr4 | 163258332 | 163258582 | 0,348 | | chr17 | 30657441 | 30657691 | 0,348 |
| chr4 | 164775219 | 164775469 | 0,348 | | chr17 | 31020026 | 31020276 | 0,312 |
| chr4 | 165581368 | 165581618 | 0,332 | | chr17 | 31982636 | 31982886 | 0,348 |
| chr4 | 166385754 | 166386004 | 0,348 | | chr17 | 33771316 | 33771566 | 0,348 |
| chr4 | 167194635 | 167194885 | 0,332 | | chr17 | 34382478 | 34382728 | 0,3 |
| chr4 | 167998589 | 167998839 | 0,296 | | chr17 | 34823051 | 34823301 | 0,348 |
| chr4 | 168813970 | 168814220 | 0,324 | | chr17 | 35632575 | 35632825 | 0,328 |
| chr4 | 169620754 | 169621004 | 0,32 | | chr17 | 36513607 | 36513857 | 0,348 |
| chr4 | 170428650 | 170428900 | 0,292 | | chr17 | 36957600 | 36957850 | 0,348 |
| chr4 | 172239593 | 172239843 | 0,308 | | chr17 | 37409895 | 37410145 | 0,348 |
| chr4 | 173054299 | 173054549 | 0,348 | | chr17 | 38280124 | 38280374 | 0,348 |
| chr4 | 173854598 | 173854848 | 0,268 | | chr17 | 38783515 | 38783765 | 0,348 |
| chr4 | 174658150 | 174658400 | 0,348 | | chr17 | 39080566 | 39080816 | 0,336 |
| chr4 | 175674466 | 175674716 | 0,348 | | chr17 | 40004497 | 40004747 | 0,348 |
| chr4 | 176773440 | 176773690 | 0,324 | | chr17 | 40695364 | 40695614 | 0,64 |
| chr4 | 177574380 | 177574630 | 0,288 | | chr17 | 40695461 | 40695711 | 0,66 |
| chr4 | 178354254 | 178354504 | 0,344 | | chr17 | 40695588 | 40695838 | 0,62 |
| chr4 | 178376420 | 178376670 | 0,348 | | chr17 | 40695755 | 40696005 | 0,632 |
| chr4 | 179177025 | 179177275 | 0,324 | | chr17 | 40695920 | 40696170 | 0,584 |
| chr4 | 179990413 | 179990663 | 0,324 | | chr17 | 40961983 | 40962233 | 0,332 |
| chr4 | 180955150 | 180955400 | 0,332 | | chr17 | 41052842 | 41053092 | 0,496 |
| chr4 | 181758151 | 181758401 | 0,348 | | chr17 | 41052881 | 41053131 | 0,484 |
| chr4 | 182559169 | 182559419 | 0,348 | | chr17 | 41062968 | 41063218 | 0,536 |
| chr4 | 183364952 | 183365202 | 0,348 | | chr17 | 41063053 | 41063303 | 0,588 |
| chr4 | 184169569 | 184169819 | 0,308 | | chr17 | 41362685 | 41362935 | 0,324 |
| chr4 | 184975478 | 184975728 | 0,328 | | chr17 | 41843202 | 41843452 | 0,348 |
| chr4 | 185777424 | 185777674 | 0,348 | | chr17 | 42736824 | 42737074 | 0,28 |
| chr4 | 186586136 | 186586386 | 0,272 | | chr17 | 43767203 | 43767453 | 0,348 |
| chr4 | 187195222 | 187195472 | 0,476 | | chr17 | 44271724 | 44271974 | 0,348 |
| chr4 | 187201287 | 187201537 | 0,512 | | chr17 | 44787702 | 44787952 | 0,324 |
| chr4 | 187833378 | 187833628 | 0,348 | | chr17 | 45676520 | 45676770 | 0,308 |
| chr4 | 188635326 | 188635576 | 0,348 | | chr17 | 46151588 | 46151838 | 0,348 |
| chr4 | 189455666 | 189455916 | 0,348 | | chr17 | 46492183 | 46492433 | 0,348 |
| chr5 | 925341 | 925591 | 0,348 | | chr17 | 47368119 | 47368369 | 0,348 |
| chr5 | 1760577 | 1760827 | 0,348 | | chr17 | 48244905 | 48245155 | 0,652 |
| chr5 | 2571762 | 2572012 | 0,348 | | chr17 | 48500926 | 48501176 | 0,332 |
| chr5 | 3908300 | 3908550 | 0,328 | | chr17 | 48925318 | 48925568 | 0,348 |
| chr5 | 5808362 | 5808612 | 0,348 | | chr17 | 49302069 | 49302319 | 0,348 |
| chr5 | 7066150 | 7066400 | 0,332 | | chr17 | 50153263 | 50153513 | 0,348 |
| chr5 | 7929390 | 7929640 | 0,348 | | chr17 | 51249262 | 51249512 | 0,308 |
| chr5 | 8884167 | 8884417 | 0,348 | | chr17 | 51650684 | 51650934 | 0,348 |
| chr5 | 9694150 | 9694400 | 0,312 | | chr17 | 52050727 | 52050977 | 0,304 |
| chr5 | 11072336 | 11072586 | 0,348 | | chr17 | 53502160 | 53502410 | 0,348 |
| chr5 | 11925598 | 11925848 | 0,348 | | chr17 | 54154294 | 54154544 | 0,348 |
| chr5 | 12734408 | 12734658 | 0,348 | | chr17 | 54514376 | 54514626 | 0,348 |
| chr5 | 14411235 | 14411485 | 0,348 | | chr17 | 55667460 | 55667710 | 0,348 |
| chr5 | 16434161 | 16434411 | 0,348 | | chr17 | 56296396 | 56296646 | 0,64 |
| chr5 | 16434161 | 16434411 | 0,348 | | chr17 | 56469489 | 56469739 | 0,348 |
| chr5 | 17378337 | 17378587 | 0,348 | | chr17 | 57271861 | 57272111 | 0,348 |
| chr5 | 18379157 | 18379407 | 0,348 | | chr17 | 58119107 | 58119357 | 0,348 |
| chr5 | 19179651 | 19179901 | 0,32 | | chr17 | 58529289 | 58529539 | 0,332 |
| chr5 | 19982105 | 19982355 | 0,272 | | chr17 | 58934643 | 58934893 | 0,348 |
| chr5 | 21021592 | 21021842 | 0,332 | | chr17 | 59737150 | 59737400 | 0,316 |
| chr5 | 22260550 | 22260800 | 0,308 | | chr17 | 60644768 | 60645018 | 0,332 |
| chr5 | 23427408 | 23427658 | 0,316 | | chr17 | 61051969 | 61052219 | 0,32 |
| chr5 | 24333150 | 24333400 | 0,252 | | chr17 | 61464852 | 61465102 | 0,348 |
| chr5 | 25638150 | 25638400 | 0,32 | | chr17 | 62271111 | 62271361 | 0,348 |
| chr5 | 26472150 | 26472400 | 0,344 | | chr17 | 62645048 | 62645298 | 0,304 |
| chr5 | 27277073 | 27277323 | 0,348 | | chr17 | 63117102 | 63117352 | 0,284 |
| chr5 | 28441164 | 28441414 | 0,328 | | chr17 | 63926144 | 63926394 | 0,348 |
| chr5 | 29277434 | 29277684 | 0,348 | | chr17 | 64736660 | 64736910 | 0,348 |
| chr5 | 30147460 | 30147710 | 0,28 | | chr17 | 65689242 | 65689492 | 0,348 |
| chr5 | 30965150 | 30965400 | 0,304 | | chr17 | 66804590 | 66804840 | 0,348 |
| chr5 | 32276489 | 32276739 | 0,348 | | chr17 | 67242150 | 67242400 | 0,34 |
| chr5 | 33120530 | 33120780 | 0,336 | | chr17 | 68050397 | 68050647 | 0,296 |
| chr5 | 33963570 | 33963820 | 0,348 | | chr17 | 69063221 | 69063471 | 0,332 |
| chr5 | 34808150 | 34808400 | 0,276 | | chr17 | 69978262 | 69978512 | 0,348 |
| chr5 | 35611221 | 35611471 | 0,34 | | chr17 | 70909150 | 70909400 | 0,336 |
| chr5 | 36450561 | 36450811 | 0,348 | | chr17 | 71861764 | 71862014 | 0,332 |
| chr5 | 38217206 | 38217456 | 0,348 | | chr17 | 72572363 | 72572613 | 0,308 |
| chr5 | 39308293 | 39308543 | 0,344 | | chr17 | 73007683 | 73007933 | 0,348 |
| chr5 | 40243400 | 40243650 | 0,348 | | chr17 | 73941279 | 73941529 | 0,348 |
| chr5 | 41069505 | 41069755 | 0,308 | | chr17 | 74441215 | 74441465 | 0,348 |
| chr5 | 42023181 | 42023431 | 0,348 | | chr17 | 75094873 | 75095123 | 0,348 |
| chr5 | 42907557 | 42907807 | 0,264 | | chr17 | 75957718 | 75957968 | 0,348 |
| chr5 | 43869212 | 43869462 | 0,348 | | chr17 | 76771456 | 76771706 | 0,348 |
| chr5 | 44811198 | 44811448 | 0,348 | | chr17 | 77357842 | 77358092 | 0,348 |
| chr5 | 45614193 | 45614443 | 0,348 | | chr17 | 77749872 | 77750122 | 0,348 |
| chr5 | 49559754 | 49560004 | 0,288 | | chr17 | 78078272 | 78078499 | 0,647577 |
| chr5 | 51558414 | 51558664 | 0,328 | | chr17 | 78078810 | 78079059 | 0,666667 |
| chr5 | 52572333 | 52572583 | 0,348 | | chr17 | 78086683 | 78086876 | 0,65285 |
| chr5 | 53692364 | 53692614 | 0,348 | | chr17 | 78090765 | 78090952 | 0,647059 |
| chr5 | 54615371 | 54615621 | 0,344 | | chr17 | 78091969 | 78092218 | 0,662651 |
| chr5 | 55613299 | 55613549 | 0,316 | | chr17 | 78184281 | 78184531 | 0,668 |
| chr5 | 56865222 | 56865472 | 0,348 | | chr17 | 78184353 | 78184603 | 0,624 |
| chr5 | 57694196 | 57694446 | 0,276 | | chr17 | 78187505 | 78187755 | 0,692 |
| chr5 | 58497233 | 58497483 | 0,252 | | chr17 | 78187877 | 78188127 | 0,644 |
| chr5 | 59329150 | 59329400 | 0,312 | | chr17 | 78188311 | 78188561 | 0,632 |
| chr5 | 60144254 | 60144504 | 0,32 | | chr17 | 78188412 | 78188662 | 0,604 |
| chr5 | 60953095 | 60953345 | 0,348 | | chr17 | 78559453 | 78559703 | 0,324 |
| chr5 | 62167485 | 62167735 | 0,348 | | chr17 | 79870994 | 79871244 | 0,348 |
| chr5 | 63311150 | 63311400 | 0,328 | | chr17 | 80358326 | 80358576 | 0,348 |
| chr5 | 64114150 | 64114400 | 0,312 | | chr18 | 352317 | 352567 | 0,348 |
| chr5 | 64921258 | 64921508 | 0,348 | | chr18 | 1376361 | 1376611 | 0,348 |
| chr5 | 65772331 | 65772581 | 0,348 | | chr18 | 1780339 | 1780589 | 0,348 |
| chr5 | 66826161 | 66826411 | 0,348 | | chr18 | 2178416 | 2178666 | 0,348 |
| chr5 | 67641208 | 67641458 | 0,348 | | chr18 | 2981417 | 2981667 | 0,348 |
| chr5 | 68472969 | 68473219 | 0,348 | | chr18 | 3133377 | 3133627 | 0,348 |
| chr5 | 70238181 | 70238430 | 0,37751 | | chr18 | 4041313 | 4041563 | 0,344 |
| chr5 | 70241800 | 70241994 | 0,35567 | | chr18 | 5367216 | 5367466 | 0,348 |
| chr5 | 70247767 | 70247866 | 0,343434 | | chr18 | 6171554 | 6171804 | 0,328 |
| chr5 | 70752734 | 70752984 | 0,348 | | chr18 | 6578629 | 6578879 | 0,348 |
| chr5 | 71556027 | 71556277 | 0,332 | | chr18 | 6977250 | 6977500 | 0,348 |
| chr5 | 72610157 | 72610407 | 0,348 | | chr18 | 8023198 | 8023448 | 0,348 |
| chr5 | 73516578 | 73516828 | 0,316 | | chr18 | 8257365 | 8257615 | 0,348 |
| chr5 | 74016348 | 74016598 | 0,428 | | chr18 | 9211150 | 9211400 | 0,308 |
| chr5 | 74321150 | 74321400 | 0,3 | | chr18 | 10066349 | 10066599 | 0,348 |
| chr5 | 75142529 | 75142779 | 0,324 | | chr18 | 10924564 | 10924814 | 0,332 |
| chr5 | 75949606 | 75949856 | 0,34 | | chr18 | 11728526 | 11728776 | 0,34 |
| chr5 | 76753702 | 76753952 | 0,308 | | chr18 | 12529626 | 12529876 | 0,312 |
| chr5 | 77564503 | 77564753 | 0,348 | | chr18 | 12968418 | 12968668 | 0,348 |
| chr5 | 78373076 | 78373326 | 0,348 | | chr18 | 13399720 | 13399970 | 0,348 |
| chr5 | 79271575 | 79271825 | 0,348 | | chr18 | 14932813 | 14933063 | 0,348 |
| chr5 | 80322374 | 80322624 | 0,348 | | chr18 | 18540358 | 18540608 | 0,316 |
| chr5 | 81143414 | 81143664 | 0,348 | | chr18 | 19342552 | 19342802 | 0,288 |
| chr5 | 82115520 | 82115770 | 0,328 | | chr18 | 20143679 | 20143929 | 0,348 |
| chr5 | 83161167 | 83161417 | 0,252 | | chr18 | 20957400 | 20957650 | 0,3 |
| chr5 | 83962946 | 83963196 | 0,34 | | chr18 | 21115324 | 21115574 | 0,5 |
| chr5 | 84798100 | 84798350 | 0,292 | | chr18 | 21115522 | 21115772 | 0,536 |
| chr5 | 85607624 | 85607874 | 0,28 | | chr18 | 21116575 | 21116825 | 0,52 |
| chr5 | 86610487 | 86610737 | 0,348 | | chr18 | 21118438 | 21118688 | 0,512 |
| chr5 | 87453348 | 87453598 | 0,348 | | chr18 | 21118490 | 21118740 | 0,508 |
| chr5 | 88723150 | 88723400 | 0,268 | | chr18 | 21119268 | 21119518 | 0,456 |
| chr5 | 90119327 | 90119577 | 0,348 | | chr18 | 21119662 | 21119912 | 0,524 |
| chr5 | 91072151 | 91072401 | 0,348 | | chr18 | 21119781 | 21120031 | 0,504 |
| chr5 | 91927392 | 91927642 | 0,316 | | chr18 | 21121194 | 21121444 | 0,484 |
| chr5 | 93066150 | 93066400 | 0,272 | | chr18 | 21136270 | 21136520 | 0,552 |
| chr5 | 94084150 | 94084400 | 0,336 | | chr18 | 21148788 | 21149038 | 0,368 |
| chr5 | 94884324 | 94884574 | 0,308 | | chr18 | 21501150 | 21501400 | 0,312 |
| chr5 | 95817279 | 95817529 | 0,348 | | chr18 | 22195155 | 22195405 | 0,34 |
| chr5 | 96673550 | 96673800 | 0,3 | | chr18 | 23350150 | 23350400 | 0,32 |
| chr5 | 97504150 | 97504400 | 0,308 | | chr18 | 24154458 | 24154708 | 0,348 |
| chr5 | 98705396 | 98705646 | 0,348 | | chr18 | 25617594 | 25617844 | 0,348 |
| chr5 | 99560435 | 99560685 | 0,32 | | chr18 | 26133235 | 26133485 | 0,276 |
| chr5 | 100365824 | 100366074 | 0,34 | | chr18 | 26944340 | 26944590 | 0,32 |
| chr5 | 101174653 | 101174903 | 0,292 | | chr18 | 27750063 | 27750313 | 0,328 |
| chr5 | 102365150 | 102365400 | 0,308 | | chr18 | 28150278 | 28150528 | 0,296 |
| chr5 | 103728150 | 103728400 | 0,332 | | chr18 | 28550925 | 28551175 | 0,308 |
| chr5 | 104531179 | 104531429 | 0,252 | | chr18 | 29794550 | 29794800 | 0,34 |
| chr5 | 105350391 | 105350641 | 0,284 | | chr18 | 30206170 | 30206420 | 0,348 |
| chr5 | 106531361 | 106531611 | 0,348 | | chr18 | 30617559 | 30617809 | 0,328 |
| chr5 | 107570373 | 107570623 | 0,348 | | chr18 | 32104550 | 32104800 | 0,312 |
| chr5 | 108926232 | 108926482 | 0,348 | | chr18 | 33485167 | 33485417 | 0,348 |
| chr5 | 110027150 | 110027400 | 0,328 | | chr18 | 34661394 | 34661644 | 0,324 |
| chr5 | 111196451 | 111196701 | 0,332 | | chr18 | 35818572 | 35818822 | 0,348 |
| chr5 | 112021809 | 112022059 | 0,348 | | chr18 | 36409564 | 36409814 | 0,348 |
| chr5 | 113013150 | 113013400 | 0,328 | | chr18 | 36817547 | 36817797 | 0,284 |
| chr5 | 114065262 | 114065512 | 0,348 | | chr18 | 37631150 | 37631400 | 0,336 |
| chr5 | 114872251 | 114872501 | 0,348 | | chr18 | 37749565 | 37749815 | 0,344 |
| chr5 | 115988311 | 115988561 | 0,348 | | chr18 | 38710272 | 38710522 | 0,348 |
| chr5 | 117001555 | 117001805 | 0,348 | | chr18 | 39747382 | 39747632 | 0,34 |
| chr5 | 117930159 | 117930409 | 0,348 | | chr18 | 40637226 | 40637476 | 0,348 |
| chr5 | 118788222 | 118788472 | 0,656 | | chr18 | 41439110 | 41439360 | 0,276 |
| chr5 | 119230150 | 119230400 | 0,328 | | chr18 | 41829773 | 41830023 | 0,348 |
| chr5 | 120473241 | 120473491 | 0,348 | | chr18 | 42239853 | 42240103 | 0,336 |
| chr5 | 121394548 | 121394798 | 0,348 | | chr18 | 43040497 | 43040747 | 0,348 |
| chr5 | 122571402 | 122571652 | 0,348 | | chr18 | 43843110 | 43843360 | 0,348 |
| chr5 | 123678502 | 123678752 | 0,348 | | chr18 | 44175715 | 44175965 | 0,348 |
| chr5 | 124774217 | 124774467 | 0,348 | | chr18 | 44654383 | 44654633 | 0,348 |
| chr5 | 125616247 | 125616497 | 0,348 | | chr18 | 45454440 | 45454690 | 0,348 |
| chr5 | 126426335 | 126426585 | 0,348 | | chr18 | 45933306 | 45933556 | 0,348 |
| chr5 | 127322174 | 127322424 | 0,336 | | chr18 | 46473309 | 46473559 | 0,348 |
| chr5 | 128479275 | 128479525 | 0,324 | | chr18 | 47313188 | 47313438 | 0,32 |
| chr5 | 129376150 | 129376400 | 0,34 | | chr18 | 48143536 | 48143786 | 0,34 |
| chr5 | 130223163 | 130223413 | 0,348 | | chr18 | 48537991 | 48538241 | 0,348 |
| chr5 | 131031617 | 131031867 | 0,28 | | chr18 | 48948147 | 48948397 | 0,348 |
| chr5 | 131713947 | 131714197 | 0,496 | | chr18 | 49765022 | 49765272 | 0,348 |
| chr5 | 131719848 | 131720098 | 0,488 | | chr18 | 50569985 | 50570235 | 0,336 |
| chr5 | 131722606 | 131722856 | 0,544 | | chr18 | 50977389 | 50977639 | 0,348 |
| chr5 | 131726399 | 131726649 | 0,524 | | chr18 | 51386182 | 51386432 | 0,344 |
| chr5 | 131726406 | 131726656 | 0,536 | | chr18 | 52359206 | 52359456 | 0,252 |
| chr5 | 131728056 | 131728306 | 0,52 | | chr18 | 53778557 | 53778807 | 0,348 |
| chr5 | 131728165 | 131728415 | 0,52 | | chr18 | 54396170 | 54396420 | 0,348 |
| chr5 | 131877011 | 131877261 | 0,348 | | chr18 | 55361574 | 55361824 | 0,348 |
| chr5 | 132746346 | 132746596 | 0,348 | | chr18 | 56168157 | 56168407 | 0,344 |
| chr5 | 133549999 | 133550249 | 0,268 | | chr18 | 56575500 | 56575750 | 0,348 |
| chr5 | 134367907 | 134368157 | 0,348 | | chr18 | 56969651 | 56969901 | 0,348 |
| chr5 | 135271150 | 135271400 | 0,328 | | chr18 | 58270587 | 58270837 | 0,252 |
| chr5 | 136109975 | 136110225 | 0,348 | | chr18 | 58864470 | 58864720 | 0,348 |
| chr5 | 136919859 | 136920109 | 0,348 | | chr18 | 59829150 | 59829400 | 0,324 |
| chr5 | 137721384 | 137721634 | 0,348 | | chr18 | 60629990 | 60630240 | 0,348 |
| chr5 | 138617755 | 138618005 | 0,348 | | chr18 | 61030027 | 61030277 | 0,348 |
| chr5 | 139437032 | 139437282 | 0,32 | | chr18 | 61430302 | 61430552 | 0,348 |
| chr5 | 140243745 | 140243995 | 0,284 | | chr18 | 62397150 | 62397400 | 0,332 |
| chr5 | 141342082 | 141342332 | 0,348 | | chr18 | 63197866 | 63198116 | 0,312 |
| chr5 | 142430497 | 142430747 | 0,348 | | chr18 | 63594781 | 63595031 | 0,324 |
| chr5 | 143263150 | 143263400 | 0,312 | | chr18 | 63997888 | 63998138 | 0,26 |
| chr5 | 144100481 | 144100731 | 0,348 | | chr18 | 64798724 | 64798974 | 0,348 |
| chr5 | 144924251 | 144924501 | 0,332 | | chr18 | 65202000 | 65202250 | 0,336 |
| chr5 | 145821033 | 145821283 | 0,348 | | chr18 | 65599811 | 65600061 | 0,288 |
| chr5 | 146723352 | 146723602 | 0,284 | | chr18 | 66403127 | 66403377 | 0,348 |
| chr5 | 148103150 | 148103400 | 0,348 | | chr18 | 67205175 | 67205425 | 0,288 |
| chr5 | 149357488 | 149357738 | 0,448 | | chr18 | 67611261 | 67611511 | 0,316 |
| chr5 | 149357622 | 149357872 | 0,416 | | chr18 | 68016661 | 68016911 | 0,344 |
| chr5 | 149360046 | 149360296 | 0,396 | | chr18 | 68818492 | 68818742 | 0,332 |
| chr5 | 149360988 | 149361238 | 0,448 | | chr18 | 69633509 | 69633759 | 0,348 |
| chr5 | 150168418 | 150168668 | 0,348 | | chr18 | 70029267 | 70029517 | 0,32 |
| chr5 | 151153336 | 151153586 | 0,312 | | chr18 | 70436499 | 70436749 | 0,348 |
| chr5 | 151974339 | 151974589 | 0,252 | | chr18 | 71240897 | 71241147 | 0,316 |
| chr5 | 153079172 | 153079422 | 0,348 | | chr18 | 71643656 | 71643906 | 0,324 |
| chr5 | 154769590 | 154769840 | 0,348 | | chr18 | 72045281 | 72045531 | 0,348 |
| chr5 | 155629221 | 155629471 | 0,348 | | chr18 | 74383168 | 74383418 | 0,348 |
| chr5 | 155771459 | 155771709 | 0,46 | | chr18 | 74798379 | 74798629 | 0,348 |
| chr5 | 156569984 | 156570234 | 0,348 | | chr18 | 75604856 | 75605106 | 0,288 |
| chr5 | 157381960 | 157382210 | 0,336 | | chr18 | 76965150 | 76965400 | 0,332 |
| chr5 | 158468169 | 158468419 | 0,348 | | chr18 | 77566578 | 77566828 | 0,348 |
| chr5 | 159465150 | 159465400 | 0,304 | | chr19 | 1364412 | 1364662 | 0,348 |
| chr5 | 160279480 | 160279730 | 0,348 | | chr19 | 2857080 | 2857330 | 0,348 |
| chr5 | 161080261 | 161080511 | 0,348 | | chr19 | 4423005 | 4423255 | 0,348 |
| chr5 | 161901345 | 161901595 | 0,344 | | chr19 | 5389785 | 5390035 | 0,348 |
| chr5 | 162917271 | 162917521 | 0,32 | | chr19 | 5612398 | 5612648 | 0,348 |
| chr5 | 163717664 | 163717914 | 0,348 | | chr19 | 6103129 | 6103379 | 0,348 |
| chr5 | 164517680 | 164517930 | 0,304 | | chr19 | 7182731 | 7182981 | 0,304 |
| chr5 | 165319177 | 165319427 | 0,348 | | chr19 | 8516971 | 8517221 | 0,348 |
| chr5 | 167015237 | 167015487 | 0,348 | | chr19 | 8923201 | 8923451 | 0,348 |
| chr5 | 167919124 | 167919374 | 0,328 | | chr19 | 9341186 | 9341436 | 0,348 |
| chr5 | 168721160 | 168721410 | 0,348 | | chr19 | 9581544 | 9581794 | 0,348 |
| chr5 | 169550080 | 169550330 | 0,336 | | chr19 | 10244002 | 10244252 | 0,344 |
| chr5 | 170379303 | 170379553 | 0,316 | | chr19 | 10677063 | 10677313 | 0,348 |
| chr5 | 171180930 | 171181180 | 0,348 | | chr19 | 10781045 | 10781295 | 0,348 |
| chr5 | 172017300 | 172017550 | 0,296 | | chr19 | 11199420 | 11199670 | 0,348 |
| chr5 | 172827926 | 172828176 | 0,348 | | chr19 | 11216102 | 11216211 | 0,59633 |
| chr5 | 173664137 | 173664387 | 0,348 | | chr19 | 11711517 | 11711767 | 0,348 |
| chr5 | 174480831 | 174481081 | 0,328 | | chr19 | 12105696 | 12105946 | 0,32 |
| chr5 | 175574930 | 175575180 | 0,348 | | chr19 | 12360810 | 12361060 | 0,296 |
| chr5 | 176381946 | 176382196 | 0,348 | | chr19 | 12625145 | 12625395 | 0,348 |
| chr5 | 177419684 | 177419934 | 0,62 | | chr19 | 13006933 | 13007183 | 0,612 |
| chr5 | 177419909 | 177420159 | 0,572 | | chr19 | 13007001 | 13007251 | 0,612 |
| chr5 | 177421022 | 177421272 | 0,588 | | chr19 | 13007639 | 13007889 | 0,612 |
| chr5 | 178424528 | 178424778 | 0,348 | | chr19 | 14730075 | 14730325 | 0,348 |
| chr5 | 179368971 | 179369221 | 0,348 | | chr19 | 15394044 | 15394294 | 0,348 |
| chr5 | 180175488 | 180175738 | 0,34 | | chr19 | 15860722 | 15860972 | 0,348 |
| chr6 | 722215 | 722465 | 0,348 | | chr19 | 16508380 | 16508630 | 0,344 |
| chr6 | 1809311 | 1809561 | 0,332 | | chr19 | 16737060 | 16737310 | 0,348 |
| chr6 | 2680150 | 2680400 | 0,288 | | chr19 | 16960936 | 16961186 | 0,3 |
| chr6 | 3484150 | 3484400 | 0,336 | | chr19 | 19135821 | 19136071 | 0,348 |
| chr6 | 4620332 | 4620582 | 0,344 | | chr19 | 19510651 | 19510901 | 0,348 |
| chr6 | 5431006 | 5431256 | 0,348 | | chr19 | 19952103 | 19952353 | 0,308 |
| chr6 | 6236906 | 6237156 | 0,348 | | chr19 | 20230842 | 20231092 | 0,336 |
| chr6 | 7045378 | 7045628 | 0,348 | | chr19 | 20462509 | 20462759 | 0,348 |
| chr6 | 7964447 | 7964697 | 0,348 | | chr19 | 21130690 | 21130940 | 0,284 |
| chr6 | 9467470 | 9467720 | 0,348 | | chr19 | 21131150 | 21131400 | 0,336 |
| chr6 | 10269525 | 10269775 | 0,348 | | chr19 | 21575132 | 21575382 | 0,348 |
| chr6 | 11927322 | 11927572 | 0,348 | | chr19 | 21961594 | 21961844 | 0,348 |
| chr6 | 12773150 | 12773400 | 0,316 | | chr19 | 22306026 | 22306276 | 0,34 |
| chr6 | 13976150 | 13976400 | 0,34 | | chr19 | 22764711 | 22764961 | 0,34 |
| chr6 | 14834528 | 14834778 | 0,348 | | chr19 | 23219468 | 23219718 | 0,268 |
| chr6 | 16162150 | 16162400 | 0,348 | | chr19 | 23594704 | 23594954 | 0,344 |
| chr6 | 18388156 | 18388406 | 0,348 | | chr19 | 23778748 | 23778998 | 0,344 |
| chr6 | 19660443 | 19660693 | 0,348 | | chr19 | 23985429 | 23985679 | 0,348 |
| chr6 | 20718512 | 20718762 | 0,348 | | chr19 | 24324202 | 24324452 | 0,288 |
| chr6 | 21534150 | 21534400 | 0,336 | | chr19 | 28272923 | 28273173 | 0,312 |
| chr6 | 22919570 | 22919820 | 0,348 | | chr19 | 28780463 | 28780713 | 0,348 |
| chr6 | 23724897 | 23725147 | 0,344 | | chr19 | 29320557 | 29320807 | 0,348 |
| chr6 | 24533878 | 24534128 | 0,348 | | chr19 | 29588492 | 29588742 | 0,348 |
| chr6 | 25335487 | 25335737 | 0,284 | | chr19 | 30033997 | 30034247 | 0,348 |
| chr6 | 26142116 | 26142366 | 0,348 | | chr19 | 30458220 | 30458470 | 0,348 |
| chr6 | 26999810 | 27000060 | 0,328 | | chr19 | 30892388 | 30892638 | 0,348 |
| chr6 | 27984245 | 27984495 | 0,348 | | chr19 | 31361227 | 31361477 | 0,348 |
| chr6 | 29227316 | 29227566 | 0,348 | | chr19 | 31763334 | 31763584 | 0,348 |
| chr6 | 30035500 | 30035750 | 0,348 | | chr19 | 32165641 | 32165891 | 0,348 |
| chr6 | 32257190 | 32257440 | 0,276 | | chr19 | 32560119 | 32560369 | 0,348 |
| chr6 | 33061928 | 33062178 | 0,348 | | chr19 | 32976483 | 32976733 | 0,312 |
| chr6 | 34249129 | 34249379 | 0,348 | | chr19 | 33350683 | 33350933 | 0,624 |
| chr6 | 35564395 | 35564645 | 0,348 | | chr19 | 33350754 | 33351004 | 0,592 |
| chr6 | 36402380 | 36402630 | 0,292 | | chr19 | 33354962 | 33355212 | 0,592 |
| chr6 | 37220892 | 37221142 | 0,344 | | chr19 | 33355018 | 33355268 | 0,58 |
| chr6 | 38024507 | 38024757 | 0,348 | | chr19 | 33413996 | 33414246 | 0,348 |
| chr6 | 38825212 | 38825462 | 0,348 | | chr19 | 33823022 | 33823272 | 0,348 |
| chr6 | 39628292 | 39628542 | 0,348 | | chr19 | 34284602 | 34284852 | 0,348 |
| chr6 | 40473041 | 40473291 | 0,348 | | chr19 | 34669651 | 34669901 | 0,336 |
| chr6 | 41760316 | 41760566 | 0,348 | | chr19 | 34965785 | 34966035 | 0,336 |
| chr6 | 42561087 | 42561337 | 0,312 | | chr19 | 35249399 | 35249649 | 0,348 |
| chr6 | 43513955 | 43514205 | 0,348 | | chr19 | 35605042 | 35605292 | 0,348 |
| chr6 | 45170150 | 45170400 | 0,272 | | chr19 | 36122870 | 36123120 | 0,348 |
| chr6 | 46210189 | 46210439 | 0,348 | | chr19 | 36322130 | 36322380 | 0,596 |
| chr6 | 47012116 | 47012366 | 0,348 | | chr19 | 36342381 | 36342631 | 0,656 |
| chr6 | 47816800 | 47817050 | 0,336 | | chr19 | 36727027 | 36727277 | 0,348 |
| chr6 | 49160150 | 49160400 | 0,296 | | chr19 | 37128748 | 37128998 | 0,288 |
| chr6 | 50331150 | 50331400 | 0,348 | | chr19 | 37563389 | 37563639 | 0,348 |
| chr6 | 51133231 | 51133481 | 0,348 | | chr19 | 37829474 | 37829724 | 0,34 |
| chr6 | 51524100 | 51524284 | 0,429348 | | chr19 | 38122751 | 38123001 | 0,34 |
| chr6 | 51524387 | 51524577 | 0,452632 | | chr19 | 38312990 | 38313240 | 0,344 |
| chr6 | 51524477 | 51524726 | 0,417671 | | chr19 | 38681154 | 38681404 | 0,348 |
| chr6 | 51612557 | 51612806 | 0,453815 | | chr19 | 38782852 | 38783102 | 0,348 |
| chr6 | 51612759 | 51613008 | 0,413655 | | chr19 | 40029441 | 40029691 | 0,32 |
| chr6 | 51613242 | 51613431 | 0,47619 | | chr19 | 40503907 | 40504157 | 0,348 |
| chr6 | 51617943 | 51618148 | 0,463415 | | chr19 | 40632336 | 40632586 | 0,348 |
| chr6 | 51637377 | 51637626 | 0,35743 | | chr19 | 40871180 | 40871430 | 0,348 |
| chr6 | 51712574 | 51712767 | 0,492228 | | chr19 | 41799074 | 41799324 | 0,296 |
| chr6 | 51747804 | 51748053 | 0,381526 | | chr19 | 41928413 | 41928663 | 0,6 |
| chr6 | 51824498 | 51824736 | 0,390756 | | chr19 | 42144707 | 42144957 | 0,348 |
| chr6 | 51882240 | 51882489 | 0,522088 | | chr19 | 43138347 | 43138597 | 0,348 |
| chr6 | 51889261 | 51889460 | 0,432161 | | chr19 | 44349101 | 44349351 | 0,312 |
| chr6 | 51889530 | 51889709 | 0,430168 | | chr19 | 44682330 | 44682580 | 0,348 |
| chr6 | 51889613 | 51889863 | 0,488 | | chr19 | 45059901 | 45060151 | 0,312 |
| chr6 | 51890671 | 51890920 | 0,566265 | | chr19 | 46056772 | 46057022 | 0,7 |
| chr6 | 51892991 | 51893208 | 0,562212 | | chr19 | 46102932 | 46103182 | 0,348 |
| chr6 | 51907748 | 51907997 | 0,37751 | | chr19 | 46560162 | 46560412 | 0,348 |
| chr6 | 51910894 | 51911114 | 0,413636 | | chr19 | 46954415 | 46954665 | 0,328 |
| chr6 | 51914920 | 51915167 | 0,538462 | | chr19 | 47389774 | 47390024 | 0,348 |
| chr6 | 51923055 | 51923235 | 0,522222 | | chr19 | 47692993 | 47693243 | 0,344 |
| chr6 | 51927226 | 51927475 | 0,473896 | | chr19 | 48475123 | 48475373 | 0,316 |
| chr6 | 51934785 | 51935035 | 0,348 | | chr19 | 48764479 | 48764729 | 0,348 |
| chr6 | 51935792 | 51936041 | 0,409639 | | chr19 | 50146477 | 50146727 | 0,348 |
| chr6 | 51944632 | 51944881 | 0,465863 | | chr19 | 51713109 | 51713359 | 0,34 |
| chr6 | 51947894 | 51948003 | 0,40367 | | chr19 | 52513511 | 52513761 | 0,288 |
| chr6 | 52866150 | 52866400 | 0,316 | | chr19 | 52920188 | 52920438 | 0,348 |
| chr6 | 53861150 | 53861400 | 0,336 | | chr19 | 53343345 | 53343595 | 0,348 |
| chr6 | 54968397 | 54968647 | 0,344 | | chr19 | 53669499 | 53669749 | 0,348 |
| chr6 | 56323494 | 56323744 | 0,348 | | chr19 | 54586305 | 54586555 | 0,3 |
| chr6 | 57132985 | 57133235 | 0,348 | | chr19 | 55068461 | 55068711 | 0,348 |
| chr6 | 58613218 | 58613468 | 0,348 | | chr19 | 55199832 | 55200082 | 0,348 |
| chr6 | 61967821 | 61968071 | 0,348 | | chr19 | 56219609 | 56219859 | 0,28 |
| chr6 | 62792394 | 62792644 | 0,344 | | chr19 | 56884255 | 56884505 | 0,316 |
| chr6 | 63594685 | 63594935 | 0,304 | | chr19 | 57437830 | 57438080 | 0,328 |
| chr6 | 64396571 | 64396821 | 0,332 | | chr19 | 58054419 | 58054669 | 0,316 |
| chr6 | 65487243 | 65487493 | 0,336 | | chr20 | 77170 | 77420 | 0,348 |
| chr6 | 66471331 | 66471581 | 0,348 | | chr20 | 519040 | 519290 | 0,32 |
| chr6 | 67540150 | 67540400 | 0,344 | | chr20 | 1147375 | 1147625 | 0,348 |
| chr6 | 68869150 | 68869400 | 0,32 | | chr20 | 1571446 | 1571696 | 0,348 |
| chr6 | 69669354 | 69669604 | 0,268 | | chr20 | 1947536 | 1947786 | 0,348 |
| chr6 | 70496481 | 70496731 | 0,332 | | chr20 | 2814622 | 2814872 | 0,348 |
| chr6 | 71310224 | 71310474 | 0,348 | | chr20 | 3211047 | 3211297 | 0,56 |
| chr6 | 72112966 | 72113216 | 0,304 | | chr20 | 3274903 | 3275153 | 0,348 |
| chr6 | 73660449 | 73660699 | 0,348 | | chr20 | 3631249 | 3631499 | 0,348 |
| chr6 | 74960150 | 74960400 | 0,324 | | chr20 | 3975347 | 3975597 | 0,348 |
| chr6 | 76010150 | 76010400 | 0,332 | | chr20 | 4449545 | 4449795 | 0,348 |
| chr6 | 76814286 | 76814536 | 0,348 | | chr20 | 5279681 | 5279931 | 0,348 |
| chr6 | 78002389 | 78002639 | 0,348 | | chr20 | 5753870 | 5754120 | 0,348 |
| chr6 | 78924155 | 78924405 | 0,348 | | chr20 | 6094874 | 6095124 | 0,328 |
| chr6 | 79983150 | 79983400 | 0,312 | | chr20 | 6498522 | 6498772 | 0,348 |
| chr6 | 81262398 | 81262648 | 0,328 | | chr20 | 6901553 | 6901803 | 0,348 |
| chr6 | 82072150 | 82072400 | 0,336 | | chr20 | 7729464 | 7729714 | 0,348 |
| chr6 | 83412551 | 83412801 | 0,348 | | chr20 | 8536150 | 8536400 | 0,324 |
| chr6 | 84224150 | 84224400 | 0,336 | | chr20 | 8928565 | 8928815 | 0,348 |
| chr6 | 85024384 | 85024634 | 0,348 | | chr20 | 9340986 | 9341236 | 0,344 |
| chr6 | 85824865 | 85825115 | 0,328 | | chr20 | 9740652 | 9740902 | 0,348 |
| chr6 | 86646607 | 86646857 | 0,348 | | chr20 | 10146600 | 10146850 | 0,348 |
| chr6 | 87730150 | 87730400 | 0,328 | | chr20 | 11388150 | 11388400 | 0,308 |
| chr6 | 89879586 | 89879836 | 0,3 | | chr20 | 11803150 | 11803400 | 0,336 |
| chr6 | 90941239 | 90941489 | 0,348 | | chr20 | 12195420 | 12195670 | 0,348 |
| chr6 | 91745267 | 91745517 | 0,348 | | chr20 | 12767453 | 12767703 | 0,348 |
| chr6 | 92548166 | 92548416 | 0,348 | | chr20 | 13141217 | 13141467 | 0,348 |
| chr6 | 93371163 | 93371413 | 0,276 | | chr20 | 13949320 | 13949570 | 0,348 |
| chr6 | 94317550 | 94317800 | 0,316 | | chr20 | 14353712 | 14353962 | 0,348 |
| chr6 | 95153242 | 95153492 | 0,348 | | chr20 | 14755112 | 14755362 | 0,348 |
| chr6 | 96076584 | 96076834 | 0,304 | | chr20 | 15359150 | 15359400 | 0,276 |
| chr6 | 96895352 | 96895602 | 0,34 | | chr20 | 15913249 | 15913499 | 0,296 |
| chr6 | 97836178 | 97836428 | 0,336 | | chr20 | 17347289 | 17347539 | 0,32 |
| chr6 | 99178177 | 99178427 | 0,348 | | chr20 | 17763020 | 17763270 | 0,348 |
| chr6 | 100312150 | 100312400 | 0,304 | | chr20 | 18167149 | 18167399 | 0,348 |
| chr6 | 101668268 | 101668518 | 0,348 | | chr20 | 18596512 | 18596762 | 0,348 |
| chr6 | 102469310 | 102469560 | 0,252 | | chr20 | 19409652 | 19409902 | 0,348 |
| chr6 | 103270888 | 103271138 | 0,348 | | chr20 | 19818614 | 19818864 | 0,308 |
| chr6 | 104071706 | 104071956 | 0,348 | | chr20 | 20210274 | 20210524 | 0,348 |
| chr6 | 104881263 | 104881513 | 0,348 | | chr20 | 20532551 | 20532801 | 0,324 |
| chr6 | 105881364 | 105881614 | 0,328 | | chr20 | 21026150 | 21026400 | 0,324 |
| chr6 | 106961211 | 106961461 | 0,348 | | chr20 | 21832473 | 21832723 | 0,348 |
| chr6 | 107880456 | 107880706 | 0,348 | | chr20 | 22237542 | 22237792 | 0,312 |
| chr6 | 108681536 | 108681786 | 0,348 | | chr20 | 22640479 | 22640729 | 0,324 |
| chr6 | 109481761 | 109482011 | 0,344 | | chr20 | 23060135 | 23060385 | 0,348 |
| chr6 | 110618500 | 110618750 | 0,348 | | chr20 | 23474780 | 23475030 | 0,34 |
| chr6 | 111427143 | 111427393 | 0,348 | | chr20 | 24275650 | 24275900 | 0,348 |
| chr6 | 112231415 | 112231665 | 0,348 | | chr20 | 24685964 | 24686214 | 0,308 |
| chr6 | 113042291 | 113042541 | 0,336 | | chr20 | 25109531 | 25109781 | 0,32 |
| chr6 | 113895590 | 113895840 | 0,348 | | chr20 | 25714681 | 25714931 | 0,296 |
| chr6 | 115006168 | 115006418 | 0,348 | | chr20 | 29847069 | 29847319 | 0,312 |
| chr6 | 115806345 | 115806595 | 0,348 | | chr20 | 30657384 | 30657634 | 0,336 |
| chr6 | 116616751 | 116617001 | 0,312 | | chr20 | 31291810 | 31292060 | 0,348 |
| chr6 | 117765567 | 117765817 | 0,348 | | chr20 | 31692805 | 31693055 | 0,348 |
| chr6 | 119110166 | 119110416 | 0,348 | | chr20 | 32588884 | 32589134 | 0,296 |
| chr6 | 119969535 | 119969785 | 0,344 | | chr20 | 32990720 | 32990970 | 0,336 |
| chr6 | 121313474 | 121313724 | 0,324 | | chr20 | 33396858 | 33397108 | 0,324 |
| chr6 | 122234150 | 122234400 | 0,316 | | chr20 | 33930796 | 33931046 | 0,332 |
| chr6 | 123082150 | 123082400 | 0,348 | | chr20 | 34021766 | 34022016 | 0,596 |
| chr6 | 123883938 | 123884188 | 0,336 | | chr20 | 34021782 | 34022032 | 0,596 |
| chr6 | 124688304 | 124688554 | 0,324 | | chr20 | 34021955 | 34022205 | 0,584 |
| chr6 | 125488626 | 125488876 | 0,34 | | chr20 | 34227958 | 34228208 | 0,348 |
| chr6 | 126295238 | 126295488 | 0,312 | | chr20 | 35300398 | 35300648 | 0,348 |
| chr6 | 127319150 | 127319400 | 0,348 | | chr20 | 35695232 | 35695482 | 0,348 |
| chr6 | 128124694 | 128124944 | 0,344 | | chr20 | 36150135 | 36150385 | 0,348 |
| chr6 | 128939956 | 128940206 | 0,32 | | chr20 | 36623218 | 36623468 | 0,348 |
| chr6 | 129747756 | 129748006 | 0,284 | | chr20 | 37117167 | 37117417 | 0,348 |
| chr6 | 130556422 | 130556672 | 0,288 | | chr20 | 37921224 | 37921474 | 0,348 |
| chr6 | 131359186 | 131359436 | 0,252 | | chr20 | 38322240 | 38322490 | 0,348 |
| chr6 | 133276306 | 133276556 | 0,348 | | chr20 | 38729196 | 38729446 | 0,348 |
| chr6 | 134312325 | 134312575 | 0,348 | | chr20 | 39510398 | 39510648 | 0,348 |
| chr6 | 135908218 | 135908468 | 0,348 | | chr20 | 40534390 | 40534640 | 0,348 |
| chr6 | 136827150 | 136827400 | 0,276 | | chr20 | 41411185 | 41411435 | 0,348 |
| chr6 | 137166633 | 137166883 | 0,356 | | chr20 | 41830566 | 41830816 | 0,348 |
| chr6 | 137219252 | 137219502 | 0,396 | | chr20 | 42275434 | 42275684 | 0,348 |
| chr6 | 137219252 | 137219502 | 0,396 | | chr20 | 42643867 | 42644117 | 0,348 |
| chr6 | 137899299 | 137899549 | 0,304 | | chr20 | 43115223 | 43115473 | 0,348 |
| chr6 | 138928325 | 138928575 | 0,348 | | chr20 | 43254061 | 43254311 | 0,676 |
| chr6 | 139961518 | 139961768 | 0,288 | | chr20 | 43254984 | 43255234 | 0,58 |
| chr6 | 141196497 | 141196747 | 0,312 | | chr20 | 44152521 | 44152771 | 0,348 |
| chr6 | 142305254 | 142305504 | 0,348 | | chr20 | 44496561 | 44496811 | 0,348 |
| chr6 | 143107636 | 143107886 | 0,336 | | chr20 | 44996290 | 44996540 | 0,348 |
| chr6 | 144133389 | 144133639 | 0,348 | | chr20 | 45529150 | 45529400 | 0,344 |
| chr6 | 145572150 | 145572400 | 0,336 | | chr20 | 45871441 | 45871691 | 0,348 |
| chr6 | 146529266 | 146529516 | 0,348 | | chr20 | 46844718 | 46844968 | 0,348 |
| chr6 | 147476386 | 147476636 | 0,348 | | chr20 | 47174893 | 47175143 | 0,348 |
| chr6 | 148521155 | 148521405 | 0,316 | | chr20 | 47650289 | 47650539 | 0,3 |
| chr6 | 149329190 | 149329440 | 0,348 | | chr20 | 47904517 | 47904767 | 0,348 |
| chr6 | 150131788 | 150132038 | 0,332 | | chr20 | 48480440 | 48480690 | 0,348 |
| chr6 | 151419594 | 151419844 | 0,348 | | chr20 | 49334830 | 49335080 | 0,348 |
| chr6 | 153113150 | 153113400 | 0,304 | | chr20 | 50145781 | 50146031 | 0,348 |
| chr6 | 153913332 | 153913582 | 0,348 | | chr20 | 50550520 | 50550770 | 0,34 |
| chr6 | 154720810 | 154721060 | 0,34 | | chr20 | 50950756 | 50951006 | 0,348 |
| chr6 | 155536244 | 155536494 | 0,348 | | chr20 | 51753303 | 51753553 | 0,336 |
| chr6 | 156340604 | 156340854 | 0,332 | | chr20 | 52324562 | 52324812 | 0,348 |
| chr6 | 157146448 | 157146698 | 0,316 | | chr20 | 53054192 | 53054442 | 0,348 |
| chr6 | 157956774 | 157957024 | 0,348 | | chr20 | 53454757 | 53455007 | 0,336 |
| chr6 | 159061159 | 159061409 | 0,348 | | chr20 | 53859987 | 53860237 | 0,348 |
| chr6 | 159870327 | 159870577 | 0,264 | | chr20 | 55091227 | 55091477 | 0,348 |
| chr6 | 160671116 | 160671366 | 0,328 | | chr20 | 55951837 | 55952087 | 0,324 |
| chr6 | 161473227 | 161473477 | 0,348 | | chr20 | 56403014 | 56403264 | 0,348 |
| chr6 | 162969538 | 162969788 | 0,336 | | chr20 | 57228245 | 57228495 | 0,348 |
| chr6 | 163775420 | 163775670 | 0,348 | | chr20 | 57694540 | 57694790 | 0,348 |
| chr6 | 164598389 | 164598639 | 0,272 | | chr20 | 58161403 | 58161653 | 0,348 |
| chr6 | 165405401 | 165405651 | 0,348 | | chr20 | 58567922 | 58568172 | 0,348 |
| chr6 | 166950248 | 166950498 | 0,348 | | chr20 | 58978600 | 58978850 | 0,348 |
| chr6 | 167769909 | 167770159 | 0,348 | | chr20 | 59778987 | 59779237 | 0,348 |
| chr6 | 168608835 | 168609085 | 0,348 | | chr20 | 60196684 | 60196934 | 0,348 |
| chr6 | 170242603 | 170242853 | 0,348 | | chr20 | 62318999 | 62319249 | 0,672 |
| chr7 | 55429 | 55679 | 0,348 | | chr20 | 62321379 | 62321629 | 0,624 |
| chr7 | 877046 | 877296 | 0,348 | | chr20 | 62326842 | 62327092 | 0,668 |
| chr7 | 2149206 | 2149456 | 0,348 | | chr20 | 62375183 | 62375433 | 0,348 |
| chr7 | 3107789 | 3108039 | 0,348 | | chr21 | 15481051 | 15481301 | 0,252 |
| chr7 | 3909387 | 3909637 | 0,348 | | chr21 | 15950150 | 15950400 | 0,344 |
| chr7 | 4730421 | 4730671 | 0,336 | | chr21 | 16081282 | 16081531 | 0,353414 |
| chr7 | 5703807 | 5704057 | 0,332 | | chr21 | 16206810 | 16207060 | 0,28 |
| chr7 | 6513490 | 6513740 | 0,276 | | chr21 | 16333315 | 16333564 | 0,381526 |
| chr7 | 7313867 | 7314117 | 0,348 | | chr21 | 16793392 | 16793642 | 0,348 |
| chr7 | 8124150 | 8124400 | 0,336 | | chr21 | 17135293 | 17135543 | 0,348 |
| chr7 | 8927574 | 8927824 | 0,348 | | chr21 | 17301983 | 17302232 | 0,381526 |
| chr7 | 9852228 | 9852478 | 0,348 | | chr21 | 17489798 | 17490048 | 0,296 |
| chr7 | 10714290 | 10714540 | 0,344 | | chr21 | 17695933 | 17696182 | 0,325301 |
| chr7 | 11521626 | 11521876 | 0,348 | | chr21 | 17823960 | 17824210 | 0,34 |
| chr7 | 12357745 | 12357995 | 0,332 | | chr21 | 17827150 | 17827400 | 0,344 |
| chr7 | 13758296 | 13758546 | 0,3 | | chr21 | 18229813 | 18230063 | 0,348 |
| chr7 | 14961489 | 14961739 | 0,336 | | chr21 | 18629909 | 18630159 | 0,348 |
| chr7 | 15766151 | 15766401 | 0,348 | | chr21 | 19024530 | 19024780 | 0,312 |
| chr7 | 16571000 | 16571250 | 0,348 | | chr21 | 19434035 | 19434285 | 0,348 |
| chr7 | 18364511 | 18364761 | 0,348 | | chr21 | 19707300 | 19707550 | 0,348 |
| chr7 | 19288150 | 19288400 | 0,296 | | chr21 | 20093300 | 20093550 | 0,276 |
| chr7 | 20093589 | 20093839 | 0,304 | | chr21 | 20474486 | 20474736 | 0,292 |
| chr7 | 20903165 | 20903415 | 0,348 | | chr21 | 20487286 | 20487536 | 0,264 |
| chr7 | 21813150 | 21813400 | 0,308 | | chr21 | 20890626 | 20890876 | 0,348 |
| chr7 | 22626926 | 22627176 | 0,34 | | chr21 | 21288139 | 21288389 | 0,348 |
| chr7 | 24588150 | 24588400 | 0,32 | | chr21 | 21685666 | 21685916 | 0,28 |
| chr7 | 25393154 | 25393404 | 0,348 | | chr21 | 22088785 | 22089035 | 0,348 |
| chr7 | 26207116 | 26207366 | 0,348 | | chr21 | 22490319 | 22490569 | 0,328 |
| chr7 | 27014593 | 27014843 | 0,296 | | chr21 | 22888860 | 22889110 | 0,328 |
| chr7 | 28180480 | 28180730 | 0,336 | | chr21 | 23136575 | 23136825 | 0,332 |
| chr7 | 29609491 | 29609741 | 0,348 | | chr21 | 23398370 | 23398620 | 0,268 |
| chr7 | 30505246 | 30505496 | 0,348 | | chr21 | 23827150 | 23827400 | 0,336 |
| chr7 | 31323318 | 31323568 | 0,288 | | chr21 | 24353533 | 24353783 | 0,3 |
| chr7 | 32129428 | 32129678 | 0,348 | | chr21 | 24895230 | 24895480 | 0,324 |
| chr7 | 32929838 | 32930088 | 0,348 | | chr21 | 25370366 | 25370616 | 0,348 |
| chr7 | 34109582 | 34109832 | 0,348 | | chr21 | 25689068 | 25689318 | 0,348 |
| chr7 | 35308294 | 35308544 | 0,348 | | chr21 | 26010566 | 26010816 | 0,308 |
| chr7 | 36109511 | 36109761 | 0,348 | | chr21 | 26339803 | 26340053 | 0,328 |
| chr7 | 37108150 | 37108400 | 0,348 | | chr21 | 26507298 | 26507547 | 0,353414 |
| chr7 | 37908361 | 37908611 | 0,348 | | chr21 | 26676239 | 26676489 | 0,348 |
| chr7 | 38864325 | 38864575 | 0,308 | | chr21 | 26969283 | 26969533 | 0,348 |
| chr7 | 39665162 | 39665412 | 0,348 | | chr21 | 27268386 | 27268636 | 0,348 |
| chr7 | 40477320 | 40477570 | 0,348 | | chr21 | 27520192 | 27520441 | 0,373494 |
| chr7 | 41459497 | 41459747 | 0,348 | | chr21 | 27761493 | 27761743 | 0,34 |
| chr7 | 43302476 | 43302726 | 0,348 | | chr21 | 28111207 | 28111457 | 0,32 |
| chr7 | 44330188 | 44330438 | 0,348 | | chr21 | 28291449 | 28291698 | 0,373494 |
| chr7 | 45260099 | 45260349 | 0,348 | | chr21 | 28420042 | 28420292 | 0,348 |
| chr7 | 46071390 | 46071640 | 0,288 | | chr21 | 28574819 | 28575068 | 0,369478 |
| chr7 | 47421295 | 47421545 | 0,348 | | chr21 | 28732222 | 28732472 | 0,316 |
| chr7 | 48228113 | 48228363 | 0,348 | | chr21 | 28919446 | 28919695 | 0,385542 |
| chr7 | 49030589 | 49030839 | 0,348 | | chr21 | 29085150 | 29085400 | 0,348 |
| chr7 | 50227150 | 50227400 | 0,336 | | chr21 | 29481927 | 29482177 | 0,256 |
| chr7 | 52176118 | 52176368 | 0,296 | | chr21 | 29886036 | 29886286 | 0,344 |
| chr7 | 53041150 | 53041400 | 0,32 | | chr21 | 30154482 | 30154732 | 0,348 |
| chr7 | 54163159 | 54163409 | 0,348 | | chr21 | 30301073 | 30301322 | 0,341365 |
| chr7 | 54971120 | 54971370 | 0,324 | | chr21 | 30415695 | 30415945 | 0,324 |
| chr7 | 55800266 | 55800516 | 0,348 | | chr21 | 30550132 | 30550381 | 0,349398 |
| chr7 | 62624136 | 62624386 | 0,34 | | chr21 | 30686458 | 30686708 | 0,34 |
| chr7 | 63426305 | 63426555 | 0,308 | | chr21 | 30810199 | 30810448 | 0,325301 |
| chr7 | 64230137 | 64230387 | 0,292 | | chr21 | 30956196 | 30956446 | 0,348 |
| chr7 | 65372594 | 65372844 | 0,344 | | chr21 | 31120815 | 31121064 | 0,341365 |
| chr7 | 67064459 | 67064709 | 0,348 | | chr21 | 31295615 | 31295865 | 0,312 |
| chr7 | 67898887 | 67899137 | 0,348 | | chr21 | 31494755 | 31495004 | 0,301205 |
| chr7 | 69412264 | 69412514 | 0,348 | | chr21 | 31631465 | 31631715 | 0,344 |
| chr7 | 70213263 | 70213513 | 0,328 | | chr21 | 31768996 | 31769245 | 0,309237 |
| chr7 | 71054944 | 71055194 | 0,28 | | chr21 | 31963626 | 31963876 | 0,284 |
| chr7 | 71874807 | 71875057 | 0,348 | | chr21 | 32291970 | 32292220 | 0,348 |
| chr7 | 72864826 | 72865076 | 0,348 | | chr21 | 32297150 | 32297400 | 0,324 |
| chr7 | 75956713 | 75956963 | 0,348 | | chr21 | 32704164 | 32704414 | 0,348 |
| chr7 | 76814033 | 76814283 | 0,32 | | chr21 | 33101826 | 33102076 | 0,348 |
| chr7 | 77713150 | 77713400 | 0,348 | | chr21 | 33555477 | 33555727 | 0,348 |
| chr7 | 78530571 | 78530821 | 0,276 | | chr21 | 33853735 | 33853983 | 0,310484 |
| chr7 | 79336977 | 79337227 | 0,348 | | chr21 | 33975120 | 33975370 | 0,348 |
| chr7 | 80660150 | 80660400 | 0,332 | | chr21 | 34142020 | 34142268 | 0,310484 |
| chr7 | 81471944 | 81472194 | 0,34 | | chr21 | 34381366 | 34381616 | 0,348 |
| chr7 | 82293809 | 82294059 | 0,348 | | chr21 | 34787647 | 34787897 | 0,348 |
| chr7 | 83095588 | 83095838 | 0,348 | | chr21 | 35034581 | 35034830 | 0,341365 |
| chr7 | 83895809 | 83896059 | 0,344 | | chr21 | 35210083 | 35210333 | 0,348 |
| chr7 | 84696541 | 84696791 | 0,336 | | chr21 | 35450331 | 35450580 | 0,401606 |
| chr7 | 85502525 | 85502775 | 0,344 | | chr21 | 35628286 | 35628536 | 0,348 |
| chr7 | 86361434 | 86361684 | 0,324 | | chr21 | 36049058 | 36049308 | 0,336 |
| chr7 | 87162681 | 87162931 | 0,348 | | chr21 | 36447052 | 36447302 | 0,292 |
| chr7 | 87968369 | 87968619 | 0,316 | | chr21 | 36789088 | 36789337 | 0,409639 |
| chr7 | 89087150 | 89087400 | 0,328 | | chr21 | 36929380 | 36929630 | 0,348 |
| chr7 | 90162150 | 90162400 | 0,312 | | chr21 | 37306439 | 37306689 | 0,348 |
| chr7 | 91015164 | 91015414 | 0,348 | | chr21 | 37620268 | 37620518 | 0,348 |
| chr7 | 91942336 | 91942586 | 0,348 | | chr21 | 37998204 | 37998454 | 0,276 |
| chr7 | 92132375 | 92132625 | 0,336 | | chr21 | 38267857 | 38268107 | 0,348 |
| chr7 | 92745515 | 92745765 | 0,348 | | chr21 | 38308910 | 38309160 | 0,532 |
| chr7 | 94032150 | 94032400 | 0,312 | | chr21 | 38537188 | 38537438 | 0,348 |
| chr7 | 94879455 | 94879705 | 0,348 | | chr21 | 39149737 | 39149987 | 0,348 |
| chr7 | 95728150 | 95728400 | 0,312 | | chr21 | 39442392 | 39442641 | 0,325301 |
| chr7 | 96535542 | 96535792 | 0,348 | | chr21 | 39679043 | 39679293 | 0,348 |
| chr7 | 97356883 | 97357133 | 0,328 | | chr21 | 39977160 | 39977409 | 0,413655 |
| chr7 | 98237766 | 98238016 | 0,324 | | chr21 | 40125556 | 40125806 | 0,348 |
| chr7 | 99150089 | 99150339 | 0,348 | | chr21 | 40427338 | 40427587 | 0,389558 |
| chr7 | 100141414 | 100141664 | 0,348 | | chr21 | 40759240 | 40759493 | 0,363636 |
| chr7 | 101399346 | 101399596 | 0,348 | | chr21 | 40972106 | 40972356 | 0,348 |
| chr7 | 102451481 | 102451731 | 0,324 | | chr21 | 41160090 | 41160343 | 0,359684 |
| chr7 | 103335561 | 103335811 | 0,296 | | chr21 | 41344027 | 41344277 | 0,348 |
| chr7 | 104416150 | 104416400 | 0,32 | | chr21 | 41592339 | 41592583 | 0,311475 |
| chr7 | 105400093 | 105400343 | 0,348 | | chr21 | 41767335 | 41767585 | 0,296 |
| chr7 | 106232404 | 106232654 | 0,252 | | chr21 | 42029142 | 42029391 | 0,393574 |
| chr7 | 107034859 | 107035109 | 0,348 | | chr21 | 42263335 | 42263585 | 0,292 |
| chr7 | 107323858 | 107324108 | 0,336 | | chr21 | 42729150 | 42729400 | 0,34 |
| chr7 | 107330445 | 107330695 | 0,468 | | chr21 | 43306231 | 43306481 | 0,348 |
| chr7 | 107330540 | 107330790 | 0,492 | | chr21 | 43667110 | 43667358 | 0,310484 |
| chr7 | 107420025 | 107420275 | 0,356 | | chr21 | 43883150 | 43883400 | 0,316 |
| chr7 | 107542660 | 107542910 | 0,356 | | chr21 | 44196371 | 44196621 | 0,348 |
| chr7 | 107557622 | 107557872 | 0,408 | | chr21 | 44424545 | 44424795 | 0,348 |
| chr7 | 107557669 | 107557919 | 0,432 | | chr21 | 44482314 | 44482564 | 0,652 |
| chr7 | 107557724 | 107557974 | 0,46 | | chr21 | 44482356 | 44482606 | 0,648 |
| chr7 | 107847587 | 107847837 | 0,348 | | chr21 | 44482953 | 44483203 | 0,636 |
| chr7 | 108648804 | 108649054 | 0,348 | | chr21 | 44483059 | 44483309 | 0,584 |
| chr7 | 109458628 | 109458878 | 0,328 | | chr21 | 44483916 | 44484166 | 0,604 |
| chr7 | 110524210 | 110524460 | 0,312 | | chr21 | 44485497 | 44485747 | 0,648 |
| chr7 | 111335538 | 111335788 | 0,332 | | chr21 | 44625010 | 44625260 | 0,348 |
| chr7 | 112398549 | 112398799 | 0,348 | | chr21 | 44948824 | 44949074 | 0,336 |
| chr7 | 113200896 | 113201146 | 0,324 | | chr21 | 45178504 | 45178754 | 0,348 |
| chr7 | 114001693 | 114001943 | 0,328 | | chr21 | 45472144 | 45472394 | 0,348 |
| chr7 | 114966262 | 114966512 | 0,348 | | chr21 | 45706431 | 45706681 | 0,636 |
| chr7 | 115771663 | 115771913 | 0,316 | | chr21 | 46000694 | 46000944 | 0,336 |
| chr7 | 116585570 | 116585820 | 0,296 | | chr21 | 46270689 | 46270939 | 0,348 |
| chr7 | 117149047 | 117149297 | 0,324 | | chr21 | 46533710 | 46533960 | 0,348 |
| chr7 | 117170889 | 117171139 | 0,412 | | chr21 | 46760919 | 46761169 | 0,348 |
| chr7 | 117171034 | 117171284 | 0,38 | | chr21 | 47065442 | 47065692 | 0,348 |
| chr7 | 117199521 | 117199770 | 0,369478 | | chr22 | 17280474 | 17280724 | 0,3 |
| chr7 | 117199607 | 117199796 | 0,354497 | | chr22 | 17413925 | 17414175 | 0,348 |
| chr7 | 117227720 | 117227969 | 0,349398 | | chr22 | 17781671 | 17781921 | 0,348 |
| chr7 | 117227791 | 117227987 | 0,357143 | | chr22 | 17935494 | 17935744 | 0,284 |
| chr7 | 117232148 | 117232398 | 0,372 | | chr22 | 18094028 | 18094278 | 0,344 |
| chr7 | 117246704 | 117246954 | 0,292 | | chr22 | 18344493 | 18344743 | 0,348 |
| chr7 | 117267461 | 117267711 | 0,344 | | chr22 | 18595330 | 18595580 | 0,348 |
| chr7 | 117267636 | 117267886 | 0,428 | | chr22 | 19219301 | 19219551 | 0,324 |
| chr7 | 117279890 | 117280140 | 0,336 | | chr22 | 19565525 | 19565775 | 0,348 |
| chr7 | 117282492 | 117282741 | 0,393574 | | chr22 | 19766919 | 19767169 | 0,348 |
| chr7 | 117622151 | 117622401 | 0,348 | | chr22 | 19947592 | 19947842 | 0,348 |
| chr7 | 118923150 | 118923400 | 0,34 | | chr22 | 20817080 | 20817330 | 0,34 |
| chr7 | 119723802 | 119724052 | 0,348 | | chr22 | 21139822 | 21140072 | 0,348 |
| chr7 | 120536847 | 120537097 | 0,296 | | chr22 | 21292445 | 21292695 | 0,316 |
| chr7 | 121388365 | 121388615 | 0,34 | | chr22 | 22024679 | 22024929 | 0,34 |
| chr7 | 122225191 | 122225441 | 0,288 | | chr22 | 22438898 | 22439148 | 0,348 |
| chr7 | 123119241 | 123119491 | 0,332 | | chr22 | 22839744 | 22839994 | 0,348 |
| chr7 | 124015307 | 124015557 | 0,348 | | chr22 | 23278902 | 23279152 | 0,348 |
| chr7 | 124862547 | 124862797 | 0,308 | | chr22 | 24248051 | 24248301 | 0,348 |
| chr7 | 125665148 | 125665398 | 0,348 | | chr22 | 24718846 | 24719096 | 0,348 |
| chr7 | 126472650 | 126472900 | 0,316 | | chr22 | 25113444 | 25113694 | 0,252 |
| chr7 | 127285399 | 127285649 | 0,348 | | chr22 | 25588970 | 25589220 | 0,348 |
| chr7 | 128300359 | 128300609 | 0,34 | | chr22 | 25966386 | 25966636 | 0,348 |
| chr7 | 129107063 | 129107313 | 0,348 | | chr22 | 26120347 | 26120597 | 0,348 |
| chr7 | 129978355 | 129978605 | 0,348 | | chr22 | 26362974 | 26363224 | 0,348 |
| chr7 | 130780700 | 130780950 | 0,308 | | chr22 | 26769343 | 26769593 | 0,304 |
| chr7 | 131649229 | 131649479 | 0,324 | | chr22 | 26922260 | 26922510 | 0,348 |
| chr7 | 132518150 | 132518400 | 0,34 | | chr22 | 27163955 | 27164205 | 0,348 |
| chr7 | 133418423 | 133418673 | 0,336 | | chr22 | 27601162 | 27601412 | 0,284 |
| chr7 | 134286655 | 134286905 | 0,332 | | chr22 | 27994362 | 27994612 | 0,312 |
| chr7 | 135090145 | 135090395 | 0,348 | | chr22 | 28270441 | 28270691 | 0,348 |
| chr7 | 135899265 | 135899515 | 0,332 | | chr22 | 28446625 | 28446875 | 0,348 |
| chr7 | 136699464 | 136699714 | 0,328 | | chr22 | 28640282 | 28640532 | 0,348 |
| chr7 | 137847153 | 137847403 | 0,348 | | chr22 | 28820811 | 28821061 | 0,348 |
| chr7 | 139280295 | 139280545 | 0,348 | | chr22 | 29045556 | 29045806 | 0,284 |
| chr7 | 140159009 | 140159259 | 0,348 | | chr22 | 29395916 | 29396166 | 0,348 |
| chr7 | 140970360 | 140970610 | 0,348 | | chr22 | 29412378 | 29412628 | 0,348 |
| chr7 | 141800731 | 141800981 | 0,348 | | chr22 | 29826594 | 29826844 | 0,308 |
| chr7 | 142632884 | 142633134 | 0,348 | | chr22 | 30000222 | 30000472 | 0,348 |
| chr7 | 143580134 | 143580384 | 0,348 | | chr22 | 30257427 | 30257677 | 0,348 |
| chr7 | 144381506 | 144381756 | 0,348 | | chr22 | 30425284 | 30425534 | 0,348 |
| chr7 | 145240150 | 145240400 | 0,324 | | chr22 | 30587479 | 30587729 | 0,348 |
| chr7 | 146040527 | 146040777 | 0,272 | | chr22 | 31117594 | 31117844 | 0,336 |
| chr7 | 146864156 | 146864406 | 0,348 | | chr22 | 31392528 | 31392778 | 0,348 |
| chr7 | 147665180 | 147665430 | 0,348 | | chr22 | 31691832 | 31692082 | 0,348 |
| chr7 | 148467130 | 148467380 | 0,348 | | chr22 | 32113185 | 32113435 | 0,348 |
| chr7 | 150033270 | 150033520 | 0,348 | | chr22 | 32353173 | 32353423 | 0,348 |
| chr7 | 150852375 | 150852625 | 0,348 | | chr22 | 32505557 | 32505807 | 0,348 |
| chr7 | 151664082 | 151664332 | 0,296 | | chr22 | 32969346 | 32969596 | 0,348 |
| chr7 | 152570612 | 152570862 | 0,348 | | chr22 | 33257698 | 33257948 | 0,348 |
| chr7 | 153378130 | 153378380 | 0,332 | | chr22 | 33562806 | 33563056 | 0,348 |
| chr7 | 154190449 | 154190699 | 0,348 | | chr22 | 33590196 | 33590446 | 0,348 |
| chr7 | 155072739 | 155072989 | 0,348 | | chr22 | 34018923 | 34019173 | 0,348 |
| chr7 | 155881209 | 155881459 | 0,328 | | chr22 | 34236108 | 34236358 | 0,328 |
| chr7 | 157364150 | 157364400 | 0,324 | | chr22 | 34418259 | 34418509 | 0,348 |
| chr7 | 158567174 | 158567424 | 0,348 | | chr22 | 34803406 | 34803656 | 0,328 |
| chr8 | 192250 | 192500 | 0,348 | | chr22 | 35219092 | 35219342 | 0,348 |
| chr8 | 1003693 | 1003943 | 0,348 | | chr22 | 35677229 | 35677479 | 0,348 |
| chr8 | 1968353 | 1968603 | 0,348 | | chr22 | 36136285 | 36136535 | 0,348 |
| chr8 | 2785184 | 2785434 | 0,348 | | chr22 | 36340176 | 36340426 | 0,348 |
| chr8 | 3594564 | 3594814 | 0,296 | | chr22 | 36559863 | 36560113 | 0,348 |
| chr8 | 4462150 | 4462400 | 0,32 | | chr22 | 36958950 | 36959200 | 0,288 |
| chr8 | 5272274 | 5272524 | 0,348 | | chr22 | 37366224 | 37366474 | 0,292 |
| chr8 | 6079383 | 6079633 | 0,348 | | chr22 | 38615284 | 38615534 | 0,348 |
| chr8 | 6896439 | 6896689 | 0,348 | | chr22 | 38882398 | 38882648 | 0,348 |
| chr8 | 8120898 | 8121148 | 0,284 | | chr22 | 39066577 | 39066827 | 0,268 |
| chr8 | 9159532 | 9159782 | 0,344 | | chr22 | 39483473 | 39483723 | 0,348 |
| chr8 | 9964948 | 9965198 | 0,348 | | chr22 | 39913116 | 39913366 | 0,348 |
| chr8 | 10849178 | 10849428 | 0,348 | | chr22 | 40285123 | 40285373 | 0,348 |
| chr8 | 11664231 | 11664481 | 0,348 | | chr22 | 40441816 | 40442066 | 0,348 |
| chr8 | 12579602 | 12579852 | 0,308 | | chr22 | 40724377 | 40724627 | 0,348 |
| chr8 | 13417150 | 13417400 | 0,328 | | chr22 | 40928202 | 40928452 | 0,348 |
| chr8 | 14462150 | 14462400 | 0,336 | | chr22 | 41164700 | 41164950 | 0,3 |
| chr8 | 15294599 | 15294849 | 0,316 | | chr22 | 41570976 | 41571226 | 0,34 |
| chr8 | 16098431 | 16098681 | 0,348 | | chr22 | 42031998 | 42032248 | 0,336 |
| chr8 | 16901802 | 16902052 | 0,348 | | chr22 | 42253945 | 42254195 | 0,348 |
| chr8 | 17701806 | 17702056 | 0,348 | | chr22 | 43414341 | 43414591 | 0,328 |
| chr8 | 18503290 | 18503540 | 0,328 | | chr22 | 43819506 | 43819756 | 0,348 |
| chr8 | 19316094 | 19316344 | 0,348 | | chr22 | 44083042 | 44083292 | 0,348 |
| chr8 | 20358596 | 20358846 | 0,336 | | chr22 | 44213665 | 44213915 | 0,348 |
| chr8 | 21164416 | 21164666 | 0,316 | | chr22 | 44359248 | 44359498 | 0,348 |
| chr8 | 22141422 | 22141672 | 0,348 | | chr22 | 44852852 | 44853102 | 0,348 |
| chr8 | 22961278 | 22961528 | 0,348 | | chr22 | 45225408 | 45225658 | 0,348 |
| chr8 | 23765128 | 23765378 | 0,348 | | chr22 | 45545508 | 45545758 | 0,312 |
| chr8 | 24589150 | 24589400 | 0,344 | | chr22 | 45714432 | 45714682 | 0,348 |
| chr8 | 25395976 | 25396226 | 0,348 | | chr22 | 46038547 | 46038797 | 0,348 |
| chr8 | 26196689 | 26196939 | 0,344 | | chr22 | 46231545 | 46231795 | 0,348 |
| chr8 | 27061150 | 27061400 | 0,348 | | chr22 | 46456342 | 46456592 | 0,344 |
| chr8 | 27954533 | 27954783 | 0,324 | | chr22 | 46757150 | 46757400 | 0,344 |
| chr8 | 28755361 | 28755611 | 0,332 | | chr22 | 46988693 | 46988943 | 0,348 |
| chr8 | 29557790 | 29558040 | 0,348 | | chr22 | 47288440 | 47288690 | 0,348 |
| chr8 | 30361271 | 30361521 | 0,348 | | chr22 | 47643065 | 47643315 | 0,348 |
| chr8 | 31611150 | 31611400 | 0,304 | | chr22 | 48053341 | 48053591 | 0,348 |
| chr8 | 32611550 | 32611800 | 0,272 | | chr22 | 48217987 | 48218237 | 0,348 |
| chr8 | 33482560 | 33482810 | 0,348 | | chr22 | 48451914 | 48452164 | 0,348 |
| chr8 | 34286695 | 34286945 | 0,292 | | chr22 | 48864259 | 48864509 | 0,316 |
| chr8 | 35095509 | 35095759 | 0,348 | | chr22 | 49252506 | 49252756 | 0,348 |
| chr8 | 35898952 | 35899202 | 0,336 | | chr22 | 49500880 | 49501130 | 0,348 |
| chr8 | 36702296 | 36702546 | 0,348 | | chr22 | 49668127 | 49668377 | 0,348 |
| chr8 | 37538504 | 37538754 | 0,348 | | chr22 | 50072698 | 50072948 | 0,348 |
| chr8 | 39232335 | 39232585 | 0,34 | | chr22 | 50281645 | 50281895 | 0,348 |
| chr8 | 40057492 | 40057742 | 0,348 | | chr22 | 50523016 | 50523266 | 0,62 |
| chr8 | 40861155 | 40861405 | 0,348 | | chr22 | 50523047 | 50523297 | 0,648 |
| chr8 | 41687779 | 41688029 | 0,348 | | chr22 | 50558794 | 50559044 | 0,348 |
| chr8 | 42488236 | 42488486 | 0,348 | | chr22 | 50979168 | 50979418 | 0,332 |
| chr8 | 43296893 | 43297143 | 0,344 | | chr22 | 51063685 | 51063935 | 0,632 |
| chr8 | 47456494 | 47456744 | 0,276 | | chr22 | 51063716 | 51063966 | 0,652 |
| chr8 | 49739152 | 49739402 | 0,348 | | chr22 | 51063856 | 51064106 | 0,636 |
| chr8 | 50539626 | 50539876 | 0,348 | | chr22 | 51064009 | 51064259 | 0,64 |
| chr8 | 51348600 | 51348850 | 0,348 | | chr22 | 51064554 | 51064804 | 0,636 |
| chr8 | 52687557 | 52687807 | 0,284 | | chr22 | 51064921 | 51065171 | 0,62 |
| chr8 | 53732150 | 53732400 | 0,328 | | chr22 | 51065196 | 51065446 | 0,644 |
| chr8 | 54556748 | 54556998 | 0,348 | | chr22 | 51065453 | 51065703 | 0,68 |
| chr8 | 55362843 | 55363093 | 0,348 | | chr22 | 51065622 | 51065872 | 0,692 |
| chr8 | 56218408 | 56218658 | 0,3 | | chr22 | 51065688 | 51065938 | 0,656 |
| chr8 | 57024628 | 57024878 | 0,348 | | chrX | 2653932 | 2654182 | 0,548 |
| chr8 | 57939209 | 57939459 | 0,348 | | chrX | 2770647 | 2770897 | 0,348 |
| chr8 | 59069598 | 59069848 | 0,348 | | chrX | 3968150 | 3968400 | 0,312 |
| chr8 | 62008281 | 62008531 | 0,348 | | chrX | 4779861 | 4780111 | 0,348 |
| chr8 | 63859170 | 63859420 | 0,348 | | chrX | 5885150 | 5885400 | 0,344 |
| chr8 | 64808260 | 64808510 | 0,308 | | chrX | 7690150 | 7690400 | 0,316 |
| chr8 | 67203150 | 67203400 | 0,328 | | chrX | 8539329 | 8539579 | 0,292 |
| chr8 | 68461150 | 68461400 | 0,348 | | chrX | 9558559 | 9558809 | 0,32 |
| chr8 | 69596150 | 69596400 | 0,34 | | chrX | 10440260 | 10440510 | 0,348 |
| chr8 | 70457150 | 70457400 | 0,308 | | chrX | 11270150 | 11270400 | 0,344 |
| chr8 | 71266150 | 71266400 | 0,348 | | chrX | 12084550 | 12084800 | 0,312 |
| chr8 | 72260292 | 72260542 | 0,34 | | chrX | 13139540 | 13139790 | 0,348 |
| chr8 | 73118183 | 73118433 | 0,316 | | chrX | 14033255 | 14033505 | 0,348 |
| chr8 | 74441212 | 74441462 | 0,348 | | chrX | 15180150 | 15180400 | 0,344 |
| chr8 | 75565150 | 75565400 | 0,336 | | chrX | 16478150 | 16478400 | 0,316 |
| chr8 | 76518550 | 76518800 | 0,292 | | chrX | 17543222 | 17543472 | 0,32 |
| chr8 | 77509332 | 77509582 | 0,348 | | chrX | 18343366 | 18343616 | 0,32 |
| chr8 | 77895920 | 77896170 | 0,36 | | chrX | 19620578 | 19620828 | 0,348 |
| chr8 | 78423319 | 78423569 | 0,324 | | chrX | 20660335 | 20660585 | 0,348 |
| chr8 | 79227653 | 79227903 | 0,328 | | chrX | 21600150 | 21600400 | 0,34 |
| chr8 | 80038233 | 80038483 | 0,296 | | chrX | 22488497 | 22488747 | 0,348 |
| chr8 | 80846150 | 80846400 | 0,336 | | chrX | 23421185 | 23421435 | 0,348 |
| chr8 | 81657150 | 81657400 | 0,292 | | chrX | 24286150 | 24286400 | 0,312 |
| chr8 | 82795491 | 82795741 | 0,348 | | chrX | 25225575 | 25225825 | 0,348 |
| chr8 | 83607557 | 83607807 | 0,312 | | chrX | 26415364 | 26415614 | 0,348 |
| chr8 | 84412845 | 84413095 | 0,288 | | chrX | 27442376 | 27442626 | 0,316 |
| chr8 | 85516336 | 85516586 | 0,336 | | chrX | 28358150 | 28358400 | 0,312 |
| chr8 | 86873337 | 86873587 | 0,32 | | chrX | 29322235 | 29322485 | 0,348 |
| chr8 | 87681621 | 87681871 | 0,332 | | chrX | 30143185 | 30143435 | 0,348 |
| chr8 | 88481908 | 88482158 | 0,348 | | chrX | 31244468 | 31244718 | 0,344 |
| chr8 | 90093162 | 90093412 | 0,348 | | chrX | 32330150 | 32330400 | 0,296 |
| chr8 | 90983285 | 90983535 | 0,288 | | chrX | 33207324 | 33207574 | 0,308 |
| chr8 | 91158369 | 91158619 | 0,348 | | chrX | 34080150 | 34080400 | 0,332 |
| chr8 | 92065468 | 92065718 | 0,348 | | chrX | 34915259 | 34915509 | 0,344 |
| chr8 | 92901150 | 92901400 | 0,328 | | chrX | 35745301 | 35745551 | 0,32 |
| chr8 | 94611550 | 94611800 | 0,296 | | chrX | 36960179 | 36960429 | 0,348 |
| chr8 | 95411550 | 95411800 | 0,348 | | chrX | 37778455 | 37778705 | 0,34 |
| chr8 | 96629348 | 96629598 | 0,272 | | chrX | 38654560 | 38654810 | 0,312 |
| chr8 | 97873150 | 97873400 | 0,292 | | chrX | 39492888 | 39493138 | 0,348 |
| chr8 | 99476228 | 99476478 | 0,344 | | chrX | 40403451 | 40403701 | 0,348 |
| chr8 | 100281617 | 100281867 | 0,348 | | chrX | 41413158 | 41413408 | 0,348 |
| chr8 | 100454657 | 100454907 | 0,412 | | chrX | 42505150 | 42505400 | 0,34 |
| chr8 | 100523378 | 100523628 | 0,348 | | chrX | 43341210 | 43341460 | 0,348 |
| chr8 | 100733076 | 100733326 | 0,392 | | chrX | 44349185 | 44349435 | 0,34 |
| chr8 | 100830576 | 100830826 | 0,356 | | chrX | 45184425 | 45184675 | 0,348 |
| chr8 | 100835931 | 100836181 | 0,3 | | chrX | 46580312 | 46580562 | 0,344 |
| chr8 | 101099280 | 101099530 | 0,344 | | chrX | 47466548 | 47466798 | 0,348 |
| chr8 | 102559150 | 102559400 | 0,332 | | chrX | 48435008 | 48435258 | 0,348 |
| chr8 | 104722173 | 104722423 | 0,304 | | chrX | 49458818 | 49459068 | 0,328 |
| chr8 | 105808550 | 105808800 | 0,348 | | chrX | 50267179 | 50267429 | 0,348 |
| chr8 | 106610467 | 106610717 | 0,348 | | chrX | 51169150 | 51169400 | 0,324 |
| chr8 | 107422394 | 107422644 | 0,308 | | chrX | 51984557 | 51984807 | 0,316 |
| chr8 | 108285311 | 108285561 | 0,348 | | chrX | 52850579 | 52850829 | 0,348 |
| chr8 | 109090412 | 109090662 | 0,348 | | chrX | 53651747 | 53651997 | 0,348 |
| chr8 | 109972262 | 109972512 | 0,344 | | chrX | 54703818 | 54704068 | 0,332 |
| chr8 | 110795150 | 110795400 | 0,316 | | chrX | 56115356 | 56115606 | 0,348 |
| chr8 | 111908174 | 111908424 | 0,264 | | chrX | 56993967 | 56994217 | 0,348 |
| chr8 | 112711020 | 112711270 | 0,252 | | chrX | 57799496 | 57799746 | 0,348 |
| chr8 | 113514731 | 113514981 | 0,348 | | chrX | 62058452 | 62058702 | 0,348 |
| chr8 | 114740243 | 114740493 | 0,348 | | chrX | 63165214 | 63165464 | 0,344 |
| chr8 | 115568249 | 115568499 | 0,324 | | chrX | 63970645 | 63970895 | 0,344 |
| chr8 | 116514193 | 116514443 | 0,348 | | chrX | 64917421 | 64917671 | 0,348 |
| chr8 | 117360167 | 117360417 | 0,348 | | chrX | 65735342 | 65735592 | 0,348 |
| chr8 | 118611150 | 118611400 | 0,348 | | chrX | 66564534 | 66564784 | 0,348 |
| chr8 | 119426175 | 119426425 | 0,348 | | chrX | 67367876 | 67368126 | 0,348 |
| chr8 | 120338150 | 120338400 | 0,328 | | chrX | 68232212 | 68232462 | 0,348 |
| chr8 | 121474337 | 121474587 | 0,348 | | chrX | 69033716 | 69033966 | 0,348 |
| chr8 | 122274620 | 122274870 | 0,336 | | chrX | 69844167 | 69844417 | 0,344 |
| chr8 | 123089214 | 123089464 | 0,348 | | chrX | 71427432 | 71427682 | 0,348 |
| chr8 | 124026007 | 124026257 | 0,348 | | chrX | 72458405 | 72458655 | 0,348 |
| chr8 | 124844530 | 124844780 | 0,348 | | chrX | 73325904 | 73326154 | 0,348 |
| chr8 | 125653991 | 125654241 | 0,348 | | chrX | 74137571 | 74137821 | 0,348 |
| chr8 | 126510926 | 126511176 | 0,348 | | chrX | 75450150 | 75450400 | 0,304 |
| chr8 | 127312793 | 127313043 | 0,34 | | chrX | 76759150 | 76759400 | 0,324 |
| chr8 | 128133591 | 128133841 | 0,34 | | chrX | 77931150 | 77931400 | 0,344 |
| chr8 | 129022031 | 129022281 | 0,348 | | chrX | 78819276 | 78819526 | 0,348 |
| chr8 | 129839921 | 129840171 | 0,328 | | chrX | 79927150 | 79927400 | 0,292 |
| chr8 | 130654835 | 130655085 | 0,348 | | chrX | 80748375 | 80748625 | 0,3 |
| chr8 | 131476472 | 131476722 | 0,292 | | chrX | 81551352 | 81551602 | 0,296 |
| chr8 | 133133387 | 133133637 | 0,348 | | chrX | 82359761 | 82360011 | 0,296 |
| chr8 | 134746852 | 134747102 | 0,348 | | chrX | 83477290 | 83477540 | 0,28 |
| chr8 | 135547920 | 135548170 | 0,312 | | chrX | 84366205 | 84366455 | 0,256 |
| chr8 | 136372136 | 136372386 | 0,348 | | chrX | 85465150 | 85465400 | 0,288 |
| chr8 | 137311150 | 137311400 | 0,328 | | chrX | 86318150 | 86318400 | 0,316 |
| chr8 | 138113279 | 138113529 | 0,348 | | chrX | 87144287 | 87144537 | 0,292 |
| chr8 | 138930098 | 138930348 | 0,348 | | chrX | 87968502 | 87968752 | 0,284 |
| chr8 | 139733141 | 139733391 | 0,348 | | chrX | 89282095 | 89282345 | 0,344 |
| chr8 | 140552082 | 140552332 | 0,304 | | chrX | 90302716 | 90302966 | 0,288 |
| chr8 | 143927698 | 143927948 | 0,348 | | chrX | 91429162 | 91429412 | 0,32 |
| chr8 | 145640016 | 145640266 | 0,648 | | chrX | 92438331 | 92438581 | 0,288 |
| chr8 | 145640554 | 145640804 | 0,688 | | chrX | 93246796 | 93247046 | 0,32 |
| chr8 | 145946790 | 145947040 | 0,348 | | chrX | 94085648 | 94085898 | 0,304 |
| chr9 | 441472 | 441722 | 0,348 | | chrX | 94891150 | 94891400 | 0,276 |
| chr9 | 1249421 | 1249671 | 0,344 | | chrX | 95711217 | 95711467 | 0,348 |
| chr9 | 2865702 | 2865952 | 0,308 | | chrX | 96648150 | 96648400 | 0,348 |
| chr9 | 3684579 | 3684829 | 0,348 | | chrX | 97471150 | 97471400 | 0,328 |
| chr9 | 4499494 | 4499744 | 0,3 | | chrX | 98557428 | 98557678 | 0,348 |
| chr9 | 5611467 | 5611717 | 0,348 | | chrX | 99863553 | 99863803 | 0,348 |
| chr9 | 6417235 | 6417485 | 0,348 | | chrX | 102182150 | 102182400 | 0,28 |
| chr9 | 7244553 | 7244803 | 0,348 | | chrX | 102990468 | 102990718 | 0,34 |
| chr9 | 8056430 | 8056680 | 0,344 | | chrX | 104258320 | 104258570 | 0,348 |
| chr9 | 8856701 | 8856951 | 0,348 | | chrX | 105096264 | 105096514 | 0,348 |
| chr9 | 9660879 | 9661129 | 0,348 | | chrX | 105974498 | 105974748 | 0,324 |
| chr9 | 10463146 | 10463396 | 0,296 | | chrX | 106776228 | 106776478 | 0,348 |
| chr9 | 11263700 | 11263950 | 0,332 | | chrX | 107581900 | 107582150 | 0,348 |
| chr9 | 12065601 | 12065851 | 0,344 | | chrX | 108432389 | 108432639 | 0,348 |
| chr9 | 12872277 | 12872527 | 0,348 | | chrX | 109447214 | 109447464 | 0,348 |
| chr9 | 13678510 | 13678760 | 0,348 | | chrX | 110387150 | 110387400 | 0,344 |
| chr9 | 14494878 | 14495128 | 0,348 | | chrX | 111297150 | 111297400 | 0,324 |
| chr9 | 15310150 | 15310400 | 0,332 | | chrX | 112213179 | 112213429 | 0,328 |
| chr9 | 16415150 | 16415400 | 0,332 | | chrX | 113024257 | 113024507 | 0,34 |
| chr9 | 17466150 | 17466400 | 0,3 | | chrX | 114142520 | 114142770 | 0,348 |
| chr9 | 21059391 | 21059641 | 0,348 | | chrX | 115476183 | 115476433 | 0,348 |
| chr9 | 22073150 | 22073400 | 0,34 | | chrX | 116479598 | 116479848 | 0,292 |
| chr9 | 22959266 | 22959516 | 0,348 | | chrX | 117493202 | 117493452 | 0,328 |
| chr9 | 24120550 | 24120800 | 0,292 | | chrX | 118384548 | 118384798 | 0,348 |
| chr9 | 25117165 | 25117415 | 0,348 | | chrX | 119427212 | 119427462 | 0,348 |
| chr9 | 26013411 | 26013661 | 0,348 | | chrX | 120380150 | 120380400 | 0,348 |
| chr9 | 26820970 | 26821220 | 0,348 | | chrX | 121219432 | 121219682 | 0,276 |
| chr9 | 27623659 | 27623909 | 0,308 | | chrX | 122166150 | 122166400 | 0,308 |
| chr9 | 28464150 | 28464400 | 0,272 | | chrX | 122994558 | 122994808 | 0,348 |
| chr9 | 29277441 | 29277691 | 0,348 | | chrX | 123971391 | 123971641 | 0,348 |
| chr9 | 30170150 | 30170400 | 0,32 | | chrX | 125186473 | 125186723 | 0,332 |
| chr9 | 31049150 | 31049400 | 0,316 | | chrX | 126046357 | 126046607 | 0,348 |
| chr9 | 33112448 | 33112698 | 0,348 | | chrX | 126855255 | 126855505 | 0,332 |
| chr9 | 33917453 | 33917703 | 0,348 | | chrX | 127687504 | 127687754 | 0,348 |
| chr9 | 34744366 | 34744616 | 0,348 | | chrX | 128495196 | 128495446 | 0,348 |
| chr9 | 36071273 | 36071523 | 0,34 | | chrX | 129596551 | 129596801 | 0,348 |
| chr9 | 38018399 | 38018649 | 0,348 | | chrX | 130429675 | 130429925 | 0,34 |
| chr9 | 71032343 | 71032593 | 0,348 | | chrX | 131260508 | 131260758 | 0,348 |
| chr9 | 71833417 | 71833667 | 0,348 | | chrX | 132208598 | 132208848 | 0,348 |
| chr9 | 72920150 | 72920400 | 0,316 | | chrX | 133010150 | 133010400 | 0,344 |
| chr9 | 73915530 | 73915780 | 0,32 | | chrX | 133923287 | 133923537 | 0,348 |
| chr9 | 75167488 | 75167738 | 0,348 | | chrX | 135091150 | 135091400 | 0,332 |
| chr9 | 76531468 | 76531718 | 0,348 | | chrX | 136037150 | 136037400 | 0,328 |
| chr9 | 77413560 | 77413810 | 0,348 | | chrX | 136863150 | 136863400 | 0,324 |
| chr9 | 78522150 | 78522400 | 0,332 | | chrX | 137744150 | 137744400 | 0,328 |
| chr9 | 79507397 | 79507647 | 0,3 | | chrX | 138579590 | 138579840 | 0,336 |
| chr9 | 80739535 | 80739785 | 0,348 | | chrX | 139824185 | 139824435 | 0,348 |
| chr9 | 81567279 | 81567529 | 0,348 | | chrX | 140904478 | 140904728 | 0,316 |
| chr9 | 82372319 | 82372569 | 0,348 | | chrX | 141983247 | 141983497 | 0,348 |
| chr9 | 83221150 | 83221400 | 0,348 | | chrX | 142791108 | 142791358 | 0,344 |
| chr9 | 84202150 | 84202400 | 0,348 | | chrX | 143621663 | 143621913 | 0,348 |
| chr9 | 85081150 | 85081400 | 0,332 | | chrX | 144958182 | 144958432 | 0,348 |
| chr9 | 85987217 | 85987467 | 0,348 | | chrX | 145759329 | 145759579 | 0,344 |
| chr9 | 86799808 | 86800058 | 0,32 | | chrX | 146589192 | 146589442 | 0,348 |
| chr9 | 87600430 | 87600680 | 0,34 | | chrX | 147591150 | 147591400 | 0,344 |
| chr9 | 88575150 | 88575400 | 0,328 | | chrX | 148452186 | 148452436 | 0,348 |
| chr9 | 89393921 | 89394171 | 0,348 | | chrX | 149357308 | 149357558 | 0,348 |
| chr9 | 90196155 | 90196405 | 0,348 | | chrX | 150240045 | 150240295 | 0,348 |
| chr9 | 91389150 | 91389400 | 0,316 | | chrX | 151043387 | 151043637 | 0,348 |
| chr9 | 92224150 | 92224400 | 0,34 | | chrX | 152016983 | 152017233 | 0,348 |
| chr9 | 93027865 | 93028115 | 0,348 | | chrX | 153113554 | 153113804 | 0,348 |
| chr9 | 93881161 | 93881411 | 0,348 | | chrX | 154180278 | 154180528 | 0,348 |
| chr9 | 94685863 | 94686113 | 0,332 | | chrY | 2660772 | 2661021 | 0,361446 |
| chr9 | 95551224 | 95551474 | 0,324 | | chrY | 2710538 | 2710787 | 0,445783 |
| chr9 | 96382913 | 96383163 | 0,348 | | chrY | 2818858 | 2819107 | 0,37751 |
| chr9 | 97206756 | 97207006 | 0,348 | | chrY | 2831578 | 2831827 | 0,349398 |
| chr9 | 98010068 | 98010318 | 0,3 | | chrY | 2846524 | 2846773 | 0,329317 |
| chr9 | 98910804 | 98911054 | 0,348 | | chrY | 2903702 | 2903952 | 0,3 |
| chr9 | 100612919 | 100613169 | 0,348 | | chrY | 3713664 | 3713914 | 0,312 |
| chr9 | 102040150 | 102040400 | 0,348 | | chrY | 6592835 | 6593085 | 0,348 |
| chr9 | 103037412 | 103037662 | 0,348 | | chrY | 6785795 | 6786044 | 0,433735 |
| chr9 | 104113326 | 104113576 | 0,348 | | chrY | 6991732 | 6991982 | 0,348 |
| chr9 | 104184056 | 104184306 | 0,512 | | chrY | 7619930 | 7620180 | 0,328 |
| chr9 | 104187132 | 104187382 | 0,516 | | chrY | 7628101 | 7628351 | 0,34 |
| chr9 | 104187690 | 104187940 | 0,52 | | chrY | 7645822 | 7646072 | 0,288 |
| chr9 | 104189655 | 104189905 | 0,512 | | chrY | 7655666 | 7655916 | 0,348 |
| chr9 | 104192058 | 104192308 | 0,544 | | chrY | 7859013 | 7859263 | 0,348 |
| chr9 | 104193030 | 104193280 | 0,524 | | chrY | 8234092 | 8234342 | 0,348 |
| chr9 | 104920172 | 104920422 | 0,336 | | chrY | 8843984 | 8844234 | 0,324 |
| chr9 | 105721671 | 105721921 | 0,304 | | chrY | 9004092 | 9004341 | 0,405622 |
| chr9 | 106523521 | 106523771 | 0,264 | | chrY | 9415984 | 9416234 | 0,332 |
| chr9 | 107485162 | 107485412 | 0,348 | | chrY | 9894154 | 9894404 | 0,328 |
| chr9 | 108536234 | 108536484 | 0,348 | | chrY | 14188444 | 14188694 | 0,336 |
| chr9 | 109563573 | 109563823 | 0,348 | | chrY | 14638059 | 14638309 | 0,348 |
| chr9 | 110626420 | 110626670 | 0,348 | | chrY | 15017843 | 15018092 | 0,445783 |
| chr9 | 111662458 | 111662708 | 0,444 | | chrY | 15021516 | 15021765 | 0,35743 |
| chr9 | 111732400 | 111732650 | 0,348 | | chrY | 15473334 | 15473583 | 0,353414 |
| chr9 | 113340650 | 113340900 | 0,332 | | chrY | 15819282 | 15819532 | 0,348 |
| chr9 | 114144997 | 114145247 | 0,308 | | chrY | 16228505 | 16228755 | 0,348 |
| chr9 | 114957028 | 114957278 | 0,348 | | chrY | 16629874 | 16630124 | 0,348 |
| chr9 | 115762575 | 115762825 | 0,308 | | chrY | 16837863 | 16838110 | 0,376518 |
| chr9 | 116577087 | 116577337 | 0,348 | | chrY | 17030033 | 17030283 | 0,296 |
| chr9 | 117441289 | 117441539 | 0,328 | | chrY | 17232877 | 17233127 | 0,308 |
| chr9 | 118963361 | 118963611 | 0,348 | | chrY | 17234803 | 17235053 | 0,308 |
| chr9 | 119460264 | 119460514 | 0,6 | | chrY | 17236192 | 17236442 | 0,288 |
| chr9 | 119888150 | 119888400 | 0,288 | | chrY | 17456632 | 17456882 | 0,348 |
| chr9 | 121125150 | 121125400 | 0,336 | | chrY | 17852621 | 17852871 | 0,304 |
| chr9 | 122426151 | 122426401 | 0,348 | | chrY | 18264936 | 18265186 | 0,308 |
| chr9 | 123520150 | 123520400 | 0,332 | | chrY | 18676622 | 18676872 | 0,304 |
| chr9 | 124683228 | 124683478 | 0,348 | | chrY | 18884770 | 18885020 | 0,3 |
| chr9 | 125485603 | 125485853 | 0,34 | | chrY | 19086733 | 19086983 | 0,348 |
| chr9 | 127505527 | 127505777 | 0,348 | | chrY | 19287757 | 19288007 | 0,348 |
| chr9 | 128307968 | 128308218 | 0,348 | | chrY | 19317270 | 19317520 | 0,348 |
| chr9 | 129109844 | 129110094 | 0,348 | | chrY | 19321140 | 19321390 | 0,28 |
| chr9 | 129916775 | 129917025 | 0,348 | | chrY | 19336515 | 19336765 | 0,348 |
| chr9 | 131244670 | 131244920 | 0,348 | | chrY | 19343744 | 19343994 | 0,308 |
| chr9 | 132573290 | 132573540 | 0,348 | | chrY | 19345482 | 19345732 | 0,348 |
| chr9 | 133333811 | 133334061 | 0,64 | | chrY | 19551092 | 19551342 | 0,34 |
| chr9 | 133413285 | 133413535 | 0,348 | | chrY | 20807527 | 20807777 | 0,34 |
| chr9 | 134486613 | 134486863 | 0,348 | | chrY | 21231948 | 21232198 | 0,328 |
| chr9 | 135288372 | 135288622 | 0,348 | | chrY | 21617999 | 21618249 | 0,304 |
| chr9 | 136280107 | 136280357 | 0,348 | | chrY | 21825259 | 21825509 | 0,34 |
| chr9 | 137460915 | 137461165 | 0,32 | | chrY | 21870303 | 21870553 | 0,348 |
| chr9 | 138342214 | 138342464 | 0,348 | | chrY | 21882430 | 21882680 | 0,348 |
| chr9 | 139306976 | 139307226 | 0,268 | | chrY | 21889152 | 21889402 | 0,312 |
| chr9 | 140524526 | 140524776 | 0,348 | | chrY | 21892914 | 21893164 | 0,336 |
| chr10 | 871150 | 871400 | 0,348 | | chrY | 21896890 | 21897140 | 0,308 |
| chr10 | 2124524 | 2124774 | 0,252 | | chrY | 22098161 | 22098411 | 0,252 |
| chr10 | 2929327 | 2929577 | 0,348 | | chrY | 22519572 | 22519822 | 0,28 |
| chr10 | 4220150 | 4220400 | 0,336 | | chrY | 22923958 | 22924208 | 0,348 |
| chr10 | 5176559 | 5176809 | 0,336 | | chrY | 23365875 | 23366125 | 0,344 |
| chr10 | 6343194 | 6343444 | 0,344 | | chrY | 23594574 | 23594824 | 0,348 |
| chr10 | 7390225 | 7390475 | 0,348 | | chrY | 23598786 | 23599036 | 0,32 |
| chr10 | 8257298 | 8257548 | 0,348 | | chrY | 23770609 | 23770859 | 0,348 |
| | | | | | chrY | 23772351 | 23772601 | 0,344 |

### Detailed Description

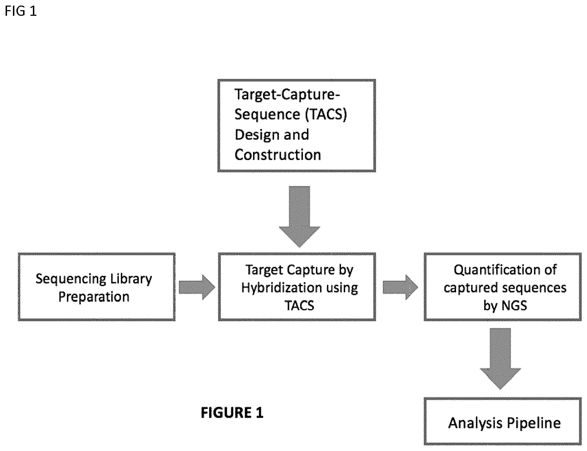

The invention pertains to a method for analyzing genetic abnormalities that involves hybridization-based enrichment of selected target regions across the human genome in a multiplexed panel assay, followed by quantification, coupled with a novel bioinformatics and mathematical analysis pipeline. An overview of the method is shown schematically in Figure 1.

In-solution hybridization enrichment has been used in the past to enrich specific regions of interest prior to sequencing (see e.g., Meyer, M and Kirchner, M. (2010) Cold Spring Harb. Protoc. 2010(6):pdbprot5448; Liao, G.J. et al. (2012) PLoS One 7:e38154; Maricic, T. et al. (2010) PLoS One 5:e14004; Tewhey, R. et al.(2009) Genome Biol. 10:R116; Tsangaras, K. et al. (2014) PLoS One 9:e109101; PCT Publication WO 2016/189388; US Patent Publication 2016/0340733; Koumbaris, G. et al. (2015) Clinical chemistry, 62(6), pp.848-855). However, for the methods of the invention, the target sequences (referred to as TArget Capture Sequences, or TACS) used to enrich for specific regions of interest have been optimized for maximum efficiency, specificity and accuracy and, furthermore, allow for analysis of very small starting amounts of fetal or embryonic DNA in samples containing only or predominantly fetal or embryonic DNA

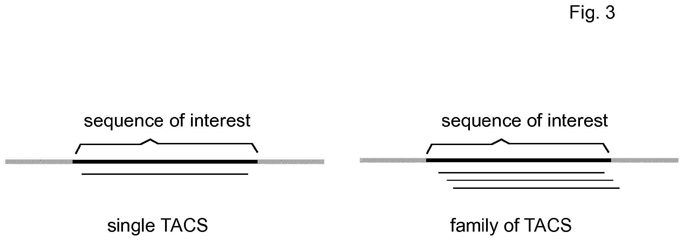

Furthermore, in certain embodiments, the TACS used in the methods are families of TACS, comprising a plurality of members that bind to the same genomic sequence but with differing start and/or stop positions, such that enrichment of the genomic sequences of interest is significantly improved compared to use of a single TACS binding to the genomic sequence. The configuration of such families of TACS is illustrated schematically in Figure 3, showing that the different start and/or stop positions of the members of the TACS family when bound to the genomic sequence of interest results in a staggered binding pattern for the family members.

The use of families of TACS with the TACS pool that bind to each target sequence of interest, as compared to use of a single TACS within the TACS pool that binds to each target sequence of interest, significantly increases enrichment for the target sequences of interest, as evidenced by a greater than 50% average increase in read-depth for the family of TACS versus a single TACS. Comparison of use of a family of TACS versus a single TACS, and the significantly improved read-depth that was observed, is described in detail in Example 5.

### Analysis of Fetal/Embryonic DNA Samples

The methods and kits of the disclosure are used in the analysis of fetal or embryonic DNA samples, e.g., for the presence of genetic abnormalities, for example for purposes of IVF Pre-implantation Genetic Screening (PGS) and Diagnosis (PGD). Accordingly, in the methods of the invention, the DNA sample comprises predominantly or only fetal or embryonic DNA. The methods can be used with samples from a single or only a few fetal or embryonic cells. As used herein "a few" fetal or embryonic cells refers to 10 fetal or embryonic cells or less. Accordingly, the methods allow for analysis of very small amounts of fetal or embryonic DNA. The fetal or embryonic DNA sample contains predominantly or only fetal/embryonic DNA, described further below in the subsection on sample preparation. An exemplification of use of the method with samples from 3-day and 5-day biopsy embryos is described in Example 6.

Accordingly, in one aspect, the invention pertains to a method of testing for risk of a genetic abnormality in a DNA sample comprising predominantly fetal or embryonic DNA and comprising genomic sequences of interest, the method comprising:a) preparing a sequencing library from the DNA sample comprising predominantly fetal or embryonic DNA;b) hybridizing the sequencing library to a pool of double-stranded TArget Capture Sequences (TACS), wherein the pool of TACS comprises a plurality of TACS families directed to different genomic sequences of interest comprising a genetic abnormality, wherein each TACS family comprises a plurality of member sequences, wherein each member sequence binds to the same genomic sequence of interest but has different start and/or stop positions with respect to a reference coordinate system for the genomic sequence of interest, wherein the start and/or stop positions for the member sequences within a TACS family, with respect to a reference coordinate system for the genomic sequence of interest are staggered by 5 to 10 base pairs, and further wherein:i) each member sequence within the pool of TACS is between 150-260 base pairs in length, each member sequence having a 5' end and a 3' end; andii) each member sequence binds to the same genomic sequence of interest at least 50 base pairs away, on both the 5' end and the 3' end, from regions harboring Copy Number Variations (CNVs), Segmental duplications or repetitive DNA elements; andiii) the GC content of the pool of TACS is between 19% and 80%, as determined by calculating the GC content of each member within the pool of TACS;c) isolating members of the sequencing library that bind to the pool of TACS to obtain an enriched library;d) amplifying and sequencing the enriched library;e) aligning, the enriched library to a reference genome thereby obtaining read-depth information and allelic counts; andf) performing statistical analysis on the enriched library sequences to thereby determine risk of a genetic abnormality in the DNA sample.

In one embodiment, the pool of TACS comprises a plurality of TACS families, wherein each member of a TACS family binds to the same target sequence of interest but with different start and/or stop positions on the sequence with respect to a reference coordinate system (i.e., binding of TACS family members to the target sequence is staggered) to thereby enrich for target sequences of interest, followed by massive parallel sequencing and statistical analysis of the enriched population. The use of families of TACS with the TACS pool that bind to each target sequence of interest, as compared to use of a single TACS within the TACS pool that binds to each target sequence of interest, significantly increases enrichment for the target sequences of interest, as evidenced by a greater than 50% average increase in read-depth for the family of TACS versus a single TACS.

Accordingly, in one embodiment, the pool of TACS comprises a plurality of TACS families directed to different genomic sequences of interest, wherein each TACS family comprises a plurality of member sequences, wherein each member sequence binds to the same genomic sequence of interest but has different start and/or stop positions with respect to a reference coordinate system for the genomic sequence of interest.

The TACS-enrichment based method of the disclosure can be used in the detection of a wide variety of genetic abnormalities. In one embodiment, the genetic abnormality is a chromosomal aneuploidy (such as a trisomy, a partial trisomy or a monosomy). In other embodiments, the genomic abnormality is a structural abnormality, including but not limited to copy number changes including microdeletions and microduplications, insertions, translocations, inversions and small-size mutations including point mutations and mutational signatures. In another embodiment, the genetic abnormality is a chromosomal mosaicism.

### TArget Capture Sequence Design

As used herein, the term "TArget Capture Sequences" or "TACS" refers to short DNA sequences that are complementary to the region(s) of interest on a genomic sequence(s) of interest (e.g., chromosome(s) of interest) and which are used as "bait" to capture and enrich the region of interest from a large library of sequences, such as a whole genomic sequencing library prepared from a biological sample. A pool of TACS is used for enrichment wherein the sequences within the pool have been optimized with regard to: (i) the length of the sequences; (ii) the distribution of the TACS across the region(s) of interest; and (iii) the GC content of the TACS. The number of sequences within the TACS pool (pool size) has also been optimized.

It has been discovered that TACS having a length of 150-260 base pairs are optimal to maximize enrichment efficiency. In various other embodiments, each sequence within the pool of TACS is between 150-260 base pairs or 200-260 base pairs, in length. In preferred embodiments, the length of the TACS within the pool is is 250 base pairs or is 260 base pairs

The distribution of the TACS across each region or chromosome of interest has been optimized to avoid high copy repeats, low copy repeats and copy number variants, while at the same time also being able to target informative single nucleotide polymorphisms (SNPs) in order to enable both aneuploidy, or structural copy number change detection, and fetal fraction (ff) estimation. Accordingly, each sequence within the TACS pool is designed such that the 5' end and the 3' end are each at least 50 base pairs away from regions in the genome that are known to harbour one or more of the following genomic elements: Copy Number Variations (CNVs), Segmental duplications and/or repetitive DNA elements (such as transposable elements or tandem repeat areas). In various other embodiments, each sequence within the TACS pool is designed such that the 5' end and the 3' end are each at least 50, 100, 150, 200, 250, 300, 400 or 500 base pairs away from regions in the genome that are known to harbour one or more of the aforementioned elements.

The term "Copy Number Variations" is a term of art that refers to a form of structural variation in the human genome in which there can be alterations in the DNA of the genome in different individuals that can result in a fewer or greater than normal number of a section(s) of the genome in certain individuals. CNVs correspond to relatively large regions of the genome that may be deleted (e.g., a section that normally is A-B-C-D can be A-B-D) or may be duplicated (e.g., a section that normally is A-B-C-D can be A-B-C-C-D). CNVs account for roughly 13% of the human genome, with each variation ranging in size from about 1 kilobase to several megabases in size.

The term "Segmental duplications" (also known as "low-copy repeats") is also a term of art that refers to blocks of DNA that range from about 1 to 400 kilobases in length that occur at more than one site within the genome and typically share a high level (greater than 90%) of sequence identity. Segmental duplications are reviewed in, for example, Eichler. E.E. (2001) Trends Genet. 17:661-669.

The term "repetitive DNA elements" (also known as "repeat DNA" or "repeated DNA") is also a term of art that refers to patterns of DNA that occur in multiple copies throughout the genome. The term "repetitive DNA element" encompasses terminal repeats, tandem repeats and interspersed repeats, including transposable elements. Repetitive DNA elements in NGS is discussed further in, for example, Todd, J. et al. (2012) Nature Reviews Genet. 13:36-46.

The TACS are designed with specific GC content characteristics in order to minimize data GC bias and to allow a custom and innovative data analysis pipeline. It has been determined that TACS with a GC content of 19-80% achieve optimal enrichment and perform best with cell free fetal DNA. Within the pool of TACS, different sequences can have different % GC content, although to be selected for inclusion with the pool, the % GC content of each sequence is chosen as between 19-80%, as determined by calculating the GC content of each member within the pool of TACS or within each family of TACS. That is, every member within the pool or within each family of TACS in the pool has a % GC content within the given percentage range (e.g., between 19-80% GC content).

In some instances, the pool of TACS (e.g., each member within each family of TACS) may be chosen so as to define a different % GC content range, deemed to be more suitable for the assessment of specific genetic abnormalities. Non-limiting examples of various % GC content ranges, can be between 19% and 80%, or between 19% and 79%, or between 19% and 78%, or between 19% and 77%, or between 19% and 76%, or between 19% and 75%, or between 19% and 74%, or between 19% and 73%, or between 19% and 72%, or between 19% and 71%, or between 19% and 70%, or between 19% and 69%, or between 19% and 68%, or between 19% and 67%, or between 19% and 66%, or between 19% and 65%, or between 19% and 64%, or between 19% and 63%, or between 19% and 62%, or between 19% and 61%, or between 19% and 60%, or between 19% and 59%, or between 19% and 58%, or between 19% and 57%, or between 19% and 56%, or between 19% and 55%, or between 19% and 54%, or between 19% and 53%, or between 19% and 52%, or between 19% and 51%, or between 19% and 50%, or between 19% and 49%, or between 19% and 48%, or between 19% and 47%, or between 19% and 46%, or between 19% and 45%, or between 19% and 44%, or between 19% and 43%, or between 19% and 42%, or between 19% and 41%, or between 19% and 40%.

As described in further detail below with respect to one embodiment of the data analysis, following amplification and sequencing of the enriched sequences, the test loci and reference loci can then be "matched" or grouped together according to their % GC content (e.g., test loci with a % GC content of 40% is matched with reference loci with a % GC content of 40%). It is appreciated that the % GC content matching procedure may allow slight variation in the allowed matched % GC range. A non-limiting instance, and with reference to the previously described example in text, a test locus with % GC content of 40% could be matched with reference loci of % GC ranging from 39-41%, thereby encompassing the test locus % GC within a suitable range.

To prepare a pool of TACS having the optimized criteria set forth above with respect to size, placement within the human genome and % GC content, both manual and computerized analysis methods known in the art can be applied to the analysis of the human reference genome. In one embodiment, a semi-automatic method is implemented where regions are firstly manually designed based on the human reference genome build 19 (hg19) ensuring that the aforementioned repetitive regions are avoided and subsequently are curated for GC-content using software that computes the % GC-content of each region based on its coordinates on the human reference genome build 19 (hg19). In another embodiment, custom-built software is used to analyze the human reference genome in order to identify suitable TACS regions that fulfill certain criteria, such as but not limited to, % GC content, proximity to repetitive regions and/or proximity to other TACS.

The number of TACS in the pool has been carefully examined and adjusted to achieve the best balance between result robustness and assay cost/throughput. The pool typically contains at least 800 or more TACS, but can include more, such as 1500 or more TACS, 2000 or more TACS or 2500 or more TACS or 3500 or more TACS or 5000 or more TACS. It has been found that an optimal number of TACS in the pool is 5000. It will be appreciated by the ordinarily skilled artisan that a slight variation in pool size typically can be used without altering the results (e.g., the addition or removal of a small number of TACS); accordingly, the number sizes of the pool given herein are to be considered "about" or "approximate", allowing for some slight variation (e.g., 1-5%) in size. Thus, for example, a pool size of "1600 sequences" is intended to refer to "about 1600 sequences" or "approximately 1600 sequences", such that, for example, 1590 or 1610 sequences is also encompassed.

In view of the foregoing, in another aspect, the invention provides a method for preparing a pool of TACS for use in the method of the invention for detecting risk of a chromosomal and/or other genetic abnormality, wherein the method for preparing the pool of TACS comprises: selecting regions in one or more chromosomes of interest having the criteria set forth above (e.g., at least 50 base pairs away on either end from the aforementioned repetitive sequences and a GC content of between 19% and 80%, as determined by calculating the GC content of each member within each family of TACS), preparing primers that amplify sequences that hybridize to the selected regions, and amplifying the sequences, wherein each sequence is 100-500 base pairs in length.

For use in the methods of the disclosure, the pool of TACS typically is fixed to a solid support, such as beads (such as magnetic beads) or a column. In one embodiment, the pool of TACS are labeled with biotin and are bound to magnetic beads coated with a biotin-binding substance, such as streptavidin or avidin, to thereby fix the pool of TACS to a solid support. Other suitable binding systems for fixing the pool of TACS to a solid support (such as beads or column) are known to the skilled artisan and readily available in the art. When magnetic beads are used as the solid support, sequences that bind to the TACS affixed to the beads can be separated magnetically from those sequences that do not bind to the TACS.

### Families of TACS

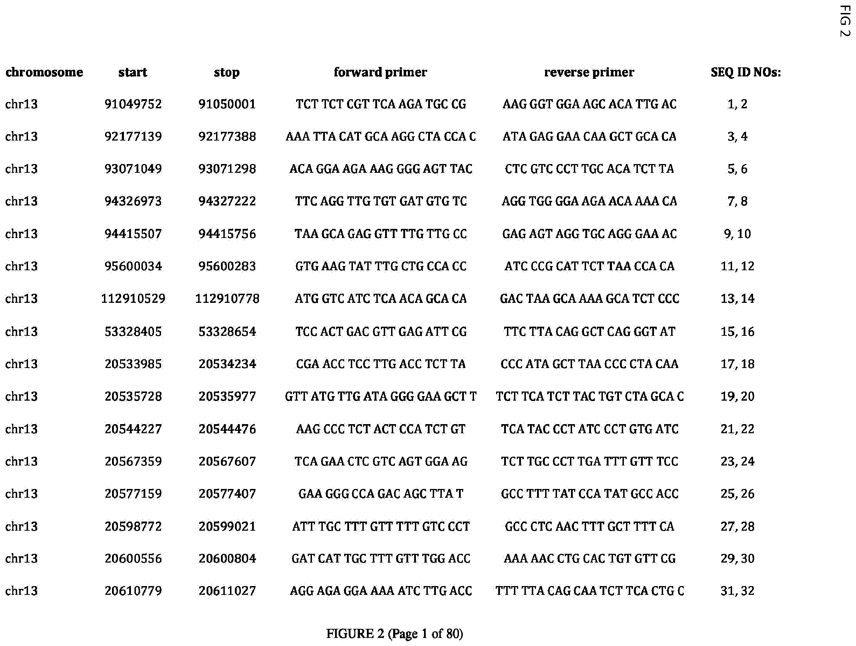

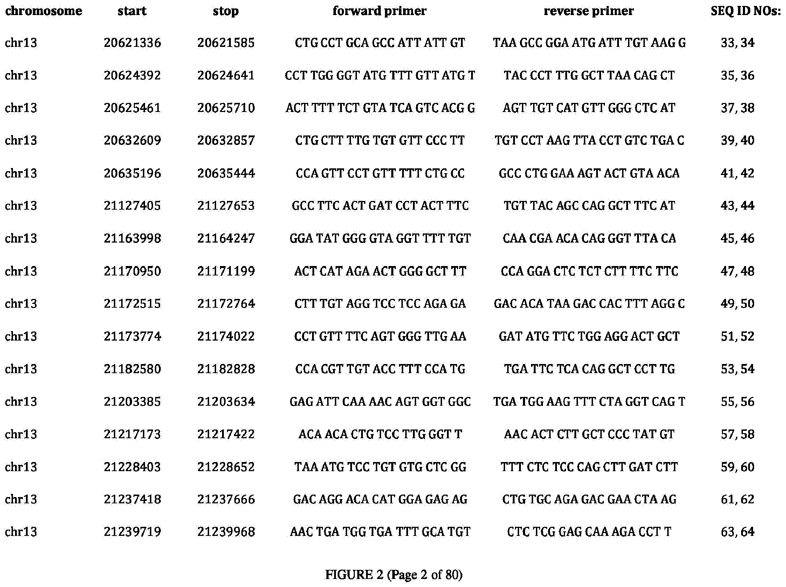

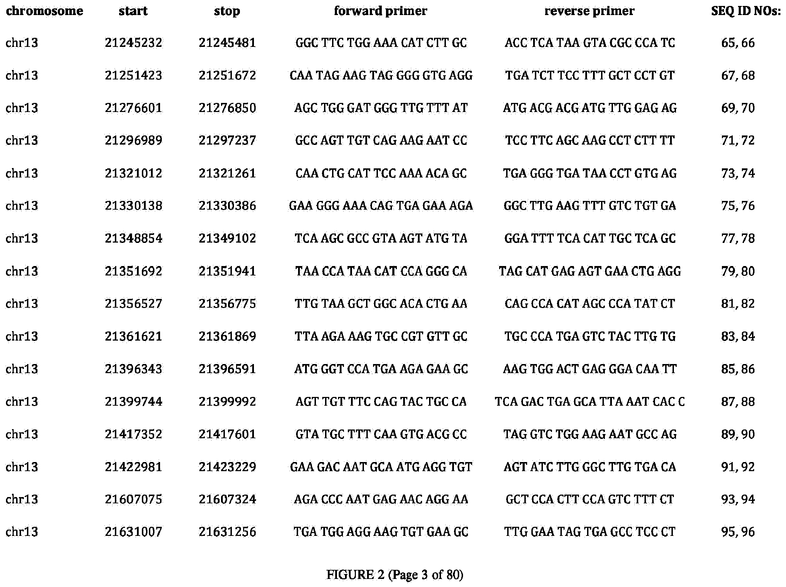

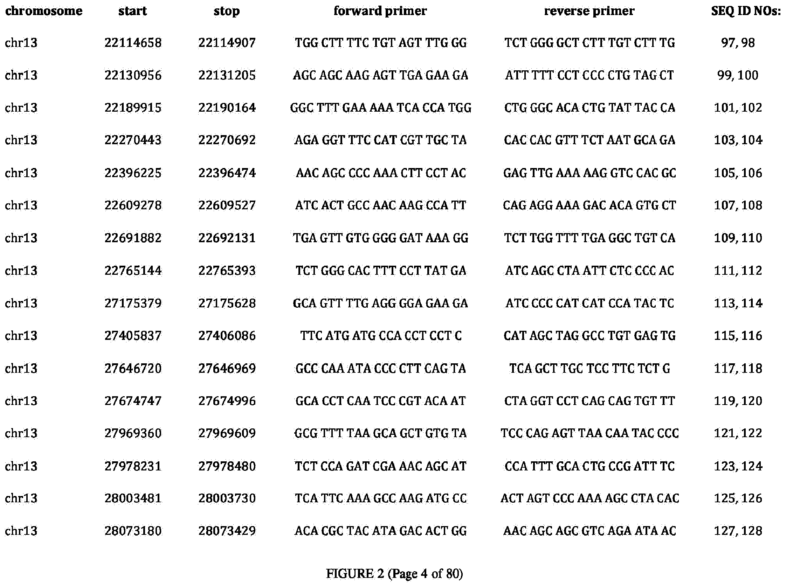

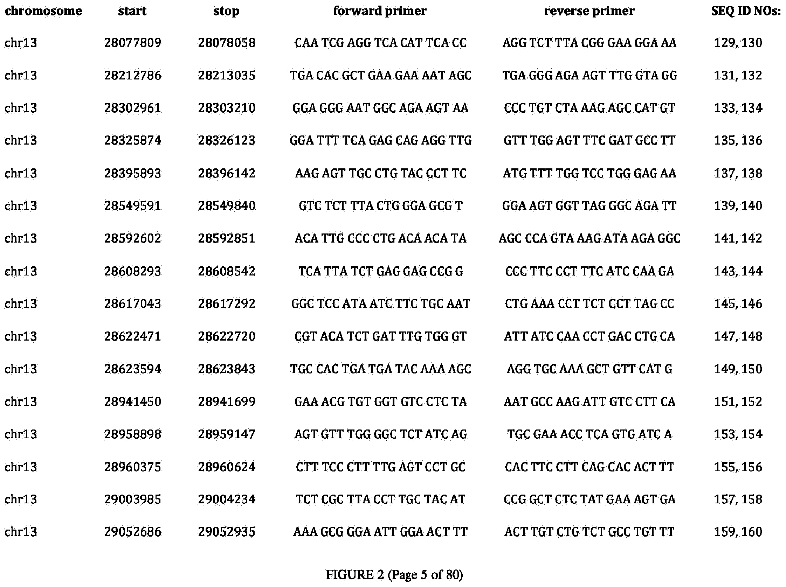

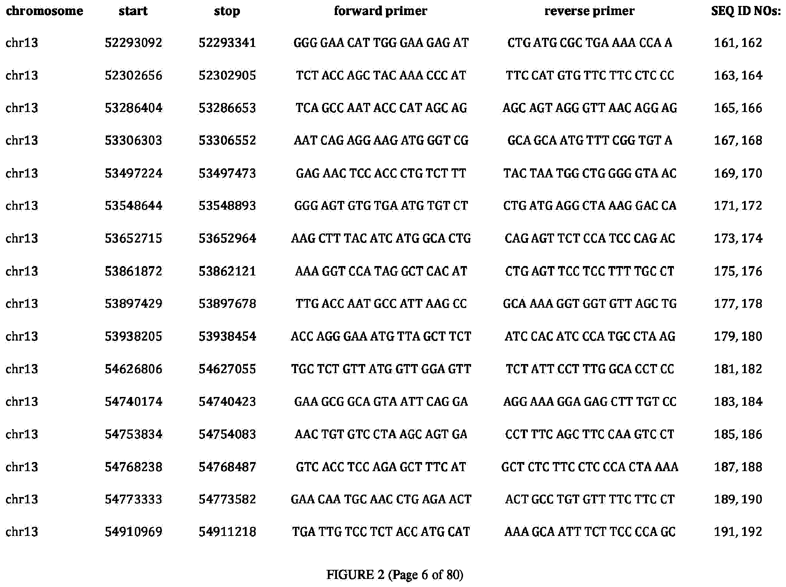

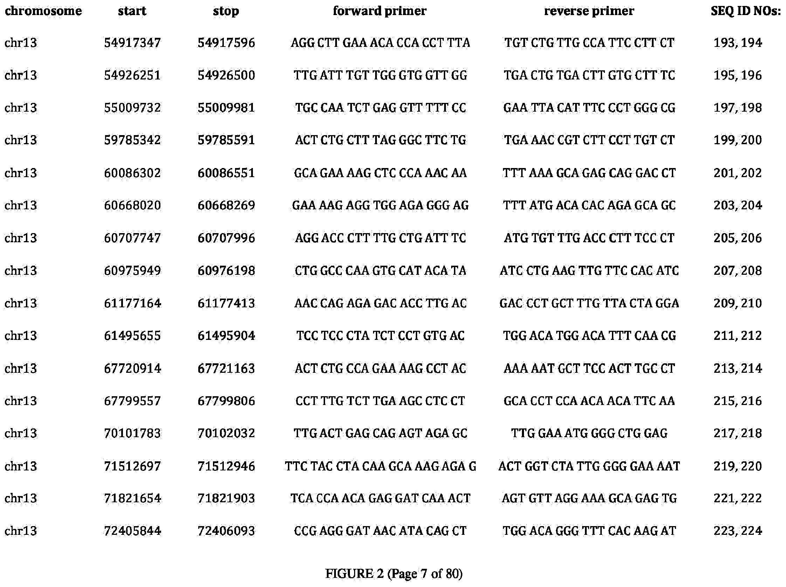

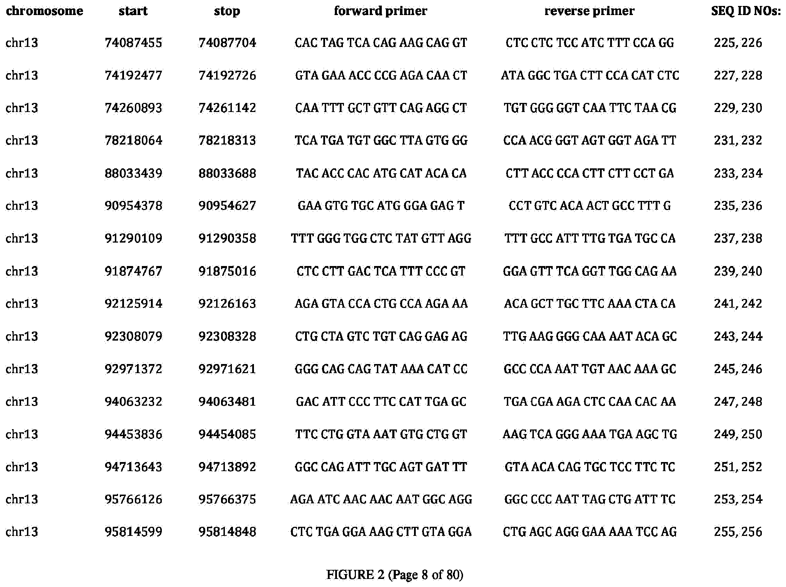

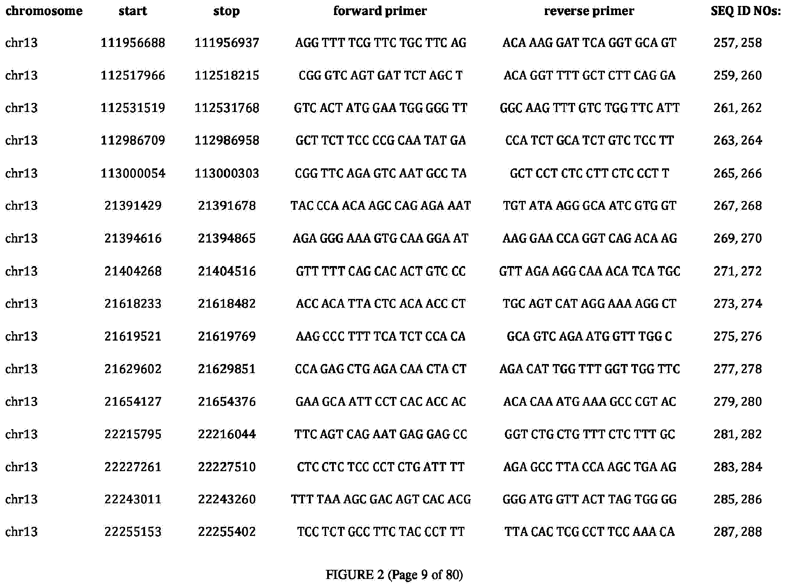

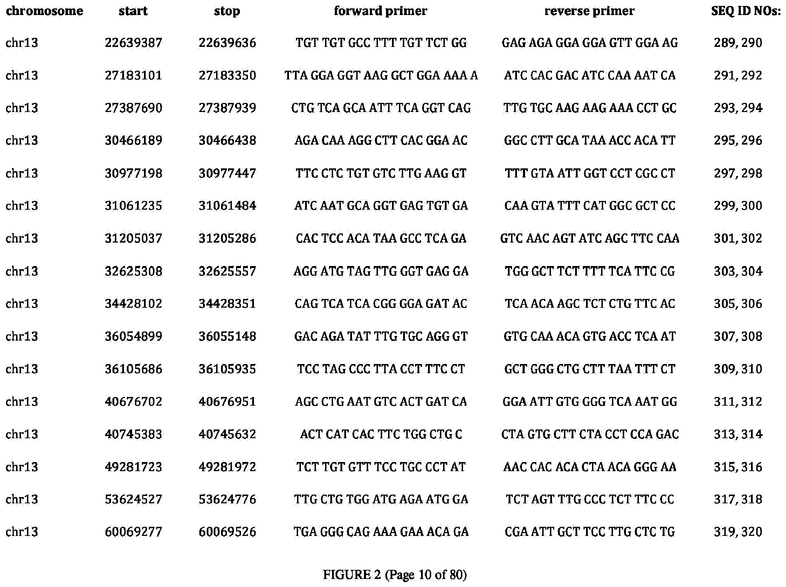

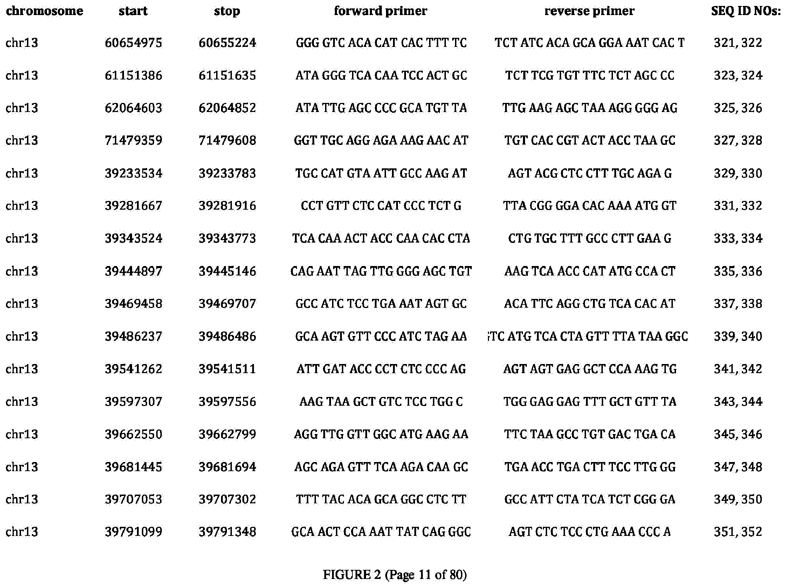

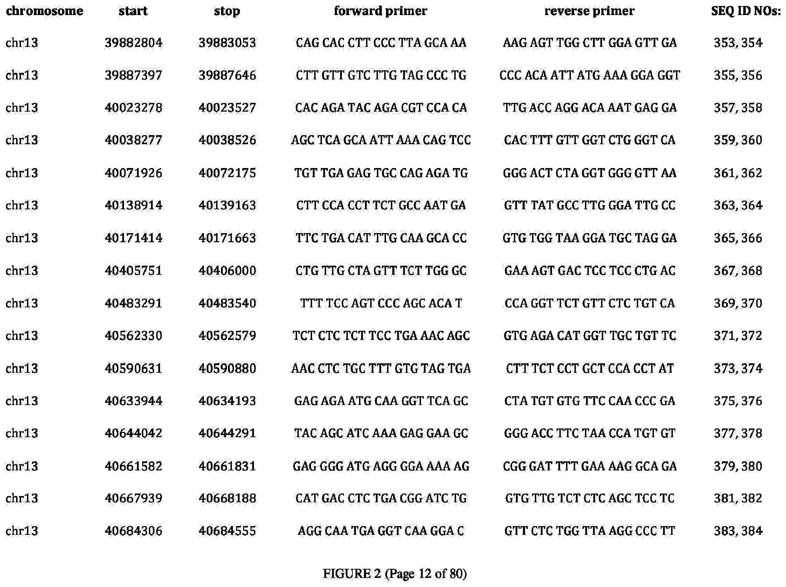

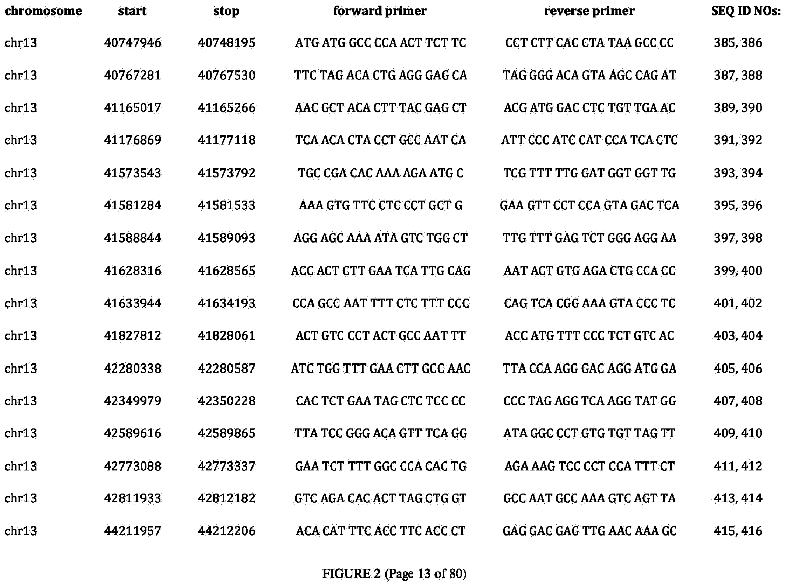

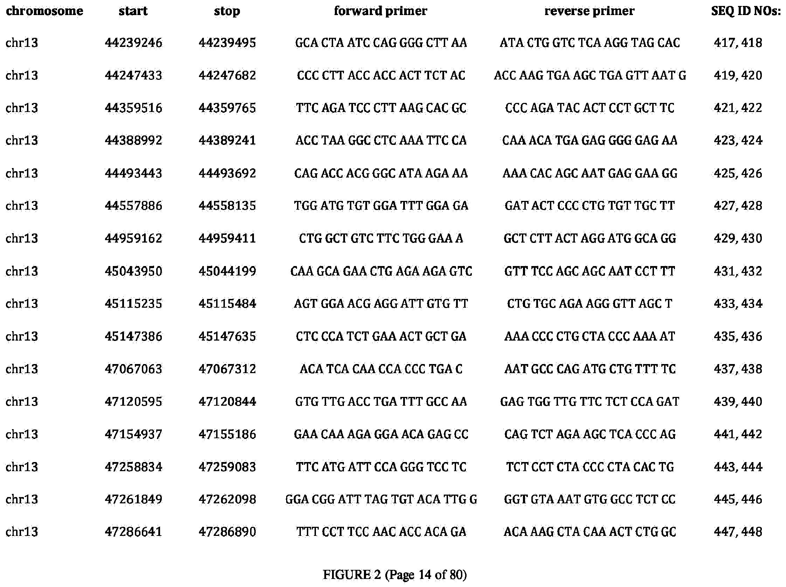

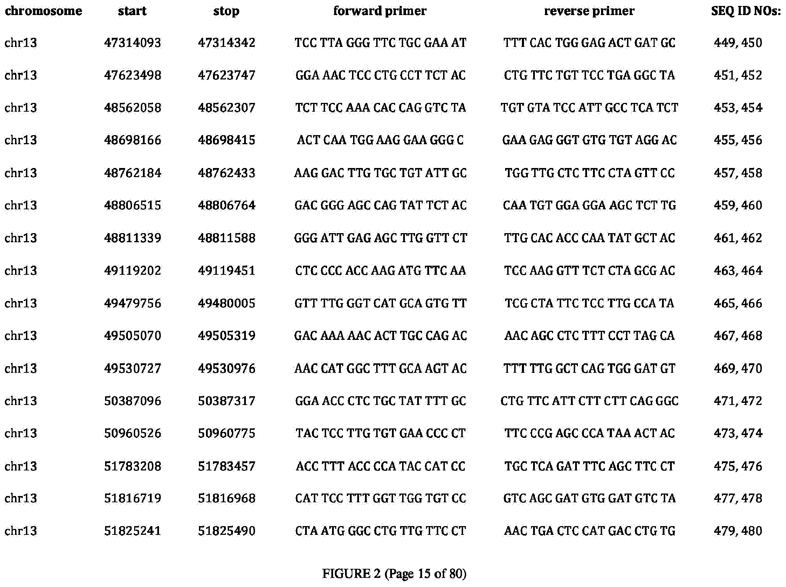

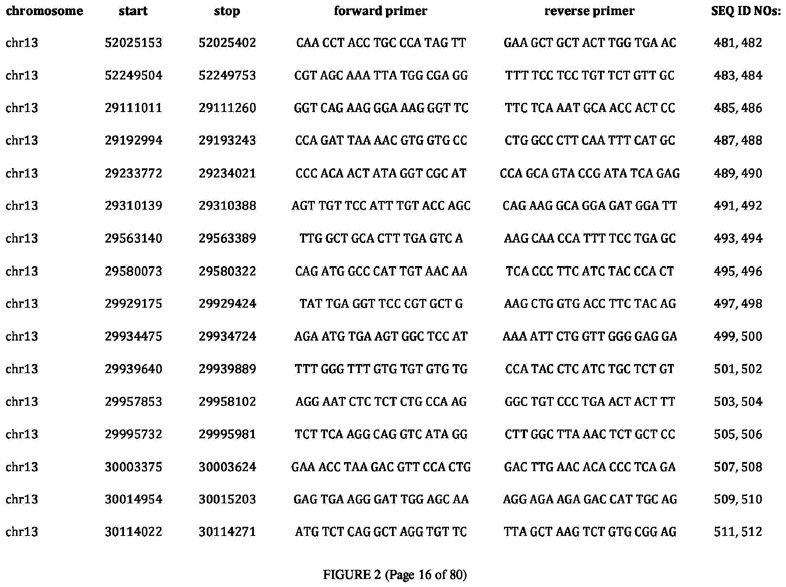

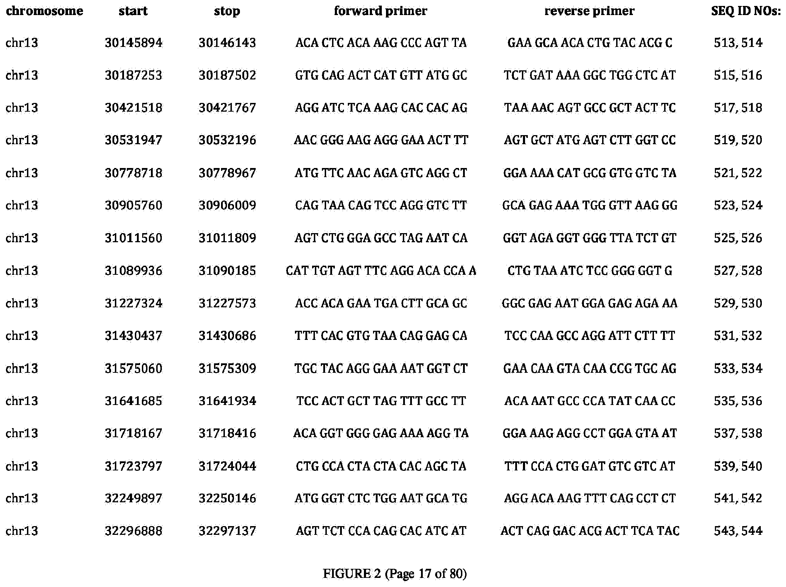

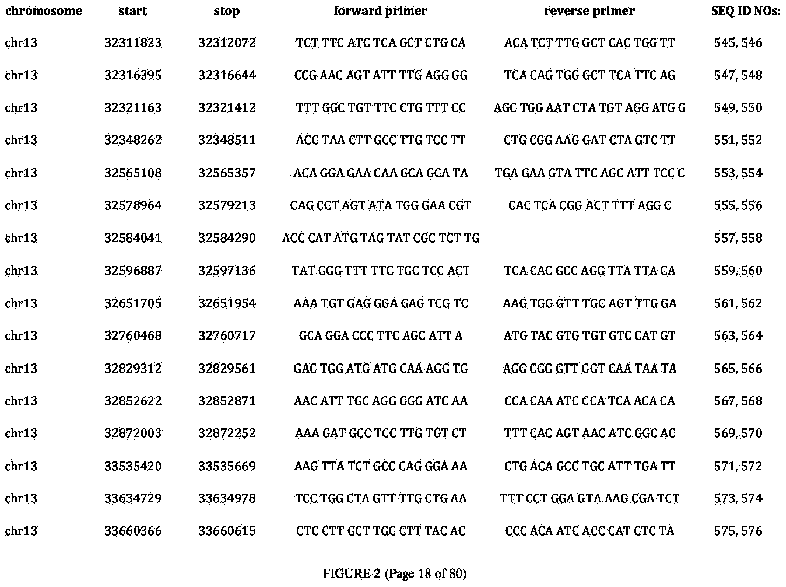

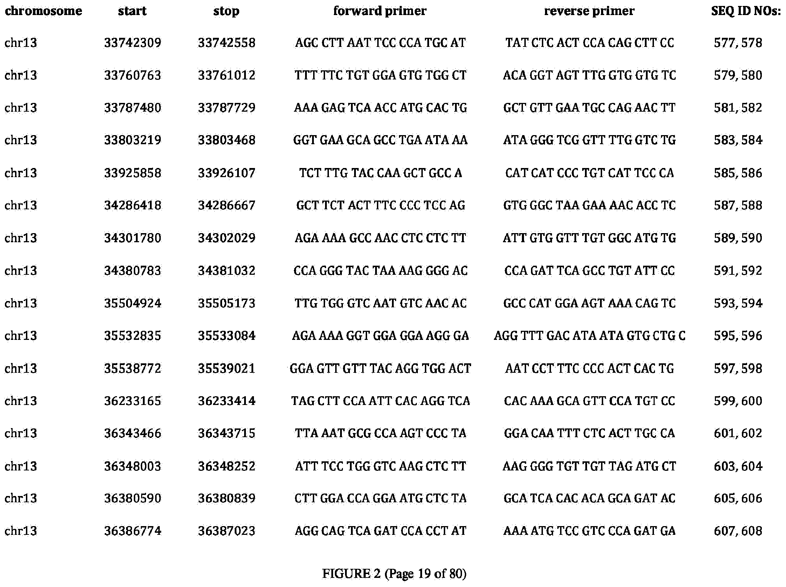

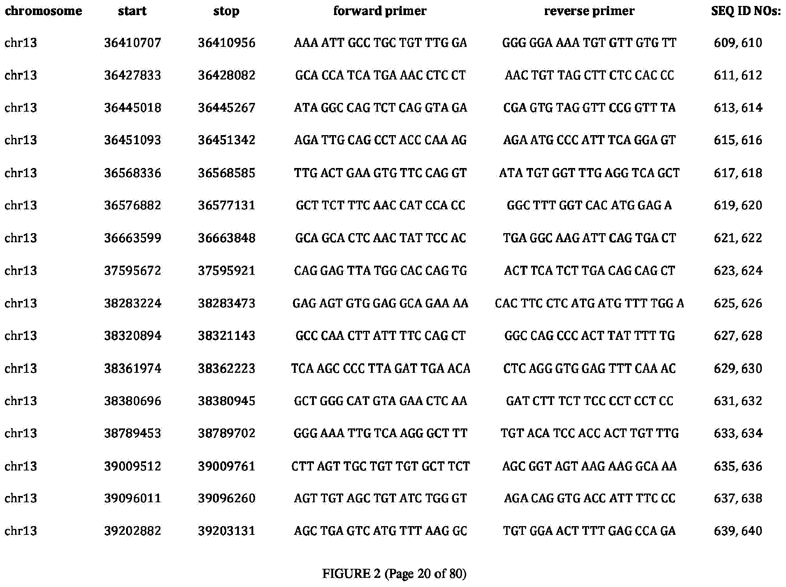

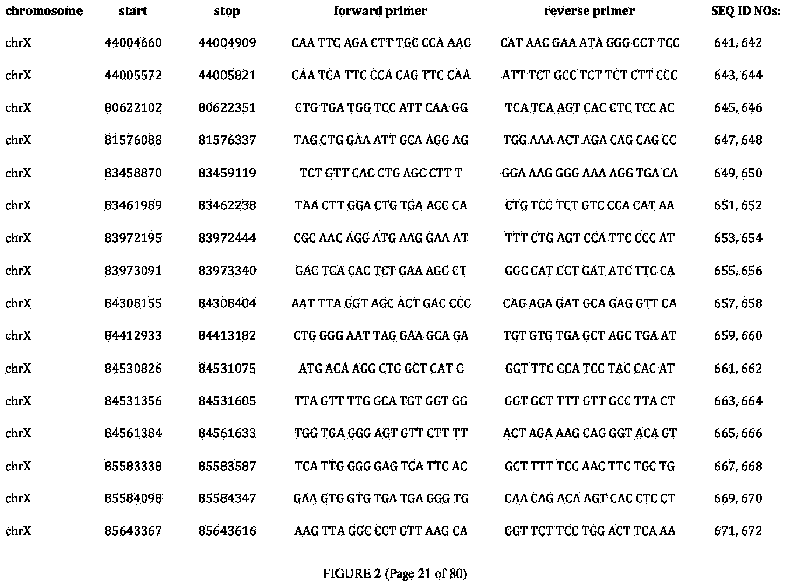

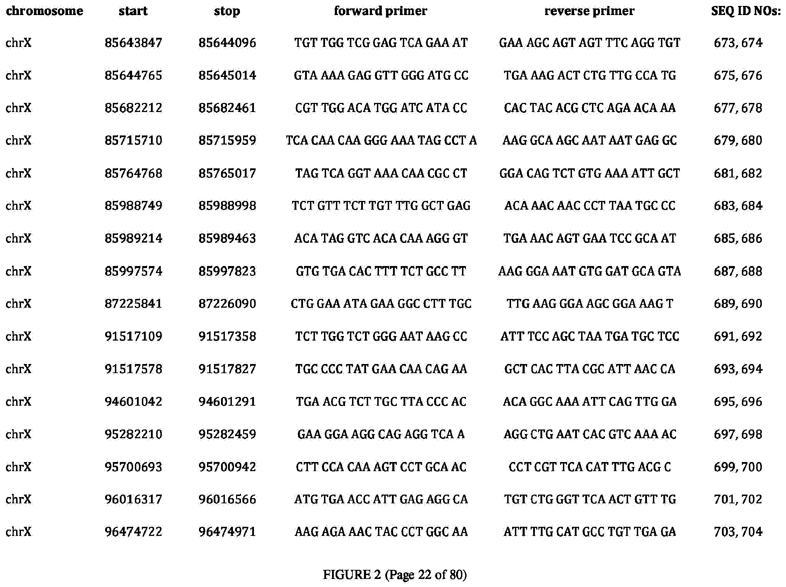

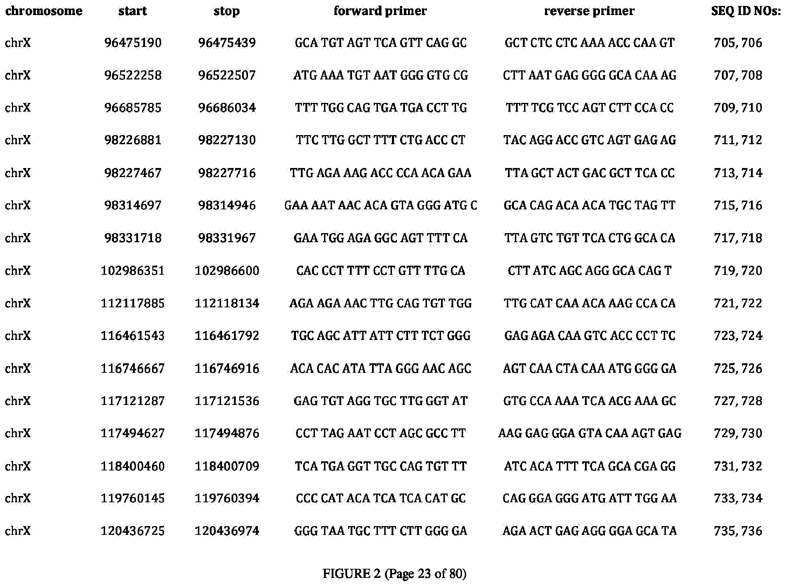

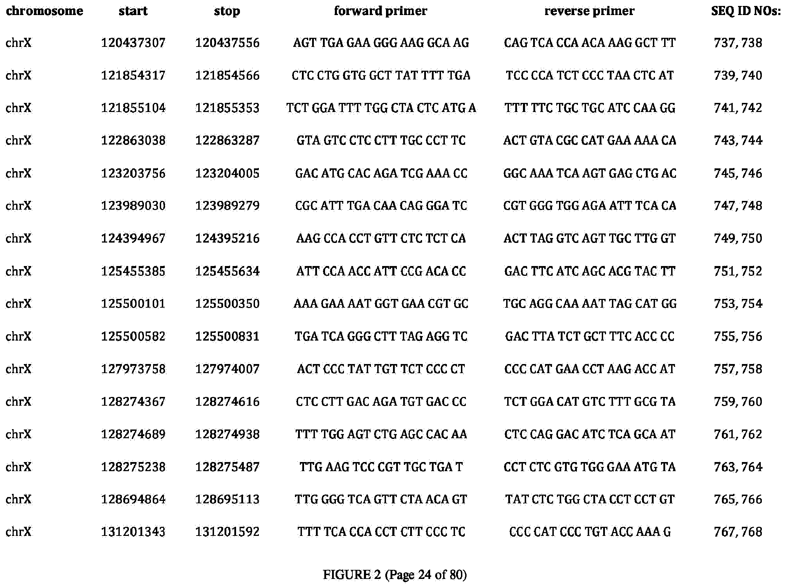

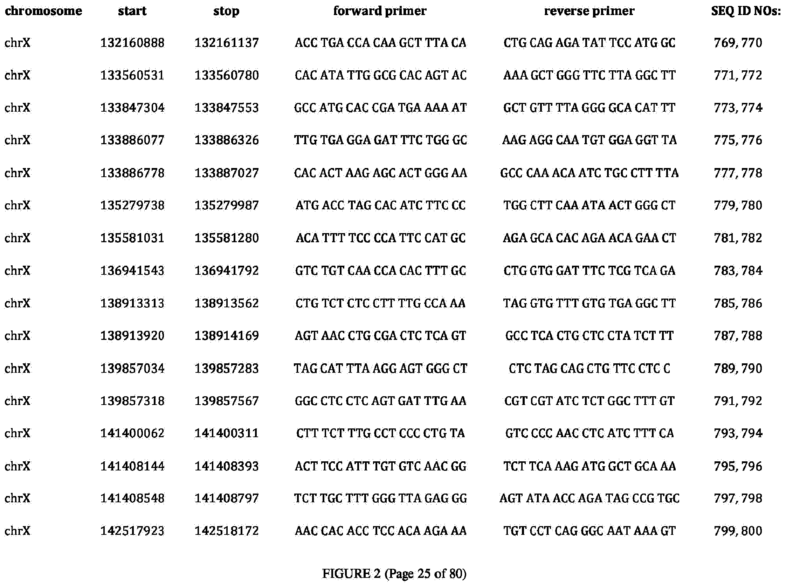

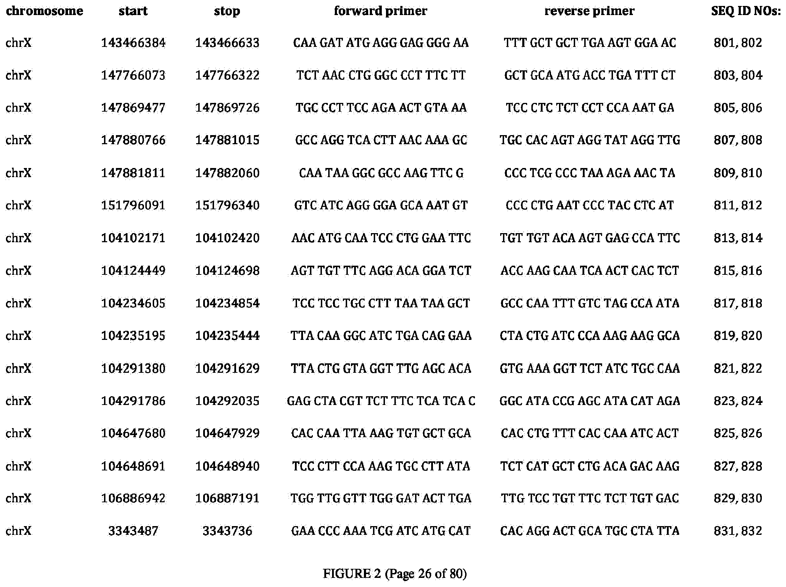

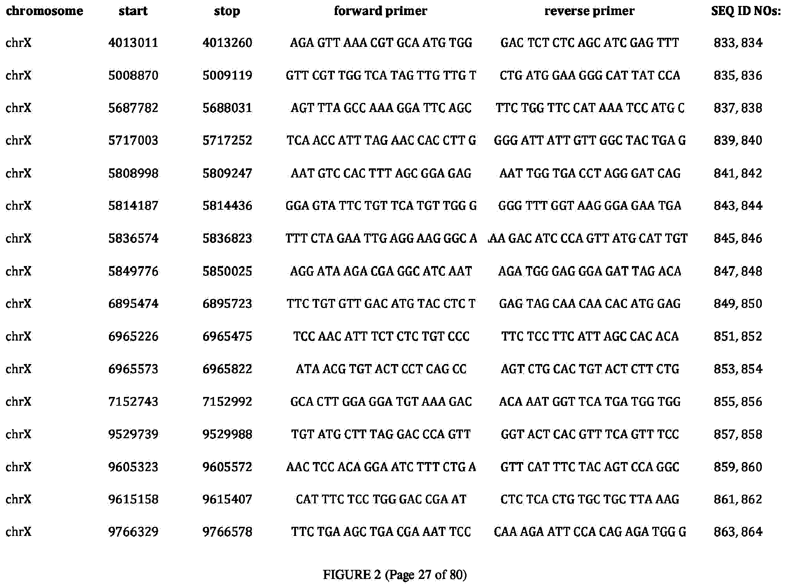

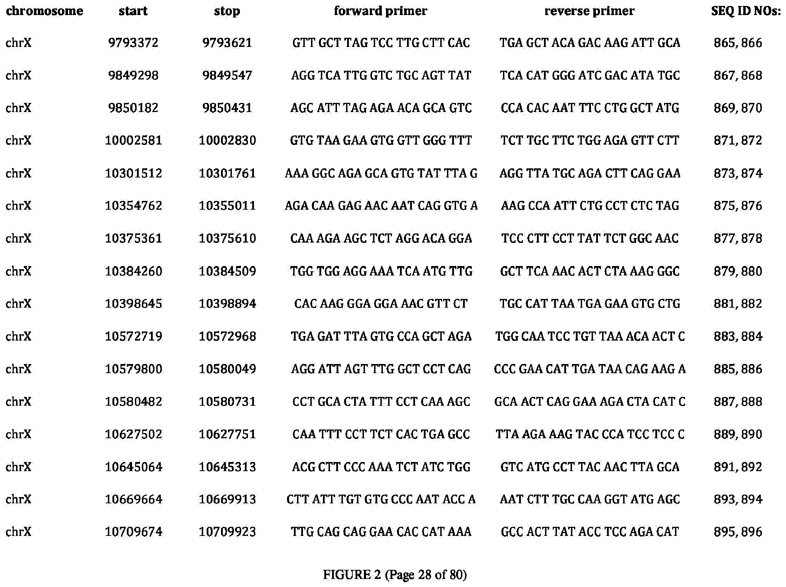

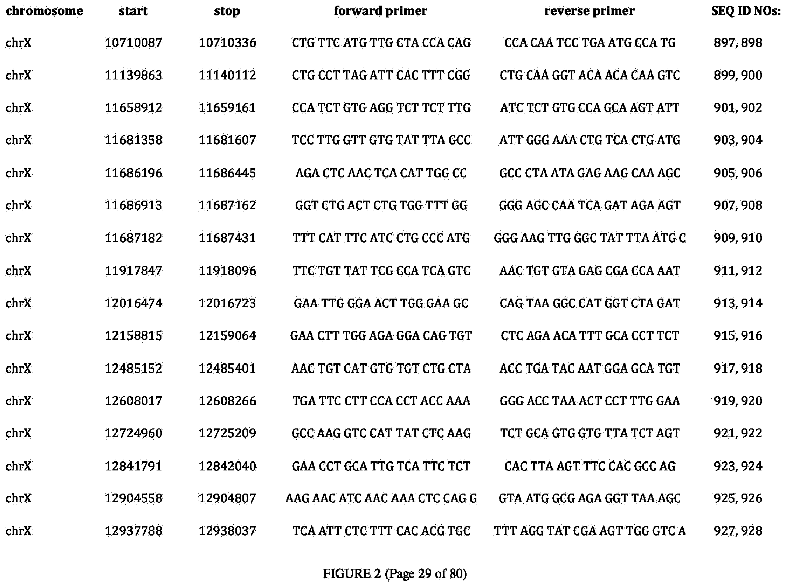

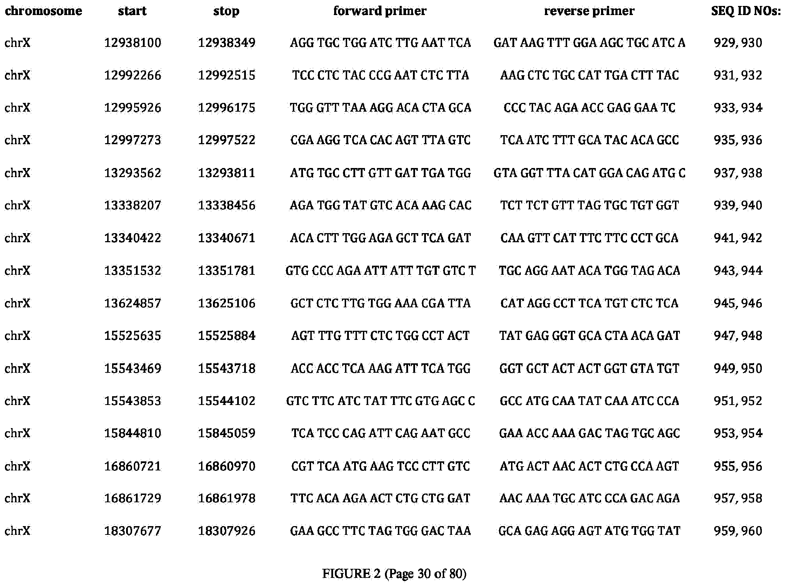

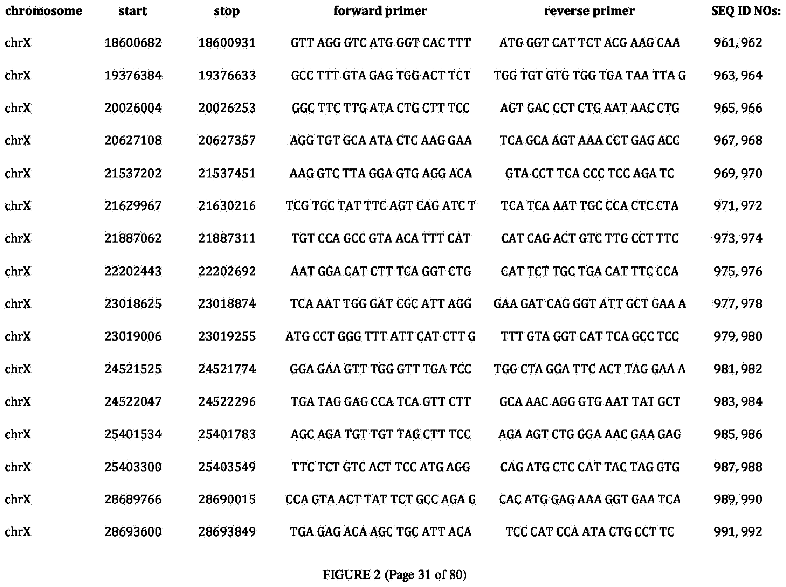

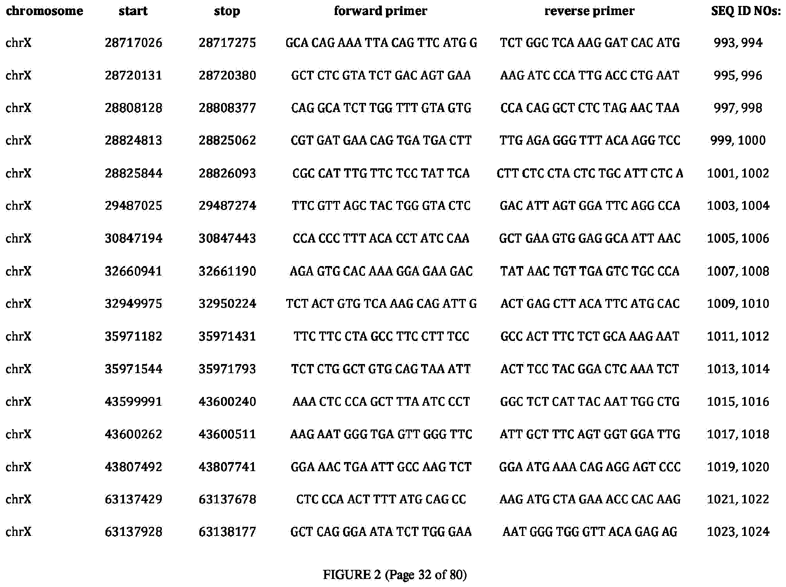

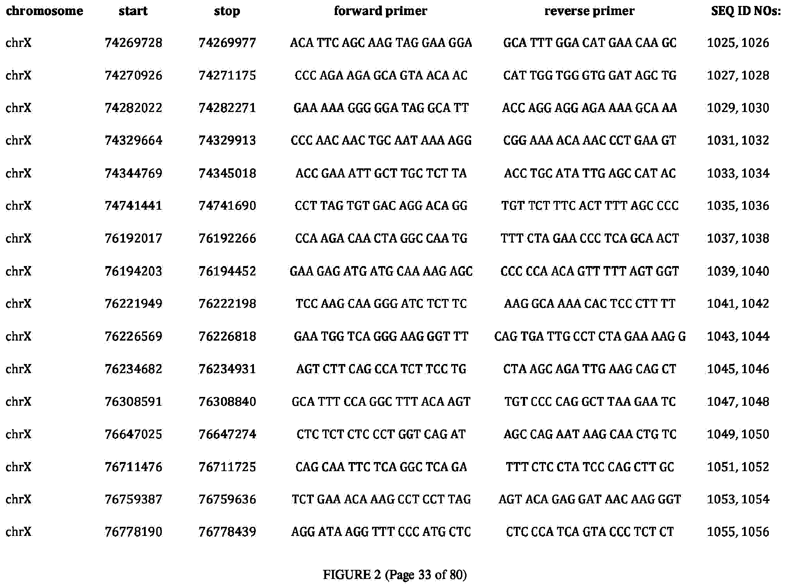

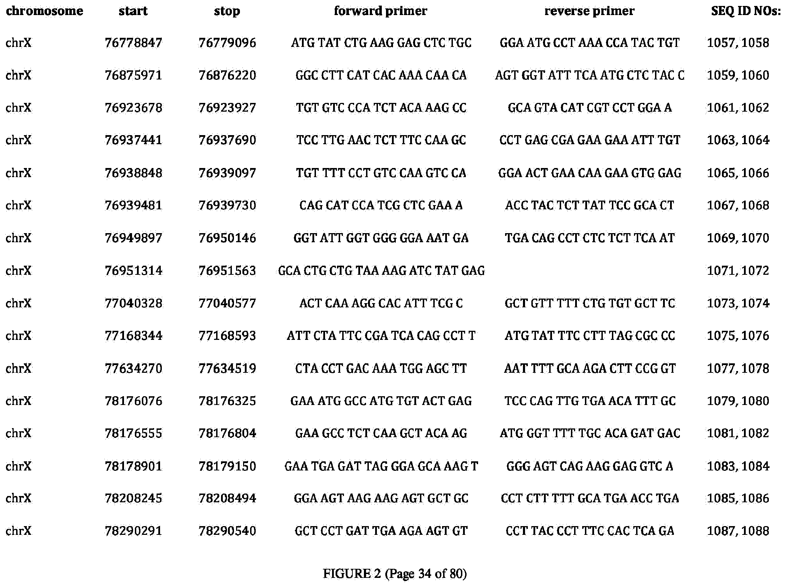

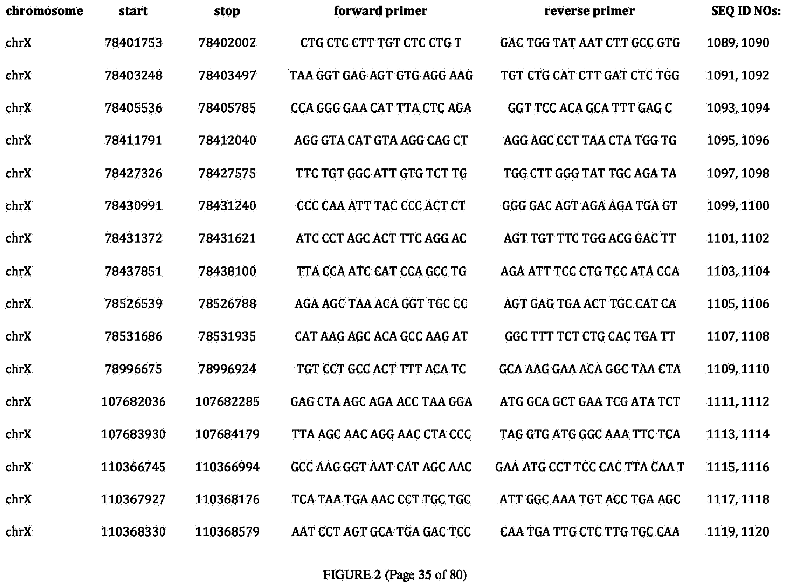

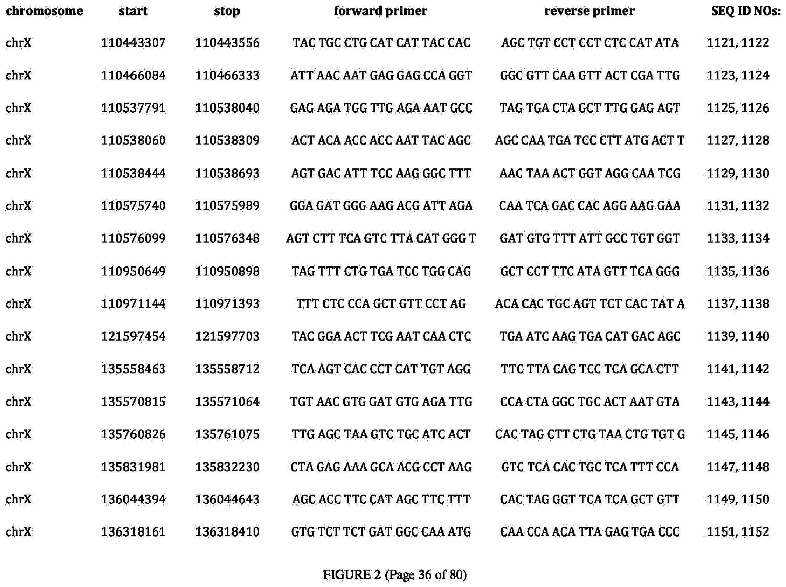

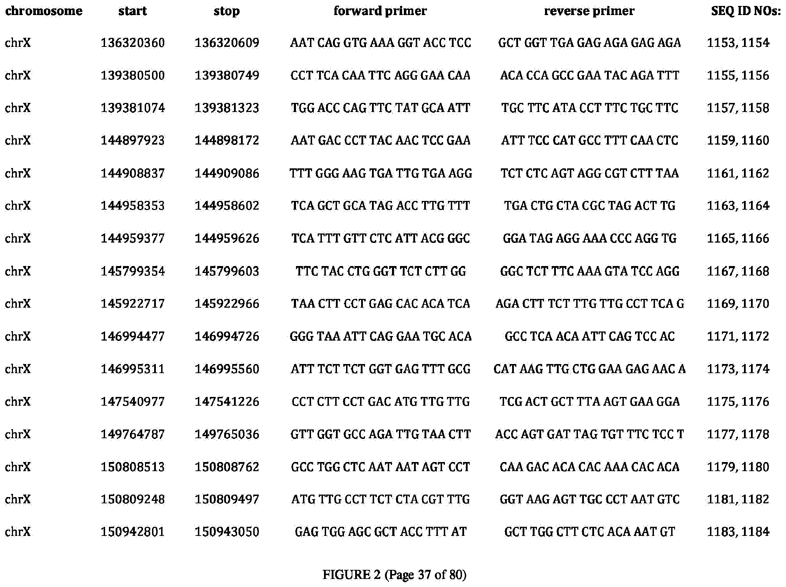

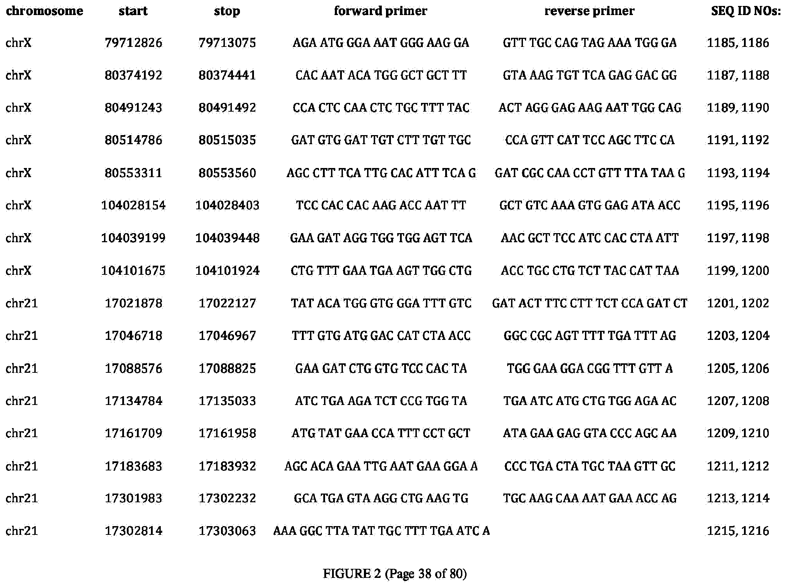

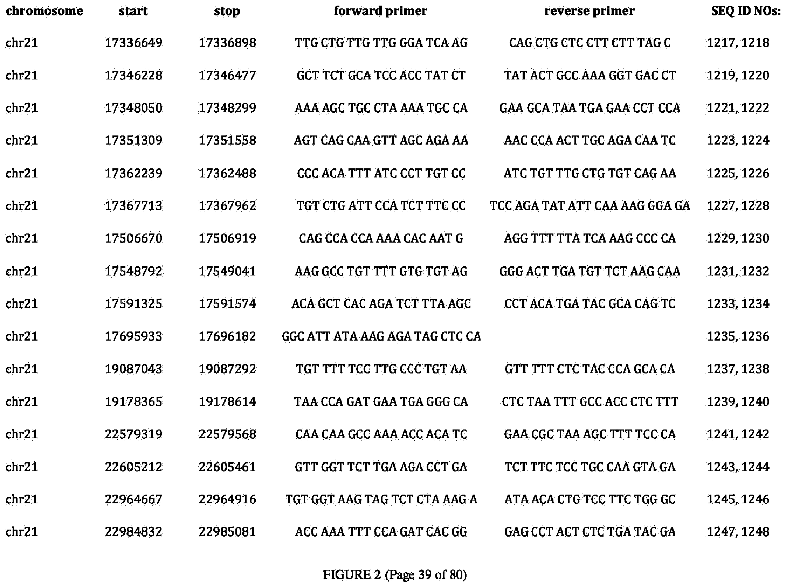

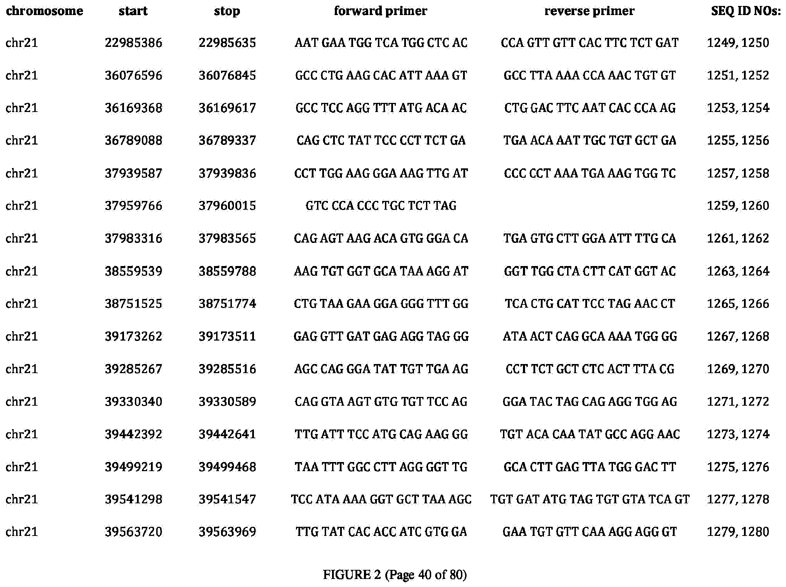

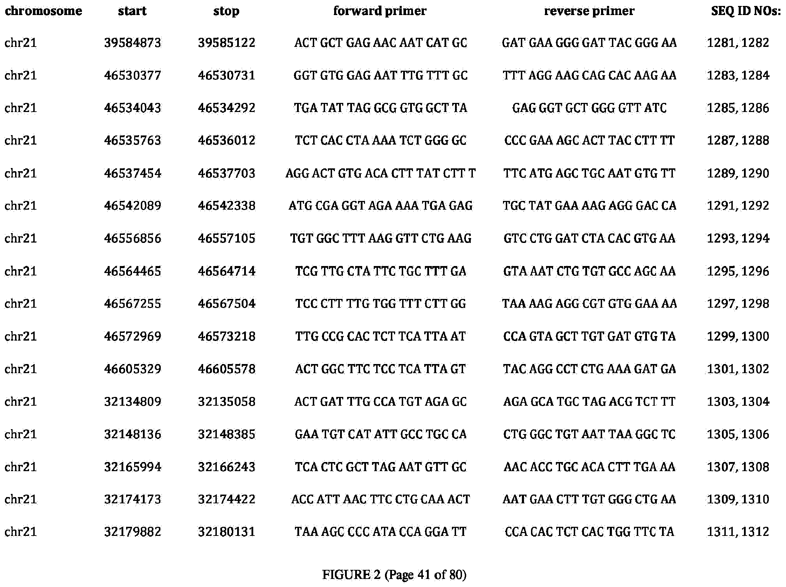

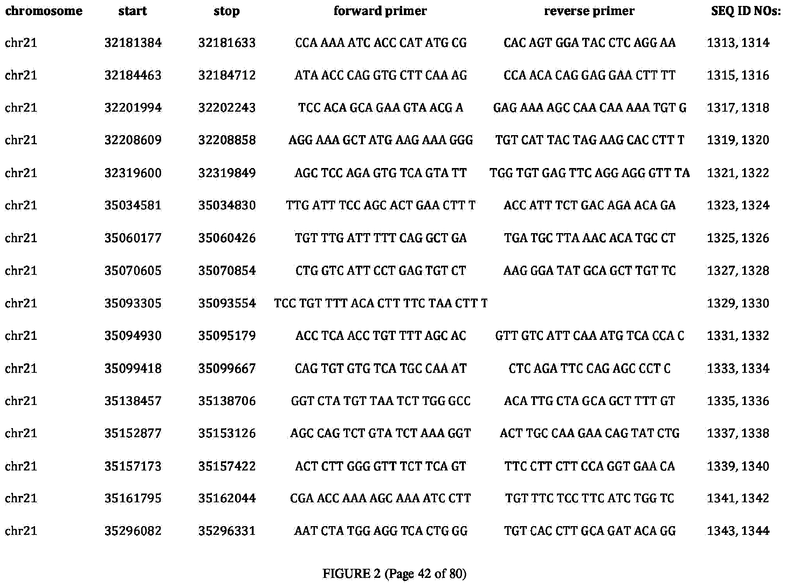

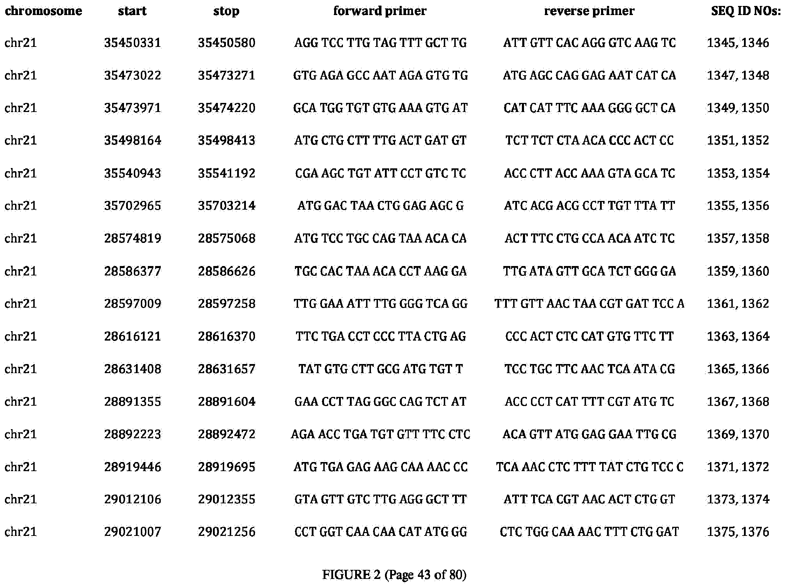

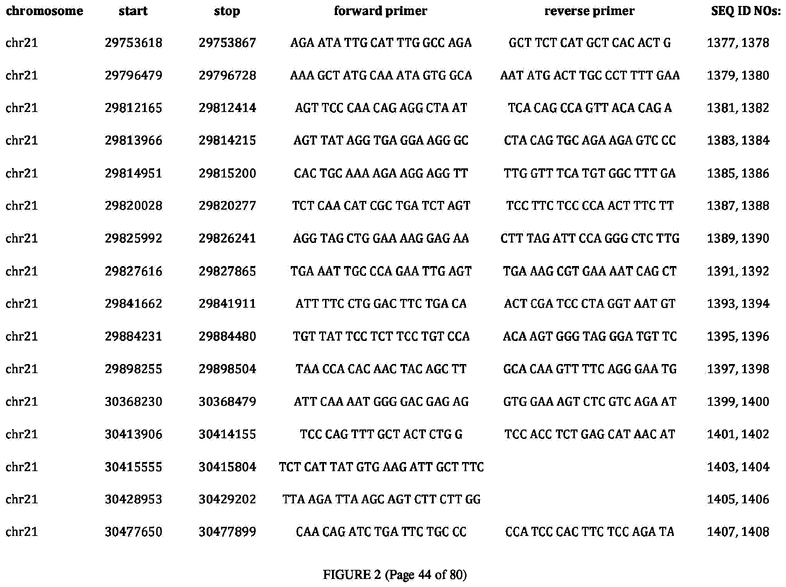

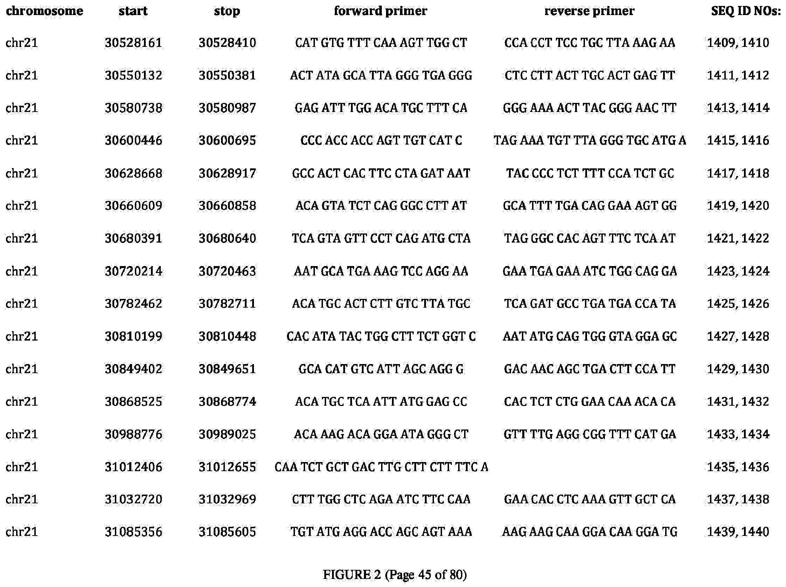

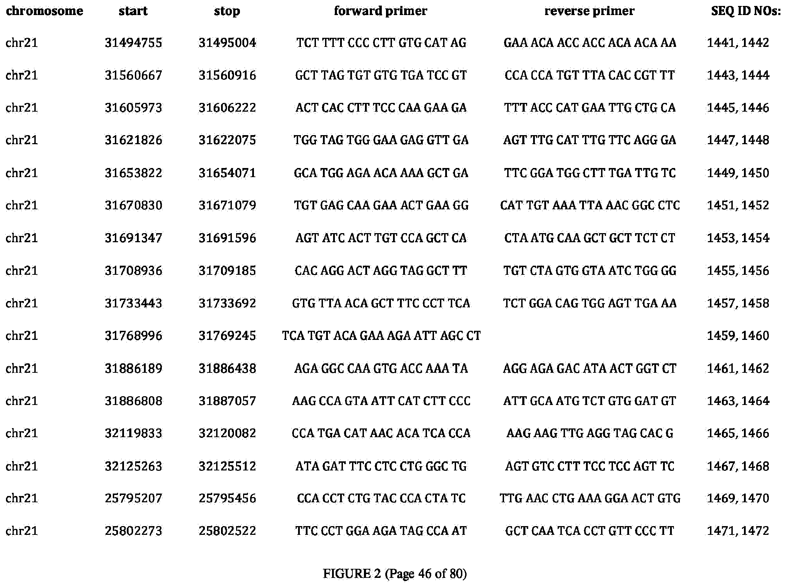

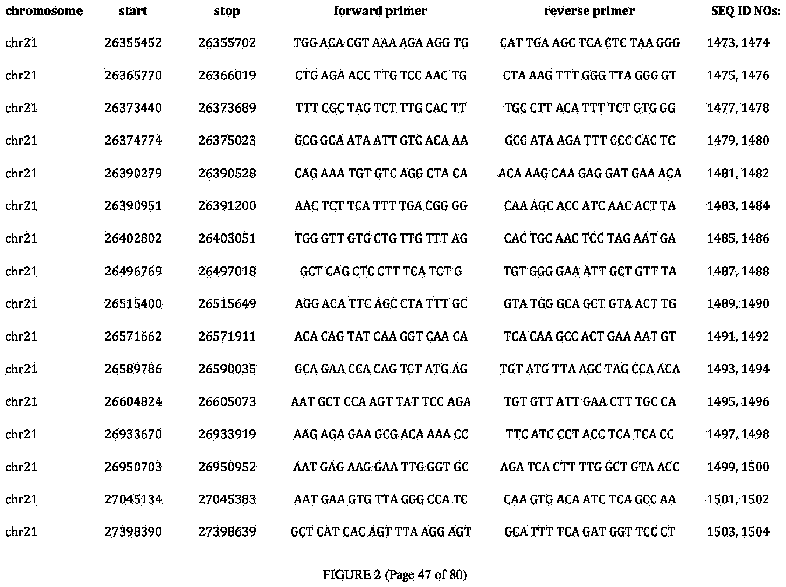

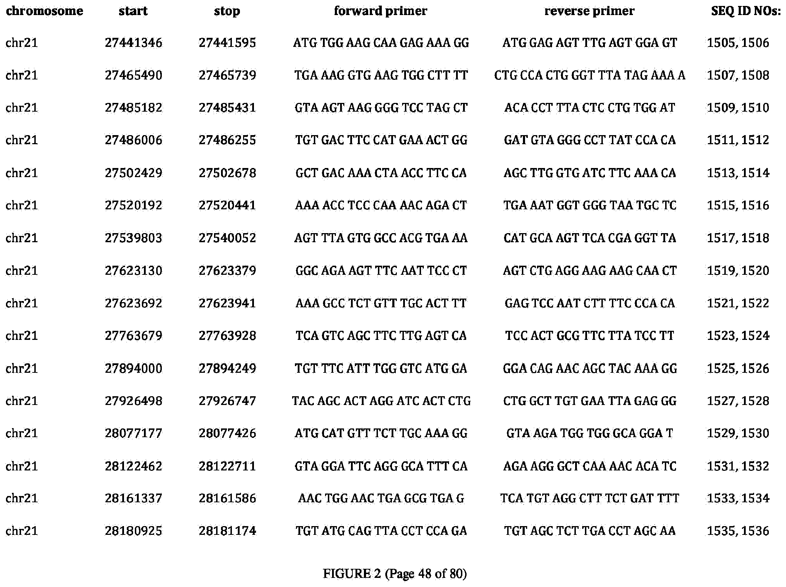

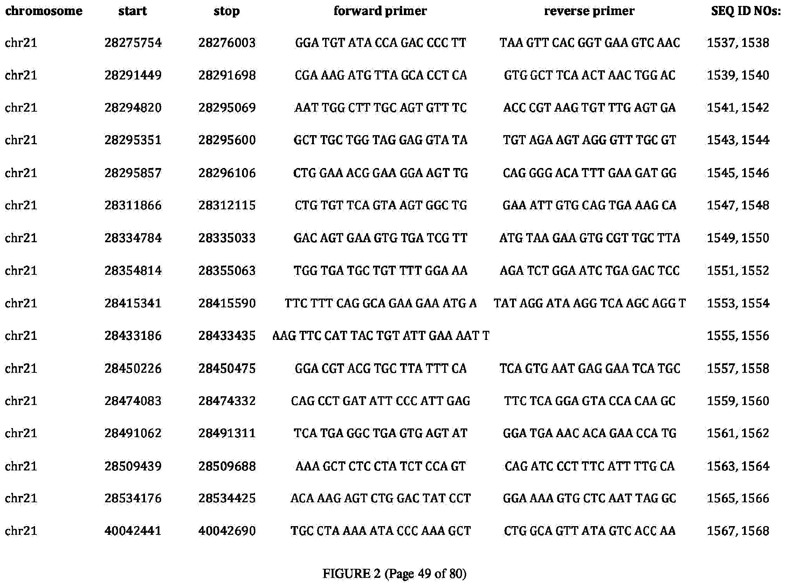

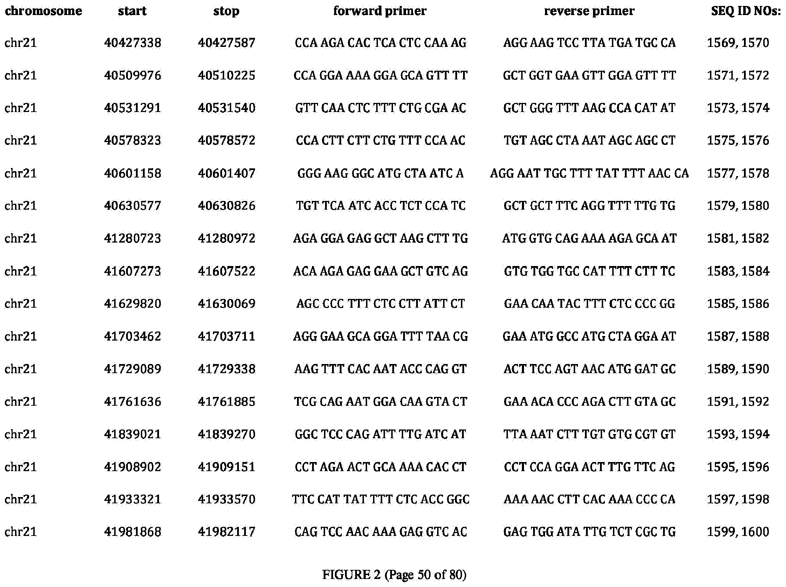

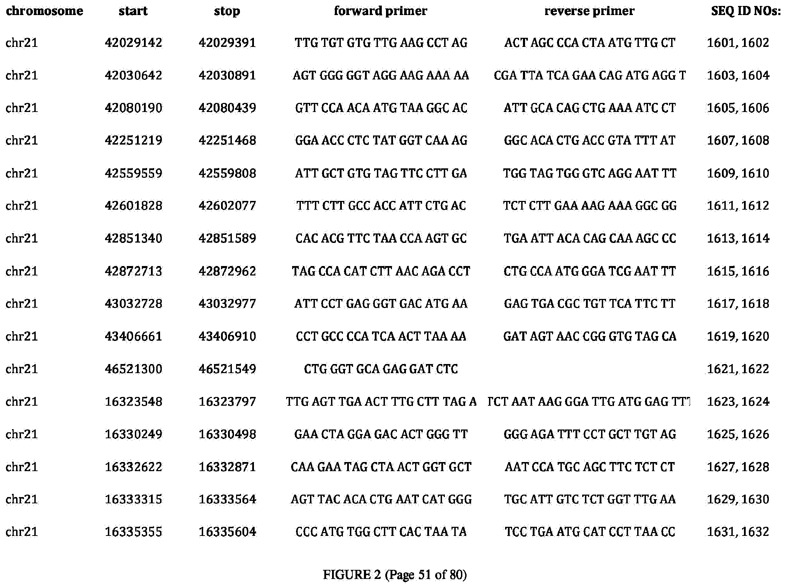

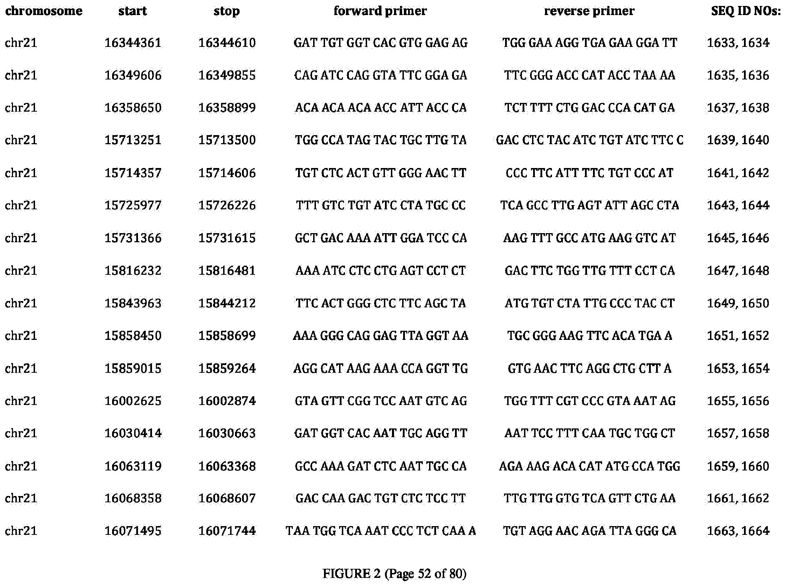

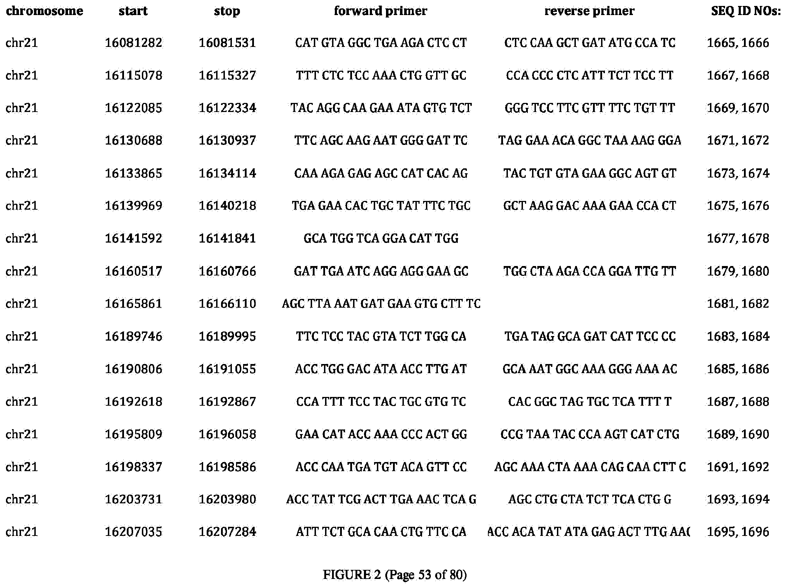

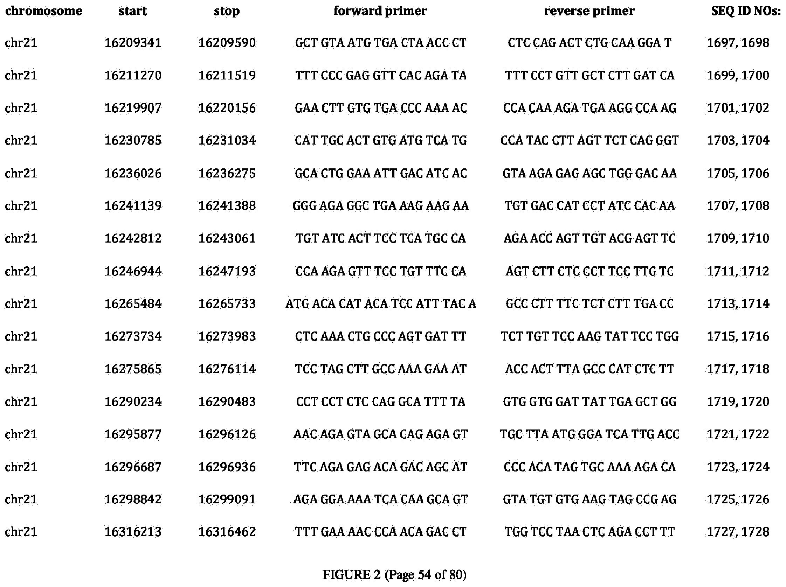

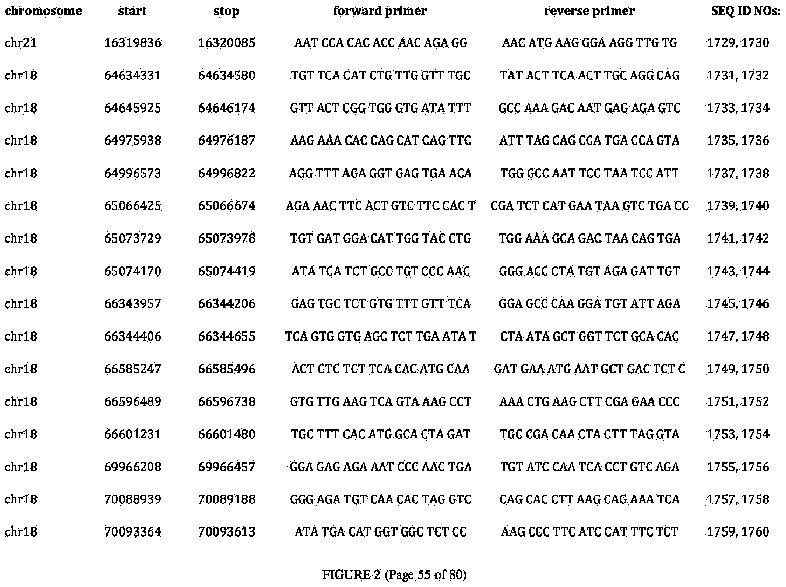

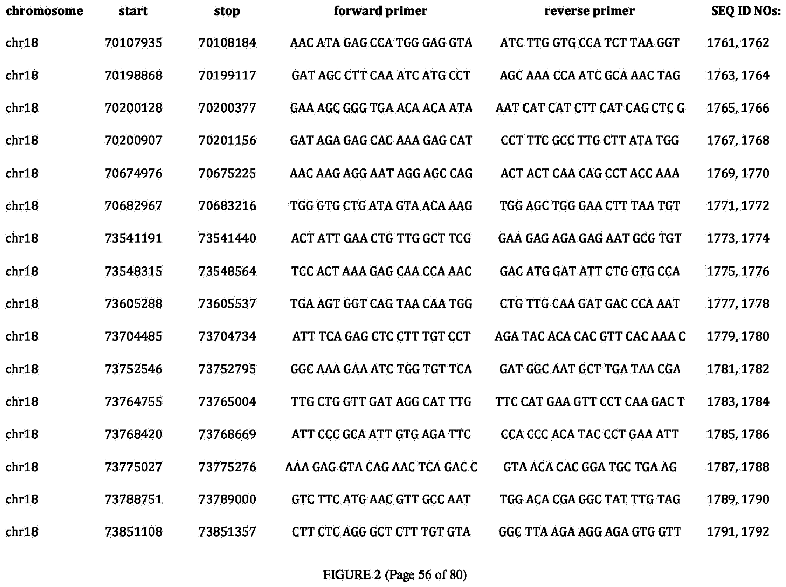

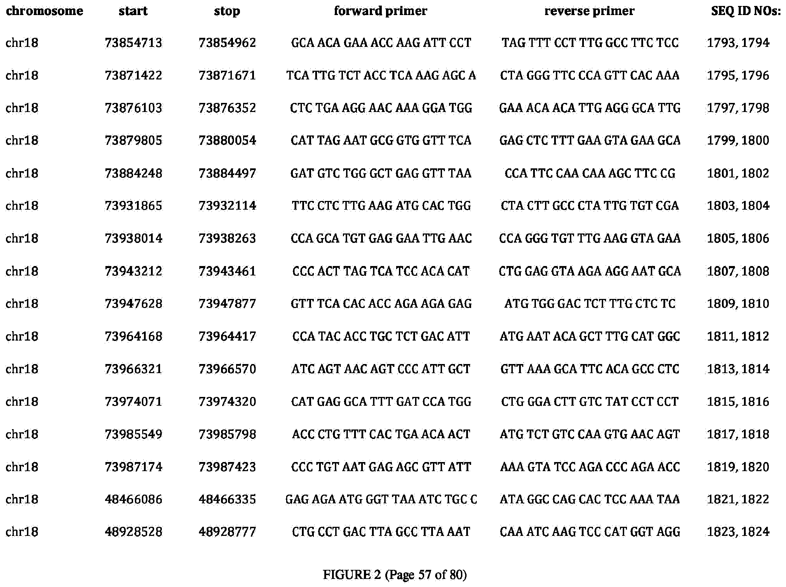

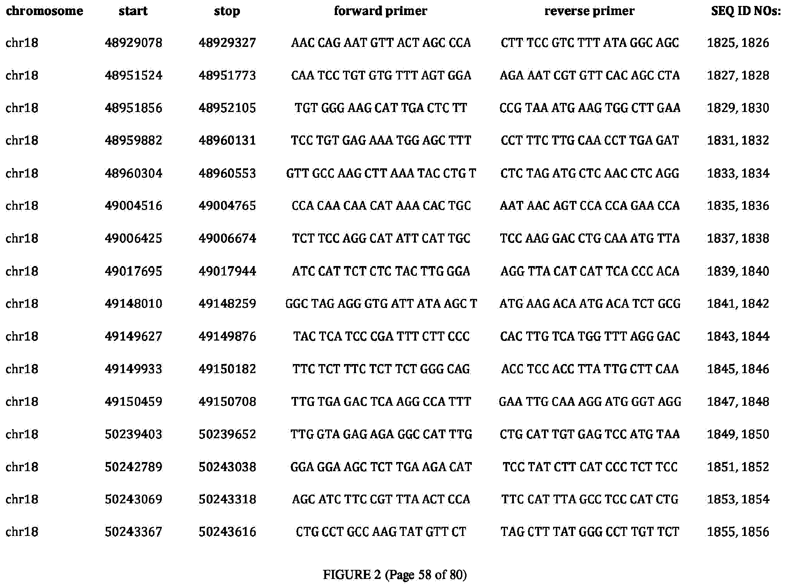

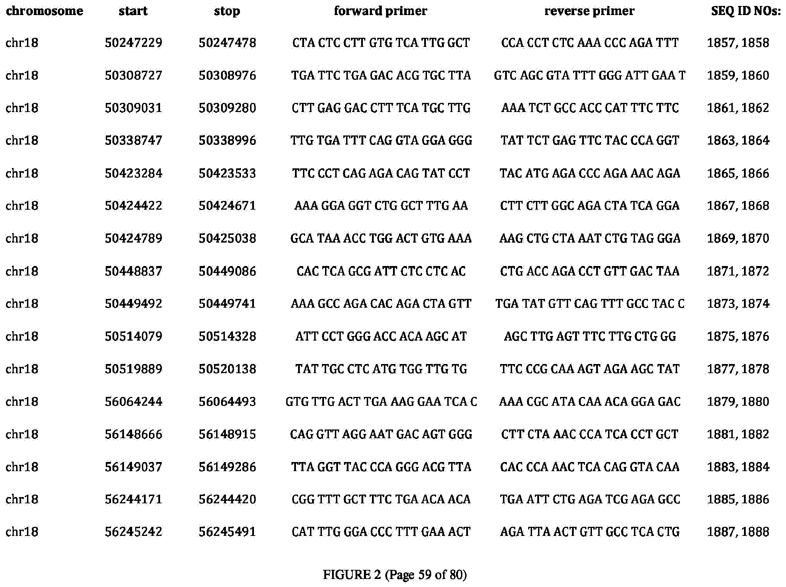

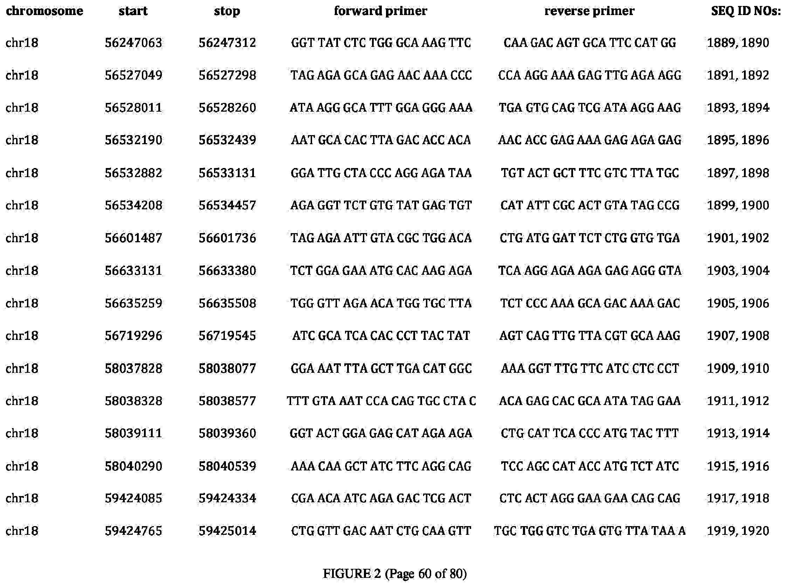

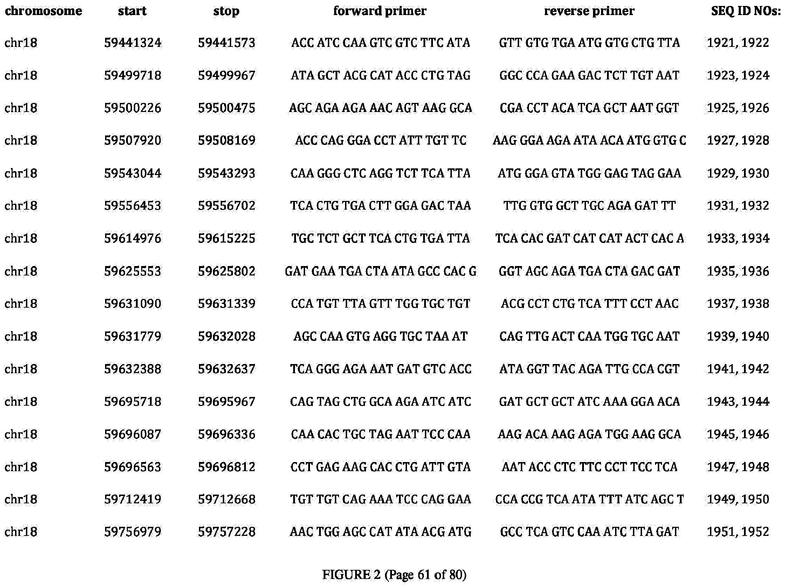

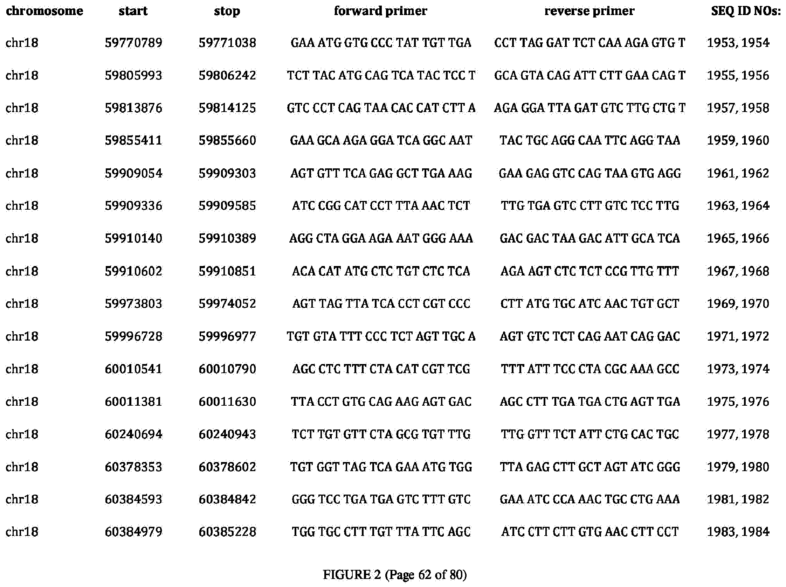

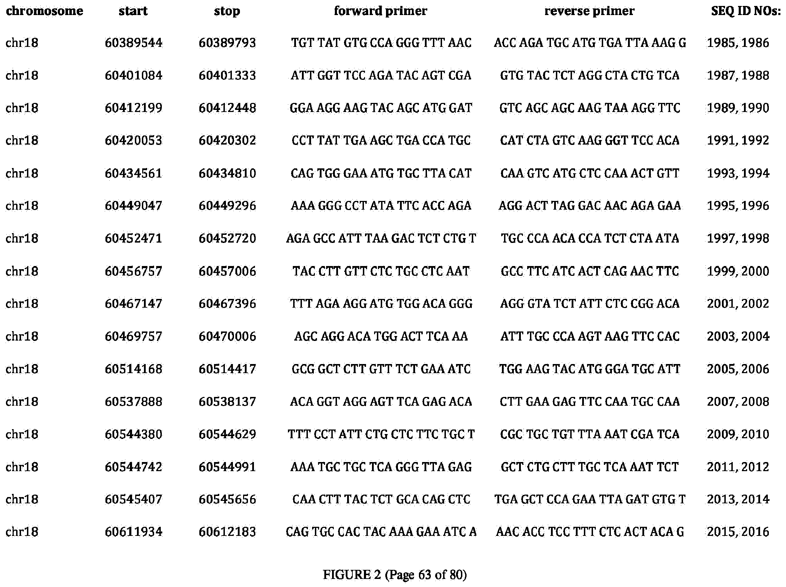

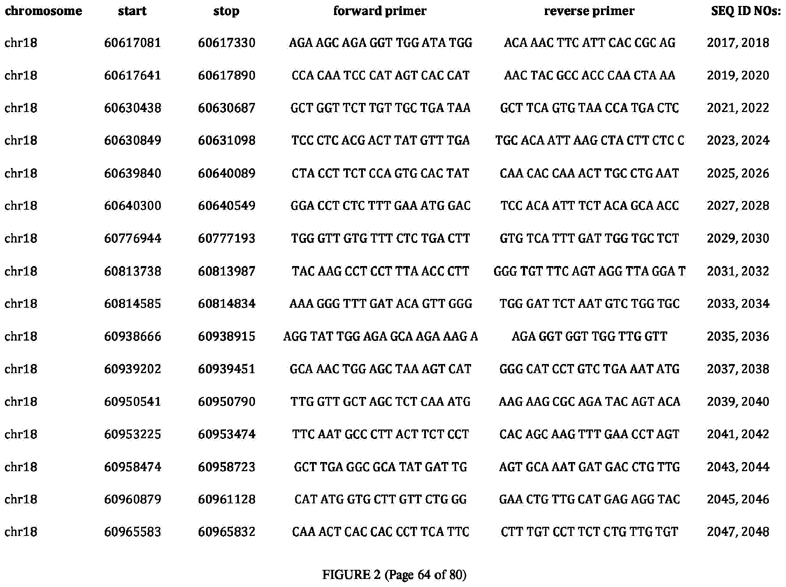

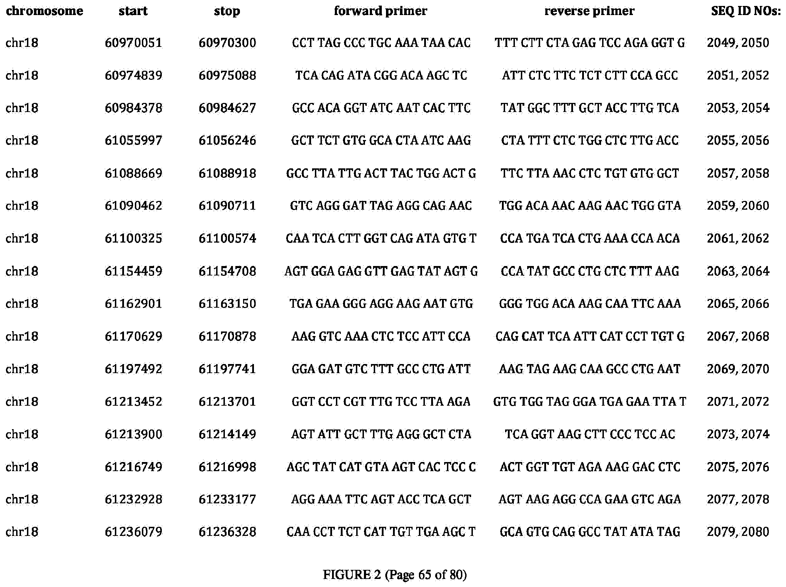

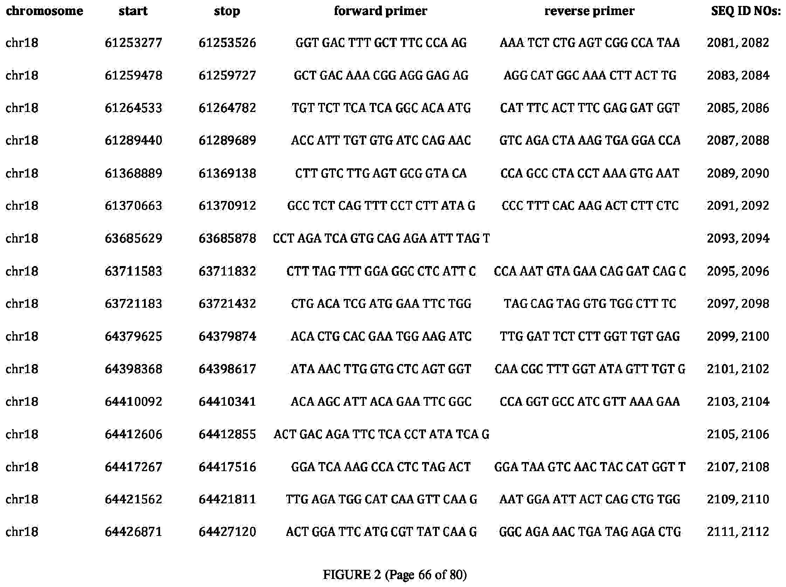

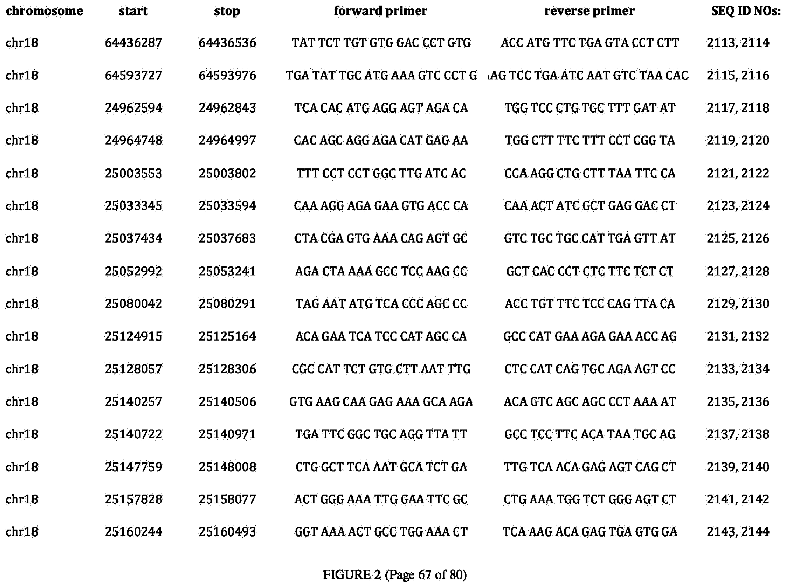

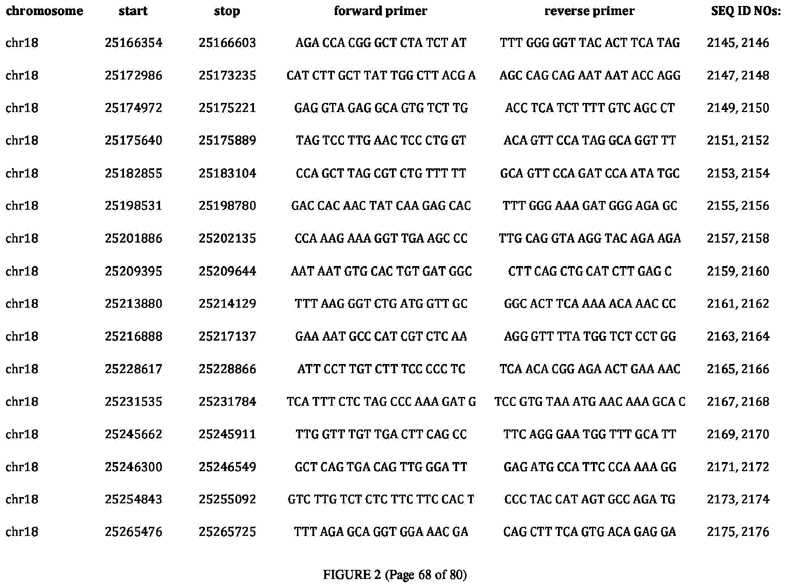

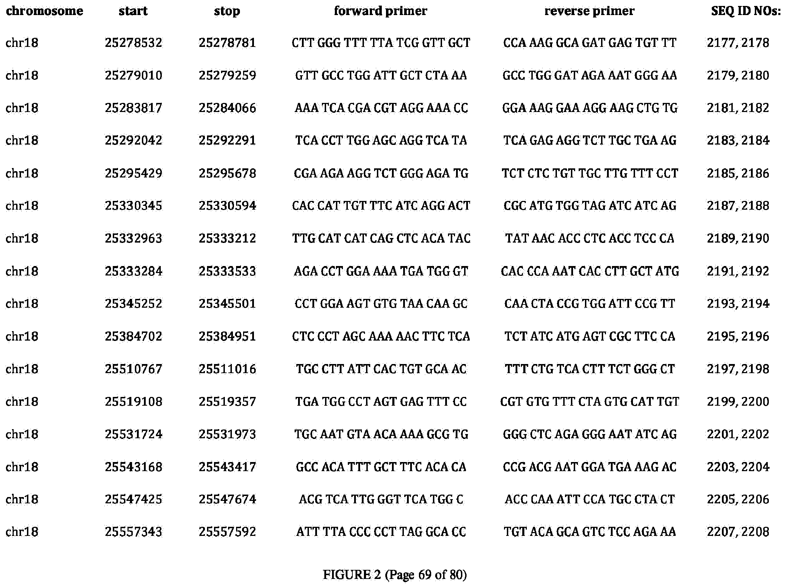

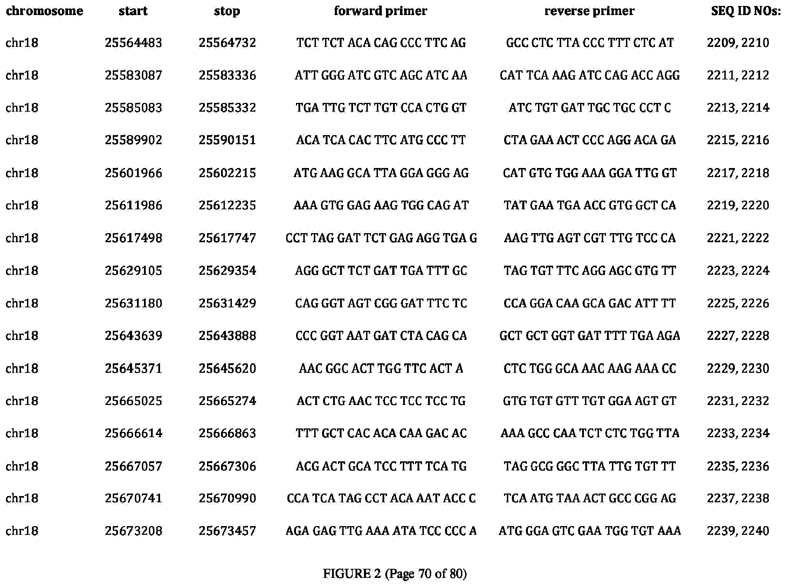

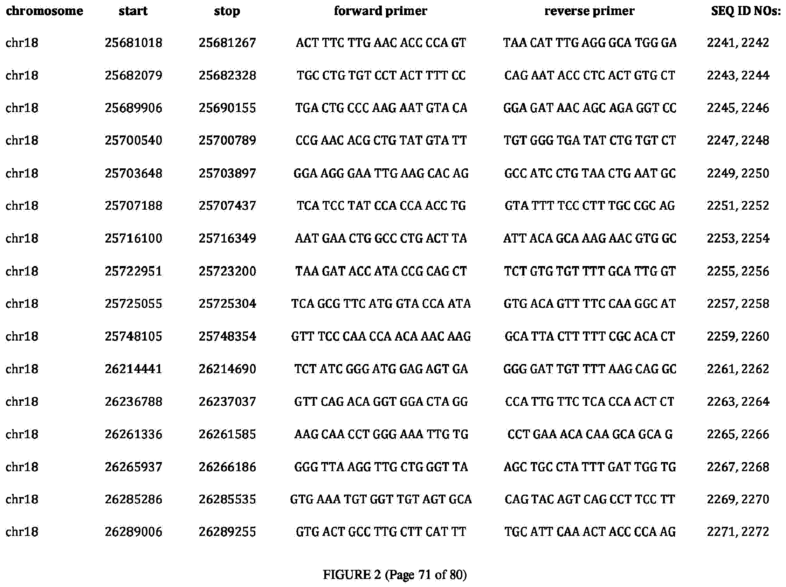

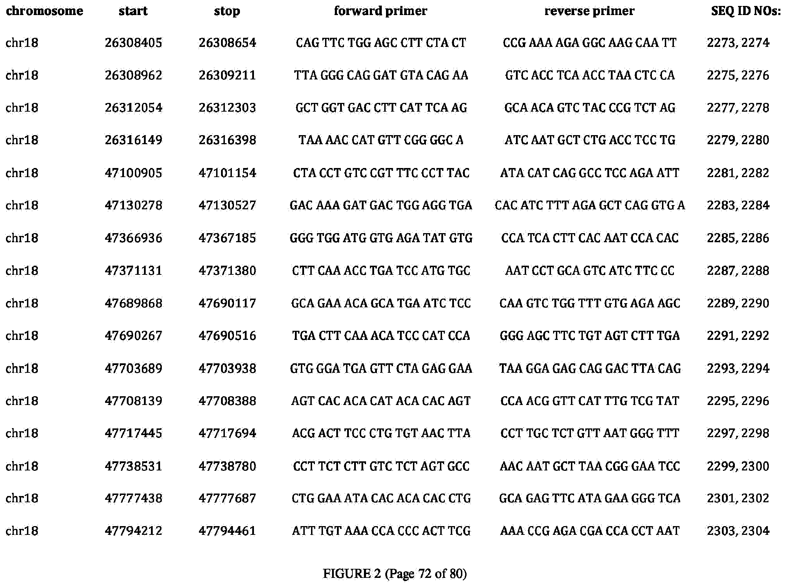

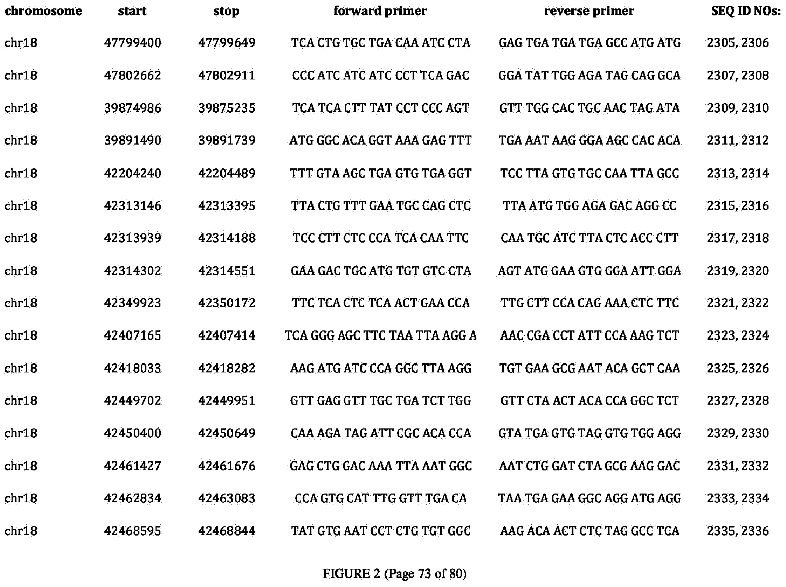

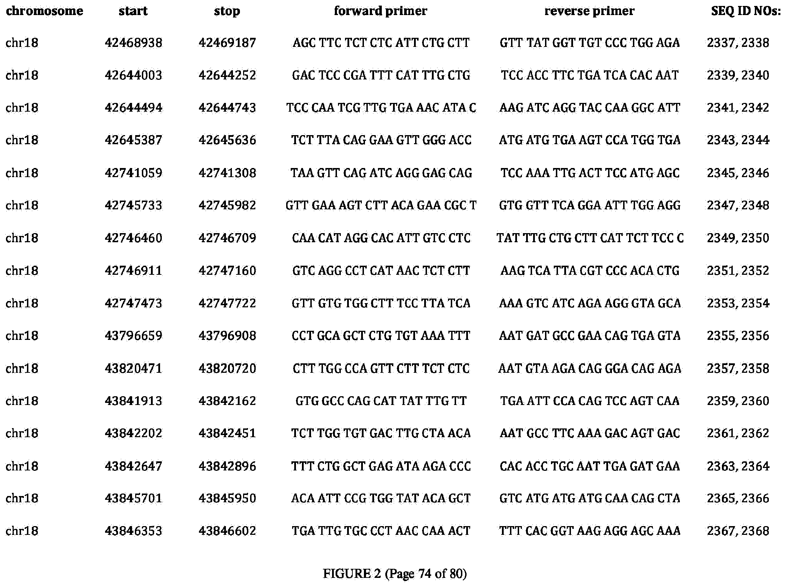

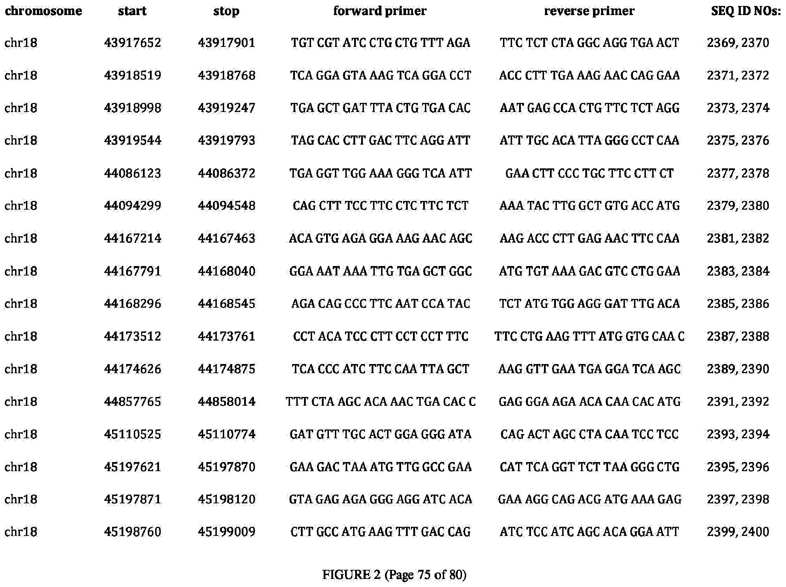

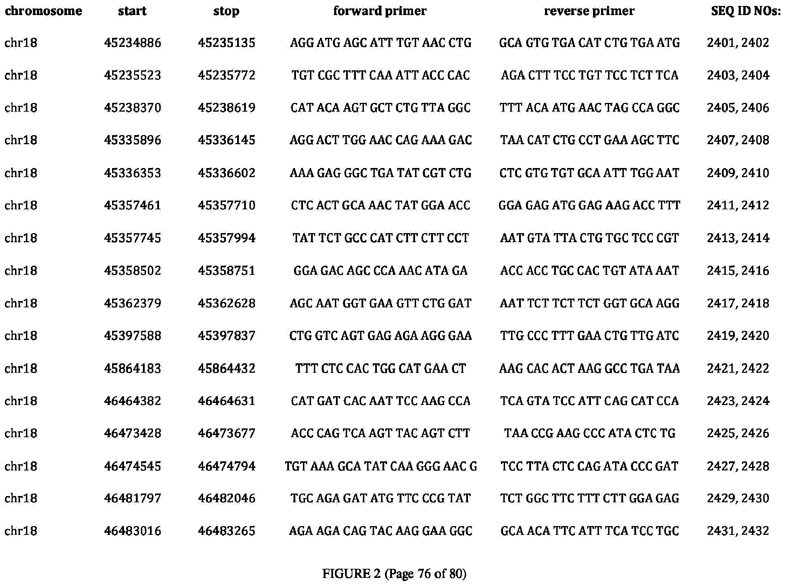

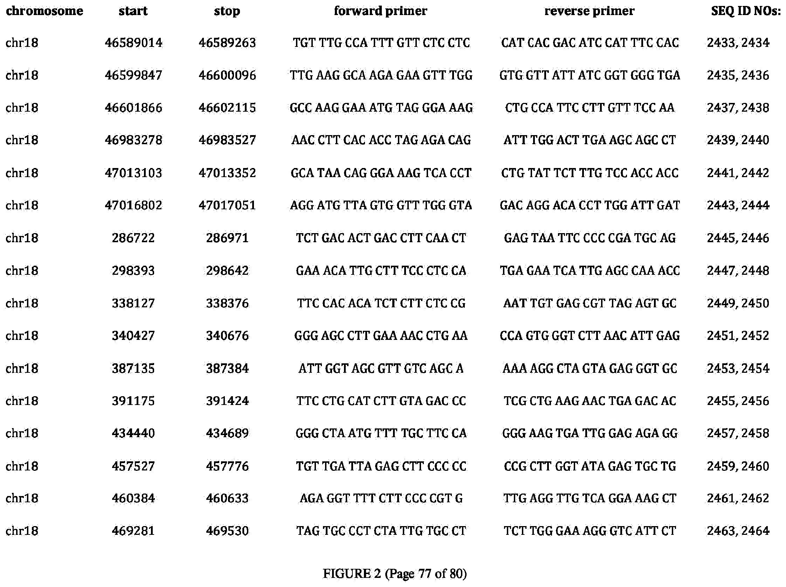

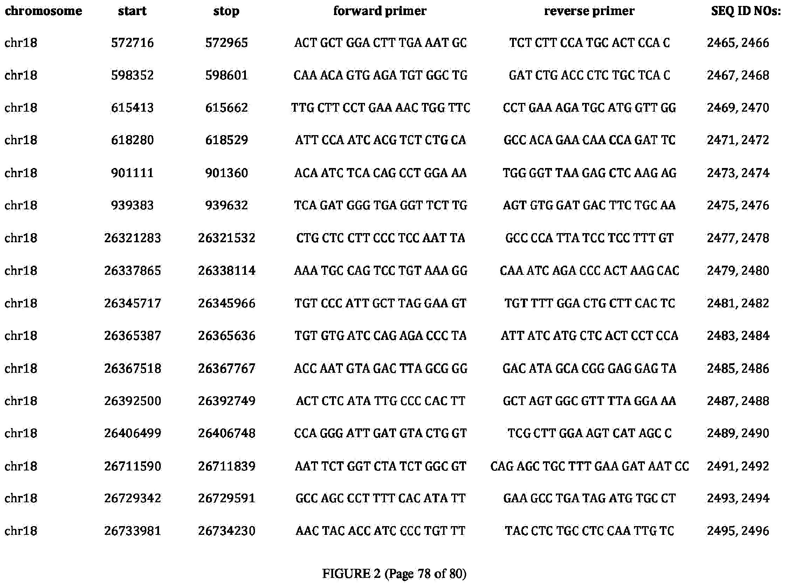

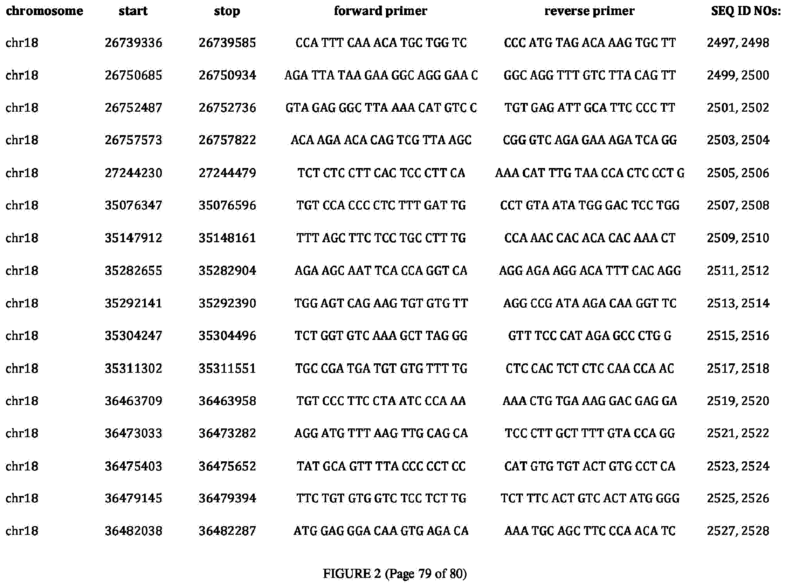

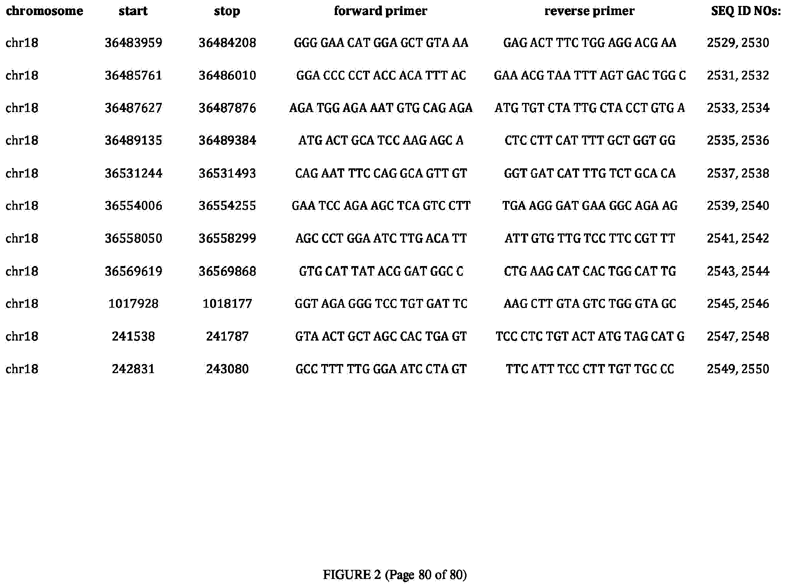

In one embodiment, the pool of TACS comprises a plurality of TACS families directed to different genomic sequences of interest. Each TACS family comprises a plurality of members that bind to the same genomic sequence of interest but having different start and/or stop positions with respect to a reference coordinate system for the genomic sequence of interest. Typically, the reference coordinate system that is used for analyzing human genomic DNA is the human reference genome built hg19, which is publically available in the art, but other coordinate systems may also be used. Alternatively, the reference coordinate system can be an artificially created genome based on built hg19 that contains only the genomic sequences of interest. Exemplary non-limiting examples of start/stop positions for TACS that bind to chromosome 13, 18, 21, X or Y are shown in Figure 2.

Each TACS family comprises at least 2 members that bind to the same genomic sequence of interest. In various embodiments, each TACS family comprises at least 2 member sequences, or at least 3 member sequences, or at least 4 member sequences, or at least 5 member sequences, or at least 6 member sequences, or at least 7 member sequences, or at least 8 member sequence, or at least 9 member sequences, or at least 10 member sequences. In various embodiments, each TACS family comprises 2 member sequences, or 3 member sequences, or 4 member sequences, or 5 member sequences, or 6 member sequences, or 7 member sequences, or 8 member sequences, or 9 member sequences, or 10 member sequences. In various embodiments, the plurality of TACS families comprises different families having different numbers of member sequences. For example, a pool of TACS can comprise one TACS family that comprises 3 member sequences, another TACS family that comprises 4 member sequences, and yet another TACS family that comprises 5 member sequences, and the like. In one embodiment, a TACS family comprises 3-5 member sequences. In another embodiment, the TACS family comprises 4 member sequences.

The pool of TACS comprises a plurality of TACS families. Thus, a pool of TACS comprises at least 2 TACS families. In various embodiments, a pool of TACS comprises at least 3 different TACS families, or at least 5 different TACS families, or at least 10 different TACS families, or at least 50 different TACS families, or at least 100 different TACS families, or at least 500 different TACS families, or at least 1000 different TACS families, or at least 2000 TACS families, or at least 4000 TACS families, or at least 5000 TACS families.

Each member within a family of TACS binds to the same genomic region of interest but with different start and/or stop positions, with respect to a reference coordinate system for the genomic sequence of interest, such that the binding pattern of the members of the TACS family is staggered (see Figure 3). In various embodiments, the start and/or stop positions are staggered by 5-10 base pairs. In one embodiment, the start and/or stop positions are staggered by 5 base pairs. In another embodiment, the start and/or stop positions are staggered by 10 base pairs.

### Sample Collection and Preparation

The methods of the invention can be used with a variety of biological samples that contain only or predominantly fetal or embryonic DNA. As used herein, a sample containing "predominantly fetal or embryonic DNA" is one that contains more than 50% fetal or embryonic DNA, and typically contains more than 90%, or 95% or 99% fetal or embryonic DNA. In one embodiment, the source of the sample that contains predominantly fetal or embryonic DNA is fetal or embryonic cells obtained from embryo biopsy of *in vitro* fertilized (IVF) pre-implantation embryos. It has been demonstrated that intact cells can be obtained from IVF pre-implantation embryos for Pre-implantation Genetic Screening (PGS) and Pre-implantation Genetic Diagnosis (PGD) processes. An ovum is fertilized through IVF and resulting cells are collected during *in vitro* growth of the embryo. For example, cells can be collected from a day 3 embryo or a day 5 embryo. Typically, if cell harvesting is performed at day 3 a single fetal cell is obtained, also known as a blastomere, and if harvesting is performed at day 5 a few cells are obtained, also known as trophectoderm cells. Typically, the genetic integrity of the grown fetal cells is interrogated using array Comparative Genomic Hybridization (aCGH), a technology that can detect genetic abnormalities of a certain genomic size and above. The method of the disclosure provides an alternative means for detecting genomic abnormalities in fetal cells obtained from an embryo, which enables higher resolution of the interrogated genome.

In another embodiment, the source of the sample that contains predominantly fetal or embryonic DNA is fetal or embryonic cells obtained non-invasively from collecting intact cells (trophoblasts) from a maternal Papanicolaou smear (pap test). Recently it has been shown that this is a simple and safe approach for obtaining fetal or embryonic genetic material non-invasively and that the cells obtained from the pap test had an abundance (near 100%) of fetal or embryonic genetic material (Jain, C.V. et al. (2016) *Science Translational Medicine* 8(363):363re4-363re4).

In another embodiment, the source of the sample that contains predominantly fetal or embryonic DNA is one or a few fetal or embryonic cells found in maternal plasma. Thus, one or a few fetal or embryonic cells present in maternal plasma can be isolated and DNA from the one or a few cells can be used as the DNA sample in the methods of the invention.

In yet other embodiments, the sample containing predominantly fetal or embryonic DNA is a DNA sample that is obtained directly from fetal tissue, or from amniotic fluid, or from chorionic villi or from medium where products of conception were grown.

In another embodiment, the DNA sample that contains predominantly fetal or embryonic DNA is obtained directly from fetal or embryonic tissue.

For the biological sample preparation, typically cells are lysed and DNA is extracted using standard techniques known in the art, a non-limiting example of which is the Qiasymphony (Qiagen) protocol.

Following isolation, the cell free DNA of the sample is used for sequencing library construction to make the sample compatible with a downstream sequencing technology, such as Next Generation Sequencing. Typically this involves ligation of adapters onto the ends of the cell free DNA fragments, followed by amplification. Sequencing library preparation kits are commercially available. A non-limiting exemplary protocol for sequencing library preparation is described in detail in Example 1.

### Enrichment by TACS Hybridization

The region(s) of interest on the chromosome(s) of interest is enriched by hybridizing the pool of TACS to the sequencing library, followed by isolation of those sequences within the sequencing library that bind to the TACS. To facilitate isolation of the desired, enriched sequences, typically the TACS sequences are modified in such a way that sequences that hybridize to the TACS can be separated from sequences that do not hybridize to the TACS. Typically, this is achieved by fixing the TACS to a solid support. This allows for physical separation of those sequences that bind the TACS from those sequences that do not bind the TACS. For example, each sequence within the pool of TACS can be labeled with biotin and the pool can then be bound to beads coated with a biotin-binding substance, such as streptavidin or avidin. In a preferred embodiment, the TACS are labeled with biotin and bound to streptavidin-coated magnetic beads. The ordinarily skilled artisan will appreciate, however, that other affinity binding systems are known in the art and can be used instead of biotin-streptavidin/avidin. For example, an antibody-based system can be used in which the TACS are labeled with an antigen and then bound to antibody-coated beads. Moreover, the TACS can incorporate on one end a sequence tag and can be bound to a solid support via a complementary sequence on the solid support that hybridizes to the sequence tag. Furthermore in addition to magnetic beads, other types of solid supports can be used, such as polymer beads and the like.

In certain embodiments, the members of the sequencing library that bind to the pool of TACS are fully complementary to the TACS. In other embodiments, the members of the sequencing library that bind to the pool of TACS are partially complementary to the TACS. For example, in certain circumstances it may be desirable to utilize and analyze data that are from DNA fragments that are products of the enrichment process but that do not necessarily belong to the genomic regions of interest (i.e., such DNA fragments could bind to the TACS because of part homologies (partial complementarity) with the TACS and when sequenced would produce very low coverage throughout the genome in non-TACS coordinates).

Following enrichment of the sequence(s) of interest using the TACS, thereby forming an enriched library, the members of the enriched library are eluted from the solid support and are amplified and sequenced using standard methods known in the art. Next Generation Sequencing is typically used, although other sequencing technologies can also be employed, which provides very accurate counting in addition to sequence information. To detect genetic abnormalities, such as but not limited to, aneuploidies or structural copy number changes requires very accurate counting and NGS is a type of technology that enables very accurate counting. Accordingly, for the detection of genetic abnormalities, such as but not limited to, aneuploidies or structural copy number changes, other accurate counting methods, such as digital PCR and microarrays can also be used instead of NGS. Non-limiting exemplary protocols for amplification and sequencing of the enriched library are described in detail in Example 3.

### Data Analysis

The information obtained from the sequencing of the enriched library can be analyzed using an innovative biomathematical/biostatistical data analysis pipeline. Details of an exemplary analysis using this pipeline are described in depth in Example 4, and in further detail below. Alternative data analysis approaches for different purposes are also provided herein. For example, data analysis approaches for analyzing fetal and/or embryonic DNA samples for genetic abnormalities are described in detail in Example 6.

The analysis pipeline described in Example 4 exploits the characteristics of the TACS, and the high-efficiency of the target capture enables efficient detection of aneuploidies or structural copy number changes, as well as other types of genetic abnormalities. In the analysis, first the sample's sequenced DNA fragments are aligned to the human reference genome. QC metrics are used to inspect the aligned sample's properties and decide whether the sample is suitable to undergo classification. These QC metrics can include, but are not limited to, analysis of the enrichment patterns of the loci of interest, such as for example the overall sequencing depth of the sample, the on-target sequencing output of the sample, TACS performance, GC bias expectation, fraction of interest quantification. For determining the risk of a chromosomal abnormality in the fetal DNA of the sample, an innovative algorithm is applied. The steps of the algorithm include, but are not limited to, removal of inadequately sequenced loci, read-depth and fragment-size information extraction at TACS-specific coordinates, genetic (GC-content) bias alleviation and ploidy status classification.

Ploidy status determination is achieved using one or more statistical methods, non-limiting examples of which include a t-test method, a bootstrap method, a permutation test and/or a binomial test of proportions and/or segmentation-based methods and/or combinations thereof. It will be appreciated by the ordinarily skilled artisan that the selection and application of tests to be included in ploidy status determination is based on the number of data points available. As such, the suitability of each test is determined by various factors such as, but not limited to, the number of TACS utilized and the respective application for GC bias alleviation, if applicable. Thus, the aforementioned methods are to be taken as examples of the types of statistical analysis that may be employed and are not the only methods suitable for the determination of ploidy status. Typically, the statistical method results in a score value for the mixed sample and risk of the chromosomal abnormality in the fetal DNA is detected when the score value for the mixed sample is above a reference threshold value.

In particular, one aspect of the statistical analysis involves quantifying and alleviating GC-content bias. In addition to the challenge of detecting small signal changes in fetal DNA in the mixed sample, and/or other components of DNA of interest part of a mixed sample (for example, but not limited to, additional or less genetic material from certain chromosomal regions), the sequencing process itself introduces certain biases that can obscure signal detection. One such bias is the preferential sequencing/amplification of genetic regions based on their GC-content. As such, certain detection methods, such as but not limited to, read-depth based methods, need to account for such bias when examining sequencing data. Thus, the bias in the data needs to be quantified and, subsequently, suitable methods are applied to account for it such that genetic context dependencies cannot affect any statistical methods that may be used to quantify fetal genetic abnormality risk.

For example, one method of quantifying the GC-content bias is to use a locally weighted scatterplot smoothing (LOESS) technique on the sequencing data. Each targeted locus may be defined by its sequencing read-depth output and its' GC-content. A line of best fit through these two variables, for a large set of loci, provides an estimate of the expected sequencing read-depth given the GC-content. Once this GC-bias quantification step is completed, the next step is to use this information to account for possible biases in the data. One method is to normalize the read-depth of all loci by their expected read-depth (based on each locus' GC-content). In principle, this unlinks the read-depth data from their genetic context and makes all data comparable. As such, data that are retrieved from different GC-content regions, such as for example, but not limited, to different chromosomes, can now be used in subsequent statistical tests for detection of any abnormalities. Thus, using the LOESS procedure, the GC bias is unlinked from the data prior to statistical testing. In one embodiment, the statistical analysis of the enriched library sequences comprises alleviating GC bias using a LOESS procedure.

In an alternative embodiment, the GC-content bias is quantified and alleviated by grouping together loci of similar (matching) GC-content. Thus, conceptually this method for alleviating GC-content bias comprises of three steps, as follows:
1) identification and calculation of GC-content in the TACS;
2) alleviation/accounting of GC-content bias using various matching/grouping procedures of the TACS; and
3) calculation of risk of any genetic abnormalities that may be present in the fetus utilizing statistical and mathematical methods on datasets produced from step 2.

For the t-test method, the dataset is split into two groups; the test loci and the reference loci. For each group, subsets of groups are created where loci are categorized according to their GC-content as illustrated in a non-limiting example in the sample Table 1 below:

**Table 1**

| GC | Reference loci read-depth | Test loci read-depth |
|---|---|---|
| 40% | ${x}_{1}^{40} , {x}_{2}^{40} , … , {x}_{nx 40}^{40}$ | ${y}_{1}^{40} , {y}_{2}^{40} , … , {y}_{ny 40}^{40}$ |
| 41% | ${x}_{1}^{41} , {x}_{2}^{41} , … , {x}_{nx 41}^{41}$ | ${y}_{1}^{41} , {y}_{2}^{41} , … , {y}_{ny 41}^{41}$ |
| 42% | ${x}_{1}^{42} , {x}_{2}^{42} , … , {x}_{nx 42}^{42}$ | ${y}_{1}^{42} , {y}_{2}^{42} , … , {y}_{ny 42}^{42}$ |
| ... | ... | ... |

It is appreciated by the ordinarily skilled artisan that subgroup creation may involve encompassing a range of appropriate GC-content and/or a subset of loci that are defined by a given GC-content and/or GC-content range. Accordingly, the % GC content given in the non-limiting example of Table 1 are to be considered "about" or "approximate", allowing for some slight variation (e.g., 1-2%). Thus, for example, a % GC content of "40%" is intended to refer to "about 40%" or "approximately 40%", such that, for example, "39%-41%" GC-content loci may also be encompassed if deemed appropriate.

Hence, when referring to a particular GC-content it is understood that the reference and test loci subgroups may comprise of any number of loci related to a particular % GC content and/or range.

Subsequently, for each GC-content subgroup, a representative read-depth is calculated. A number of methods may be utilized to choose this such as, but not limited to, the mean, median or mode of each set. Thus, two vectors of representative read-depth are created where one corresponds to the reference loci and the other to the test loci (e.g., Xm, Ym). In one embodiment, the two vectors may be tested against each other to identify significant differences in read-depth. In another embodiment, the difference of the two vectors may be used to assess if there are significant discrepancies between the test and reference loci. The sample is attributed the score of the test.

For statistical analysis using a bootstrap approach, the dataset is split into two groups, the test loci and the reference loci. The GC-content of each locus is then calculated. Then the following procedure is performed:
A random locus is selected from the reference loci; its read-depth and GC-content are recorded. Subsequently, a random locus from the test loci is selected, with the only condition being that its' GC-content is similar to that of the reference locus. Its read-depth is recorded. It is appreciated by the ordinarily skilled artisan that GC-content similarity may encompass a range of suitable GC-content. As such, referral to a specific % GC content may be considered as "approximate" or "proximal" or "within a suitable range" (e.g., 1%-2%) encompassing the specific % GC content under investigation. Thus, a reference-test locus pair of similar GC-content is created. The difference of the reference-test pair is recorded, say E1. The loci are then replaced to their respective groups. This process is repeated until a bootstrap sample of the same size as the number of test TACS present is created. A representative read-depth of the bootstrap sample is estimated, say E_mu, and recorded. A number of methods may be utilized to do so, such as but not limited to, the mean, mode or median value of the vector, and/or multiples thereof.

The process described above is repeated as many times as necessary and a distribution of E_mu is created. The sample is then attributed a score that corresponds to a percentile of this distribution.

For statistical analysis using a permutation test, the dataset is sorted firstly into two groups, the test-loci and the reference loci. For each group, subsets of groups are created, where loci are categorized according to their GC-content similarity (see columns 2 and 3 of the non-limiting sample Table 2 below). The number of loci present in each test subgroup is also recorded. The loci of the test group are utilized to calculate an estimate of the test-group's read-depth, say Yobs. A representative number from each GC-content subgroup may be selected to do so. Any number of methods may be used to provide a read-depth estimate, such as but not limited to, the mean, median or mode of the chosen loci.

**Table 2**

| GC | Reference loci read- | Test loci read-depth | test loci | Merging of loci |
|---|---|---|---|---|
| 40% | ${x}_{1}^{40} , {x}_{2}^{40} , … , {x}_{nx 40}^{40}$ | ${y}_{1}^{40} , {y}_{2}^{40} , … , {y}_{ny 40}^{40}$ | ny40 | ${x}_{1}^{40} ,.. , {x}_{nx 40}^{40} , {y}_{1}^{40} , … , {y}_{ny 40}^{40}$ |
| 41% | ${x}_{1}^{41} , {x}_{2}^{41} , … , {x}_{nx 41}^{41}$ | ${y}_{1}^{41} , {y}_{2}^{41} , … , {y}_{ny 41}^{41}$ | ny41 | ${x}_{1}^{41} ,.. , {x}_{nx 41}^{41} , {y}_{1}^{41} ,.. , {y}_{ny 41}^{41}$ |
| 42% | ${x}_{1}^{42} , {x}_{2}^{42} , … , {x}_{nx 42}^{42}$ | ${y}_{1}^{42} , {y}_{2}^{42} , … , {y}_{ny 42}^{42}$ | ny42 | ${x}_{1}^{42} ,.. , {x}_{nx 42}^{42} , {y}_{1}^{42} ,.. , {y}_{ny 42}^{42}$ |
| ... | ... | ... | ... | ... |

A distribution to test Yobs is then built utilizing loci irrespective of their test or reference status as follows. The test and reference loci of each GC-content subgroup (see last column of sample Table 2) are combined to allow for calculation of a new read-depth estimate. From each merged subgroup a number of loci are chosen at random, where this number is upper-bounded by the number of test-loci utilized in the original calculation of Yobs (e.g., for GC content 40%, and in the context of the non-limiting sample Table 2, this number of loci may be in the range [1,ny40]). The new read-depth estimate is calculated from all the chosen loci. The procedure is iterated as many times as necessary in order to build a distribution of observed means. A sample is then attributed a score that corresponds to the position of Yobs in this distribution using a suitable transformation that accounts for the moments of the built distribution. As with the already described methods, it is appreciated that slight variation in % GC content is allowed (e.g., 1%-2%), if deemed appropriate. Hence, reference to a specific GC-content could be taken as "about" or "approximate", so that for example when referring to a 40% GC-content, loci that are "approximately" or "about" 40% (e.g., 39%-41%) may be utilized in the method.

For statistical analysis using a binomial test of proportions, fragment-sizes aligned to TACS-specific genomic coordinates are used. It has been shown that fragments of cell free genetic material originating from the placenta tend to be smaller in length when compared to other cell free genetic material (Chan, K.C. (2004) Clin. Chem. 50:88-92). Hence, the statistic of interest is whether the proportion of small-size fragments aligned to a TACS-specific test-region deviates significantly from what is expected when comparing it to the respective proportion of other TACS-specific reference-regions, as this would indicate fetal genetic abnormalities.

Thus, fragment-sizes are assigned into two groups. Sizes related to the test loci are assigned to one group and fragment-sizes related to the reference loci are assigned to the other group. Subsequently, in each group, fragment sizes are distributed into two subgroups, whereby small-size fragments are assigned into one subgroup and all remaining fragments are designated to the remaining subgroup. The last step computes the proportion of small-sized fragments in each group and uses these quantities in a binomial test of proportions. The score of the test is attributed to the sample under investigation.

The final result of a sample may be given by combining one or more scores derived from the different statistical methods, non-limiting examples of which are given in Example 4.

For statistical analysis using segmentation methods, the read-depth and sequence composition of non-overlapping genomic regions of interest of fixed-size is obtained. On the obtained dataset, GC-content read-depth bias alleviation may be performed, but is not limited to, using a local polynomial fitting method in order to estimate the expected read-depth of regions based on their GC content. The expected value, dependent on GC-content, is then used to normalize regions using suitable methods known to those skilled in the art. The normalized dataset is subsequently processed using one or more segmentation-based classification routines. To do so the algorithms process consecutive data points to detect the presence of read-depth deviations which manifest in the form of a "jump/drop" from their surrounding data points. Depending on the segmentation routine used, data points are given a score which is used towards assigning membership into segments of similar performing read-depths. For example, consecutive data points with score values within a suitable range may be classified as one segment, whereas consecutive data points with score values which exceed the set thresholds may be assigned to a different segment. Details of segmentation-based routines are given in Example 6.

### Kits of the Invention

In another aspect, the invention provides kits for carrying out the methods of the disclosure. In one embodiment, the kit comprises a container consisting of the pool of TACS and instructions for performing the method. In one embodiment, the TACS are provided in a form that allows them to be bound to a solid support, such as biotinylated TACS. In another embodiment, the TACS are provided together with a solid support, such as biotinylated TACS provided together with streptavidin-coated magnetic beads.

In one embodiment, the kit comprises a container comprising the pool of TACS and instructions for performing the method, wherein the pool of TACS comprises a plurality of member sequences, wherein:
(i) each member sequence within the TACS pool is between 100-500 base pairs in length, each member sequence having a 5' end and a 3' end;
(ii) each member sequence binds to the same genomic sequence of interest at least 50 base pairs away, on both the 5' end and the 3' end, from regions harboring Copy Number Variations (CNVs), Segmental duplications or repetitive DNA elements; and
(iii) the GC content of the pool of TACS is between 19% and 80%, as determined by calculating the GC content of each member within the pool of TACS.

In one embodiment, the pool of TACS comprises a plurality of TACS families, wherein each TACS family comprises a plurality of member sequences, wherein each member sequence binds to the same genomic sequence of interest but has different start and/or stop positions with respect to a reference coordinate system for the genomic sequence of interest,

Furthermore, any of the various features described herein with respect to the design and structure of the TACS can be incorporated into the TACS that are included in the kit.

In various other embodiments, the kit can comprise additional components for carrying out other aspects of the method. For example, in addition to the pool of TACS, the kit can comprise one or more of the following (i) one or more components for isolating cell free DNA from a biological sample (e.g., as described in Example 1); (ii) one or more components for preparing the sequencing library (e.g., primers, adapters, buffers, linkers, restriction enzymes, ligation enzymes, polymerase enzymes and the like as described in detail in Example 1); (iii) one or more components for amplifying and/or sequencing the enriched library (e.g., as described in Example 3); and/or (iv) software for performing statistical analysis (e.g., as described in Example 4).

### Fragment-Based Analysis

In another aspect, the invention pertains to fragment based analysis of samples, described further in Example 7. There is evidence from the literature that fetal cell free DNA can be found in the medium of IVF products of conception and it can be used for the assessment of chromosomal abnormalities (Liu, WeiQiang, et al. (2017)). Furthermore, specific types of genetic abnormalities can be characterized by and/or associated with fragments of a smaller size than the expected size of fragments originating from healthy tissues (Jiang et al, (2015), Proceedings of the National Academy of Sciences, 112(11), ppE1317-E1325).

Thus, fragments-based detection may be used to detect abnormalities. For example, a binomial test of proportions, as described Example 4, can be used for the detection of increased presence of nucleic acid material originating from abnormal cells based on fragment size. In particular, under the null hypothesis that the distribution of fragment sizes originating from both euploid and aneuploid cells is the same, a binomial test for proportions (as described in Example 4) using continuity correction can be utilized to quantify any evidence against it.

### EXAMPLES

The present invention is further illustrated by the following examples, which should not be construed as further limiting.

### Example 1: Sample Collection and Library Preparation

The general methodology for the TACS-based multiplexed parallel analysis approach for genetic assessment is shown schematically in Figure 1. In this example, methods for collecting and processing a fetal or embryonic DNA sample, followed by sequencing library preparation for use in the methodology of Figure 1 are described.

### Sample Collection

Fetal cell samples were obtained from 3-day and 5-day biopsy embryos respectively were subjected to the TACS methodology shown in Figure 1 to determine the status of genetic abnormalities. Protocols used for collecting samples for our study were approved by the Cyprus National Bioethics Committee, and informed consent was obtained from all participants.

### Sequencing library preparation

Collected fetal cells were initially lysed and DNA extracted using the Rubicon Genomics PicoPLEX WGA Kit (Liang, L. et al. (2013) PLoS One 8(4), p. e61838). Following a pre-amplification step, the lysed material was amplified using amplification enzyme and buffer supplied by the manufacturer. Subsequently, DNA was purified followed by fragmentation using sonication. Following fragmentation, standard library preparation methods were used with the following modifications. A negative control extraction library was prepared separately to monitor any contamination introduced during the experiment. During this step, 5' and 3' overhangs were filled-in, by adding 12 units of T4 polymerase (NEB) while 5' phosphates were attached using 40 units of T4 polynucleotide kinase (NEB) in a 100µl reaction and subsequent incubation at 25° C for 15 minutes and then 12° C for 15 minutes. Reaction products were purified using the MinElute kit (Qiagen). Subsequently, adaptors P5 and P7 (see adaptor preparation) were ligated at 1:10 dilution to both ends of the DNA using 5 units of T4 DNA ligase (NEB) in a 40µl reaction for 20 minutes at room temperature, followed by purification using the MinElute kit (Qiagen). Nicks were removed in a fill-in reaction with 16 units of Bst polymerase (NEB) in a 40 µl reaction with subsequent incubation at 65 °C for 25 minutes and then 12 °C for 20 minutes. Products were purified using the MinElute kit (Qiagen). Library amplification was performed using a Fusion polymerase (Herculase II Fusion DNA polymerase (Agilent Technologies) or Pfusion High Fidelity Polymerase (NEB)) in 50 µl reactions and with the following cycling conditions, 95°C for 3 minutes; followed by 10 cycles at 95°C for 30 seconds, 60°C for 30 seconds, 72°C for 30 seconds and finally 72°C for 3 minutes (Koumbaris, G. et al. (2015) Clinical chemistry, 62(6), pp.848-855). The final library products were purified using the MinElute Purification Kit (Qiagen) and measured by spectrophotometry.

### Adaptor preparation

Hybridization mixtures for adapter P5 and P7 were prepared separately and incubated for 10 seconds at 95° C followed by a ramp from 95° C to 12° C at a rate of 0.1° C /second. P5 and P7 reactions were combined to obtain a ready-to-use adapter mix (100 µM of each adapter). Hybridization mixtures were prepared as follows: P5 reaction mixture contained adaptor P5_F (500 µM) at a final concentration of 200 µM, adaptor P5+P7_R (500 µM) at a final concentration of 200 µM with 1X oligo hybridization buffer. In addition, P7 reaction mixture contained adaptor P7_F (500 µM) at a final concentration of 200 µM, adapter P5+P7_R(500 µM) at a final concentration of 200 µM with 1X oligo hybridization buffer (Koumbaris, G. et al. (2015) Clinical chemistry, 62(6), pp.848-855). Sequences were as follows, wherein ^{∗} = a phosphorothioate bond (PTO) (Integrated DNA Technologies):
adaptor P5_F:
   A^{∗}C^{∗}A^{∗}C^{∗}TCTTTCCCTACACGACGCTCTTCCG^{∗}A^{∗}T^{∗}C^{∗}T (SEQ ID NO: XX)
adaptor P7_F:
   G^{∗}T^{∗}G^{∗}A^{∗}CTGGAGTTCAGACGTGTGCTCTTCCG^{∗}A^{∗}T^{∗}C^{∗}T (SEQ ID NO: YY),
adaptor_P5+P7_R:
   A^{∗}G^{∗}A^{∗}T^{∗}CGGAA^{∗}G^{∗}A^{∗}G^{∗}C (SEQ ID NO: ZZ)

### Example 2: TArget Capture Sequences (TACS) Design and Preparation

This example describes preparation of custom TACS for the detection of whole or partial chromosomal abnormalities for chromosomes 1-22, X and Y or any other chromosome, as well as other genetic abnormalities, such as but not limited to, chromosomal mosaicism, microdeletion/microduplication syndromes, translocations, inversions, insertions, and other point or small size mutations. The genomic target-loci used for TACS design were selected based on their GC content and their distance from repetitive elements (minimum 50 bp away). TACS size can be variable. In one embodiment of the method the TACS range from 100-500 bp in size and are generated through a PCR-based approach as described below. The TACS were prepared by simplex polymerase chain reaction using standard Taq polymerase, primers designed to amplify the target-loci, and normal DNA used as template.

All custom TACS were generated using the following cycling conditions: 95°C for 3 minutes; 40 cycles at 95°C for 15 seconds, 60°C for 15 seconds, 72°C for 12 seconds; and 72°C for 12 seconds, followed by verification via agarose gel electrophoresis and purification using standard PCR clean up kits such as the Qiaquick PCR Purification Kit (Qiagen) or the NucleoSpin 96 PCR clean-up (Mackerey Nagel) or the Agencourt AMPure XP for PCR Purification (Beckman Coulter). Concentration was measured by Nanodrop (Thermo Scientific).

### Example 3: TACS Hybridization and Amplification

This example describes the steps schematically illustrated in Figure 1 of target capture by hybridization using TACS, followed by quantitation of captured sequences by Next Generation Sequencing (NGS).

### TACS Biotinylation

TACS were prepared for hybridization, as previously described (Koumbaris, G. et al. (2015) Clinical chemistry, 62(6), pp.848-855), starting with blunt ending with the Quick Blunting Kit (NEB) and incubation at room temperature for 30 minutes. Reaction products were subsequently purified using the MinElute kit (Qiagen) and were ligated with a biotin adaptor using the Quick Ligation Kit (NEB) in a 40µl reaction at RT for 15 minutes. The reaction products were purified with the MinElute kit (Qiagen) and were denatured into single stranded DNA prior to immobilization on streptavidin coated magnetic beads (Invitrogen).

### TACS Hybridization

Amplified libraries were mixed with blocking oligos (Koumbaris, G. et al. (2105) Clinical chemistry, 62(6), pp.848-855) (200 µM), 5µg of Cot-1 DNA (Invitrogen), 50 µg of Salmon Sperm DNA (Invitrogen), Agilent hybridization buffer 2x, Agilent blocking agent 10X, and were heated at 95°C for 3 minutes to denature the DNA strands. Denaturation was followed by 30 minute incubation at 37°C to block repetitive elements and adaptor sequences. The resulting mixture was then added to the biotinylated TACS. All samples were incubated in a rotating incubator for 12- 48 hours at 66°C. After incubation, the beads were washed as described previously and DNA was eluted by heating (Koumbaris, G. et al. (2105) Clinical chemistry, 62(6), pp.848-855). Eluted products were amplified using outer-bound adaptor primers. Enriched amplified products were pooled equimolarly and sequenced on a suitable platform.

### Example 4: Bioinformatics Sample Analysis

This example describes representative statistical analysis approaches for use in the methodology illustrated in Figure 1 ("analysis pipeline" in Figure 1).

### Human Genome Alignment

For each sample, the bioinformatic pipeline routine described below was applied in order to align the sample's sequenced DNA fragments to the human reference genome. Targeted paired-end read fragments obtained from NGS results were processed to remove adaptor sequences and poor quality reads (Q-score<25) using the cutadapt software (Martin, M. et al. (2011) EMB.netJournal 17.1). The quality of the raw and/or processed reads as well as any descriptive statistics which aid in the assessment of quality check of the sample's sequencing output were obtained using the FastQC software (Babraham Institute (2015) *FastQC*) and/or other custom-built software. Processed reads which were at least 25 bases long were aligned to the human reference genome built hg19 (UCSC Genome Bioinformatics) using the Burrows-Wheel Alignment algorithm (Li, H. and Durbin, R. (2009) Bioinformatics 25:1754-1760) but other algorithms known to those skilled in the art may be used as well. If relevant, duplicate reads were removed post-alignment. Where applicable, sequencing output pertaining to the same sample but processed on separate sequencing lanes, was merged to a single sequencing output file. The removal of duplicates and merging procedures were performed using the Picard tools software suite (Broad Institute (2015) *Picard*) and/or the Sambamba tools software suite (Tarasov, Artem, et al. "Sambamba: fast processing of NGS alignment formats." Bioinformatics 31.12 (2015): 2032-2034.).

The above software analysis resulted in a final aligned version of a sequenced sample against the human reference genome and all subsequent steps were based on this aligned version. Information in terms of Short Nucleotide Polymorphisms (SNPs) at loci of interest was obtained using bcftools from the SAMtools software suite (Li, H. et al. (2009) Bioinformatics 25:2078-2079) and/or other software known to those skilled in the art. The read-depth per base, at loci of interest, was obtained using the mpileup option of the SAMtools software suite, from here on referred to as the mpileup file. Information pertaining to the size of the aligned fragments was obtained using the view option of the SAMtools software suite, from here on referred to as the fragment-sizes file and/or other software known to those skilled in the art.

The mpileup file and the fragment-sizes file were processed using custom-build application programming interfaces (APIs) written in the Python and R programming languages (Python Software Foundation (2015) *Python*; The R Foundation (2015) *The R Project for Statistical Computing*). The APIs were used to determine the ploidy state of chromosomes of interest, and/or other genetic abnormalities in regions of interest across the human genome, using a series of steps (collectively henceforth referred to as the "algorithm") and to also collect further descriptive statistics to be used as quality check metrics, such as but not limited to fetal fraction quantification (collectively henceforth referred to as the "QC metrics"). The APIs can also be used for the assessment of genetic abnormalities from data generated when applying the described method in cases of multiple gestation pregnancies, as well as other genetic abnormalities such as, but not limited to, microdeletions, microduplications, copy number variations, translocations, inversions, insertions, point mutations and mutational signatures.

### QC Metrics

QC metrics were used to inspect an aligned sample's properties and decide whether the sample was suitable to undergo classification. These metrics were, but are not limited to, the enrichment of a sample. The patterns of enrichment are indicative of whether a sample has had adequate enrichment across loci of interest in a particular sequencing experiment (herein referred to as a "run"). To assess this, various metrics are assessed, non-limiting examples of which are:
(i) overall sample on-target read depth,
(ii) sample on-target sequencing output with respect to total mapped reads,
(iii) individual TACS performance in terms of achieved read-depth,
(iv) kurtosis and skewness of individual TACS enrichment,
(v) kurtosis and skewness moments that arise from all TACS,
(vi) fragment size distribution,
(vii) percentage of duplication
(viii) percentage of paired reads and,
(ix) percentage of aligned reads,
if applicable.

The above checks are also taken into consideration with regards to GC-bias enrichment. Samples that fail to meet one or more of the criteria given above are flagged for further inspection, prior to classification.

### The Algorithm

The algorithm is a collection of data processing, mathematical and statistical model routines arranged as a series of steps. The algorithm's steps aim in deciding the relative ploidy state of a chromosome of interest with respect to all other chromosomes of the sequenced sample and is used for the detection of whole or partial chromosomal abnormalities for chromosomes 1-22, X and Y or any other chromosome, as well as other genetic abnormalities such as, but not limited to, chromosomal mosaicism, microdeletion/microduplication syndromes and other point or small size mutations. As such the algorithm can be used, but is not limited to, the detection of whole or partial chromosomal abnormalities for chromosomes 13, 18, 21, X, Y or any other chromosome, as well as other genetic abnormalities such as, but not limited to, microdeletions, microduplications, copy number variations, translocations, inversions, insertions, point mutations and other mutational signatures.

For read-depth associated tests, the algorithm compares sequentially the read-depth of loci from each chromosome of interest (herein referred to as the test chromosome) against the read-depth of all other loci (herein referred to as the reference loci) to classify its ploidy state. For each sample, these steps were, but are not limited to:
(a) Removal of inadequately sequenced loci. The read-depth of each locus was retrieved. Loci that have not achieved a minimum number of reads, were considered as inadequately enriched and were removed prior to subsequent steps.
(b) Genetic (GC-content) bias alleviation. The sequencing procedure may introduce discrepancies in read-depth across the loci of interest depending on their GC content. To account for such bias, a novel sequence-matching approach that increases both sensitivity and specificity to detect chromosomal aneuploidies was employed. The GC content of each locus on the test chromosome was identified and similar genetic loci were grouped together to form genetically matched groups. The procedure was repeated for the reference loci. Then, genetically matched groups from the test chromosome were conditionally paired with their genetically matched group counterparts on the reference chromosome(s). The groups may have any number of members. The conditionally matched groups were then used to assess the ploidy status of test chromosomes.
(c) Genetic abnormality determination. Ploidy status determination, or other genetic abnormalities of interest such as but not limited to microdeletions, microduplications, copy number variations, translocations, inversions, insertions, point mutations and other mutational signatures was achieved using a single statistical method and/or a weighted score approach on the result from the following, but not limited to, statistical methods:
   Statistical Method 1: The differences in read-depth of the conditionally paired groups were tested for statistical significance using the t-test formula: $t = \frac{\hat{x} - μ}{s / \sqrt{n}}$ where t is the result of the t-test, *x̂*is the average of the differences of the conditionally paired groups, µ is the expected read-depth and is set to a value that represents insignificant read-depth differences between the two groups, *s* the standard deviation of the differences of the conditionally paired groups and n the length of the vector of the conditionally paired differences. The magnitude of the t-score was then used to identify evidence, if any, against the null hypothesis of same ploidy between reference and test chromosomes. Specifically, t>=c1 (where c1 is a predefined threshold belonging to the set of all positive numbers) shows evidence against the null hypothesis of no difference.
   Statistical Method 2: Bivariate nonparametric bootstrap. The bootstrap method depends on the relationship between the random variables X (read-depth of reference loci) and Y (read-depth of test loci). Here, the read depth of baits on the reference group (random variable denoted by X) were treated as the independent covariate. The first step of the iterative procedure involved random sampling with replacement (bootstrapping) of the read-depths of loci on the reference chromosomes, i.e., (x1,g1),...,(xn,gn), where the parameter g is known and denotes the GC-content of the chosen bait. Then, for each randomly selected reference bait (xi,gi), a corresponding read depth was generated for a genetically matched locus i.e., (y1,g1),...,(yn,gn). Thus, the bivariate data (x1,y1), (x2,y2),...,(xn,yn) was arrived at, which was conditionally matched on their GC-content (parameter gi). The differences between the read depths of the genetically matched bootstrapped values xi and yi were used to compute the statistic of interest in each iteration. In one embodiment this statistical measure can be, but is not limited to, the mode, mean or median of the recorded differences. and/or multi Dies thereof. The procedure was repeated as necessary to build up the distribution of the statistic of interest from these differences. The sample was assigned a score that corresponds to a specific percentile of the built distribution (e.g. 5^{th} percentile). Under the null hypothesis the ploidy between chromosomes in the reference and test groups is not different. As such, samples whose score for a particular chromosome, was greater than a predefined threshold, say c2, were classified as statistically unlikely to have the same ploidy. Other statistical measures may be employed.
   Statistical Method 3: Stratified permutation test. The statistic of interest is the read-depth estimate of the test chromosome, denoted by, *Ŷ_{obs}* which is calculated using all loci of the test chromosome's genetically matched groups as follows: ${\hat{Y}}_{obs} = \frac{{\sum }_{j = 1}^{j = T} {\sum }_{i = 1}^{i = Nj} {y}_{ij}}{{\sum }_{j = 1}^{j = T} Nj}$ where *yᵢⱼ* is the read-depth of locus *i* part of the genetically matched group *j* (i.e., loci belonging to a specific group based on their GC-content), *Nj* is the number of test loci part of the genetically matched group j and T is the number of genetically matched groups.
      Subsequently, a null distribution to test *Ŷ_{obs}* was built. To do so, for each group *j*, the test and reference loci were combined (exchangeability under the null hypothesis), and each group *j* was sampled randomly up to *Nj* times without replacement (stratified permutation). This created a vector of values, say yi, and from this the vector's average value, say ,was calculated. The procedure was repeated as necessary to build the null distribution. Finally, *Ŷ_{obs}* was studentised against the null distribution using the formula: ${Z}_{Yobs} = \frac{{Y}_{obs}^{∧} - \hat{Y}}{{σ}_{Y}}$ where *Ŷ* and *σ_{Y}* are the first and square root of the second moment of all permuted *ýᵢ* statistic values. Samples whose *Z_{Yobs}* was greater than a predefined threshold, say c3, were statistically less likely to have the same ploidy in the reference and test groups.
      In the case of fragment-size associated tests, the algorithm computes the proportion of small-size fragments found in test-loci and compares it with the respective proportion in reference-loci as described in Statistical Method 4 below.
   Statistical Method 4: Fragment Size Proportions. For each sample the number and size of fragments aligned onto the human reference genome at the corresponding TACS coordinates, is extracted. The data is subsequently filtered so as to remove fragment-sizes considered statistical outliers using the median outlier detection method. Specifically, outliers are defined as those fragments whose size is above or below the thresholds, *Fₜₕᵣ,* set by equation : ${F}_{thr} = {F}_{median} \pm \left(X\times IQR\right)$ where *F_{median}* is the median fragment-size of all fragments of a sample, X is a variable that can take values from the set of *R* +, and *IQR* is the interquartile range of fragment sizes. Thereafter, a binomial test of proportions is carried out to test for supporting evidence against the null hypothesis, H0, where this is defined as:
      H0: The proportion of small fragments of the test-region is not different from the proportion of small-fragments of the reference region.

In various embodiments of the invention, small fragments are defined as those fragments whose size is less than or equal to a subset of *Z* + that is upper-bounded by 160bp. If the set of all TACS are defined as T, then the test region can be any proper subset S which defines the region under investigation, and the reference region is the relative complement of S in T. For example, in one embodiment of the invention, the set S is defined by all TACS-captured sequences of chromosome 21 and thus the reference set is defined by all TACS-captured fragments on the reference chromosomes, and/or other reference loci

The alternative hypothesis, H1, is defined as:
H1: The proportion of small fragments of the test-region is not equal to the proportion of test fragments of the reference region.

As such, and taking into account continuity correction, the following score is computed (Brown et. al, Harrel): ${W}_{test} = \left({p}^{′}-{p}_{ref}\right) / \sqrt{\frac{{p}^{′} \left(1-{p}^{′}\right)}{{N}_{test}}}$ where ${p}^{′} = \frac{\left({F}^{′}+0.5\right)}{\left({N}_{test}+1\right)}$ ${p}_{ref} = \frac{\left({F}_{ref}+0.5\right)}{\left({N}_{ref}+1\right)}$

*F́* is the number of small-size fragments on the test-region, *F_{ref}* the number of small size fragments on the reference region, *Nₜₑₛₜ* the number of all fragments on the test region and *N_{ref}* the number of all fragments on the reference region.

For each sample, the algorithm tests sequentially the proportion of fragment sizes of regions under investigation (for example, but not limited to, chromosome 21, chromosome 18, chromosome 13 or other (sub)chromosomal regions of interest) against reference regions; those not under investigation at the time of testing. For each sample a score is assigned for each test. Scores above a set-threshold, say c4, provide evidence against the null hypothesis.

Weighted Score method 1: In one embodiment of the method, a weighted score was attributed to each sample *s*, computed as a weighted sum of all statistical methods using the formula: ${V}_{s} \left(R, F\right) = {z}_{1} max \left\{{R}_{s}, {F}_{s}\right\} + \left(1-{z}_{1}\right) min \left\{{R}_{s}, {F}_{s}\right\}$ where *Rₛ* is the run-specific corrected score arising from a weighted contribution of each read-depth related statistical method for sample s and is defined as: ${R}_{s} = \frac{\left({\sum }_{i} {w}_{i}{S}_{is}-{{R}^{′}}_{r}\right)}{{σ}_{r}}$ and *Ŕᵣ* is the run-specific median value calculated from the vector of all unadjusted read-depth related weighted scores that arise from a single sequencing run, and *σᵣ* is a multiple of the standard deviation of R scores calculated from a reference set of 100 euploid samples. The terms *max*{*Rₛ,Fₛ*}and *min*{*Rₛ,Fₛ*} denote the maximum and minimum values of the bracketed set, respectively.

*Fₛ* is the run-specific corrected score arising from the fragment-size related statistical method and is defined as: ${F}_{s} = \frac{\left({W}_{test}-{{R}^{′}}_{f}\right)}{{σ}_{f}}$ where *Wₜₑₛₜ* is as defined earlier, *Ŕ_{f}* is the run specific median calculated from the vector of all unadjusted fragment-related statistical scores that arise from a single sequencing run, and *σ_{f}* is a multiple of the standard deviation of *F* scores calculated from a reference set of 100 euploid samples.

A unique classification score of less than a predefined value indicates that there is no evidence from the observed data that a sample has a significant risk of aneuploidy.

Weighted Score method 2: In another embodiment of the method, the weighted score arising from the statistical methods described above was used to assign each sample a unique genetic abnormality risk score using the formula: $R \left(t, c\right) = {\sum }_{j = 0}^{j = N} {w}_{j} \frac{{t}_{j}}{{c}_{j}}$ where R is the weighted score result, *wⱼ* the weight assigned to method *j*, *tⱼ* the observed score resulting from method *j,* and*cⱼ* the threshold of method *j.*

A unique classification score of less than a predefined value indicates that there is no evidence from the observed data that a sample has a significant risk of aneuploidy.

Since all read depths from baits in the reference group were assumed to be generated from the same population, and in order to have a universal threshold, run-specific adjustments were also employed to alleviate run-specific biases.

The aforementioned method(s), are also suitable for the detection of other genetic abnormalities, such as but not limited to, subchromosomal abnormalities. A non-limiting example is the contiguous partial loss of chromosomal material leading to a state of microdeletion, or the contiguous partial gain of chromosomal material leading to a state of microduplication. A known genetic locus subject to both such abnormalities is 7q11.23. In one embodiment of statistical method 1, synthetic plasma samples of 5%, 10% and 20% fetal material were tested for increased risk of microdeletion and/or microduplication states for the genetic locus 7q11.23.

For point mutations various binomial tests are carried out that take into consideration the fetal fraction estimate of the sample, f, the read-depth of the minor allele, r, and the total read-depth of the sequenced base, n. Two frequent, yet non-limiting examples involve assessment of the risk when the genetic abnormality is a recessive point mutation or a dominant point mutation.

In addition to the above, fetal sex determination methods were also developed, with non-limiting examples given below. In one embodiment of the invention, fetal sex was assigned to a sample using a Poisson test using the formula: $Pr \left({r}_{y}\leq k\right) = {e}^{- λ} {\sum }_{i = 0}^{i = k} \frac{{λ}^{i}}{i !}$ where $λ = \frac{fBμ}{2}$ and f is the fetal fraction estimate of the sample, B is the number of target sequences on chromosome Y, µ is the read-depth of the sample and k is the sum of reads obtained from all targets B. The null hypothesis of the Poisson test was that the sample is male. A value of Pr(r_{y}) less than a threshold c_{y} was considered as enough evidence to reject the null hypothesis, i.e. the sample is not male. If any of the terms for computing Pr(r_{y}) were unavailable, then the sample's sex was classified as NA (not available).

In another embodiment of the invention, fetal sex was assigned using the average read-depth of target sequences on chromosome Y. If the average read-depth of the target-sequences was over a predefined threshold, where such threshold may be defined using other sample-specific characteristics such as read-depth and fetal-fraction estimate, the fetal sex was classified as male. If the average read-depth was below such threshold then the sample was classified as female.

### Example 5: Target Enrichment Using Families of TACS

In this example, a family of TACS, containing a plurality of members that all bind to the same target sequence of interest, was used for enrichment, compared to use of a single TACS binding to a target sequence of interest. Each member of the family of TACS bound to the same target sequence of interest but had a different start and/or stop coordinates with respect to a reference coordinate system for that target sequence (e.g., the human reference genome built hg19). Thus, when aligned to the target sequence, the family of TACS exhibit a staggered binding pattern, as illustrated in Figure 3. Typically, the members of a TACS family were staggered approximately 5-10 base pairs.

A family of TACS containing four members (i.e., four sequences that bound to the same target sequence but having different start/stop positions such that the binding of the members to the target sequence was staggered) was prepared. Single TACS hybridization was also prepared as a control. The TACS were fixed to a solid support by labelling with biotin and binding to magnetic beads coated with a biotin-binding substance (e.g., streptavidin or avidin) as described in Example 3. The family of TACS and single TACS were then hybridized to a sequence library, bound sequences were eluted and amplified, and these enriched amplified products were then pooled equimolarly and sequenced on a suitable sequencing platform, as described in Example 3.

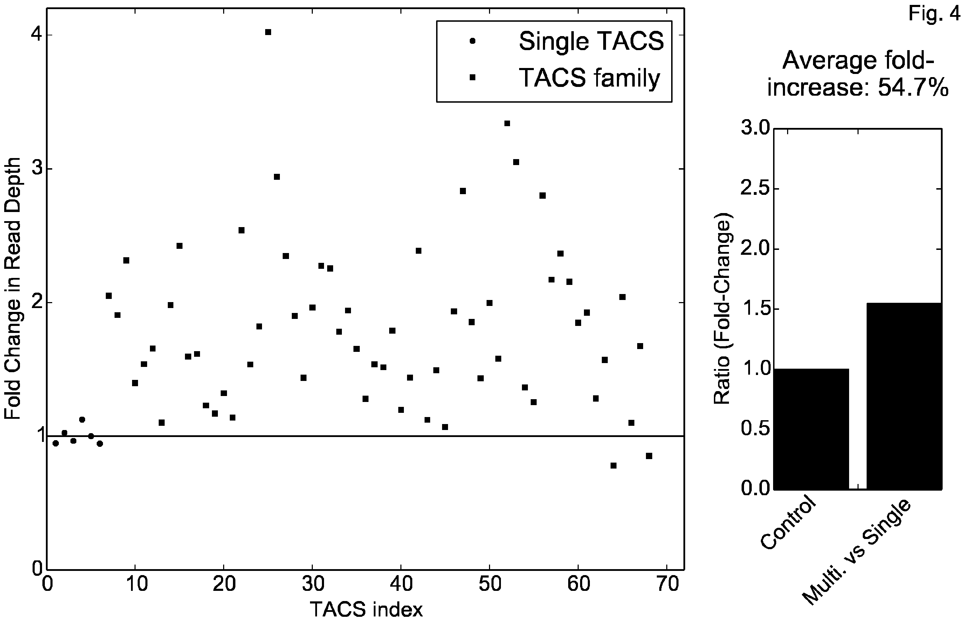

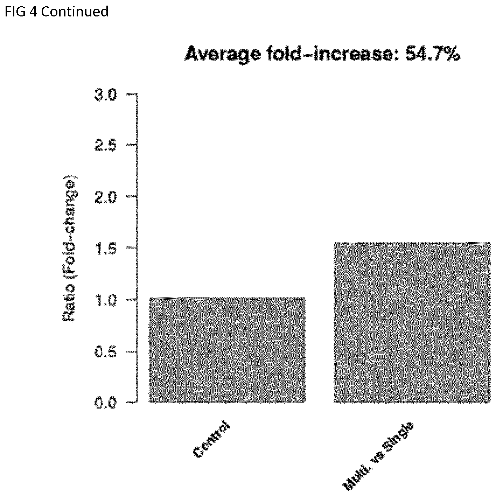

The enriched sequences from the family of TACS sample and the single TACS sample were analyzed for read-depth. The results are shown in Figures 4A and 4B. As shown in Figure 4A, target sequences of interest enriched using the family of four TACS (red dots) exhibited a fold-change in read-depth when compared to control sequences that were subjected to enrichment using only a single TACS (blue dots). Fold-change was assessed by normalizing the read-depth of each locus by the average read-depth of a sample, wherein the average read-depth was calculated from all loci enriched with a single TACS. As shown in Figure 4B, an overall 54.7% average increase in read-depth was observed using the family of four TACS.

This example demonstrates that use of a family of TACS, as compared to a single TACS, results in significantly improved enrichment of a target sequence of interest resulting in significantly improved read-depth of that sequence.

### Example 6: Analysis of Fetal DNA Samples from Embryo Biopsy

In this example, fetal DNA samples obtained from fetal cells from embryo biopsy were analyzed using the TACS-based methodology shown in Figure 1 to detect chromosomal abnormalities in the fetal samples.

### Fetal Sample Collection, Library Preparation and TACS Enrichment

Fetal cell samples were obtained from 3-day and 5-day biopsy embryos respectively were subjected to the TACS methodology shown in Figure 1 to determine the status of genetic abnormalities. All samples were previously referred for Pre-implantation Genetic Screening (PGS) and subjected to array Comparative Genomic Hybridization (aCGH) as part of the routine screening test. Results of aCGH were used as a reference standard for the results obtained.

Collected fetal cells were initially lysed and DNA extracted using the Rubicon Genomics PicoPLEX WGA Kit (Liang, L. et al. (2013) PLoS One 8(4), p. e61838).

For certain samples in which whole-genome sequencing was to be performed, the lysed material was subjected to whole genome amplification using commercial whole genome amplification kits. Briefly, following a pre-amplification step, the lysed material was then amplified using amplification enzyme and buffer supplied by the manufacturer. Subsequently, DNA was purified followed by fragmentation using sonication. Fragmented DNA was then processed using standard sequencing library preparation methods such as described in Example 1, typically involving ligation of adapters onto the ends of the cell free DNA fragments, followed by amplification. In addition to the description provided in Example 1, sequencing library preparation kits are commercially available for this purpose.

For samples in which TACS-based enrichment was to be performed, then the sequencing library obtained from the above methods underwent TACS hybridization essentially as described in Example 3. The region(s) of interest on the chromosome(s) of interest were enriched by hybridizing the pool of TACS to the sequencing library, followed by isolation of those sequences within the sequencing library that bind to the TACS. To facilitate isolation of the desired, enriched sequences, typically the TACS sequences were modified such that sequences that hybridized to the TACS were separable from sequences that did not hybridize to the TACS. Typically this was achieved by fixing the TACS to a solid support such as described in Example 3, thereby allowing for physical separation of those sequences that bind the TACS from those sequences that do not bind the TACS. The pools of TACS used either can contain a plurality of single TACS that bind to different target sequences of interest or, alternatively, can contain a plurality of families of TACS containing a plurality of members that each bind to the same target sequence of interest but with different start and/or stop positions on the target sequence, as described in Example 5.

For analysis of fetal DNA samples by TACS-based enrichment, the pool of TACS can contain TACS that target a subset of chromosomes of interest (e.g., chromosomes 13, 18, 21, X and Y). More preferably, however, the pool of TACS contains various TACS that target every chromosome within the human genome (chromosomes 1-22, X and Y) such that the entire genome is encompassed, allowing for determination of chromosomal abnormalities in any chromosome within the human genome.

Next Generation Sequencing (NGS) typically was used to sequence the TACS-enriched sequences (or the whole genome for samples analyzed by whole genome sequencing), thereby providing very accurate counting as well as sequence information. Library products were pooled equimolarly and then subjected to sequencing.

### Data Analysis

Sequencing data obtained from NGS were processed to remove adaptor sequences and poor quality reads. Reads whose length was at least 25 bases long post adaptor-removal were aligned to the human reference genome built hg19. If relevant, duplicate reads were removed post-alignment. Where applicable, sequencing output pertaining to the same sample but processed on separate sequencing lanes, was merged to a single sequencing output file. Software analysis provides a final aligned version of a sequenced sample against the human reference genome from which information was extracted in terms of Short Nucleotide Polymorphisms (SNPs) at loci of interest, read-depth per base and the size of aligned fragments.

For whole-genome sequencing and TACS-based whole-genome sequencing, the read-depth of non-overlapping genomic regions of fixed size (e.g. 50kb or 1Mb) was obtained by using the samtools bedcov tool, which provides the sum of all reads across a specified genomic region. The obtained value was divided by the length of the windows. For TACS targeted-based sequencing, the read-depth was obtained by using the samtools mpileup tool, which provides information on the read-depth per base, across specified contiguous sequences or the bedcov tool. The median value of the obtained information was assigned as the read-depth of a given locus. Removal of read-depth outliers was performed using either a median-based or mean-based outlier detection approach. Finally, GC-content read-depth bias alleviation was achieved using a local polynomial fitting method to estimate the expected read-depth of regions based on their GC content and then normalize regions using this expected value accordingly.

The normalized read-depth from all regions was used as input into
(a) various segmentation-based classification algorithms (described further below), and/or
(b) score-based classification algorithms (described further below),
which were then used to determine the ploidy status of the interrogated regions, as well as the size of any genetic aneuploidies. Score-based classification algorithms were used only with targeted enrichment sequencing data.

### Ploidy Status Determination Using Segmentation Algorithms

Three different types of segmentation algorithms were developed and applied to fetal DNA sample analysis: (i) Likelihood-based segmentation; (ii) Segmentation using small overlapping windows; and (iii) Segmentation using parallel pairwise testing, each of which is described further below, along with the results for application of the algorithm.

Each algorithm is a collection of data processing and statistical modeling routines arranged as a series of steps with aim to decide if the observed sequencing data does not support the null hypothesis, H0 defined as:
H0= There are no ploidy deviations from the expected ploidy state.

For human genomes the expected ploidy state is the diploid state. The segmentation approach aims to discover breakpoints in consecutive data where there is a clear distinction between read-depths, which in turn indicates that there is a change in ploidy state. The algorithms are described below.

### A. Likelihood-based segmentation

Given a set of ordered data points {*x_{1},x_{2},x_{3},x_{4},..,x_{N}*}*,* that describe read-depth, the aim was to infer at which point *x_{i}* the data changes distribution (i.e. there is a significant and consecutive change in read-depth). This was labeled as the break point ϑ_*{1}*. For example, if the data changes distribution after *x_{3}* then ϑ*_{1}=x_{3}.* If more than one break point exists, then the algorithm will label the next discovered break point as ϑ*_{2}*. The algorithm steps were as follows:
(a) Given a sequence of data (*i,x_{i}),* where *i=1..N,* the algorithm estimates the number of modes in the data. To this end, a process known as bivariate kernel density estimation was utilized. For example, if there was a single breakpoint, then the algorithm returned that there were 2 modes in our data distribution.
(b) Decide the position of the break point(s) in the data, if such point(s) exist(s). This was achieved with the following algorithm:
   (1) Based on the number of breakpoints found in (a) define the probability density function (p.d.f) of the data, which depends on the unknown values of the breakpoints. This may be, but not limited to, a mixture of Normal distributions.
   (2) Calculate the maximum likelihood estimate of the p.d.f in (1) for a fixed set of value(s) for the breakpoints.
   (3) Repeat (2) for different sets of break point value(s).
   (4) Select as estimated break point(s) the values that maximizes step (2).

It was noted that the algorithm does this by assigning membership in all combinations for all break-points estimated in part (a). As an example, if the probability is maximized when data points *x_{1}* to *x_{3}* come from the first distribution then ϑ*_{1}=x_{3}* and membership of *x_{1}* to *x_{3}* is assigned to the first distribution and *x_{4}* to *x_{N}* to the next identified distribution(s). If the likelihood is maximized with all data points *x_{i}* assigned to the same mode then no breakpoint is defined and all data points are assigned to the same distribution. Various distributions and computational methods known to those skilled in the art can be used to implement this.

Representative results of fetal DNA analysis using the likelihood-based segmentation algorithm are shown in Figure 5. These results demonstrate that likelihood-based segmentation analysis can classify whole-chromosome aberrations in fetal DNA samples (e.g., from PGD/PGS products of conception). At the top panel of Figure 5, a sample without any ploidy abnormalities subjected to whole-genome sequencing is presented. The expected read-depth of each chromosome (blue horizontal bars) lies within the red lines that indicate the range of values of normal ploidy, as decided from the data. Even if on occasion individual data points (grey dots) deviate from the confidence intervals this is not sufficient evidence of ploidy aberrations according to the probabilistic metric used. Conversely, if enough data points deviate from the confidence intervals then the probabilistic measure used can assign a different ploidy state. Such a case is presented at the bottom of Figure 5, where the sample has been determined to have monosomy 18 and monosomy 20.

In similar fashion, Figure 10 presents results from the algorithm utilizing data derived from TACS specific coordinates combined with data from products of partial complementarity to the TACS that align to non-TACS coordinates thus producing low coverage throughout the genome. In the top panel of Figure 10 a normal male sample is presented, whereas in the bottom panel the male sample is classified as having trisomy for chromosome 13 and monosomy for chromosome 21.

Figure 11 presents results from the algorithm utilizing data from TACS specific coordinates only. As with Figure 10, in the top panel of Figure 11 a normal male sample is presented, whereas in the bottom panel the male sample is classified as having trisomy for chromosome 13 and monosomy for chromosome 21.

Thus, it can be seen that the algorithm successfully classifies TACS-based enrichment and TACS-based whole genome sequencing data, allowing for correct classification of chromosomal abnormalities and at the same time requiring significantly less sequencing than massively parallel shotgun sequencing approaches.

### B. Segmentation using small overlapping windows

Given a set of data points the aim was to decide membership of each data point into a set of clusters, based on a thresholding scheme. The algorithm does so as follows:
(a) Given a set of consecutive read-depth data *x_{i}* (*i=1* to *N*) the data are divided into overlapping windows of fixed size. For example let *w_{1}* = *{x_{1}* , ..., *x{10}*} denote the first window, then *w_{2}* = {*x_{2},* ..., *x_{11}*}, *w_{3}* = {*x_{3},* ..., *x_{12}*} etc.
(b) For each window *w_{k},* a score *S(k) = (X_{k} - m)*/*m* is computed, where X*_{k}* is the median of *w_{k}* and *m* is the median from all *x_{i}* from all chromosomes.
(c) Assign cluster membership based on a thresholding value *s*, whereby:
   if *S(k) < s ,* assign to cluster1
   if *s <= S(k) < C_{1}s* are assigned to cluster 2,
   if *2s <= S(k) < C_{2}s* are assigned to cluster 3 etc.
   where *C_{j}*. are positive real numbers greater than one. For example, if *s* is a particular threshold value then all consecutive *w_{k}* where *S(k) < s* are assigned to cluster 1. All consecutive *w_{k}* where *s <= S(k) < C_{1}s* are assigned to cluster 2. All consecutive *w_{k}* where *2s <= S(k) <C_{2}s* are assigned to cluster 3 etc. The threshold *s* can be either decided from the data or treated as a tuning parameter.

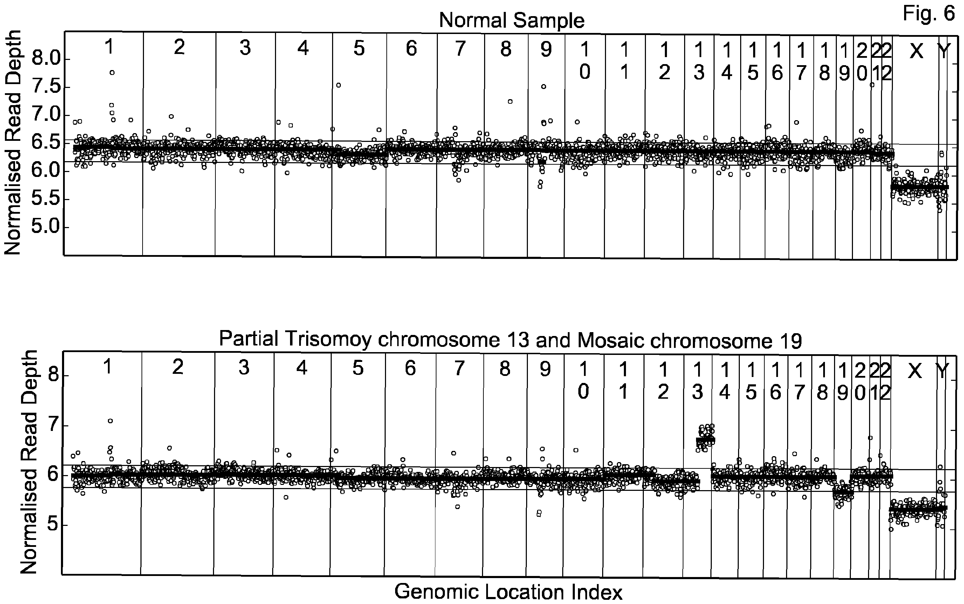

Representative results of ploidy determination for fetal DNA samples (e.g., PGS/PGD products of conception) using whole genome sequencing and small overlapping windows segmentation are shown in Figure 6. The top panel illustrates a normal sample. As with Figure 5, the expected read-depth of each chromosome (blue horizontal bars) lies within the red lines, which indicate the range of values of normal ploidy. The expected read-depth is calculated from the individual data points (grey dots). The average read-depth and data points of chromosomes X and Y lie below the bottom red-line, indicating that there is only a single copy of each chromosome, as expected for a male sample. An aneuploid sample is presented at the bottom of Figure 6 where the sample is classified with trisomy 13 and mosaicism on chromosome 19.

### C. Segmentation using Parallel Pairwise Testing

This segmentation approach firstly performs full chromosome ploidy determination and then a sub-chromosomal ploidy determination as follows:
(a) Read-depth data from one candidate chromosome are compared with read-depth data from other chromosomes using non-parametric statistical tests. The process is repeated until all candidate chromosomes are tested.
(b) Perform a multiple comparisons adjustment on the results of the statistical tests to avoid false positive results.
(c) Depending on the statistical test result from the adjusted data, assign the relevant ploidy to candidate chromosomes that illustrate significant evidence against the null hypothesis
(d) Once full-chromosomal ploidy is determined then sub-chromosomal ploidy is tested by randomly splitting regions of each chromosome into smaller sizes. Each sub-chromosomal region is then tested for significant deviations from its expected full-chromosomal read-depth using similar statistical tests as in steps (a)-(c).

Representative results of ploidy determination for fetal DNA samples (e.g., PGS/PGD products of conception) using whole genome sequencing and small overlapping windows segmentation are presented in Figure 7. The top panel illustrates a normal sample. As with Figures 5, 6, 10 and 11, the expected read-depth of each chromosome is illustrated using blue horizontal bars. In this instance, confidence interval bars have been omitted. A normal sample is presented at the top Figure 7 whilst a sample presenting many abnormalities is presented at the bottom panel.

### Ploidy Status Determination Using Score-Based Classification

Additionally or alternatively to the segmentation-based algorithms described above, fetal DNA samples can be analyzed using score-based classification. The read-depth data were firstly transformed using square root or logarithmic transformation in order to minimize variance biases. Then methods such as those described in Example 4 were performed to decide on the ploidy status of each tested region (chromosomal and sub-chromosomal regions may be tested).

Representative results using a score-based classification system on the fetal DNA samples (e.g., PGS/PGD products of conception) are shown in Figure 8. Green dots illustrate normal ploidy samples whilst all others that lie above or below the normal ploidy thresholds illustrate some type of abnormality. Specifically, blue dots illustrate trisomy samples, cyan dots illustrate partial trisomy samples and red dots illustrate monosomy samples.

In summary, this example demonstrates the successful analysis of fetal DNA samples (e.g., PGS/PGD products of conception) for chromosomal abnormalities using either whole genome sequencing data, TACS-based whole genome sequencing data and TACS-based enrichment data, using a variety of statistical analysis approaches. Furthermore, the example illustrates that the methods used with whole genome sequencing data can be successfully applied to TACS-based whole genome sequencing data and TACS-based enrichment data.

### Example 7: Fragment Size Based Tests

There is evidence from the literature that unhealthy tissue can be characterized by and/or associated with fragments in the plasma having a smaller size than the expected size of fragments originating from healthy tissues (Jiang et al, (2015), Proceedings of the National Academy of Sciences, 112(11), ppE1317-E1325). Furthermore, it has been shown that fetal cell free DNA can be found in the spent medium of embryo culture of PGS/PGD products of conception and that it can be used for the assessment of chromosomal abnormalities (Liu, WeiQiang, et al. (2017). Thus, a fragments-size based test can be utilized to detect the presence of somatic copy number variations. To this effect, a binomial test of proportions, as described Example 4, can be used for the detection of increased presence of nucleic acid material originating from non-healthv tissue based on fragment size. In particular, under the null hypothesis that the distribution of fragment sizes originating from both healthy and non-healthy cells is the same, a binomial test for proportions (as described in Example 4) using continuity correction can be utilized to quantify any evidence against it.

The same hypothesis holds true for fragments originating from the placenta/fetus (Chan, K.C. (2004) Clin. Chem. 50:88-92). Specifically, placenta derived fragments are generally of smaller size when compared to fragments originating from maternal tissues/cells. Accordingly, assessment of the fragment size-based test was performed using maternal plasma samples (i.e., mixed samples where cell free DNA is of maternal and fetal origin). The size of fragments that have aligned to TACS-enriched regions can be obtained from the aligned data. Subsequently, the proportion of fragments under a specific threshold from a test region is compared respective proportion of fragments from a reference region for evidence against the null hypothesis H0,
H0: The proportion of small fragments of the test-region is not different from the proportion of small-fragments of the reference region.

Figure 9 shows results when applying the fragment sizes method to the mixed sample containing maternal and fetal DNA. The black dots are individual samples. The x-axis shows the sample index. The y-axis shows the score result of the fragments-based method. A score result greater than the one indicated by the threshold, illustrated as a grey line, indicates a deviation from the expected size of fragments illustrating the presence of aneuploidy. The results demonstrate that an aneuploid sample, having an estimated fetal fraction equal to 2.8%, was correctly identified, illustrating that fragments-based detection may be used to detect abnormalities in samples with low signal-to-noise ratio (e.g., as is the case in detection of cancer).

Accordingly, this example demonstrates the successful ability of the fragments-based detection method in detecting genetic abnormalities present in diminutive amounts. In addition to this, since small-sized fragments are associated with fragments from non-healthy tissues (Jiang et al, (2015), Proceedings of the National Academy of Sciences, 112(11), ppE1317-E1325) they can also be leveraged for the detection of small-sized mutations, such as point mutations and mutational signatures.

## Claims

1. A method of testing for risk of a genetic abnormality in a DNA sample comprising predominantly fetal or embryonic DNA and comprising genomic sequences of interest, the method comprising:
a) preparing a sequencing library from the DNA sample comprising predominantly fetal or embryonic DNA;
b) hybridizing the sequencing library to a pool of double-stranded TArget Capture Sequences (TACS), wherein the pool of TACS comprises a plurality of TACS families directed to different genomic sequences of interest comprising a genetic abnormality, wherein each TACS family comprises a plurality of member sequences, wherein each member sequence binds to the same genomic sequence of interest but has different start and/or stop positions with respect to a reference coordinate system for the genomic sequence of interest, wherein the start and/or stop positions for the member sequences within a TACS family, with respect to a reference coordinate system for the genomic sequence of interest are staggered by 5 to 10 base pairs, and further wherein:
i) each member sequence within the pool of TACS is between 150-260 base pairs in length, each member sequence having a 5' end and a 3' end; and
ii) each member sequence binds to the same genomic sequence of interest at least 50 base pairs away, on both the 5' end and the 3' end, from regions harboring Copy Number Variations (CNVs), Segmental duplications or repetitive DNA elements; and
iii) the GC content of the pool of TACS is between 19% and 80%, as determined by calculating the GC content of each member within the pool of TACS;
c) isolating members of the sequencing library that bind to the pool of TACS to obtain an enriched library;
d) amplifying and sequencing the enriched library;
e) aligning, the enriched library to a reference genome thereby obtaining read-depth information and allelic counts; and
f) performing statistical analysis on the enriched library sequences to thereby determine risk of a genetic abnormality in the DNA sample.

2. The method of claim 1, wherein the DNA sample is from a pre-implantation embryo, or from intact trophoblasts collected from a maternal Papanicolaou smear, or from a fetal cell found in maternal plasma.

3. The method of any one of claims 1 to 2, wherein the DNA sample is obtained directly from fetal tissue, or amniotic fluid, or chorionic villi, or medium where products of conception were grown

4. The method of any one of claims 1 to 3, wherein the pool of TACS comprises members that bind to chromosomes 1-22, X and Y of the human genome.

5. The method of claim 1, wherein the pool of TACS comprises at least 5 different TACS families, optionally wherein each family comprises at least 3 member sequences.

6. The method of any one of claims 1 to 5, wherein the genetic abnormality is a chromosomal aneuploidy or wherein the genetic abnormality is a structural abnormality, including but not limited to copy number changes including microdeletions and microduplications, insertions, deletions, translocations, inversions and small-size mutations including point mutations and mutational signatures.

7. The method of any one of claims 1 to 6, wherein the pool of TACS is fixed to a solid support, optionally wherein the TACS are biotinylated and are bound to streptavidin-coated magnetic beads.

8. The method of any one of claims 1 to 7, wherein amplification of the enriched library is performed in the presence of blocking sequences that inhibit amplification of wild-type sequences.

9. The method of any one of claims 1 to 8, wherein members of the sequencing library that bind to the pool of TACS are partially complementary to the TACS.

10. The method of any of claims 1 to 9, wherein the statistical analysis comprises a segmentation algorithm, optionally wherein the segmentation algorithm is selected from the group consisting of likelihood-based segmentation, segmentation using small overlapping windows, segmentation using parallel pairwise testing, and combinations thereof.

11. The method of any one of claims 1 to 10, wherein sequencing of the enriched library provides a read-depth for the genomic sequences of interest and read-depths for reference loci and the statistical analysis comprises applying an algorithm that tests sequentially the read-depth of the loci of from the genomic sequences of interest against the read-depth of the reference loci, the algorithm comprising steps for: (a) removal of inadequately sequenced loci; (b) GC-content bias alleviation; and (c) ploidy status determination, optionally wherein GC-content bias is alleviated by grouping together loci of matching GC content.

12. The method of any one of the preceding claims, wherein sequencing of the enriched library provides the number and size of sequenced fragments for TACS-specific coordinates and the statistical analysis comprises applying an algorithm that tests sequentially the fragment-size proportion for the genomic sequence of interest against the fragment-size proportion of the reference loci, the algorithm comprising steps for: (a) removal of fragment-size outliers; (b) fragment-size proportion calculation; and (c) ploidy status determination.

## Patentansprüche

1. Ein Verfahren zum Testen auf das Risiko einer genetischen Anomalie in einer DNA-Probe, die überwiegend fötale oder embryonale DNA umfasst und genomische Sequenzen von Interesse umfasst, wobei das Verfahren umfasst:
a) Herstellen einer Sequenzierbibliothek aus der DNA-Probe, die vorwiegend fötale oder embryonale DNA umfasst;
b) Hybridisieren der Sequenzierbibliothek an einen Pool von doppelsträngigen Zielerfassungssequenzen (TArget Capture Sequences: TACS), wobei der Pool von TACS eine Vielzahl von TACS-Familien umfasst, die an verschiedene genomische Sequenzen von Interesse gerichtet sind, die eine genetische Anomalie umfassen, wobei jede TACS-Familie eine Vielzahl von Mitgliedssequenzen umfasst, wobei jede Mitgliedssequenz an dieselbe genomische Sequenz von Interesse bindet, aber unterschiedliche Start- und/oder Stopp-Positionen in Bezug auf ein Referenzkoordinatensystem für die genomische Sequenz von Interesse aufweist, wobei die Start- und/oder Stopp-Positionen für die Mitgliedssequenzen innerhalb einer TACS-Familie in Bezug auf ein Referenzkoordinatensystem für die genomische Sequenz von Interesse um 5 bis 10 Basenpaare versetzt sind, und wobei ferner:
i) jede Mitgliedssequenz innerhalb des TACS-Pools zwischen 150-260 Basenpaare lang ist, wobei jede Mitgliedssequenz ein 5'-Ende und ein 3'-Ende hat; und
ii) jede Mitgliedssequenz an dieselbe genomische Sequenz von Interesse bindet, sowohl am 5'-Ende als auch am 3'-Ende mindestens 50 Basenpaare entfernt von Regionen, die Kopienzahlvariationen (CNVs), segmentale Duplikationen oder repetitive DNA-Elemente beherbergen; und
iii) der GC-Gehalt des TACS-Pools zwischen 19 % und 80 % liegt, wie durch Berechnung des GC-Gehalts jedes Mitglieds innerhalb des TACS-Pools bestimmt;
c) Isolieren von Mitgliedern der Sequenzierbibliothek, die an den TACS-Pool binden, um eine angereicherte Bibliothek zu erhalten;
d) Amplifizieren und Sequenzieren der angereicherten Bibliothek;
e) Alignment der angereicherten Bibliothek an ein Referenzgenom, wodurch Sequenziertiefeinformationen und Allelanzahlen erhalten werden; und
f) Durchführung einer statistischen Analyse der angereicherten Bibliothekssequenzen, um dadurch das Risiko einer genetischen Anomalie in der DNA-Probe zu bestimmen.

2. Das Verfahren nach Anspruch 1, wobei die DNA-Probe von einem Präimplantationsembryo oder von intakten Trophoblasten, die aus einem mütterlichen Papanicolaou-Abstrich entnommen wurden, oder von einer fötalen Zelle stammt, die im mütterlichen Plasma gefunden wurde.

3. Das Verfahren nach einem der Ansprüche 1 bis 2, wobei die DNA-Probe direkt aus fötalem Gewebe oder Fruchtwasser oder Chorionzotten oder dem Medium, in dem die Empfängnisprodukte gewachsen sind, gewonnen wird.

4. Das Verfahren nach einem der Ansprüche 1 bis 3, wobei der Pool von TACS Mitglieder umfasst, die an die Chromosomen 1-22, X und Y des menschlichen Genoms binden.

5. Das Verfahren nach Anspruch 1, wobei der TACS-Pool mindestens 5 verschiedene TACS-Familien umfasst, wobei optional jede Familie mindestens 3 Mitgliedssequenzen umfasst.

6. Das Verfahren nach einem der Ansprüche 1 bis 5, wobei die genetische Anomalie eine chromosomale Aneuploidie ist oder wobei die genetische Anomalie eine strukturelle Anomalie ist, einschließlich, aber nicht beschränkt auf Kopienzahlveränderungen einschließlich Mikrodeletionen und Mikroduplikationen, Insertionen, Deletionen, Translokationen, Inversionen und Mutationen kleiner Größe einschließlich Punktmutationen und Mutationssignaturen.

7. Das Verfahren nach einem der Ansprüche 1 bis 6, wobei der TACS-Pool auf einem festen Träger fixiert ist, wobei die TACS optional biotinyliert und an Streptavidin-beschichtete magnetische Kügelchen gebunden sind.

8. Das Verfahren nach einem der Ansprüche 1 bis 7, wobei die Amplifikation der angereicherten Bibliothek in Gegenwart von Blockiersequenzen durchgeführt wird, die die Amplifikation von Wildtypsequenzen hemmen.

9. Das Verfahren nach einem der Ansprüche 1 bis 8, wobei Mitglieder der Sequenzierbibliothek, die an den Pool von TACS binden, teilweise komplementär zu den TACS sind.

10. Das Verfahren nach einem der Ansprüche 1 bis 9, wobei die statistische Analyse einen Segmentierungsalgorithmus umfasst, wobei der Segmentierungsalgorithmus optional aus der Gruppe ausgewählt wird, die aus der wahrscheinlichkeitsbasierten Segmentierung, der Segmentierung unter Verwendung kleiner überlappender Ausschnitte, der Segmentierung unter Verwendung paralleler paarweiser Tests und Kombinationen davon besteht.

11. Das Verfahren nach einem der Ansprüche 1 bis 10, wobei die Sequenzierung der angereicherten Bibliothek eine Sequenziertiefe für die genomischen Sequenzen von Interesse und Sequenziertiefen für Referenzloci liefert und die statistische Analyse die Anwendung eines Algorithmus umfasst, der sequentiell die Sequenziertiefe der Loci aus den genomischen Sequenzen von Interesse gegen die Sequenziertiefe der Referenzloci testet, wobei der Algorithmus Schritte umfasst für: (a) das Entfernen unzureichend sequenzierter Loci; (b) die Verringerung der GC-Gehalt-Verzerrung; und (c) die Bestimmung des Ploidie-Status, wobei optional die GC-Gehalt-Verzerrung durch Gruppierung von Loci mit vergleichbarem GC-Gehalt verringert wird.

12. Das Verfahren nach einem der vorhergehenden Ansprüche, wobei die Sequenzierung der angereicherten Bibliothek die Anzahl und Größe sequenzierter Fragmente für TACS-spezifische Koordinaten liefert und die statistische Analyse die Anwendung eines Algorithmus umfasst, der sequentiell das Fragmentgrößenverhältnis für die genomische Sequenz von Interesse gegen das Fragmentgrößenverhältnis der Referenzloci testet, wobei der Algorithmus Schritte umfasst für: (a) das Entfernen von Ausreißern in der Fragmentgröße; (b) die Berechnung des Fragmentgrößenverhältnisses; und (c) die Bestimmung des Ploidie-Status.

## Revendications

1. Procédé de test pour un risque d'anomalie génétique dans un échantillon d'ADN comprenant de manière prédominante un ADN fœtal ou embryonnaire et comprenant des séquences génomiques d'intérêt, le procédé comprenant :
(a) la préparation d'une bibliothèque de séquençage à partir de l'échantillon d'ADN comprenant de manière prédominante un ADN fœtal ou embryonnaire ;
(b) l'hybridation de la bibliothèque de séquençage à un groupe de séquences de capture cibles (TACS) à double brin, le groupe de TACS comprenant une pluralité de familles de TACS dirigées sur des séquences génomiques d'intérêt différentes, chaque famille de TACS comprenant une pluralité de séquences d'élément, chaque séquence d'élément se liant aux mêmes séquences génomiques d'intérêt mais possédant des positions de départ et/ou d'arrêt différentes par rapport à un système de coordonnées de référence pour la séquence génomique d'intérêt, les positions de départ et/ou d'arrêt pour les séquences d'élément à l'intérieur d'une famille de TACS, par rapport à un système de référence de coordonnées de référence pour la séquence génomique d'intérêt, étant échelonnées par 5 à 10 paires de bases, et en outre :
(i) chaque séquence d'élément à l'intérieur du groupe de TACS étant d'une longueur de 150 à 260 paires de bases, chaque séquence d'élément possédant une extrémité 5' et une extrémité 3' ; et
(ii) chaque séquence d'élément se liant à la même séquence génomique d'intérêt au moins 50 paires de bases à l'écart, à la fois sur l'extrémité 5' et l'extrémité 3', de régions abritant des variations du nombre de copies (CNV), des duplications segmentaires ou des éléments d'ADN répétitifs ; et
(iii) la teneur en GC du groupe de TACS étant comprise entre 19 % et 80 %, telle que déterminée en calculant la teneur en GC de chaque élément à l'intérieur du groupe de TACS ;
(c) l'isolement d'éléments de la bibliothèque de séquençage qui se lient au groupe de TACS pour obtenir une bibliothèque enrichie ;
(d) l'amplification et le séquençage de la bibliothèque enrichie ;
(e) la comparaison de la bibliothèque enrichie à un génome de référence, obtenant ainsi des informations de profondeur de lecture et des comptes alléliques ; et
(f) la réalisation d'une analyse statistique sur les séquences de la bibliothèque enrichie pour ainsi déterminer le risque d'une anomalie génétique dans l'échantillon d'ADN.

2. Procédé selon la revendication 1, l'échantillon d'ADN étant d'un embryon pré-implantation ou de trophoblastes intacts collectés d'un frottis maternel de Papanicolaou ou d'une cellule fœtale trouvée dans un plasma maternel.

3. Procédé selon l'une quelconque des revendications 1 à 2, l'échantillon d'ADN étant obtenu directement d'un tissu fœtal ou d'un fluide amniotique ou de villosités choriales ou d'un milieu où des produits de conception ont crû.

4. Procédé selon l'une quelconque des revendications 1 à 3, le groupe de TACS comprenant des éléments qui se lient aux chromosomes 1 à 22, X et Y du génome humain.

5. Procédé selon la revendication 1, le groupe de TACS comprenant au moins 5 familles de TACS différentes, éventuellement chaque famille de TACS comprenant au moins 3 séquences d'élément.

6. Procédé selon l'une quelconque des revendications 1 à 5, l'anomalie génétique étant une aneuploïdie chromosomique ou l'anomalie génétique étant une anomalie structurale, comprenant, sans s'y limiter, des changements du nombre de copies comprenant des microdélétions et des microduplications, des insertions, des délétions, des translocations, des inversions et des mutations de petite taille y compris des mutations ponctuelles et des signatures mutationnelles.

7. Procédé selon l'une quelconque des revendications 1 à 6, le groupe de TACS étant fixé à un support solide, éventuellement, les TACS étant biotinylées et étant liées à des billes magnétiques revêtues par de la streptavidine.

8. Procédé selon l'une quelconque des revendications 1 à 7, l'amplification de la bibliothèque enrichie étant réalisée en la présence de séquences de blocage qui inhibent l'amplification de séquences de type sauvage.

9. Procédé selon l'une quelconque des revendications 1 à 8, des éléments de la bibliothèque de séquençage qui se lient au groupe de TACS étant partiellement complémentaires aux TACS.

10. Procédé selon l'une quelconque des revendications 1 à 9, l'analyse statistique comprenant un algorithme de segmentation, éventuellement l'algorithme de segmentation étant choisi dans le groupe constitué par une segmentation basée sur la probabilité, une segmentation utilisant de petites fenêtres chevauchantes, une segmentation utilisant un test par paires en parallèle et des combinaisons correspondantes.

11. Procédé selon l'une quelconque des revendications 1 à 10, le séquençage de la bibliothèque enrichie fournissant une profondeur de lecture pour les séquences génomiques d'intérêt et des profondeurs de lecture pour des loci de référence et l'analyse statistique comprenant l'application d'un algorithme qui teste séquentiellement la profondeur de lecture des loci des séquences génomiques d'intérêt par rapport à la profondeur de lecture des loci de référence, l'algorithme comprenant des étapes pour : (a) l'élimination de loci séquencés de manière inadéquate ; (b) l'atténuation des biais de la teneur en GC ; et (c) la détermination du statut de la ploïdie, éventuellement, le biais de la teneur en GC étant atténué en regroupant ensemble des loci à teneur en GC correspondante.

12. Procédé selon l'une quelconque des revendications précédentes, le séquençage de la bibliothèque enrichie fournissant le nombre et la taille de fragments séquencés pour des coordonnées TACS-spécifiques et l'analyse statistique comprenant l'application d'un algorithme qui teste séquentiellement la proportion fragmentaire pour la séquence génomique d'intérêt par rapport à la proportion fragmentaire des loci de référence, l'algorithme comprenant des étapes pour : (a) l'élimination de valeurs fragmentaires aberrantes ; (b) le calcul de la proportion fragmentaire ; et (c) la détermination du statut de la ploïdie.
